# EUROPEAN PATENT APPLICATION

(11) **EP 3 508 499 A1**
(43) Date of publication of application: **10.07.2019**
(21) Application number: 18150632.0
(22) Date of filing: 08.01.2018
(51) Int. Cl.: C07K 16/28, A61K 39/395

(54) **ANTIBODIES TARGETING, AND OTHER MODULATORS OF, AN IMMUNOGLOBULIN GENE ASSOCIATED WITH RESISTANCE AGAINST ANTI-TUMOUR IMMUNE RESPONSES, AND USES THEREOF**

(71) Applicant: iOmx Therapeutics AG, 82152 Martinsried (DE)
(72) Inventor: Beckhove, Philipp, 93053 Regensburg (DE); Michels, Tillmann, 82152 Martinsried (DE); Menevse, Ayse Nur, 93053 Regensburg (DE); Rathinasamy, Anchana, 93053 Regensburg (DE); Volpin, Valentina, 93053 Regensburg (DE); Khandelwal, Nisit, 82152 Martinsried (DE); Urlinger, Stefanie, 82152 Martinsried (DE); Zantow, Jonas, 82152 Martinsried (DE); Frenzel, Andre, 38124 Braunschweig (DE); Kuhn, Philipp, 38124 Braunschweig (DE)
(74) Representative: Kuttenkeuler, David

(57) **Abstract**

The invention is based on the surprising finding of antibodies that bind to IGSF11 (VSIG3) can also inhibit the interaction between IGSF11 and IGSF11 receptors such as VSIR (VISTA), the inhibition of such interaction can sensitise tumour cells to anti-tumour immune responses. In particular, the invention provides products, compositions and methods for treating diseases using modulators of IGSF11, especially antigen binding proteins targeting IGSF11, including those being inhibitors of IGSF11-interaction with VSIR. Also provided are methods of sensitising cells involved with a proliferative disorder against the cytotoxic effect of cell-mediated immune responses, and/or to kill such cells and/or methods for treating proliferative diseases, using an IGSF11 inhibitor such as an IGSF11-binding antibody, as well as certain related aspects including detection, diagnostic and screening methods

SEQUENCE LISTING

<110>  iOmx Therapeutics AG

<120>  ANTIBODIES TARGETING, AND OTHER MODULATORS OF, AN IMMUNOGLOBULIN
       GENE ASSOCIATED WITH RESISTANCE AGAINST ANTI-TUMOUR IMMUNE
       RESPONSES, AND USES THEREOF

<130>  I33137EP

<160>  387

<170>  PatentIn version 3.5

<210>  1
<211>  5
<212>  PRT
<213>  Homo sapiens

<400>  1

Ser Tyr Ser Met Asn
1               5

<210>  2
<211>  17
<212>  PRT
<213>  Homo sapiens

<400>  2

Ser Ile Ser Ser Ser Ser Ser Tyr Ile Tyr Tyr Ala Asp Ser Val Lys
1               5                   10                  15

Gly

<210>  3
<211>  15
<212>  PRT
<213>  Homo sapiens

<400>  3

Ser Ile Ile Val Gln Ala Leu Gly Ile Thr Ser Val Phe Asp Ile
1               5                   10                  15

<210>  4
<211>  124
<212>  PRT
<213>  Homo sapiens

<400>  4

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr

                20                  25                  30

Ser Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Val Arg Ser Ile Ile Val Gln Ala Leu Gly Ile Thr Ser Val Phe Asp
            100                 105                 110

Ile Trp Gly Gln Gly Thr Met Val Thr Val Ser Ser
        115                 120

<210>  5
<211>  11
<212>  PRT
<213>  Homo sapiens

<400>  5

Arg Ala Ser Gln Ile Ile Ser Asn His Leu Asn
1               5                   10

<210>  6
<211>  7
<212>  PRT
<213>  Homo sapiens

<400>  6

Ala Ala Ser Arg Leu Gln Thr
1               5

<210>  7
<211>  9
<212>  PRT
<213>  Homo sapiens

<400>  7

Gln Gln Ser Tyr Ser Asn Pro Arg Thr
1               5

<210>  8
<211>  107
<212>  PRT

<213>  Homo sapiens

<400>  8

Ala Ile Arg Leu Thr Gln Ser Pro Ser Ser Leu Ser Ala Ser Val Gly
1               5                   10                  15

Asp Arg Val Thr Ile Ser Cys Arg Ala Ser Gln Ile Ile Ser Asn His
            20                  25                  30

Leu Asn Trp Tyr Gln Gln Lys Pro Gly Lys Ala Pro Lys Leu Leu Ile
        35                  40                  45

Tyr Ala Ala Ser Arg Leu Gln Thr Gly Val Pro Ser Arg Phe Ser Gly
    50                  55                  60

Ser Gly Ser Glu Thr Asp Tyr Thr Leu Thr Ile Ser Ser Leu Gln Pro
65                  70                  75                  80

Glu Asp Phe Ala Thr Tyr Tyr Cys Gln Gln Ser Tyr Ser Asn Pro Arg
                85                  90                  95

Thr Phe Gly His Gly Thr Lys Val Glu Ile Lys
            100                 105

<210>  9
<211>  372
<212>  DNA
<213>  Homo sapiens

<400>  9
gaggtgcagc tggtggagtc tgggggaggc ctggtcaagc ctggggggtc cctgagactc      60

tcctgtgcag cctctggatt caccttcagt agctatagca tgaactgggt ccgccaggct     120

ccagggaagg ggctggagtg ggtctcatcc attagtagta gtagtagtta catatactac     180

gcagactcag tgaagggccg attcaccatc tccagagaca acgccaagaa ctcactgtat     240

ctgcaaatga acagcctgag agccgaggac acggctgtat attactgtgt caggtctatt     300

atagttcaag cacttggcat aacgtccgtt tttgatatct ggggccaagg gacaatggtc     360

accgtctctt ca                                                         372

<210>  10
<211>  321
<212>  DNA
<213>  Homo sapiens

<400>  10
gccatccggt tgacccagtc tccatcctcc ctgtctgcat ctgtaggaga cagagtcacc      60

atctcttgcc gggcaagtca gatcattagc aatcatttaa attggtatca gcagaaacca     120

ggaaaagccc ctaagctcct gatctatgct gcatccagat tgcaaactgg ggtcccatca 180

aggttcagtg gcagtggctc tgagacagac tacactctca ccatcagcag tctgcaacct 240

gaagattttg caacttacta ctgtcaacag agttacagta acccccggac gttcggccac 300

gggaccaagg tggaaatcaa a 321

<210> 11
<211> 5
<212> PRT
<213> Homo sapiens

<400> 11

Ser Asn Tyr Met Ser
1               5

<210> 12
<211> 16
<212> PRT
<213> Homo sapiens

<400> 12

Val Ile Tyr Ser Gly Gly Ser Thr Tyr Tyr Ala Asp Ser Val Lys Gly
1               5                   10                  15

<210> 13
<211> 15
<212> PRT
<213> Homo sapiens

<400> 13

Gly Asn Pro Tyr Tyr Tyr Gly Asp Leu Gln Val Asn Phe Asp Tyr
1               5                   10                  15

<210> 14
<211> 123
<212> PRT
<213> Homo sapiens

<400> 14

Glu Val Gln Leu Val Glu Ser Gly Gly Gly Leu Ile Gln Pro Gly Gly
1               5                   10                  15

Ser Leu Arg Leu Ser Cys Ala Ala Ser Gly Phe Thr Val Ser Ser Asn
            20                  25                  30

Tyr Met Ser Trp Val Arg Gln Ala Pro Gly Lys Gly Leu Glu Trp Val
        35                  40                  45

Ser Val Ile Tyr Ser Gly Gly Ser Thr Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Arg Gly Asn Pro Tyr Tyr Tyr Gly Asp Leu Gln Val Asn Phe Asp Tyr
            100                 105                 110

Trp Gly Gln Gly Thr Leu Val Thr Val Ser Ser
        115                 120

<210>  15
<211>  14
<212>  PRT
<213>  Homo sapiens

<400>  15

Gly Ser Ser Thr Gly Ala Val Thr Ser Gly Asn Leu Pro Asn
1               5                   10

<210>  16
<211>  7
<212>  PRT
<213>  Homo sapiens

<400>  16

Ser Thr Ser Asn Lys His Ser
1               5

<210>  17
<211>  9
<212>  PRT
<213>  Homo sapiens

<400>  17

Leu Leu Tyr Tyr Gly Gly Ala Trp Val
1               5

<210>  18
<211>  109
<212>  PRT
<213>  Homo sapiens

<400>  18

Gln Ala Val Val Thr Gln Glu Pro Ser Leu Thr Val Ser Pro Gly Gly
1               5                   10                  15

Thr Val Thr Pro Thr Cys Gly Ser Ser Thr Gly Ala Val Thr Ser Gly

20                  25                  30

Asn Leu Pro Asn Trp Phe Gln Gln Lys Pro Gly Gln Ala Pro Arg Ala
        35                  40                  45

Leu Ile Tyr Ser Thr Ser Asn Lys His Ser Trp Thr Pro Ala Arg Ile
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Leu Leu Tyr Tyr Gly Gly
                85                  90                  95

Ala Trp Val Phe Gly Gly Gly Thr Lys Leu Thr Val Leu
            100                 105

<210>  19
<211>  369
<212>  DNA
<213>  Homo sapiens

<400>  19
gaagtgcagc tggtggagtc tggaggaggc ttgatccagc ctggggggtc cctgagactc      60

tcctgtgcag cctctgggtt caccgtcagt agcaactaca tgagctgggt ccgccaggct     120

ccagggaagg ggctggagtg ggtctcagtt atttatagcg gtggtagcac atactacgca     180

gactccgtga agggccgatt caccatctcc agagacaatt ccaagaacac gctgtatctt     240

caaatgaaca gcctgagagc cgaggacacg gccgtgtatt actgtgcgag aggtaaccca     300

tattactacg gtgacctcca ggtgaacttt gactactggg gccagggaac cctggtcacc     360

gtctcctca                                                             369

<210>  20
<211>  327
<212>  DNA
<213>  Homo sapiens

<400>  20
caggctgtgg tgactcagga gccctcactg actgtgtccc caggagggac agtcactccc      60

acctgtggtt ccagcactgg agcagtcacc agtggtaacc ttccaaactg gttccagcag     120

aaacctggac aagcacccag ggcactgatt tatagtacaa gcaacaaaca ctcctggacc     180

cctgcccgga tctcaggctc cctccttggg ggcaaagctg ccctgacact gtcaggtgtg     240

cagcctgagg acgaggctga atattactgt ctactctatt atggtggtgc ttgggtgttc     300

ggcggaggga ccaagctgac cgtccta                                         327

<210>  21
<211>  5
<212>  PRT
<213>  Homo sapiens

<400>  21

Ser Tyr Ala Ile Ser
1               5


<210>  22
<211>  17
<212>  PRT
<213>  Homo sapiens

<400>  22

Gly Ile Ile Pro Ile Phe Gly Thr Ala Asn Tyr Ala Gln Lys Phe Gln
1               5                   10                  15


Gly



<210>  23
<211>  13
<212>  PRT
<213>  Homo sapiens

<400>  23

Pro Arg Ile Gln Leu Trp Ala Ala Gly Gly Phe Asp Tyr
1               5                   10


<210>  24
<211>  121
<212>  PRT
<213>  Homo sapiens

<400>  24

Val Gln Leu Val Gln Ser Gly Ala Glu Val Lys Lys Pro Gly Ser Ser
1               5                   10                  15


Val Lys Val Ser Cys Lys Ala Ser Gly Gly Thr Phe Ser Ser Tyr Ala
            20                  25                  30


Ile Ser Trp Val Arg Gln Ala Pro Gly Gln Gly Leu Glu Trp Met Gly
        35                  40                  45


Gly Ile Ile Pro Ile Phe Gly Thr Ala Asn Tyr Ala Gln Lys Phe Gln
    50                  55                  60


Gly Arg Val Thr Ile Thr Ala Asp Glu Ser Thr Ser Thr Ala Tyr Met
65                  70                  75                  80

Glu Leu Ser Ser Leu Arg Ser Glu Asp Thr Ala Val Tyr Tyr Cys Ala
                85                  90                  95

Ser Pro Arg Ile Gln Leu Trp Ala Ala Gly Gly Phe Asp Tyr Trp Gly
            100                 105                 110

Gln Gly Thr Leu Val Thr Val Ser Ser
        115                 120

<210>  25
<211>  14
<212>  PRT
<213>  Homo sapiens

<400>  25

Thr Gly Thr Ser Ser Asp Val Gly Gly Tyr Asn Leu Val Ser
1               5                   10

<210>  26
<211>  7
<212>  PRT
<213>  Homo sapiens

<400>  26

Asp Val Ser Asn Arg Pro Ser
1               5

<210>  27
<211>  10
<212>  PRT
<213>  Homo sapiens

<400>  27

Ser Ser Phe Thr Thr Ser Thr Thr Leu Val
1               5                   10

<210>  28
<211>  110
<212>  PRT
<213>  Homo sapiens

<400>  28

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Leu Val Ser Trp Tyr Gln Gln His Pro Gly Lys Ala Pro Lys Leu
        35                  40                  45

Met Ile Tyr Asp Val Ser Asn Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Phe Thr Thr Ser
                85                  90                  95

Thr Thr Leu Val Phe Gly Gly Gly Thr Lys Leu Thr Val Leu
            100                 105                 110

<210>  29
<211>  363
<212>  DNA
<213>  Homo sapiens

<400>  29
gtgcagctgg tgcagtctgg ggctgaggtg aagaagcctg ggtcctcggt gaaggtctcc      60

tgcaaggctt ctggaggcac cttcagcagc tatgctatca gctgggtgcg acaggcccct     120

ggacaagggc ttgagtggat gggagggatc atccctatct ttggtacagc aaactacgca     180

cagaagttcc agggcagagt cacgattacc gcggacgaat ccacgagcac agcctacatg     240

gagctgagca gcctgagatc tgaggacacg gccgtgtatt actgtgcgag ccccaggata     300

cagctatggg ccgccggggg ctttgactac tggggccagg gaaccctggt cactgtctcc     360

tca                                                                   363

<210>  30
<211>  330
<212>  DNA
<213>  Homo sapiens

<400>  30
cagtctgccc tgactcagcc tgcctccgtg tctgggtctc ctggacagtc gatcaccatc      60

tcctgcactg gaaccagcag tgacgttggt ggttataacc ttgtctcctg gtaccaacag     120

cacccaggca aagcccccaa actcatgatt tatgatgtca gtaatcggcc ctcaggggtt     180

tctaatcgct tctctggctc caagtctggc aacacggcct ccctgaccat ctctgggctc     240

caggctgagg acgaggctga ttattattgc agctcattta caactagcac cactctagta     300

ttcggcggag ggaccaaact gaccgtccta                                      330

<210>  31
<211>  5
<212>  PRT
<213>  Homo sapiens

<400>  31

Ser Tyr Ser Met Asn
1               5

<210>  32
<211>  17
<212>  PRT
<213>  Homo sapiens

<400>  32

Ser Ile Ser Ser Ser Ser Ser Tyr Ile Tyr Tyr Ala Asp Ser Val Lys
1               5                   10                  15

Gly

<210>  33
<211>  17
<212>  PRT
<213>  Homo sapiens

<400>  33

Gly Gln Leu Leu Trp Phe Gly Glu Ser Ala Leu Ile Asp Ala Phe Asp
1               5                   10                  15

Ile

<210>  34
<211>  126
<212>  PRT
<213>  Homo sapiens

<400>  34

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Gly Gln Leu Leu Trp Phe Gly Glu Ser Ala Leu Ile Asp Ala
            100                 105                 110

Phe Asp Ile Trp Gly Gln Gly Thr Met Val Thr Val Ser Ser
        115                 120                 125

<210>  35
<211>  11
<212>  PRT
<213>  Homo sapiens

<400>  35

Arg Ala Ser Gln Ser Ile Ser Ser Tyr Leu Asn
1               5                   10

<210>  36
<211>  7
<212>  PRT
<213>  Homo sapiens

<400>  36

Ala Ala Ser Ile Leu Gln Ser
1               5

<210>  37
<211>  9
<212>  PRT
<213>  Homo sapiens

<400>  37

Gln Gln Ser Tyr Ser Thr Pro Arg Thr
1               5

<210>  38
<211>  107
<212>  PRT
<213>  Homo sapiens

<400>  38

Glu Ile Val Leu Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Leu Lys Pro Gly Lys Ala Pro Lys Leu Leu Ile
        35                  40                  45

Tyr Ala Ala Ser Ile Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Ala Tyr Tyr Cys Gln Gln Ser Tyr Ser Thr Pro Arg
                85                  90                  95

Thr Phe Gly Gln Gly Thr Lys Leu Glu Ile Lys
            100                 105

<210>  39
<211>  378
<212>  DNA
<213>  Homo sapiens

<400>  39
gaagtgcagc tggtggagtc tgggggaggc ctggtcaagc ctggggggtc cctgagactc      60

tcctgtgcag cctctggatt caccttcagt agctatagca tgaactgggt ccgccaggct     120

ccagggaagg ggctggagtg ggtctcatcc attagtagta gtagtagtta catatactac     180

gcagactcag tgaagggccg attcaccatc tccagagaca acgccaagaa ctcactgtat     240

ctgcaaatga acagcctgag agccgaggac acggctgtgt attactgtgc gagaggtcaa     300

ttactatggt tcggggagtc agcactgatt gatgcttttg atatctgggg ccaagggaca     360

atggtcaccg tctcttca                                                   378

<210>  40
<211>  321
<212>  DNA
<213>  Homo sapiens

<400>  40
gaaattgtgt tgacgcagtc tccatcctcc ctgtctgcat ctgtaggaga cagagtcacc      60

atcacttgcc gggcaagtca gagcattagc agctatttaa attggtatca gctgaaacca     120

gggaaagccc ctaaactcct gatctatgct gcatccattc tgcaaagtgg ggtcccatca     180

aggttcagtg gcagtggatc tgggacagat ttcactctca ccatcagcag tctgcaacct     240

gaagattttg cagcttacta ctgtcaacag agttacagta cccctcggac ttttggccag     300

gggaccaagc tggagatcaa a                                               321

<210>  41
<211>  5
<212>  PRT
<213>  Homo sapiens

<400>  41

Ser Asn Tyr Met Ser
1               5

<210>  42
<211>  16
<212>  PRT
<213>  Homo sapiens

<400>  42

Val Ile Tyr Ser Gly Gly Ser Thr Tyr Tyr Ala Asp Ser Val Lys Gly
1               5                   10                  15

<210>  43
<211>  15
<212>  PRT
<213>  Homo sapiens

<400>  43

Gly Asn Pro Tyr Tyr Tyr Gly Asp Leu Gln Val Asn Phe Asp Tyr
1               5                   10                  15

<210>  44
<211>  123
<212>  PRT
<213>  Homo sapiens

<400>  44

Glu Val Gln Leu Val Glu Ser Gly Gly Gly Leu Ile Gln Pro Gly Gly
1               5                   10                  15

Ser Leu Arg Leu Ser Cys Ala Ala Ser Gly Phe Thr Val Ser Ser Asn
            20                  25                  30

Tyr Met Ser Trp Val Arg Gln Ala Pro Gly Lys Gly Leu Glu Trp Val
        35                  40                  45

Ser Val Ile Tyr Ser Gly Gly Ser Thr Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Arg Gly Asn Pro Tyr Tyr Tyr Gly Asp Leu Gln Val Asn Phe Asp Tyr
            100                 105                 110

Trp Gly Gln Gly Thr Leu Val Thr Val Ser Ser

115                 120


<210>  45
<211>  14
<212>  PRT
<213>  Homo sapiens

<400>  45

Ala Ser Ser Thr Gly Ala Val Thr Ser Gly Tyr Tyr Pro Asn
1               5                   10


<210>  46
<211>  7
<212>  PRT
<213>  Homo sapiens

<400>  46

Ser Thr Ser Asn Lys Asp Ser
1               5


<210>  47
<211>  9
<212>  PRT
<213>  Homo sapiens

<400>  47

Leu Leu Tyr Tyr Gly Gly Ala Trp Val
1               5


<210>  48
<211>  109
<212>  PRT
<213>  Homo sapiens

<400>  48

Gln Ala Val Val Thr Gln Glu Pro Ser Leu Thr Val Ser Pro Gly Glu
1               5                   10                  15


Thr Val Thr Leu Thr Cys Ala Ser Ser Thr Gly Ala Val Thr Ser Gly
            20                  25                  30


Tyr Tyr Pro Asn Trp Phe Gln Gln Arg Pro Gly Gln Ala Pro Arg Ala
        35                  40                  45


Leu Ile Tyr Ser Thr Ser Asn Lys Asp Ser Trp Thr Pro Ala Arg Phe
    50                  55                  60


Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Leu Leu Tyr Tyr Gly Gly
                85                  90                  95

Ala Trp Val Phe Gly Gly Gly Thr Lys Leu Thr Val Leu
            100                 105

<210>  49
<211>  369
<212>  DNA
<213>  Homo sapiens

<400>  49
gaggtgcagc tggtggagtc tggaggaggc ttgatccagc ctggggggtc cctgagactc      60

tcctgtgcag cctctgggtt caccgtcagt agcaactaca tgagctgggt ccgccaggct     120

ccagggaagg ggctggagtg ggtctcagtt atttatagcg gtggtagcac atactacgca     180

gactccgtga agggccgatt caccatctcc agagacaatt ccaagaacac gctgtatctt     240

caaatgaaca gcctgagagc cgaggacacg gccgtgtatt actgtgcgag aggtaaccca     300

tattactacg gtgacctcca ggtgaacttt gactactggg gccagggaac cctggtcacc     360

gtctcctca                                                             369

<210>  50
<211>  327
<212>  DNA
<213>  Homo sapiens

<400>  50
caggctgtgg tgactcagga gccctcactg actgtgtccc caggagagac ggtcactctc      60

acctgtgctt ccagcactgg agcagtcacc agtggttact atccaaactg gttccagcag     120

agacctggac aagcacccag ggcactgatt tatagtacaa gcaacaaaga ctcctggacc     180

cctgcccggt tctcaggctc cctccttggg ggcaaagctg ccctgacact gtcaggtgtg     240

cagcctgagg acgaggctga gtattactgc ctgctctact atggtggtgc ttgggtgttc     300

ggcggaggga ccaagctgac cgtccta                                         327

<210>  51
<211>  5
<212>  PRT
<213>  Homo sapiens

<400>  51

Ser Asn Tyr Met Ser
1               5

<210>  52
<211>  16
<212>  PRT
<213>  Homo sapiens

<400>  52

Val Ile Tyr Ser Gly Gly Ser Thr Tyr Tyr Ala Asp Ser Val Lys Gly
1               5                   10                  15


<210>  53
<211>  15
<212>  PRT
<213>  Homo sapiens

<400>  53

Gly Asn Pro Tyr Tyr Tyr Gly Asp Leu Gln Val Asn Phe Asp Tyr
1               5                   10                  15


<210>  54
<211>  123
<212>  PRT
<213>  Homo sapiens

<400>  54

Glu Val Gln Leu Val Glu Ser Gly Gly Gly Leu Ile Gln Pro Gly Gly
1               5                   10                  15


Ser Leu Arg Leu Ser Cys Ala Ala Ser Gly Phe Thr Val Ser Ser Asn
            20                  25                  30


Tyr Met Ser Trp Val Arg Gln Ala Pro Gly Lys Gly Leu Glu Trp Val
        35                  40                  45


Ser Val Ile Tyr Ser Gly Gly Ser Thr Tyr Tyr Ala Asp Ser Val Lys
    50                  55                  60


Gly Arg Phe Thr Ile Ser Arg Asp Asn Ser Lys Asn Thr Leu Tyr Leu
65                  70                  75                  80


Gln Met Asn Ser Leu Arg Ala Glu Asp Thr Ala Val Tyr Tyr Cys Ala
                85                  90                  95


Arg Gly Asn Pro Tyr Tyr Tyr Gly Asp Leu Gln Val Asn Phe Asp Tyr
            100                 105                 110


Trp Gly Gln Gly Thr Leu Val Thr Val Ser Ser
        115                 120


<210>  55
<211>  14
<212>  PRT
<213>  Homo sapiens

<400>  55

Ala Ser Ser Thr Gly Ala Val Thr Ser Gly Tyr Tyr Pro Asn
1               5                   10

<210>  56
<211>  7
<212>  PRT
<213>  Homo sapiens

<400>  56

Ser Thr Ser Asn Lys His Ser
1               5

<210>  57
<211>  9
<212>  PRT
<213>  Homo sapiens

<400>  57

Leu Leu Tyr Tyr Gly Gly Asp Trp Val
1               5

<210>  58
<211>  109
<212>  PRT
<213>  Homo sapiens

<400>  58

Gln Ala Val Val Thr Gln Glu Pro Ser Leu Thr Val Ser Pro Gly Gly
1               5                   10                  15

Thr Val Thr Leu Thr Cys Ala Ser Ser Thr Gly Ala Val Thr Ser Gly
            20                  25                  30

Tyr Tyr Pro Asn Trp Phe Gln Gln Lys Pro Gly Gln Ala Pro Arg Pro
        35                  40                  45

Leu Ile Tyr Ser Thr Ser Asn Lys His Ser Trp Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Asp Asp Glu Ala Glu Tyr Tyr Cys Leu Leu Tyr Tyr Gly Gly
                85                  90                  95

Asp Trp Val Phe Gly Gly Gly Thr Lys Leu Thr Val Leu
            100                 105

<210>  59

<211>  369
<212>  DNA
<213>  Homo sapiens

<400>  59
gaggtgcagc tggtggagtc tggaggaggc ttgatccagc ctggggggtc cctgagactc      60

tcctgtgcag cctctgggtt caccgtcagt agcaactaca tgagctgggt ccgccaggct     120

ccagggaagg ggctggagtg ggtctcagtt atttatagcg gtggtagcac atactacgca     180

gactccgtga agggccgatt caccatctcc agagacaatt ccaagaacac gctgtatctt     240

caaatgaaca gcctgagagc cgaggacacg gccgtgtatt actgtgcgag aggtaaccca     300

tattactacg gtgacctcca ggtgaacttt gactactggg gccagggaac cctggtcacc     360

gtctcctca                                                             369


<210>  60
<211>  327
<212>  DNA
<213>  Homo sapiens

<400>  60
caggctgtgg tgactcagga gccctcactg actgtgtccc caggagggac agtcactctc      60

acctgtgctt ccagcactgg agcagtcacc agtggttact atccaaactg gttccagcag     120

aaacctggac aagcacccag gccactgatt tatagtacaa gcaacaaaca ctcctggacc     180

cctgcccggt tctcaggctc cctccttggg ggcaaagctg ccctgacact gtcaggtgtg     240

cagcctgacg acgaggctga atattactgc ctgctctact atggtggtga ctgggtgttc     300

ggcggaggga ccaagctgac cgtccta                                         327


<210>  61
<211>  5
<212>  PRT
<213>  Homo sapiens

<400>  61

Gly Tyr Tyr Met His
1               5


<210>  62
<211>  17
<212>  PRT
<213>  Homo sapiens

<400>  62

Trp Ile Asn Pro Asn Ser Gly Gly Thr Asn Tyr Ala Gln Lys Phe Gln
1               5                   10                  15


Gly

<210>  63
<211>  6
<212>  PRT
<213>  Homo sapiens

<400>  63

Gly Ser Asn Phe Asp Tyr
1               5


<210>  64
<211>  115
<212>  PRT
<213>  Homo sapiens

<400>  64

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Ala Gly Ser Asn Phe Asp Tyr Trp Gly Gln Gly Thr Leu Val Thr
            100                 105                 110

Val Ser Ser
        115


<210>  65
<211>  14
<212>  PRT
<213>  Homo sapiens

<400>  65

Thr Gly Ser Ser Ser Asp Ile Gly Ser Phe Ser Tyr Val Ser
1               5                   10

<210>  66
<211>  7
<212>  PRT
<213>  Homo sapiens

<400>  66

Gly Val Asn Asn Arg Pro Leu
1               5


<210>  67
<211>  10
<212>  PRT
<213>  Homo sapiens

<400>  67

Ser Ser Tyr Thr Arg Arg Ser Thr Val Ile
1               5                   10


<210>  68
<211>  110
<212>  PRT
<213>  Homo sapiens

<400>  68

Gln Ser Ala Leu Thr Gln Pro Ala Ser Val Ser Gly Ser Pro Gly Gln
1               5                   10                  15


Ser Ile Thr Ile Ala Cys Thr Gly Ser Ser Ser Asp Ile Gly Ser Phe
            20                  25                  30


Ser Tyr Val Ser Trp Tyr Gln Gln Arg Pro Gly Lys Ala Pro Thr Leu
        35                  40                  45


Ile Ile Tyr Gly Val Asn Asn Arg Pro Leu Gly Val Ser Arg Arg Phe
    50                  55                  60


Ser Gly Ser Lys Ser Gly Asn Thr Ala Ser Leu Ser Ile Ser Gly Leu
65                  70                  75                  80


Gln Ala Glu Asp Glu Ala Asp Tyr Tyr Cys Ser Ser Tyr Thr Arg Arg
                85                  90                  95


Ser Thr Val Ile Phe Gly Gly Gly Thr Lys Leu Thr Val Leu
            100                 105                 110


<210>  69
<211>  345
<212>  DNA
<213>  Homo sapiens

<400>  69

caggttcagc tggtgcagtc tggggctgag gtgaagaagc ctggggcctc agtgaaggtc 60

tcctgcaagg cttctggata caccttcacc ggctactata tgcactgggt gcgacaggcc 120

cctggacaag ggcttgagtg gatgggatgg atcaacccta acagtggtgg cacaaactat 180

gcacagaagt ttcagggcag ggtcaccatg accagggaca cgtccatcag cacagcctac 240

atggagctga gcaggctgag atctgacgac acggccgtgt attactgtgc tgcggggtct 300

aactttgact actggggcca gggaaccctg gtcaccgtct cctca 345

<210> 70
<211> 330
<212> DNA
<213> Homo sapiens

<400> 70
cagtctgccc tgactcagcc tgcctccgtg tctgggtctc cgggacaatc gatcaccatc 60

gcctgcactg gaagcagtag tgacattggt agtttttctt atgtctcctg gtaccaacag 120

cgccccggca aagcccccac actcatcatt tatggggtca ataatcgacc cttaggggtg 180

tctcggcgct tctctggctc caagtctggc aacacggcct ccctaagcat ctctgggctc 240

caggctgagg acgaggctga ttattactgc agttcctata cacgcagaag cactgtgatc 300

ttcggcggcg ggaccaagtt gaccgtccta 330

<210> 71
<211> 7
<212> PRT
<213> Homo sapiens

<400> 71

Ser Ser Gly Tyr Tyr Trp Gly
1               5

<210> 72
<211> 16
<212> PRT
<213> Homo sapiens

<400> 72

Ser Ile Tyr Tyr Ser Gly Ser Thr Tyr Tyr Asn Pro Ser Leu Lys Ser
1               5                   10                  15

<210> 73
<211> 13
<212> PRT
<213> Homo sapiens

<400> 73

His Arg Val Arg Phe Gly Glu Phe Asp Ala Phe Asp Ile
1               5                   10

<210>  74
<211>  123
<212>  PRT
<213>  Homo sapiens

<400>  74

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Ser
            20                  25                  30

Gly Tyr Tyr Trp Gly Trp Ile Arg Gln Pro Pro Gly Lys Gly Leu Glu
        35                  40                  45

Trp Ile Gly Ser Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg His Arg Val Arg Phe Gly Glu Phe Asp Ala Phe Asp Ile
            100                 105                 110

Trp Gly Gln Gly Thr Met Val Thr Val Ser Ser
        115                 120


<210>  75
<211>  14
<212>  PRT
<213>  Homo sapiens

<400>  75

Thr Gly Thr Ser Ser Asn Val Gly Ala Asp Phe Asp Val His
1               5                   10


<210>  76
<211>  7
<212>  PRT
<213>  Homo sapiens

<400>  76

Gly Ser Asp Asn Arg Pro Ser
1               5

<210>  77
<211>  11
<212>  PRT
<213>  Homo sapiens

<400>  77

Gln Ala Tyr Asp Val Arg Leu Ser Gly Trp Val
1               5                   10


<210>  78
<211>  111
<212>  PRT
<213>  Homo sapiens

<400>  78

Gln Ala Val Leu Thr Gln Pro Pro Ser Val Ser Gly Ala Pro Gly Glu
1               5                   10                  15


Arg Val Thr Leu Ser Cys Thr Gly Thr Ser Ser Asn Val Gly Ala Asp
            20                  25                  30


Phe Asp Val His Trp Tyr Gln Gln Phe Pro Gly Thr Ala Pro Arg Leu
        35                  40                  45


Leu Ile Phe Gly Ser Asp Asn Arg Pro Ser Gly Val Pro Asp Arg Phe
    50                  55                  60


Ser Gly Ser Lys Ser Gly Thr Ser Ala Ser Leu Ala Ile Thr Gly Leu
65                  70                  75                  80


Gln Val Glu Asp Glu Ala Asp Tyr Tyr Cys Gln Ala Tyr Asp Val Arg
                85                  90                  95


Leu Ser Gly Trp Val Phe Gly Gly Gly Thr Lys Leu Thr Val Leu
            100                 105                 110


<210>  79
<211>  369
<212>  DNA
<213>  Homo sapiens

<400>  79
cagctgcagc tgcaggagtc gggcccagga ctggtgaagc cttcggagac cctgtccctc      60

acctgcaccg tctctggtgg ctccatcagc agtagtggtt actactgggg ctggatccgc     120

cagcccccag ggaaggggct ggagtggatt gggagtatct attatagtgg gagcacctac     180

tacaacccgt ccctcaagag tcgagtcacc atatccgtag acacgtctaa gaaccagttc     240

tccctgaagc tgagctctgt gaccgccgca gacacggctg tgtattactg tgcgagacat     300

agggtacggt tcggggagtt cgatgctttt gatatctggg gccaagggac aatggtcacc     360

gtctcttca                                                          369

<210>  80
<211>  333
<212>  DNA
<213>  Homo sapiens

<400>  80
caggctgtgc tgactcagcc accctcagtg tctggggccc ccggagagag ggtcaccctc      60

tcttgtacag ggaccagctc gaacgtcggg gcagattttg atgtacattg gtaccagcag     120

tttccaggaa cagcccccag actcctcatc tttggttccg acaatcggcc ctcaggagtc     180

cctgaccgat tctctggctc caagtctggc acctcagcct ccctggccat cactgggctc     240

caggttgagg atgaggctga ttattattgc caggcttatg acgtcaggct gagtggctgg     300

gtgttcggcg gggggaccaa gctgaccgtc cta                                  333

<210>  81
<211>  5
<212>  PRT
<213>  Homo sapiens

<400>  81

Ser Tyr Ala Ile Ser
1               5

<210>  82
<211>  17
<212>  PRT
<213>  Homo sapiens

<400>  82

Gly Ile Ile Pro Ile Phe Gly Thr Ala Asn Tyr Ala Gln Lys Phe Gln
1               5                   10                  15

Gly

<210>  83
<211>  11
<212>  PRT
<213>  Homo sapiens

<400>  83

Gly Arg Gly Phe Gly Glu Leu Tyr Phe Asp Tyr
1               5                   10

<210>  84
<211>  120
<212>  PRT

<213>  Homo sapiens

<400>  84

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Arg Gly Phe Gly Glu Leu Tyr Phe Asp Tyr Trp Gly Gln
            100                 105                 110

Gly Thr Leu Val Thr Val Ser Ser
        115                 120

<210>  85
<211>  11
<212>  PRT
<213>  Homo sapiens

<400>  85

Arg Ala Ser Gln Gly Val Arg Ser Asn Ile Ala
1               5                   10

<210>  86
<211>  7
<212>  PRT
<213>  Homo sapiens

<400>  86

Asp Ser Ser Thr Arg Ala Thr
1               5

<210>  87
<211>  9
<212>  PRT
<213>  Homo sapiens

<400>  87

Gln Gln Tyr Lys Asn Trp Pro Arg Thr
1               5

<210>  88
<211>  107
<212>  PRT
<213>  Homo sapiens

<400>  88

Glu Thr Thr Leu Thr Gln Ser Pro Ala Thr Leu Ser Val Ser Pro Gly
1               5                   10                  15

Glu Arg Ala Thr Leu Ser Cys Arg Ala Ser Gln Gly Val Arg Ser Asn
            20                  25                  30

Ile Ala Trp Tyr Gln Gln Lys Pro Gly Gln Ala Pro Arg Leu Leu Ile
        35                  40                  45

Tyr Asp Ser Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Ser Cys Gln Gln Tyr Lys Asn Trp Pro Arg
                85                  90                  95

Thr Phe Gly Gln Gly Thr Lys Val Glu Ile Lys
            100                 105

<210>  89
<211>  360
<212>  DNA
<213>  Homo sapiens

<400>  89
caggttcagc tggtgcagtc tggggctgag gtgaagaagc ctgggtcctc ggtgaaggtc      60

tcctgcaagg cttctggagg caccttcagc agctatgcta tcagctgggt gcgacaggcc     120

cctggacaag ggcttgagtg gatgggaggg atcatcccta tctttggtac agcaaactac     180

gcacagaagt tccagggcag agtcacgatt accgcggacg aatccacgag cacagcctac     240

atggagctga gcagcctgag atctgaggac acggccgtgt attactgtgc gagagggcga     300

gggttcgggg agttatactt tgactactgg ggccagggaa ccctggtcac cgtctcctca     360

<210>  90
<211>  321
<212>  DNA

<213>  Homo sapiens

<400>  90
gaaacgacac tcacgcagtc tccagccacc ctgtctgtgt ctccagggga aagagccacc      60

ctctcctgca gggccagtca gggtgttaga agcaatatag cctggtacca gcagaaacct     120

ggccaggctc ccaggctcct catctatgat tcatccacca gggccactgg tatcccagcc     180

aggttcagtg gcagtgggtc tgggacagag ttcactctca ccatcagcag cctgcagtct     240

gaagattttg cagtttattc ctgtcagcag tataagaact ggcctcggac gttcggccaa     300

gggaccaagg tggaaatcaa a                                               321


<210>  91
<211>  5
<212>  PRT
<213>  Homo sapiens

<400>  91

Ser Tyr Gly Ile Ser
1               5


<210>  92
<211>  17
<212>  PRT
<213>  Homo sapiens

<400>  92

Trp Ile Ser Ala Tyr Asn Gly Asn Thr Asn Tyr Ala Gln Lys Leu Gln
1               5                   10                  15


Gly



<210>  93
<211>  10
<212>  PRT
<213>  Homo sapiens

<400>  93

Val Pro Ala Trp Ser Gly Gln Phe Asp Tyr
1               5                   10


<210>  94
<211>  119
<212>  PRT
<213>  Homo sapiens

<400>  94

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Trp Ile Ser Ala Tyr Asn Gly Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Val Pro Ala Trp Ser Gly Gln Phe Asp Tyr Trp Gly Gln Gly
            100                 105                 110

Thr Leu Val Thr Val Ser Ser
        115

<210>  95
<211>  14
<212>  PRT
<213>  Homo sapiens

<400>  95

Thr Gly Ser Ser Ser Asp Val Gly Gly Tyr Asn Tyr Val Ser
1               5                   10

<210>  96
<211>  7
<212>  PRT
<213>  Homo sapiens

<400>  96

Asp Val Arg Ser Arg Pro Ser
1               5

<210>  97
<211>  11
<212>  PRT
<213>  Homo sapiens

<400>  97

Thr Ser Tyr Thr Ser Ser Asn Thr Leu Val Ile
1               5                   10

<210>  98

<211>  111
<212>  PRT
<213>  Homo sapiens

<400>  98

Gln Ser Ala Leu Thr Gln Pro Ala Ser Val Ser Gly Ser Pro Gly Gln
1               5                   10                  15

Ser Ile Thr Ile Ser Cys Thr Gly Ser Ser Ser Asp Val Gly Gly Tyr
            20                  25                  30

Asn Tyr Val Ser Trp Tyr Gln Gln Tyr Pro Gly Lys Ala Pro Lys Leu
        35                  40                  45

Met Ile Tyr Asp Val Arg Ser Arg Pro Ser Gly Val Ser Asn Arg Phe
    50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Thr Ser Tyr Thr Ser Ser
                85                  90                  95

Asn Thr Leu Val Ile Phe Gly Gly Gly Thr Lys Val Thr Val Leu
            100                 105                 110

<210>  99
<211>  357
<212>  DNA
<213>  Homo sapiens

<400>  99
caggttcagc tggtgcagtc tggagctgag gtgaagaagc ctggggcctc agtgaaggtc      60

tcctgcaagg cttctggtta cacctttacc agctatggta tcagctgggt gcgacaggcc     120

cctggacaag ggcttgagtg gatgggatgg atcagcgcct acaatggtaa cacaaactat     180

gcacagaagc tccagggcag agtcaccatg accacagaca catccacgag cacagcctac     240

atggagctga ggagcctgag atctgacgac acggccgtgt attactgtgc gagagtccct     300

gcgtggagtg gtcaatttga ctactggggc cagggaaccc tggtcaccgt ctcctca        357

<210>  100
<211>  333
<212>  DNA
<213>  Homo sapiens

<400>  100
cagtctgccc tgactcagcc tgcctccgtg tctgggtctc ctggacagtc gatcaccatc      60

tcctgcactg gatccagcag tgacgttggt ggttataact atgtctcctg gtaccagcag     120

tacccaggca aagcccccaa actcatgatt tatgatgtca gaagtcggcc ctcaggggtt 180

tctaatcgct tctctggctc caagtctggc aacacggcct ccctgaccat ctctggactc 240

caggctgagg acgaggctga ttattactgc acctcatata caagcagcaa cactcttgtg 300

atattcggcg gagggaccaa ggtgaccgtc cta 333

<210> 101
<211> 5
<212> PRT
<213> Homo sapiens

<400> 101

Ser Tyr Ala Met His
1               5

<210> 102
<211> 17
<212> PRT
<213> Homo sapiens

<400> 102

Val Ile Ser Tyr Asp Gly Ser Asn Lys Tyr Tyr Ala Asp Ser Val Lys
1               5                   10                  15

Gly

<210> 103
<211> 11
<212> PRT
<213> Homo sapiens

<400> 103

Gly Gly Ala Leu Asn Tyr Tyr Gly Met Asp Val
1               5                   10

<210> 104
<211> 120
<212> PRT
<213> Homo sapiens

<400> 104

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Glu Gly Gly Ala Leu Asn Tyr Tyr Gly Met Asp Val Trp Gly Gln
            100                 105                 110

Gly Thr Thr Val Thr Val Ser Ser
        115                 120

<210>  105
<211>  11
<212>  PRT
<213>  Homo sapiens

<400>  105

Gln Gly Asp Ser Leu Arg His Phe Tyr Ala Thr
1               5                   10

<210>  106
<211>  7
<212>  PRT
<213>  Homo sapiens

<400>  106

Gly Lys Asn Asn Arg Pro Ser
1               5

<210>  107
<211>  11
<212>  PRT
<213>  Homo sapiens

<400>  107

Gln Ser Arg Asp Pro Arg Asn Asn His Leu Ile
1               5                   10

<210>  108
<211>  108
<212>  PRT
<213>  Homo sapiens

<400>  108

Ser Ser Glu Leu Thr Gln Asp Pro Gly Val Ser Val Ala Leu Gly Gln

1               5                   10                  15

Thr Val Arg Ile Thr Cys Gln Gly Asp Ser Leu Arg His Phe Tyr Ala
            20                  25                  30

Thr Trp Tyr Gln Gln Lys Pro Gly Gln Ala Pro Ile Leu Val Ile Tyr
        35                  40                  45

Gly Lys Asn Asn Arg Pro Ser Gly Ile Pro Asp Arg Phe Ser Gly Ser
    50                  55                  60

Ser Ser Glu Asp Thr Ala Ser Leu Thr Ile Thr Gly Ala Gln Ala Glu
65                  70                  75                  80

Asp Glu Ala Asp Tyr Tyr Cys Gln Ser Arg Asp Pro Arg Asn Asn His
                85                  90                  95

Leu Ile Phe Gly Gly Gly Thr Lys Leu Ile Val Leu
            100                 105

<210>  109
<211>  360
<212>  DNA
<213>  Homo sapiens

<400>  109
caggtgcagc tggtggagtc tgggggaggc gtggtccagc ctgggaggtc cctgagactc      60

tcctgtgcag cctctggatt caccttcagt agctatgcta tgcactgggt ccgccaggct     120

ccaggcaagg ggctggagtg ggtggcagtt atatcatatg atggaagcaa taaatactac     180

gcagactccg tgaagggccg attcaccatc tccagagaca attccaagaa cacgctgtat     240

ctgcaaatga acagcctgag agctgaggac acggctgtgt attactgtgc ggagggcgga     300

gcgttgaact actacggtat ggacgtctgg ggccaaggga ccacggtcac cgtctcctca     360

<210>  110
<211>  324
<212>  DNA
<213>  Homo sapiens

<400>  110
tcttctgagc tgactcagga ccctggtgtg tctgtggcct tgggacagac agtcaggatc      60

acatgccagg gagacagcct cagacacttt tatgcaacct ggtaccagca gaagccagga     120

caggccccta tacttgtcat ctatggtaaa aacaaccggc cctcagggat cccagaccga     180

ttctctggct ccagttctga agacacagcc tccttgacca tcactggggc tcaggcggag     240

gatgaggctg actactactg tcaatcccgg gaccccagga ataaccattt gattttcggc      300

ggcgggacca aactgatcgt cctc                                            324

<210>  111
<211>  5
<212>  PRT
<213>  Homo sapiens

<400>  111

Ser Asn Tyr Met Ser
1               5


<210>  112
<211>  16
<212>  PRT
<213>  Homo sapiens

<400>  112

Val Ile Tyr Ser Gly Gly Ser Thr Tyr Tyr Ala Asp Ser Val Lys Gly
1               5                   10                  15


<210>  113
<211>  15
<212>  PRT
<213>  Homo sapiens

<400>  113

Gly Asn Pro Tyr Tyr Tyr Gly Asp Leu Gln Val Asn Phe Asp Tyr
1               5                   10                  15


<210>  114
<211>  123
<212>  PRT
<213>  Homo sapiens

<400>  114

Glu Val Gln Leu Leu Glu Ser Gly Gly Gly Leu Ile Gln Pro Gly Gly
1               5                   10                  15


Ser Leu Arg Leu Ser Cys Ala Val Ser Gly Phe Thr Val Ser Ser Asn
            20                  25                  30


Tyr Met Ser Trp Val Arg Gln Ala Pro Gly Lys Gly Leu Glu Trp Val
        35                  40                  45


Ser Val Ile Tyr Ser Gly Gly Ser Thr Tyr Tyr Ala Asp Ser Val Lys
    50                  55                  60


Gly Arg Phe Thr Ile Ser Arg Asp Asn Ser Lys Asn Thr Leu Tyr Leu
65                  70                  75                  80


Gln Met Asn Ser Leu Arg Ala Glu Asp Thr Ala Val Tyr Tyr Cys Ala

85                      90                      95

Arg Gly Asn Pro Tyr Tyr Tyr Gly Asp Leu Gln Val Asn Phe Asp Tyr
            100                 105                 110

Trp Gly Gln Gly Thr Leu Val Thr Val Ser Ser
        115                 120

<210>  115
<211>  14
<212>  PRT
<213>  Homo sapiens

<400>  115

Ala Ser Ser Thr Gly Ala Val Thr Ser Gly Tyr Tyr Pro Asn
1               5                   10

<210>  116
<211>  7
<212>  PRT
<213>  Homo sapiens

<400>  116

Ser Thr Ser Asn Lys His Ser
1               5

<210>  117
<211>  9
<212>  PRT
<213>  Homo sapiens

<400>  117

Leu Leu Tyr Tyr Gly Gly Ala Trp Val
1               5

<210>  118
<211>  109
<212>  PRT
<213>  Homo sapiens

<400>  118

Gln Ala Val Val Thr Gln Glu Pro Ser Leu Thr Val Ser Pro Gly Gly
1               5                   10                  15

Thr Val Thr Leu Thr Cys Ala Ser Ser Thr Gly Ala Val Thr Ser Gly
            20                  25                  30

Tyr Tyr Pro Asn Trp Phe Gln Gln Lys Pro Gly Gln Ala Pro Arg Ala
        35                  40                  45

Leu Ile Tyr Ser Thr Ser Asn Lys His Ser Trp Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Leu Leu Tyr Tyr Gly Gly
                85                  90                  95

Ala Trp Val Phe Gly Gly Gly Thr Lys Leu Thr Val Leu
            100                 105

<210>  119
<211>  369
<212>  DNA
<213>  Homo sapiens

<400>  119
gaggtgcagc tgttggagtc tggaggaggc ttgatccagc ctggggggtc cctgagactc      60

tcctgtgcag tctctgggtt caccgtcagt agcaactaca tgagctgggt ccgccaggct     120

ccagggaagg ggctggagtg ggtctcagtt atttatagcg gtggtagcac atactacgca     180

gactccgtga agggccgatt caccatctcc agagacaatt ccaagaacac gctgtatctt     240

caaatgaaca gcctgagagc cgaggacacg gccgtgtatt actgtgcgag aggtaaccca     300

tattactacg gtgacctcca ggtgaacttt gactactggg gccagggaac cctggtcacc     360

gtctcctca                                                             369

<210>  120
<211>  327
<212>  DNA
<213>  Homo sapiens

<400>  120
caggctgtgg tgactcagga gccctcactg actgtgtccc caggagggac agtcactctc      60

acctgtgctt ccagcactgg agcagtcacc agtggttact atccaaactg gttccagcag     120

aaacctggac aagcacccag ggcactgatt tatagtacaa gcaacaaaca ctcctggacc     180

cctgcccggt tctcaggctc cctccttggg ggcaaagctg ccctgacact gtcaggtgtg     240

cagcctgagg acgaggctga gtattactgc ctgctctact atggtggtgc ttgggtgttc     300

ggcggaggga ccaagctgac cgtccta                                         327

<210>  121
<211>  5
<212>  PRT
<213>  Homo sapiens

<400>  121

Ser Asn Tyr Met Ser
1               5

<210>  122
<211>  16
<212>  PRT
<213>  Homo sapiens

<400>  122

Val Ile Tyr Ser Gly Gly Ser Thr Tyr Tyr Ala Asp Ser Val Lys Gly
1               5                   10                  15

<210>  123
<211>  15
<212>  PRT
<213>  Homo sapiens

<400>  123

Gly Asn Pro Tyr Tyr Tyr Gly Asp Leu Gln Val Asn Phe Asp Tyr
1               5                   10                  15

<210>  124
<211>  123
<212>  PRT
<213>  Homo sapiens

<400>  124

Glu Val Gln Leu Val Glu Ser Gly Gly Gly Leu Ile Gln Pro Gly Gly
1               5                   10                  15

Ser Leu Arg Leu Ser Cys Ala Ala Ser Gly Phe Thr Val Ser Ser Asn
            20                  25                  30

Tyr Met Ser Trp Val Arg Gln Ala Pro Gly Lys Gly Leu Glu Trp Val
        35                  40                  45

Ser Val Ile Tyr Ser Gly Gly Ser Thr Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Arg Gly Asn Pro Tyr Tyr Tyr Gly Asp Leu Gln Val Asn Phe Asp Tyr
            100                 105                 110

Trp Gly Gln Gly Thr Leu Val Thr Val Ser Ser
        115                 120

<210>  125
<211>  14
<212>  PRT
<213>  Homo sapiens

<400>  125

Ala Ser Ser Thr Gly Ala Val Thr Ser Ala Tyr Tyr Pro Asn
1               5                   10


<210>  126
<211>  7
<212>  PRT
<213>  Homo sapiens

<400>  126

Ser Thr Ser Asn Lys His Ser
1               5


<210>  127
<211>  9
<212>  PRT
<213>  Homo sapiens

<400>  127

Leu Leu Tyr Tyr Gly Gly Ala Trp Val
1               5


<210>  128
<211>  109
<212>  PRT
<213>  Homo sapiens

<400>  128

Gln Ala Val Val Thr Gln Glu Pro Ser Leu Thr Val Ser Pro Gly Gly
1               5                   10                  15

Thr Val Thr Leu Thr Cys Ala Ser Ser Thr Gly Ala Val Thr Ser Ala
            20                  25                  30

Tyr Tyr Pro Asn Trp Phe Gln Gln Lys Pro Gly Gln Ala Pro Arg Ala
        35                  40                  45

Leu Ile Tyr Ser Thr Ser Asn Lys His Ser Trp Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Asp Tyr Tyr Cys Leu Leu Tyr Tyr Gly Gly

85                  90                  95

Ala Trp Val Phe Gly Gly Gly Thr Lys Leu Thr Val Leu
            100                 105

<210>  129
<211>  369
<212>  DNA
<213>  Homo sapiens

<400>  129
gaagtgcagc tggtggagtc tggaggaggc ttgatccagc ctggggggtc cctgagactc      60

tcctgtgcag cctctgggtt caccgtcagt agcaactaca tgagctgggt ccgccaggct     120

ccagggaagg ggctggagtg ggtctcagtt atttatagcg gtggtagcac atactacgca     180

gactccgtga agggccgatt caccatctcc agagacaatt ccaagaacac gctgtatctt     240

caaatgaaca gcctgagagc cgaggacacg gccgtgtatt actgtgcgag aggtaaccca     300

tattactacg gtgacctcca ggtgaacttt gactactggg gccagggaac cctggtcacc     360

gtctcctca                                                             369

<210>  130
<211>  327
<212>  DNA
<213>  Homo sapiens

<400>  130
caggctgtgg tgactcagga gccctcactg actgtgtccc caggagggac agtcactctc      60

acctgtgctt ccagcactgg agcagtcacc agtgcttact atccaaactg gttccagcag     120

aaacctggac aagcacccag ggcactgatt tatagtacaa gcaacaaaca ctcctggacc     180

cctgcccggt tctcaggctc cctccttggg ggcaaagctg ccctgacact gtcaggtgtg     240

cagcctgagg acgaggctga ctattactgc ctgctctact atggtggtgc ttgggtgttc     300

ggcggaggga ccaagctgac cgtccta                                         327

<210>  131
<211>  5
<212>  PRT
<213>  Homo sapiens

<400>  131

Asp Tyr Tyr Ile His
1               5

<210>  132
<211>  17
<212>  PRT
<213>  Homo sapiens

<400>  132

Trp Phe Asn Pro Ser Thr Gly Gly Ala Asn Tyr Ala Gln Lys Phe Gln
1               5                   10                  15

Gly

<210>  133
<211>  8
<212>  PRT
<213>  Homo sapiens

<400>  133

Gly Asn Ser Pro Asp Leu Asp Tyr
1               5

<210>  134
<211>  117
<212>  PRT
<213>  Homo sapiens

<400>  134

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Asn Asp Tyr
            20                  25                  30

Tyr Ile His Trp Val Arg Gln Ala Pro Gly Gln Gly Leu Glu Trp Met
        35                  40                  45

Gly Trp Phe Asn Pro Ser Thr Gly Gly Ala Asn Tyr Ala Gln Lys Phe
    50                  55                  60

Gln Gly Arg Val Thr Met Thr Arg Asp Thr Ser Ile Ser Thr Ala Tyr
65                  70                  75                  80

Leu Glu Val Ser Ser Leu Arg Ser Asp Asp Thr Ala Val Tyr Tyr Cys
                85                  90                  95

Ala Arg Gly Asn Ser Pro Asp Leu Asp Tyr Trp Gly Gln Gly Thr Leu
            100                 105                 110

Val Thr Val Ser Ser
        115

<210>  135
<211>  17
<212>  PRT

<213>  Homo sapiens

<400>  135

Lys Ser Ser Gln Ser Leu Leu His Ser Ser Asn Asn Lys Asn Tyr Leu
1               5                   10                  15

Ala

<210>  136
<211>  7
<212>  PRT
<213>  Homo sapiens

<400>  136

Trp Ala Ser Thr Arg Gln Ser
1               5

<210>  137
<211>  10
<212>  PRT
<213>  Homo sapiens

<400>  137

Gln Gln Tyr Tyr Thr Thr Thr Pro Asn Thr
1               5                   10

<210>  138
<211>  114
<212>  PRT
<213>  Homo sapiens

<400>  138

Asp Ile Gln Met Thr Gln Ser Pro Asp Ser Leu Thr Val Ser Leu Gly
1               5                   10                  15

Glu Arg Ala Thr Ile Asn Cys Lys Ser Ser Gln Ser Leu Leu His Ser
            20                  25                  30

Ser Asn Asn Lys Asn Tyr Leu Ala Trp Tyr Gln Gln Arg Pro Gly Gln
        35                  40                  45

Pro Pro Lys Leu Leu Ile Tyr Trp Ala Ser Thr Arg Gln Ser Gly Val
    50                  55                  60

Pro Asp Arg Phe Ser Gly Ser Gly Ser Gly Thr Asp Phe Thr Leu Thr
65                  70                  75                  80

Ile Asn Ser Leu Gln Ala Asp Asp Met Ala Val Tyr Tyr Cys Gln Gln
                85                  90                  95

Tyr Tyr Thr Thr Thr Pro Asn Thr Phe Gly Gln Gly Thr Lys Leu Glu
            100                 105                 110

Ile Lys

<210>  139
<211>  351
<212>  DNA
<213>  Homo sapiens

<400>  139
caagtccagc tggtgcagtc tggggctgag gtgaagaagc ctggggcctc agtgaaggtc      60

tcctgcaagg cttctggata caccttcaac gactactata tacactgggt gcggcaggcc     120

cctggacaag ggcttgagtg gatggggtgg ttcaacccta gcactggtgg cgcaaattat     180

gcacagaagt ttcagggcag ggtcaccatg accagggaca cgtccatcag cacagcctac     240

ttggaagtga gcagcctgag atctgacgac acggccgtgt attactgtgc gagaggtaat     300

agcccggacc ttgactactg gggccaggga accctggtca ccgtctcctc a              351

<210>  140
<211>  342
<212>  DNA
<213>  Homo sapiens

<400>  140
gacatccaga tgacccagtc tccagactcc ctgactgtgt ctctgggcga gagggccacc      60

atcaactgca agtcgagcca gagtctttta cacagctcca acaataagaa ttacttggct     120

tggtaccagc agagaccagg acagcctcct aaactgctca tttactgggc atccacccgg     180

caatccgggg tcccggaccg cttcagtggc agcgggtctg ggacagattt cactctcacc     240

atcaacagcc tgcaggctga cgacatggca gtttattact gccagcagta ttatactact     300

actccgaaca cttttggcca ggggaccaag ctggagatca aa                        342

<210>  141
<211>  5
<212>  PRT
<213>  Homo sapiens

<400>  141

Gly Tyr Tyr Met His
1               5

<210>  142
<211>  17
<212>  PRT
<213>  Homo sapiens

<400>  142

Trp Ile Asn Pro Asn Ser Gly Gly Thr Asn Tyr Ala Gln Lys Phe Gln
1               5                   10                  15

Gly

<210>  143
<211>  14
<212>  PRT
<213>  Homo sapiens

<400>  143

Asp Pro Asp Gly Ser Gly Gly Ser Ser Arg Trp Phe Asp Pro
1               5                   10

<210>  144
<211>  123
<212>  PRT
<213>  Homo sapiens

<400>  144

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Asp Pro Asp Gly Ser Gly Gly Ser Ser Arg Trp Phe Asp Pro
            100                 105                 110

Trp Gly Gln Gly Thr Leu Val Thr Val Ser Ser
        115                 120

<210>  145
<211>  12

<212>  PRT
<213>  Homo sapiens

<400>  145

Thr Leu Ser Ser Gly His Thr Asn Tyr Ala Ile Ala
1               5                   10


<210>  146
<211>  11
<212>  PRT
<213>  Homo sapiens

<400>  146

Leu Asn Ser Asp Gly Ser His Thr Arg Gly Gly
1               5                   10


<210>  147
<211>  9
<212>  PRT
<213>  Homo sapiens

<400>  147

Met Ile Trp His Asn Asn Ala Trp Val
1               5


<210>  148
<211>  111
<212>  PRT
<213>  Homo sapiens

<400>  148

Gln Pro Val Leu Thr Gln Ser Pro Ser Ala Ser Ala Ser Leu Gly Ala
1               5                   10                  15


Ser Val Lys Leu Thr Cys Thr Leu Ser Ser Gly His Thr Asn Tyr Ala
            20                  25                  30


Ile Ala Trp Arg Gln Gln Gln Pro Gly Lys Ala Pro Arg Tyr Leu Met
        35                  40                  45


Leu Leu Asn Ser Asp Gly Ser His Thr Arg Gly Gly Gly Ile Pro Asp
    50                  55                  60


Arg Phe Ser Gly Ser Ser Ser Gly Ala Glu Arg Tyr Leu Thr Ile Ser
65                  70                  75                  80


Ser Leu Gln Ser Glu Asp Glu Ala Asp Tyr Tyr Cys Met Ile Trp His
                85                  90                  95


Asn Asn Ala Trp Val Phe Gly Gly Gly Thr Lys Leu Ala Val Leu

            100                 105                 110

<210> 149
<211> 369
<212> DNA
<213> Homo sapiens

<400> 149
caagtgcagc tggtgcagtc tggggctgag gtgaagaagc ctggggcctc agtgaaggtc 60

tcctgcaagg cttctggata caccttcacc ggctactata tgcactgggt gcgacaggcc 120

cctggacaag ggcttgagtg gatgggatgg atcaacccta acagtggtgg cacaaactat 180

gcacagaagt ttcagggcag ggtcaccatg accagggaca cgtccatcag cacagcctac 240

atggagctga gcaggctgag atctgacgac acggccgtgt attactgtgc gagagatcct 300

gatgggagtg gtggtagttc ccggtggttc gacccctggg gccagggaac cctggtcacc 360

gtctcctca 369

<210> 150
<211> 333
<212> DNA
<213> Homo sapiens

<400> 150
cagcctgtgc tgactcaatc gccctctgcc tctgcctccc tgggagcctc ggtcaagctc 60

acctgcactc tgagcagtgg gcacaccaac tacgccatcg cttggcgtca gcaacagcct 120

gggaaggccc ctcgatattt gatgctgctt aacagtgatg gcagccacac gagggggggc 180

gggatccctg atcgcttctc aggctccagt tctggggctg agcgctacct caccatctcc 240

agcctccagt ctgaggatga ggctgactat tactgtatga tttggcacaa caacgcttgg 300

gtgttcggcg gagggaccaa gctggccgtc ctt 333

<210> 151
<211> 5
<212> PRT
<213> Homo sapiens

<400> 151

Gly Tyr Tyr Met His
1               5

<210> 152
<211> 17
<212> PRT
<213> Homo sapiens

<400> 152

Gly Ile Ile Pro Ile Phe Gly Thr Ala Asn Tyr Ala Gln Lys Phe Gln
1               5                   10                  15

Gly

<210> 153
<211> 18
<212> PRT
<213> Homo sapiens

<400> 153

Ala Gly Met Glu Leu Thr Arg Ser Gly Ala Tyr Tyr Tyr Tyr Gly Met
1               5                   10                  15

Asp Val

<210> 154
<211> 127
<212> PRT
<213> Homo sapiens

<400> 154

Gln Val Gln Leu Gln Glu Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Gly Ala Gly Met Glu Leu Thr Arg Ser Gly Ala Tyr Tyr Tyr Tyr
            100                 105                 110

Gly Met Asp Val Trp Gly Gln Gly Thr Thr Val Thr Val Ser Ser
        115                 120                 125

<210> 155
<211> 14
<212> PRT

<213>  Homo sapiens

<400>  155

Thr Gly Thr Ser Ser Asp Ile Gly Gly Tyr Thr Phe Val Ser
1               5                   10


<210>  156
<211>  7
<212>  PRT
<213>  Homo sapiens

<400>  156

Asp Val Asn Asn Arg Pro Ser
1               5


<210>  157
<211>  10
<212>  PRT
<213>  Homo sapiens

<400>  157

Ser Ser Val Thr Ser Thr Asn Thr Tyr Val
1               5                   10


<210>  158
<211>  110
<212>  PRT
<213>  Homo sapiens

<400>  158

Gln Ser Ala Leu Thr Gln Pro Ala Ser Val Ser Gly Ser Pro Gly Gln
1               5                   10                  15


Ser Ile Thr Ile Ser Cys Thr Gly Thr Ser Ser Asp Ile Gly Gly Tyr
            20                  25                  30


Thr Phe Val Ser Trp Tyr Gln Gln His Pro Gly Lys Ala Pro Lys Val
        35                  40                  45


Met Ile His Asp Val Asn Asn Arg Pro Ser Gly Ile Ser Asn Arg Phe
    50                  55                  60


Ser Gly Ser Lys Ser Gly Asn Thr Ala Ser Leu Thr Ile Ser Gly Leu
65                  70                  75                  80


Gln Ala Glu Asp Glu Ala Asp Tyr Tyr Cys Ser Ser Val Thr Ser Thr
                85                  90                  95


Asn Thr Tyr Val Phe Gly Thr Gly Thr Lys Val Thr Val Leu
            100                 105                 110

<210>  159
<211>  381
<212>  DNA
<213>  Homo sapiens

<400>  159
caggtgcagc tgcaggagtc gggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggaggg atcatcccta tctttggtac agcaaactac     180

gcacagaagt tccagggcag agtcacgatt accgcggacg aatccacgag cacagcctac     240

atggagctga gcagcctgag atctgaggac acggccgtgt attactgtgc cggggcgggg     300

atggagctta ctcgctcggg tgcttactac tactacggta tggacgtctg gggccaaggg     360

accacggtca ccgtctcctc a                                               381


<210>  160
<211>  330
<212>  DNA
<213>  Homo sapiens

<400>  160
cagtctgccc tgactcagcc tgcctccgtg tctgggtctc ctggacagtc gatcaccatc      60

tcctgcactg gaaccagcag tgacattggt ggttatacct ttgtctcctg gtaccaacaa     120

cacccaggca aagcccccaa agtcatgatt catgatgtca ataatcggcc ctcagggatt     180

tctaatcgct tctctggctc caagtctggc aacacggcct ccctgaccat ctctgggctc     240

caggctgagg acgaggctga ttattactgc agctcagtta caagcaccaa cacttatgtc     300

ttcggaactg ggacaaaggt caccgtccta                                      330


<210>  161
<211>  5
<212>  PRT
<213>  Homo sapiens

<400>  161

Gly Tyr Tyr Met His
1               5


<210>  162
<211>  17
<212>  PRT
<213>  Homo sapiens

<400>  162

Trp Ile Asn Pro Asn Ser Gly Asn Thr Gly Tyr Ala Gln Lys Phe Gln
1               5                   10                  15

Gly

<210>  163
<211>  11
<212>  PRT
<213>  Homo sapiens

<400>  163

Gly Gly Gln Gln Gln Leu Val Leu Asp Asp Tyr
1               5                   10

<210>  164
<211>  120
<212>  PRT
<213>  Homo sapiens

<400>  164

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Gln Gln Gln Leu Val Leu Asp Asp Tyr Trp Gly Gln
            100                 105                 110

Gly Thr Leu Val Thr Val Ser Ser
        115                 120

<210>  165
<211>  11
<212>  PRT
<213>  Homo sapiens

<400>  165

Gln Ala Ser Gln Asp Ile Arg His His Leu Asn

1               5                   10


<210>  166
<211>  7
<212>  PRT
<213>  Homo sapiens

<400>  166

Asp Ser Ser Asn Leu Glu Thr
1               5


<210>  167
<211>  9
<212>  PRT
<213>  Homo sapiens

<400>  167

Gln Gln Tyr Asp Ser Leu Pro Arg Thr
1               5


<210>  168
<211>  107
<212>  PRT
<213>  Homo sapiens

<400>  168

Asp Ile Val Leu Thr Gln Ser Pro Ala Ser Leu Ser Ala Ser Val Gly
1               5                   10                  15

Asp Arg Ile Thr Ile Thr Cys Gln Ala Ser Gln Asp Ile Arg His His
            20                  25                  30

Leu Asn Trp Phe Gln His Lys Pro Gly Lys Ala Pro Lys Leu Leu Ile
        35                  40                  45

Ser Asp Ser Ser Asn Leu Glu Thr Gly Val Pro Ser Arg Phe Ser Gly
    50                  55                  60

Ser Gly Ser Gly Thr Asp Phe Ser Phe Thr Ile Ser Arg Leu Gln Pro
65                  70                  75                  80

Glu Asp Ile Ala Thr Tyr Tyr Cys Gln Gln Tyr Asp Ser Leu Pro Arg
                85                  90                  95

Thr Phe Gly Gln Gly Thr Lys Leu Glu Ile Lys
            100                 105


<210>  169
<211>  360
<212>  DNA

<213>  Homo sapiens

<400>  169
caggtgcagc tggtgcaatc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggatgg atcaacccta acagtggtaa cacaggctat     180

gcacagaagt tccagggcag agtcaccatg accaggaaca cctccataag cacagcctac     240

atggagctga gcagcctgag atctgaggac acggccgtgt attactgtgc gagaggcgga     300

cagcagcagc tggtactgga cgactactgg ggccagggaa ccctggtcac cgtctcctca     360


<210>  170
<211>  321
<212>  DNA
<213>  Homo sapiens

<400>  170
gatattgtgc tgacacagtc tccagcctcc ctgtctgcat ctgtcggcga caggatcacc      60

atcacctgcc aggcgagtca ggacattagg catcatttaa attggtttca gcacaaacca     120

gggaaagccc ccaagctcct gatctccgat tcatccaacc tggaaacagg agtcccgtca     180

aggttcagtg gaagtgggtc tgggacagat ttttctttca ccatcagccg cctgcagcct     240

gaagatattg cgacttatta ctgtcaacaa tatgatagtc tgcctcgaac ctttggccag     300

gggaccaaac tggagatcaa a                                               321


<210>  171
<211>  5
<212>  PRT
<213>  Homo sapiens

<400>  171

Ser Tyr Ala Met His
1               5


<210>  172
<211>  17
<212>  PRT
<213>  Homo sapiens

<400>  172

Val Ile Ser Tyr Asp Gly Ser Asn Lys Tyr Tyr Ala Asp Ser Val Lys
1               5                   10                  15


Gly


<210>  173
<211>  12

<212>  PRT
<213>  Homo sapiens

<400>  173

Ala Ser Pro Ser Gln Trp Leu Val Leu Gly His Tyr
1               5                   10


<210>  174
<211>  121
<212>  PRT
<213>  Homo sapiens

<400>  174

Gln Val Gln Leu Val Glu Ser Gly Gly Gly Val Val Gln Pro Gly Arg
1               5                   10                  15


Ser Leu Arg Leu Ser Cys Ala Ala Ser Gly Phe Thr Phe Ser Ser Tyr
            20                  25                  30


Ala Met His Trp Val Arg Gln Ala Pro Gly Lys Gly Leu Glu Trp Val
        35                  40                  45


Ala Val Ile Ser Tyr Asp Gly Ser Asn Lys Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Ala Ser Pro Ser Gln Trp Leu Val Leu Gly His Tyr Trp Gly
            100                 105                 110


Gln Gly Thr Leu Val Thr Val Ser Ser
        115                 120


<210>  175
<211>  12
<212>  PRT
<213>  Homo sapiens

<400>  175

Arg Ala Ser Gln Ser Val Ser Ser Ser Tyr Leu Ala
1               5                   10


<210>  176
<211>  7
<212>  PRT
<213>  Homo sapiens

<400>  176

Gly Ala Ser Ser Arg Ala Thr
1               5


<210>  177
<211>  9
<212>  PRT
<213>  Homo sapiens

<400>  177

Gln Gln Tyr Gly Ser Ser Pro Trp Thr
1               5


<210>  178
<211>  108
<212>  PRT
<213>  Homo sapiens

<400>  178

Glu Thr Thr Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Trp Thr Phe Gly Gln Gly Thr Lys Val Glu Ile Lys
            100                 105


<210>  179
<211>  363
<212>  DNA
<213>  Homo sapiens

<400>  179
caggtgcagc tggtggagtc tgggggaggc gtggtccagc ctgggaggtc cctgagactc      60

tcctgtgcag cctctggatt caccttcagt agctatgcta tgcactgggt ccgccaggct     120

ccaggcaagg ggctggagtg ggtggcagtt atatcatatg atggaagcaa taaatactac 180

gcagactccg tgaagggccg attcaccatc tccagagaca attccaagaa cacgctgtat 240

ctgcaaatga acagcctgag agctgaggac acggctgtgt attactgtgc gagagcctct 300

ccatcacagt ggctggtact cgggcactac tggggccagg gaaccctggt caccgtctcc 360

tca 363

<210> 180
<211> 324
<212> DNA
<213> Homo sapiens

<400> 180
gaaacgacac tcacgcagtc tccaggcacc ctgtctttgt ctccagggga aagagccacc 60

ctctcctgca gggccagtca gagtgttagc agcagctact tagcctggta ccagcagaaa 120

cctggccagg ctcccaggct cctcatctat ggtgcatcca gcagggccac tggcatccca 180

gacaggttca gtggcagtgg gtctgggaca gacttcactc tcaccatcag cagactggag 240

cctgaagatt ttgcagtgta ttactgtcag cagtatggta gctcaccgtg gacgttcggc 300

caagggacca aggtggaaat caaa 324

<210> 181
<211> 5
<212> PRT
<213> Homo sapiens

<400> 181

Gly Tyr Tyr Met His
1               5

<210> 182
<211> 17
<212> PRT
<213> Homo sapiens

<400> 182

Trp Ile Asn Pro Asn Ser Gly Asn Thr Gly Tyr Ala Gln Lys Phe Gln
1               5                   10                  15

Gly

<210> 183
<211> 11
<212> PRT
<213> Homo sapiens

<400> 183

Gly Gly Gln Gln Gln Leu Val Leu Asp Asp Tyr
1               5                   10

<210>  184
<211>  120
<212>  PRT
<213>  Homo sapiens

<400>  184

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Gln Gln Gln Leu Val Leu Asp Asp Tyr Trp Gly Gln
            100                 105                 110

Gly Thr Leu Val Thr Val Ser Ser
        115                 120

<210>  185
<211>  11
<212>  PRT
<213>  Homo sapiens

<400>  185

Arg Ala Ser Gln Ser Val Ser Ser Asn Leu Ala
1               5                   10

<210>  186
<211>  7
<212>  PRT
<213>  Homo sapiens

<400>  186

Gly Ala Ser Thr Arg Ala Thr
1               5

<210>  187
<211>  9
<212>  PRT
<213>  Homo sapiens

<400>  187

Gln Gln Tyr Asn Asn Trp Pro Trp Thr
1               5

<210>  188
<211>  107
<212>  PRT
<213>  Homo sapiens

<400>  188

Glu Thr Thr Leu Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Asn Trp Pro Trp
                85                  90                  95

Thr Phe Gly Gln Gly Thr Lys Val Glu Thr Lys
            100                 105

<210>  189
<211>  360
<212>  DNA
<213>  Homo sapiens

<400>  189
caggtgcagc tggtgcagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggatgg atcaacccta acagtggtaa cacaggctat     180

gcacagaagt tccagggcag agtcaccatg accaggaaca cctccataag cacagcctac     240

atggagctga gcagcctgag atctgaggac acggccgtgt attactgtgc gagaggcgga     300

cagcagcagc tggtactgga cgactactgg ggccagggaa ccctggtcac cgtctcctca 360

<210> 190
<211> 321
<212> DNA
<213> Homo sapiens

<400> 190
gaaacgacac tcacgcagtc tccagccacc ctgtctgtgt ctccagggga aagagccacc 60

ctctcctgca gggccagtca gagtgttagc agcaacttag cctggtacca gcagaaacct 120

ggccaggctc ccaggctcct catctatggt gcatccacca gggccactgg tatcccagcc 180

aggttcagtg gcagtgggtc tgggacagag ttcactctca ccatcagcag cctgcagtct 240

gaagattttg cagtttatta ctgtcagcag tataataact ggccttggac gttcggccaa 300

gggaccaagg tggaaaccaa a 321

<210> 191
<211> 7
<212> PRT
<213> Homo sapiens

<400> 191

Ser Gly Gly Tyr Tyr Trp Ser
1               5

<210> 192
<211> 16
<212> PRT
<213> Homo sapiens

<400> 192

Tyr Ile Tyr Tyr Ser Gly Ser Thr Tyr Tyr Asn Pro Ser Leu Lys Ser
1               5                   10                  15

<210> 193
<211> 18
<212> PRT
<213> Homo sapiens

<400> 193

Gly Gly Ile Ser Pro Ser Gly Ser Ser Ile Tyr Tyr Tyr Tyr Gly Met
1               5                   10                  15

Asp Val

<210> 194
<211> 128
<212> PRT

<213>  Homo sapiens

<400>  194

Gln Leu Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Gly Gly Ile Ser Pro Ser Gly Ser Ser Ile Tyr Tyr Tyr
            100                 105                 110

Tyr Gly Met Asp Val Trp Gly Gln Gly Thr Thr Val Thr Val Ser Ser
        115                 120                 125

<210>  195
<211>  14
<212>  PRT
<213>  Homo sapiens

<400>  195

Thr Gly Thr Ser Asn Asp Val Gly Gly Tyr Asn Tyr Val Ser
1               5                   10

<210>  196
<211>  7
<212>  PRT
<213>  Homo sapiens

<400>  196

Glu Val Asn Lys Arg Pro Ser
1               5

<210>  197
<211>  9
<212>  PRT
<213>  Homo sapiens

<400>  197

Ser Ser Tyr Ala Gly Thr Lys Glu Val
1               5

<210>  198
<211>  109
<212>  PRT
<213>  Homo sapiens

<400>  198

Gln Pro Val Leu Thr Gln Pro Pro Ser Ala Ser Gly Ser Pro Gly Gln
1               5                   10                  15

Ser Val Thr Ile Ser Cys Thr Gly Thr Ser Asn Asp Val Gly Gly Tyr
            20                  25                  30

Asn Tyr Val Ser Trp Tyr Gln Gln His Pro Gly Lys Ala Pro Lys Leu
        35                  40                  45

Ile Ile Tyr Glu Val Asn Lys Arg Pro Ser Gly Val Pro Asp Arg Phe
    50                  55                  60

Ser Gly Ser Lys Ser Gly Asn Thr Ala Ser Leu Thr Val Ser Gly Leu
65                  70                  75                  80

Gln Ala Val Asp Glu Ser Asp Tyr Tyr Cys Ser Ser Tyr Ala Gly Thr
                85                  90                  95

Lys Glu Val Phe Gly Gly Gly Thr Lys Leu Thr Val Leu
            100                 105

<210>  199
<211>  384
<212>  DNA
<213>  Homo sapiens

<400>  199
cagctgcagc tgcaggagtc cggcccagga ctggtgaagc cttcacagac cctgtccctc      60

acctgcactg tctctggtgg ctccatcagc agtggtggtt actactggag ctggatccgc     120

cagcacccag ggaagggcct ggagtggatt gggtacatct attacagtgg gagcacctac     180

tacaacccgt ccctcaagag tcgagttacc atatcagtag acacgtctaa gaaccagttc     240

tccctgaagc tgagctctgt gactgccgcg gacacggccg tgtattactg tgcgagaggt     300

gggatatcac cctccgggtc ctcgatctac tactactacg gtatggacgt ctggggccaa     360

gggaccacgg tcaccgtctc ctca                                            384

<210>  200

<211>  327
<212>  DNA
<213>  Homo sapiens

<400>  200
cagcctgtgc tgactcagcc accctccgcg tccgggtctc ctggacagtc agtcaccatc      60

tcctgcactg gaaccagcaa tgacgttggt ggttataact atgtctcctg gtaccaacaa     120

cacccaggca aagcccccaa actcataatt tatgaggtca ataagcggcc ctcaggggtc     180

cctgatcgct tctctggctc caagtctggc aacacggcct ccctgaccgt ctctgggctc     240

caggctgtgg atgagtctga ttattactgc agctcatatg caggcaccaa ggaggtcttc     300

ggcggaggga ccaagctgac cgtccta                                         327


<210>  201
<211>  5
<212>  PRT
<213>  Homo sapiens

<400>  201

Ser Tyr Gly Ile Ser
1               5


<210>  202
<211>  17
<212>  PRT
<213>  Homo sapiens

<400>  202

Trp Ile Ser Ala Tyr Asn Gly Asn Thr Asn Tyr Ala Gln Lys Leu Gln
1               5                   10                  15


Gly




<210>  203
<211>  10
<212>  PRT
<213>  Homo sapiens

<400>  203

Val Pro Ala Trp Ser Gly Gln Phe Asp Tyr
1               5                   10


<210>  204
<211>  119
<212>  PRT
<213>  Homo sapiens

<400>  204

Gln Val Gln Leu Val Gln Ser Gly Ala Glu Val Lys Lys Pro Gly Ala

1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Trp Ile Ser Ala Tyr Asn Gly Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Val Pro Ala Trp Ser Gly Gln Phe Asp Tyr Trp Gly Gln Gly
            100                 105                 110

Thr Leu Val Thr Val Ser Ser
        115

<210>  205
<211>  14
<212>  PRT
<213>  Homo sapiens

<400>  205

Thr Gly Thr Ser Ser Asp Val Gly Gly Tyr Asn Tyr Val Ser
1               5                   10

<210>  206
<211>  7
<212>  PRT
<213>  Homo sapiens

<400>  206

Glu Val Thr Asn Arg Pro Ser
1               5

<210>  207
<211>  11
<212>  PRT
<213>  Homo sapiens

<400>  207

Asn Ser Tyr Thr Ser Gly Pro Thr Tyr Val Leu
1               5                   10

<210>  208
<211>  111
<212>  PRT
<213>  Homo sapiens

<400>  208

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Glu Val Thr Asn Arg Pro Ser Gly Val Ser Asp Arg Phe
    50                  55                  60

Ser Gly Ser Lys Ser Ala Asn Thr Ala Ser Leu Thr Ile Ser Glu Leu
65                  70                  75                  80

Gln Ala Glu Asp Glu Ala Val Tyr Tyr Cys Asn Ser Tyr Thr Ser Gly
                85                  90                  95

Pro Thr Tyr Val Leu Phe Gly Gly Gly Thr Gln Leu Thr Val Leu
            100                 105                 110

<210>  209
<211>  357
<212>  DNA
<213>  Homo sapiens

<400>  209
caggtgcagc tggtgcagtc tggagctgag gtgaagaagc ctggggcctc agtgaaggtc      60

tcctgcaagg cttctggtta cacctttacc agctatggta tcagctgggt gcgacaggcc     120

cctggacaag ggcttgagtg gatgggatgg atcagcgctt acaatggtaa cacaaactat     180

gcacagaagc tccagggcag agtcaccatg accacagaca catccacgag cacagcctac     240

atggagctga ggagcctgag atctgacgac acggccgtgt attactgtgc gagagtccct     300

gcgtggagtg gtcaatttga ctactggggc cagggaaccc tggtcaccgt ctcctca        357

<210>  210
<211>  333
<212>  DNA
<213>  Homo sapiens

<400>  210
cagtctgccc tgactcagcc tgcctccgtg tctgggtctc ctggacagtc gatcaccatc      60

tcctgcactg gaaccagcag tgacgttggt ggttataact atgtctcctg gtaccaacag 120

cacccaggca aagcccccaa actcatgatt tatgaggtca ctaatcggcc ctcaggggtt 180

tccgatcgct tctctggctc caagtctgcc aacacggcct ccctgaccat ctctgagctc 240

caggctgaag acgaggctgt ttattactgc aactcataca caagcggccc cacttatgtg 300

ctgttcggcg gagggaccca gctgaccgtc cta 333

<210> 211
<211> 7
<212> PRT
<213> Homo sapiens

<400> 211

Ser Gly Gly Tyr Tyr Trp Ser
1               5

<210> 212
<211> 16
<212> PRT
<213> Homo sapiens

<400> 212

Tyr Ile Tyr Tyr Ser Gly Ser Thr Tyr Tyr Asn Pro Ser Leu Lys Ser
1               5                   10                  15

<210> 213
<211> 11
<212> PRT
<213> Homo sapiens

<400> 213

Ala Ser Arg Ser Thr Asp Tyr Tyr Phe Asp Tyr
1               5                   10

<210> 214
<211> 121
<212> PRT
<213> Homo sapiens

<400> 214

Glu Val Gln Leu Leu Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Ala Ser Arg Ser Thr Asp Tyr Tyr Phe Asp Tyr Trp Gly
            100                 105                 110

Gln Gly Thr Leu Val Thr Val Ser Ser
        115                 120

<210>  215
<211>  13
<212>  PRT
<213>  Homo sapiens

<400>  215

Thr Gly Asn Arg Asn Asn Ile Gly Asp Gln Gly Ala Ala
1               5                   10

<210>  216
<211>  7
<212>  PRT
<213>  Homo sapiens

<400>  216

Arg Asn Asn Asn Gly Pro Ser
1               5

<210>  217
<211>  11
<212>  PRT
<213>  Homo sapiens

<400>  217

Ser Ala Trp Asp Ser Ser Leu Arg Ala Trp Val
1               5                   10

<210>  218
<211>  110
<212>  PRT
<213>  Homo sapiens

<400>  218

Gln Pro Gly Leu Thr Gln Pro Pro Ser Met Ser Tyr Gly Leu Gly Gln
1               5                   10                  15

Thr Ala Thr Leu Thr Cys Thr Gly Asn Arg Asn Asn Ile Gly Asp Gln
            20                  25                  30

Gly Ala Ala Trp Leu Gln Gln His Gln Gly His Pro Pro Lys Leu Leu
        35                  40                  45

Ser Tyr Arg Asn Asn Asn Gly Pro Ser Gly Ile Ser Glu Arg Leu Ser
    50                  55                  60

Ala Ser Arg Ser Gly Asn Thr Ala Ser Leu Thr Ile Ser Gly Leu Gln
65                  70                  75                  80

Pro Glu Asp Glu Ala Asp Tyr Tyr Cys Ser Ala Trp Asp Ser Ser Leu
                85                  90                  95

Arg Ala Trp Val Phe Gly Gly Gly Thr Lys Leu Thr Val Leu
            100                 105                 110

<210>  219
<211>  363
<212>  DNA
<213>  Homo sapiens

<400>  219
gaagtgcagc tgttggagtc tggcccagga ctggtgaagc cttcacagac cctgtccctc      60

acctgcactg tctctggtgg ctccatcagc agtggtggtt actactggag ctggatccgc     120

cagcacccag ggaagggcct ggagtggatt gggtacatct attacagtgg gagcacctac     180

tacaacccgt ccctcaagag tcgagttacc atatcagtag acacgtctaa gaaccagttc     240

tccctgaagc tgagctctgt gactgccgcg gacacggccg tgtattactg tgcgagggct     300

tcacgatcga ccgattacta ctttgactac tggggccagg gaaccctggt caccgtctcc     360

tca                                                                   363

<210>  220
<211>  330
<212>  DNA
<213>  Homo sapiens

<400>  220
cagccagggc tgactcagcc accctcgatg tcctacggct tgggacagac cgccacactc      60

acctgcactg ggaacagaaa caatattggc gaccaaggag cagcttggct gcagcagcac     120

cagggccacc ctcccaaact cctatcctac aggaataaca acgggccctc agggatctca     180

gagagattat ctgcatccag gtcaggaaac acagcctccc tgaccattag tggactccag     240

cctgaggacg aggctgacta ttactgctca gcatgggaca gcagcctcag ggcttgggtg     300

ttcggcggag ggaccaagct gaccgtcctc                                      330

<210>  221
<211>  5
<212>  PRT
<213>  Homo sapiens

<400>  221

Ser Tyr Ala Ile Ser
1               5


<210>  222
<211>  17
<212>  PRT
<213>  Homo sapiens

<400>  222

Gly Ile Ile Pro Ile Phe Gly Thr Ala Asn Tyr Ala Gln Lys Phe Gln
1               5                   10                  15


Gly


<210>  223
<211>  15
<212>  PRT
<213>  Homo sapiens

<400>  223

Pro Lys Tyr Ser Ser Gly Trp Phe Tyr Tyr Tyr Gly Met Asp Val
1               5                   10                  15


<210>  224
<211>  124
<212>  PRT
<213>  Homo sapiens

<400>  224

Gln Val Gln Leu Val Gln Ser Gly Ala Glu Val Lys Lys Pro Gly Ser
1               5                   10                  15


Ser Val Lys Val Ser Cys Lys Ala Ser Gly Gly Thr Phe Ser Ser Tyr
            20                  25                  30


Ala Ile Ser Trp Val Arg Gln Ala Pro Gly Gln Gly Leu Glu Trp Met
        35                  40                  45


Gly Gly Ile Ile Pro Ile Phe Gly Thr Ala Asn Tyr Ala Gln Lys Phe
    50                  55                  60


Gln Gly Arg Val Thr Ile Thr Ala Asp Glu Ser Thr Ser Thr Ala Tyr

65                  70                  75                  80

Met Glu Leu Ser Ser Leu Arg Ser Glu Asp Thr Ala Val Tyr Tyr Cys
                85                  90                  95

Ala Arg Pro Lys Tyr Ser Ser Gly Trp Phe Tyr Tyr Tyr Gly Met Asp
            100                 105                 110

Val Trp Gly Gln Gly Thr Thr Val Thr Val Ser Ser
        115                 120

<210>  225
<211>  13
<212>  PRT
<213>  Homo sapiens

<400>  225

Ser Gly Ser Ser Ser Asn Ile Gly Asn Asn Tyr Val Ser
1               5                   10

<210>  226
<211>  7
<212>  PRT
<213>  Homo sapiens

<400>  226

Asp Asn Asn Lys Arg Pro Ser
1               5

<210>  227
<211>  10
<212>  PRT
<213>  Homo sapiens

<400>  227

Gly Thr Trp Asp Ser Ser Leu Ser Val Val
1               5                   10

<210>  228
<211>  109
<212>  PRT
<213>  Homo sapiens

<400>  228

Gln Ser Val Leu Thr Gln Pro Pro Ser Val Ser Ala Ala Pro Gly Gln
1               5                   10                  15

Lys Val Thr Ile Ser Cys Ser Gly Ser Ser Ser Asn Ile Gly Asn Asn
            20                  25                  30

Tyr Val Ser Trp Tyr Gln Gln Leu Pro Gly Thr Ala Pro Lys Leu Leu
        35                  40                  45

Ile Tyr Asp Asn Asn Lys Arg Pro Ser Gly Ile Pro Asp Arg Phe Ser
    50                  55                  60

Gly Ser Lys Ser Gly Thr Ser Ala Thr Leu Gly Ile Thr Gly Leu Gln
65                  70                  75                  80

Thr Gly Asp Glu Ala Asp Tyr Tyr Cys Gly Thr Trp Asp Ser Ser Leu
                85                  90                  95

Ser Val Val Phe Gly Gly Gly Thr Lys Leu Thr Val Leu
            100                 105

<210> 229
<211> 372
<212> DNA
<213> Homo sapiens

<400> 229
caggtccagc tggtgcagtc tggggctgag gtgaagaagc ctgggtcctc ggtgaaggtc 60

tcctgcaagg cttctggagg caccttcagc agctatgcta tcagctgggt gcgacaggcc 120

cctggacaag ggcttgagtg gatgggaggg atcatcccta tctttggtac agcaaactac 180

gcacagaagt tccagggcag agtcacgatt accgcggacg aatccacgag cacagcctac 240

atggagctga gcagcctgag atctgaggac acggccgtgt attactgtgc gagaccaaag 300

tatagcagtg gctggttcta ctactacggt atggacgtct ggggccaagg gaccacggtc 360

accgtctcct ca 372

<210> 230
<211> 327
<212> DNA
<213> Homo sapiens

<400> 230
cagtctgtgc tgactcagcc accctcagtg tctgcggccc caggacagaa ggtcaccatc 60

tcctgctctg gaagcagctc caacattggg aataattatg tatcctggta ccagcagctc 120

ccaggaacag cccccaaact cctcatttat gacaataata agcgaccctc agggattcct 180

gaccgattct ctggctccaa gtctggcacg tcagccaccc tgggcatcac cggactccag 240

actggggacg aggccgatta ttactgcgga acatgggata gcagcctgag tgtggtattc 300

ggcggaggga ccaagctgac cgtccta 327

<210> 231
<211> 5
<212> PRT

<213>  Homo sapiens

<400>  231

Ser Tyr Ala Ile Ser
1               5


<210>  232
<211>  17
<212>  PRT
<213>  Homo sapiens

<400>  232

Trp Ile Ser Ala Tyr Asn Gly Asn Thr Asn Tyr Ala Gln Lys Leu Gln
1               5                   10                  15


Gly


<210>  233
<211>  12
<212>  PRT
<213>  Homo sapiens

<400>  233

Leu Gly Ser Tyr Gly Tyr Thr Gly Ala Phe Asp Ile
1               5                   10


<210>  234
<211>  121
<212>  PRT
<213>  Homo sapiens

<400>  234

Gln Met Gln Leu Val Gln Ser Gly Ala Glu Val Lys Lys Pro Gly Ser
1               5                   10                  15


Ser Val Lys Val Ser Cys Lys Ala Ser Gly Gly Thr Phe Ser Ser Tyr
            20                  25                  30


Ala Ile Ser Trp Val Arg Gln Ala Pro Gly Gln Gly Leu Glu Trp Met
        35                  40                  45


Gly Trp Ile Ser Ala Tyr Asn Gly Asn Thr Asn Tyr Ala Gln Lys Leu
    50                  55                  60


Gln Gly Arg Val Thr Met Thr Thr Asp Thr Ser Thr Ser Thr Ala Tyr
65                  70                  75                  80


Met Glu Leu Arg Ser Leu Arg Ser Asp Asp Thr Ala Val Tyr Tyr Cys
                85                  90                  95

Ala Ser Leu Gly Ser Tyr Gly Tyr Thr Gly Ala Phe Asp Ile Trp Gly
            100                 105                 110

Gln Gly Thr Met Ala Thr Val Ser Ser
        115                 120

<210> 235
<211> 14
<212> PRT
<213> Homo sapiens

<400> 235

Thr Gly Thr Ser Ser Asp Val Gly Gly Tyr Asn Tyr Val Ser
1               5                   10

<210> 236
<211> 7
<212> PRT
<213> Homo sapiens

<400> 236

Glu Val Ser Asn Arg Pro Ser
1               5

<210> 237
<211> 11
<212> PRT
<213> Homo sapiens

<400> 237

Ser Ser Tyr Thr Ser Ser Ser Thr Leu Val Val
1               5                   10

<210> 238
<211> 111
<212> PRT
<213> Homo sapiens

<400> 238

Gln Ser Ala Leu Thr Gln Pro Ala Ser Val Ser Gly Ser Pro Gly Gln
1               5                   10                  15

Ser Ile Thr Ile Ser Cys Thr Gly Thr Ser Ser Asp Val Gly Gly Tyr
            20                  25                  30

Asn Tyr Val Ser Trp Tyr Gln Gln His Pro Gly Lys Ala Pro Lys Leu
        35                  40                  45

Met Ile Tyr Glu Val Ser Asn Arg Pro Ser Gly Val Ser Asn Arg Phe

50                  55                  60

Ser Gly Ser Lys Ser Gly Asn Thr Ala Ser Leu Thr Ile Ser Gly Leu
65                  70                  75                  80

Gln Ala Glu Asp Glu Ala Asp Tyr Tyr Cys Ser Ser Tyr Thr Ser Ser
                85                  90                  95

Ser Thr Leu Val Val Phe Gly Gly Gly Thr Lys Leu Thr Val Leu
            100                 105                 110

<210>  239
<211>  363
<212>  DNA
<213>  Homo sapiens

<400>  239
caaatgcagc tggtacaatc tggggctgag gtgaagaagc ctgggtcctc ggtgaaggtc      60

tcctgcaagg cttctggagg caccttcagc agctatgcta tcagctgggt gcgacaggcc     120

cctggacaag ggcttgagtg gatgggatgg atcagcgctt acaatggtaa cacaaactat     180

gcacagaagc tccagggcag agtcaccatg accacagaca catccacgag cacagcctac     240

atggagctga ggagcctgag atctgacgac acggccgtgt attactgtgc gagcctgggg     300

agctatggtt atacaggggc ttttgatatc tggggccaag ggacaatggc caccgtctct     360

tca                                                                   363

<210>  240
<211>  333
<212>  DNA
<213>  Homo sapiens

<400>  240
cagtctgccc tgactcagcc tgcctccgtg tctgggtctc ctggacagtc gatcaccatc      60

tcctgcactg gaaccagcag tgacgttggt ggttataact atgtctcctg gtaccaacag     120

cacccaggca aagcccccaa actcatgatt tatgaggtca gtaatcggcc ctcaggggtt     180

tctaatcgct tctctggctc caagtctggc aacacggcct ccctgaccat ctctgggctc     240

caggctgagg acgaggctga ttattactgc agctcatata caagcagcag cactctcgtg     300

gtattcggcg gagggaccaa gctgaccgtc cta                                  333

<210>  241
<211>  5
<212>  PRT
<213>  Homo sapiens

<400>  241

Ser Tyr Ala Ile Ser

1               5


<210>  242
<211>  17
<212>  PRT
<213>  Homo sapiens

<400>  242

Gly Ile Ile Pro Ile Phe Gly Thr Ala Asn Tyr Ala Gln Lys Phe Gln
1               5                   10                  15


Gly




<210>  243
<211>  11
<212>  PRT
<213>  Homo sapiens

<400>  243

Gly Gly Trp Leu Arg Gln Asn Trp Phe Asp Pro
1               5                   10


<210>  244
<211>  120
<212>  PRT
<213>  Homo sapiens

<400>  244

Gln Val Gln Leu Val Gln Ser Gly Ala Glu Val Lys Lys Pro Gly Ser
1               5                   10                  15


Ser Val Lys Val Ser Cys Lys Ala Ser Gly Gly Thr Phe Ser Ser Tyr
            20                  25                  30


Ala Ile Ser Trp Val Arg Gln Ala Pro Gly Gln Gly Leu Glu Trp Met
        35                  40                  45


Gly Gly Ile Ile Pro Ile Phe Gly Thr Ala Asn Tyr Ala Gln Lys Phe
    50                  55                  60


Gln Gly Arg Val Thr Ile Thr Ala Asp Glu Ser Thr Ser Thr Ala Tyr
65                  70                  75                  80


Met Glu Leu Thr Ser Leu Arg Ser Glu Asp Thr Ala Val Tyr Tyr Cys
                85                  90                  95


Ala Arg Gly Gly Trp Leu Arg Gln Asn Trp Phe Asp Pro Trp Gly Gln
            100                 105                 110

Gly Thr Leu Val Thr Val Ser Ser
        115                 120

<210>  245
<211>  11
<212>  PRT
<213>  Homo sapiens

<400>  245

Gln Gly Asp Ser Leu Arg Ser Tyr Tyr Ala Ser
1               5                   10

<210>  246
<211>  7
<212>  PRT
<213>  Homo sapiens

<400>  246

Gly Lys Asn Asn Arg Pro Ser
1               5

<210>  247
<211>  12
<212>  PRT
<213>  Homo sapiens

<400>  247

Asn Ser Arg Asp Ser Ser Gly Asn His Pro Arg Val
1               5                   10

<210>  248
<211>  109
<212>  PRT
<213>  Homo sapiens

<400>  248

Ser Ser Glu Leu Thr Gln Asp Pro Ala Val Ser Val Ala Leu Gly Gln
1               5                   10                  15

Thr Val Arg Ile Thr Cys Gln Gly Asp Ser Leu Arg Ser Tyr Tyr Ala
            20                  25                  30

Ser Trp Tyr Gln Gln Lys Pro Gly Gln Ala Pro Val Leu Val Ile Tyr
        35                  40                  45

Gly Lys Asn Asn Arg Pro Ser Gly Ile Pro Asp Arg Phe Ser Gly Ser
    50                  55                  60

Ser Ser Gly Asn Thr Ala Ser Leu Thr Ile Thr Gly Ala Gln Ala Glu
65                  70                  75                  80

Asp Glu Ala Asp Tyr Tyr Cys Asn Ser Arg Asp Ser Ser Gly Asn His
                85                  90                  95

Pro Arg Val Phe Gly Gly Gly Thr Lys Leu Thr Val Leu
            100                 105

<210>  249
<211>  360
<212>  DNA
<213>  Homo sapiens

<400>  249
caggtgcagc tggtgcagtc tggggctgag gtgaagaagc ctgggtcctc ggtgaaggtc      60

tcctgcaagg cttctggagg caccttcagc agctatgcta tcagctgggt gcgacaggcc     120

cctggacaag ggcttgagtg gatgggaggg atcatcccta tctttggtac agcaaactac     180

gcacagaagt tccagggcag agtcacgata accgcggacg aatccacgag cacagcctac     240

atggagctga ccagcctgag atctgaggac acggccgtgt attactgtgc gagagggggg     300

tggctacgac agaactggtt cgacccctgg ggccagggaa ccctggtcac cgtctcctca     360

<210>  250
<211>  327
<212>  DNA
<213>  Homo sapiens

<400>  250
tcttctgagc tgactcagga ccctgctgtg tctgtggcct tgggacaaac agtcaggatc      60

acatgccaag gagacagcct cagaagctat tatgcaagct ggtaccagca gaagccagga     120

caggcccctg tacttgtcat ctatggtaaa aacaaccggc cctcagggat cccagaccga     180

ttctctggct ccagctcagg aaacacagct tccttgacca tcactggggc tcaggcggaa     240

gatgaggctg actattactg taactcccgg gacagcagtg gtaaccatcc aagggtattc     300

ggcggaggga ccaagctgac cgtccta                                         327

<210>  251
<211>  7
<212>  PRT
<213>  Homo sapiens

<400>  251

Ser Gly Gly Tyr Tyr Trp Ser
1               5

<210>  252
<211>  16
<212>  PRT
<213>  Homo sapiens

<400>  252

Tyr Ile Tyr Tyr Ser Gly Ser Thr Tyr Tyr Asn Pro Ser Leu Lys Ser
1               5                   10                  15


<210>  253
<211>  13
<212>  PRT
<213>  Homo sapiens

<400>  253

Trp Ser Leu Gly Thr Ser Asn His Gly Trp Phe Asp Pro
1               5                   10


<210>  254
<211>  123
<212>  PRT
<213>  Homo sapiens

<400>  254

Gln Val Gln Leu Gln Glu Ser Gly Pro Gly Leu Val Lys Pro Ser Gln
1               5                   10                  15


Thr Leu Ser Leu Thr Cys Thr Val Ser Gly Gly Ser Ile Ser Ser Gly
            20                  25                  30


Gly Tyr Tyr Trp Ser Trp Ile Arg Gln His Pro Gly Lys Gly Leu Glu
        35                  40                  45


Trp Ile Gly Tyr Ile Tyr Tyr Ser Gly Ser Thr Tyr Tyr Asn Pro Ser
    50                  55                  60


Leu Lys Ser Arg Val Thr Ile Ser Val Asp Thr Ser Lys Asn Gln Phe
65                  70                  75                  80


Ser Leu Lys Leu Ser Ser Val Thr Ala Ala Asp Thr Ala Val Tyr Tyr
                85                  90                  95


Cys Ala Ser Trp Ser Leu Gly Thr Ser Asn His Gly Trp Phe Asp Pro
            100                 105                 110


Trp Gly Gln Gly Thr Leu Val Thr Val Ser Ser
        115                 120


<210>  255
<211>  13
<212>  PRT
<213>  Homo sapiens

<400>  255

Ser Gly Ser Ser Ser Asn Ile Gly Ser Asn Thr Val Asn
1               5                   10

<210>  256
<211>  7
<212>  PRT
<213>  Homo sapiens

<400>  256

Asp Asn Asn Asn Arg Pro Ser
1               5

<210>  257
<211>  11
<212>  PRT
<213>  Homo sapiens

<400>  257

Gln Ser Tyr Asp Ser Asn Leu Ser Gly Trp Val
1               5                   10

<210>  258
<211>  110
<212>  PRT
<213>  Homo sapiens

<400>  258

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Thr Val Asn Trp Tyr Gln Gln Leu Pro Gly Thr Ala Pro Lys Leu Leu
        35                  40                  45

Ile Ser Asp Asn Asn Asn Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ala Glu Asp Glu Ala Asp Tyr Tyr Cys Gln Ser Tyr Asp Ser Asn Leu
                85                  90                  95

Ser Gly Trp Val Phe Gly Gly Gly Thr Lys Leu Thr Val Leu
            100                 105                 110

<210>  259

<211>  369
<212>  DNA
<213>  Homo sapiens

<400>  259
caggtgcaac tgcaggagtc gggcccagga ctggtgaagc cttcacagac cctgtccctc      60

acctgcactg tctctggtgg ctccatcagc agtggtggtt actactggag ctggatccgc     120

cagcacccag ggaagggcct ggagtggatt gggtacatct attacagtgg gagcacctac     180

tacaacccgt ccctcaagag tcgagttacc atatcagtag acacgtctaa gaaccagttc     240

tccctgaagc tgagctctgt gaccgccgca gacacggctg tgtattactg tgcgagctgg     300

tccctaggta ccagcaacca tggttggttc gacccctggg gccagggaac cctggtcacc     360

gtctcctca                                                             369


<210>  260
<211>  330
<212>  DNA
<213>  Homo sapiens

<400>  260
cagtctgtgc tgactcagcc accctcagcg tctgggaccc ccgggcagag ggtcaccatc      60

tcttgttctg gaagcagctc caacatcgga agtaatactg taaactggta ccagcagctt     120

ccaggaacag cccccaaact cctcatctct gataataaca atcggccctc aggggtccct     180

gaccgattct ctggctccaa gtctggcacc tcagcctccc tggccatcac tgggctccag     240

gctgaggatg aggctgatta ttactgccag tcctatgaca gtaacctgag tggttgggtg     300

ttcggcggag ggaccaagct gaccgtccta                                      330


<210>  261
<211>  5
<212>  PRT
<213>  Homo sapiens

<400>  261

Ser Tyr Ala Ile Ser
1               5


<210>  262
<211>  17
<212>  PRT
<213>  Homo sapiens

<400>  262

Gly Ile Ile Pro Ile Phe Gly Thr Ala Asn Tyr Ala Gln Lys Phe Gln
1               5                   10                  15


Gly

<210> 263
<211> 11
<212> PRT
<213> Homo sapiens

<400> 263

Ala Arg Gly Ser Thr Trp Gly Tyr Phe Asp Tyr
1               5                   10


<210> 264
<211> 120
<212> PRT
<213> Homo sapiens

<400> 264

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ala Arg Gly Ser Thr Trp Gly Tyr Phe Asp Tyr Trp Gly Gln
            100                 105                 110

Gly Thr Leu Val Thr Val Ser Ser
        115                 120


<210> 265
<211> 11
<212> PRT
<213> Homo sapiens

<400> 265

Arg Ala Ser Gln Ser Val Ser Asn Tyr Leu Ala
1               5                   10

<210>  266
<211>  7
<212>  PRT
<213>  Homo sapiens

<400>  266

Asp Ala Ser Asn Arg Ala Thr
1               5


<210>  267
<211>  9
<212>  PRT
<213>  Homo sapiens

<400>  267

Gln Gln Arg Asp Asn Trp Pro Leu Thr
1               5


<210>  268
<211>  107
<212>  PRT
<213>  Homo sapiens

<400>  268

Glu Ile Val Leu Thr Gln Ser Pro Ala Ser Leu Ser Leu Ser Pro Gly
1               5                   10                  15


Glu Arg Val Thr Leu Ser Cys Arg Ala Ser Gln Ser Val Ser Asn Tyr
            20                  25                  30


Leu Ala Trp Tyr Gln Gln Lys Pro Gly Gln Ala Pro Arg Leu Leu Ile
        35                  40                  45


Tyr Asp Ala Ser Asn Arg Ala Thr Gly Ile Pro Ala Arg Phe Ser Gly
    50                  55                  60


Ser Gly Ser Gly Thr Asp Phe Thr Leu Thr Ile Ser Ser Leu Glu Pro
65                  70                  75                  80


Glu Asp Phe Ala Val Tyr Tyr Cys Gln Gln Arg Asp Asn Trp Pro Leu
                85                  90                  95


Thr Phe Gly Gly Gly Thr Lys Val Glu Ile Lys
            100                 105


<210>  269
<211>  360
<212>  DNA
<213>  Homo sapiens

<400>  269

caggtgcagc tggtacaatc tggggctgag gtgaagaagc ctgggtcctc ggtgaaggtc 60

tcctgcaagg cttctggagg caccttcagc agctatgcta tcagctgggt gcgacaggcc 120

cctggacaag ggcttgagtg gatgggaggg atcatcccta tctttggtac agcaaactac 180

gcacagaagt tccagggcag agtcacgatt accgcggaca aatccacgag cacagcctac 240

atggagctga gcagcctgag atctgaggac acggccgtgt attactgtgc gagagcgagg 300

ggcagcacct ggggctactt tgactactgg ggccagggaa ccctggtcac cgtctcctca 360

<210> 270
<211> 321
<212> DNA
<213> Homo sapiens

<400> 270
gaaatagtgt tgacgcagtc tccagcctcc ctgtctttgt ctccagggga aagagtcacc 60

ctctcctgca gggccagtca gagtgttagc aattacttag cctggtatca acagaaacct 120

ggccaggctc ccaggctcct catctatgat gcatccaaca gggccactgg catcccagcc 180

aggttcagtg gcagtgggtc tgggacagac ttcactctca ccatcagcag cctagagccc 240

gaagattttg cagtttatta ctgtcagcag cgtgacaact ggcccctcac tttcggcgga 300

gggaccaagg tggagatcaa a 321

<210> 271
<211> 5
<212> PRT
<213> Homo sapiens

<400> 271

Ser Tyr Ala Ile Ser
1               5

<210> 272
<211> 17
<212> PRT
<213> Homo sapiens

<400> 272

Gly Ile Ile Pro Ile Phe Gly Thr Ala Asn Tyr Ala Gln Lys Phe Gln
1               5                   10                  15

Gly

<210> 273
<211> 13
<212> PRT
<213> Homo sapiens

<400>  273

Val Gly Val Glu Tyr Gln Leu Leu Trp Tyr Phe Asp Tyr
1               5                   10


<210>  274
<211>  122
<212>  PRT
<213>  Homo sapiens

<400>  274

Gln Val Gln Leu Val Gln Ser Gly Ala Glu Val Lys Lys Pro Gly Ser
1               5                   10                  15


Ser Val Lys Val Ser Cys Lys Ala Ser Gly Gly Thr Phe Ser Ser Tyr
            20                  25                  30


Ala Ile Ser Trp Val Arg Gln Ala Pro Gly Gln Gly Leu Glu Trp Met
        35                  40                  45


Gly Gly Ile Ile Pro Ile Phe Gly Thr Ala Asn Tyr Ala Gln Lys Phe
    50                  55                  60


Gln Gly Arg Val Thr Ile Thr Ala Asp Glu Ser Thr Ser Thr Ala Tyr
65                  70                  75                  80


Met Glu Leu Ser Ser Leu Arg Ser Glu Asp Thr Ala Val Tyr Tyr Cys
                85                  90                  95


Ala Arg Val Gly Val Glu Tyr Gln Leu Leu Trp Tyr Phe Asp Tyr Trp
            100                 105                 110


Gly Gln Gly Thr Leu Val Thr Val Ser Ser
        115                 120


<210>  275
<211>  11
<212>  PRT
<213>  Homo sapiens

<400>  275

Arg Thr Ser Gln Thr Ile Ser Asn Tyr Leu Asn
1               5                   10


<210>  276
<211>  7
<212>  PRT
<213>  Homo sapiens

<400>  276

Ala Ala Ser Asn Leu Gln Ser
1               5

<210>  277
<211>  7
<212>  PRT
<213>  Homo sapiens

<400>  277

Gln Gln Ser Tyr Asn Ala Ser
1               5

<210>  278
<211>  105
<212>  PRT
<213>  Homo sapiens

<400>  278

Asp Ile Gln Met Thr Gln Ser Pro Ser Ser Leu Ser Ala Ser Val Gly
1               5                   10                  15

Asp Arg Val Thr Leu Thr Cys Arg Thr Ser Gln Thr Ile Ser Asn Tyr
            20                  25                  30

Leu Asn Trp Tyr Gln Gln Lys Pro Gly Lys Ala Pro Lys Leu Leu Val
        35                  40                  45

Tyr Ala Ala Ser Asn Leu Gln Ser Trp Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Asn Ala Ser Phe
                85                  90                  95

Gly Gly Gly Thr Lys Val Glu Phe Lys
            100                 105

<210>  279
<211>  366
<212>  DNA
<213>  Homo sapiens

<400>  279
caagtgcagc tggtacagtc tggggctgag gtgaagaagc ctgggtcctc ggtgaaggtc      60

tcctgcaagg cttctggagg caccttcagc agctatgcaa tcagctgggt gcgacaggcc     120

cctggacaag ggcttgagtg gatgggaggg atcatcccta tctttggtac agcaaactac     180

gcacagaagt tccagggcag agtcacgatt accgcggacg aatccacgag cacagcctac     240

atggagctga gcagcctgag atctgaggac acggccgtgt attactgtgc gagagtaggg 300

gttgagtacc agctgctatg gtactttgac tactggggcc agggaaccct agtcaccgtc 360

tcctca 366

<210> 280
<211> 315
<212> DNA
<213> Homo sapiens

<400> 280
gacatccaga tgacccagtc tccatcctcc ctgtctgcat ctgtaggaga cagagtcacc 60

ctcacttgcc ggacaagtca gaccattagc aactatttaa attggtatca gcagaaacca 120

gggaaagccc ctaagctcct ggtctatgct gcatccaatt tgcaaagttg ggtcccatca 180

aggttcagtg gcagtgggtc tgggacagat ttcactctca ccatcagcag tctgcagcct 240

gaagattttg caacttacta ctgtcaacag agttacaatg cctctttcgg cggagggacc 300

aaggtggagt tcaaa 315

<210> 281
<211> 5
<212> PRT
<213> Homo sapiens

<400> 281

Ser Tyr Ala Ile Ser
1               5

<210> 282
<211> 17
<212> PRT
<213> Homo sapiens

<400> 282

Gly Ile Ile Pro Ile Phe Gly Thr Ala Asn Tyr Ala Gln Lys Phe Gln
1               5                   10                  15

Gly

<210> 283
<211> 10
<212> PRT
<213> Homo sapiens

<400> 283

Ser Tyr Tyr Tyr Tyr Tyr Gly Met Asp Val
1               5                   10

<210>  284
<211>  119
<212>  PRT
<213>  Homo sapiens

<400>  284

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Gly Ile Ile Pro Ile Phe Gly Thr Ala Asn Tyr Ala Gln Lys Phe
    50                  55                  60

Gln Gly Arg Val Thr Ile Thr Ala Asp Glu Ser Thr Gly Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Tyr Tyr Tyr Tyr Tyr Gly Met Asp Val Trp Gly Gln Gly
            100                 105                 110

Thr Thr Val Thr Val Ser Ser
        115


<210>  285
<211>  16
<212>  PRT
<213>  Homo sapiens

<400>  285

Arg Ser Ser Gln Ser Leu Leu His Ser Asn Gly Tyr Asn Tyr Leu Asp
1               5                   10                  15


<210>  286
<211>  7
<212>  PRT
<213>  Homo sapiens

<400>  286

Leu Gly Ser Asn Arg Ala Ser
1               5


<210>  287

<211>  9
<212>  PRT
<213>  Homo sapiens

<400>  287

Met Gln Ala Val Asp Thr Pro Arg Thr
1               5


<210>  288
<211>  112
<212>  PRT
<213>  Homo sapiens

<400>  288

Asp Ile Gln Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Tyr Leu Asp Trp Tyr Leu Gln Lys Pro Gly Gln Ser
        35                  40                  45

Pro Gln Leu Leu Ile His Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ala
                85                  90                  95

Val Asp Thr Pro Arg Thr Phe Gly Gln Gly Thr Lys Val Asp Ile Lys
            100                 105                 110


<210>  289
<211>  357
<212>  DNA
<213>  Homo sapiens

<400>  289
caggtccagc tggtgcagtc tggggctgag gtgaagaagc ctgggtcctc ggtgaaggtc      60

tcctgcaagg cttctggagg caccttcagc agctatgcta tcagctgggt gcgacaggcc     120

cctggacaag ggcttgagtg gatgggaggg atcatcccta tctttggtac agcaaactac     180

gcacagaagt tccagggcag agtcacgatt accgcggacg aatccacggg cacagcctac     240

atggagctga gcagcctgag atctgaggac acggccgtgt attactgtgc gagaagttac     300

tactactact acggtatgga cgtctggggc caagggacca cggtcaccgt ctcctca        357

<210>  290
<211>  336
<212>  DNA
<213>  Homo sapiens

<400>  290
gacatccaga tgacccagtc tccactctcc ctgcccgtca cccctggaga gccggcctcc      60

atctcctgca ggtctagtca gagcctcctg catagtaatg gatacaacta tttggattgg     120

tacctgcaga agccagggca gtcaccacag ctcctgatcc atttgggttc taatcgggcc     180

tccggggtcc ctgacaggtt cagtggcagt ggatcaggca cagattttac actgaaaatt     240

agcagagtgg aggctgagga tgttggggtt tattactgca tgcaagctgt agatactcct     300

cggacgttcg gccaagggac caaggtggac atcaaa                               336


<210>  291
<211>  5
<212>  PRT
<213>  Homo sapiens

<400>  291

Gly Tyr Tyr Met His
1               5


<210>  292
<211>  17
<212>  PRT
<213>  Homo sapiens

<400>  292

Trp Ile Asn Pro Asn Ser Gly Asn Thr Gly Tyr Ala Gln Lys Phe Gln
1               5                   10                  15


Gly




<210>  293
<211>  11
<212>  PRT
<213>  Homo sapiens

<400>  293

Gly Gly Gln Gln Gln Leu Val Leu Asp Asp Tyr
1               5                   10


<210>  294
<211>  120
<212>  PRT
<213>  Homo sapiens

<400>  294

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Gln Gln Gln Leu Val Leu Asp Asp Tyr Trp Gly Gln
            100                 105                 110

Gly Thr Leu Val Thr Val Ser Ser
        115                 120

<210>  295
<211>  11
<212>  PRT
<213>  Homo sapiens

<400>  295

Arg Ala Ser Gln Ser Ile Gly Gly Trp Leu Ala
1               5                   10

<210>  296
<211>  7
<212>  PRT
<213>  Homo sapiens

<400>  296

Ala Ala Ser Ser Leu Gln Ser
1               5

<210>  297
<211>  9
<212>  PRT
<213>  Homo sapiens

<400>  297

Arg Gln Ser Tyr Ser Thr Pro Pro Thr

1               5

<210>  298
<211>  107
<212>  PRT
<213>  Homo sapiens

<400>  298

Asp Ile Gln Leu Thr Gln Ser Pro Ser Thr Leu Ser Ala Ser Val Gly
1               5                   10                  15

Asp Arg Val Thr Ile Thr Cys Arg Ala Ser Gln Ser Ile Gly Gly Trp
            20                  25                  30

Leu Ala Trp Tyr Gln His Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Arg Gln Ser Tyr Ser Thr Pro Pro
                85                  90                  95

Thr Phe Gly Gln Gly Thr Lys Val Glu Ile Lys
            100                 105

<210>  299
<211>  360
<212>  DNA
<213>  Homo sapiens

<400>  299
caggtgcagc tggtgcagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggatgg atcaacccta acagtggtaa cacaggctat     180

gcacagaagt tccagggcag agtcaccatg accaggaaca cctccataag cacagcctac     240

atggagctga gcagcctgag atctgaggac acggccgtgt attactgtgc gagaggcgga     300

cagcagcagc tggtactgga cgactactgg ggccagggaa ccctggtcac cgtctcctca     360

<210>  300
<211>  321
<212>  DNA
<213>  Homo sapiens

<400>  300

gacatccagt tgacccagtc tccttccacc ctgtcagcat ctgtaggcga cagagtcacc 60

atcacttgcc gggccagtca gtctattggt ggttggttgg cctggtatca gcacaaacca 120

gggaaagccc ccaagctcct gatctatgct gcatccagtt tgcaaagtgg ggtcccatca 180

aggttcagtg gcagtggatc tgggacagat ttcactctca ccatcagcag tctgcaacct 240

gaagattttg caacttacta ctgtcgacag agttacagta cccctccgac gttcggccaa 300

gggaccaagg tggaaatcaa a 321

<210> 301
<211> 5
<212> PRT
<213> Homo sapiens

<400> 301

Ser Tyr Ala Met His
1               5

<210> 302
<211> 17
<212> PRT
<213> Homo sapiens

<400> 302

Val Ile Ser Tyr Asp Gly Ser Asn Lys Tyr Tyr Ala Asp Ser Val Lys
1               5                   10                  15

Gly

<210> 303
<211> 13
<212> PRT
<213> Homo sapiens

<400> 303

Val Val Ala Ala Ala Asp Leu Thr Arg Tyr Phe Asp Tyr
1               5                   10

<210> 304
<211> 122
<212> PRT
<213> Homo sapiens

<400> 304

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Ala Met His Trp Val Arg Gln Ala Pro Gly Lys Gly Leu Glu Trp Val
        35                  40                  45

Ala Val Ile Ser Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Val Ala Ala Ala Asp Leu Thr Arg Tyr Phe Asp Tyr Trp
            100                 105                 110

Gly Gln Gly Thr Leu Val Thr Val Ser Ser
        115                 120

<210>  305
<211>  12
<212>  PRT
<213>  Homo sapiens

<400>  305

Arg Ala Ser Gln Ser Val Pro Lys Asn Tyr Leu Ala
1               5                   10

<210>  306
<211>  7
<212>  PRT
<213>  Homo sapiens

<400>  306

Thr Ala Ser Ser Arg Ala Pro
1               5

<210>  307
<211>  9
<212>  PRT
<213>  Homo sapiens

<400>  307

Gln Gln Tyr Gly Thr Ser Pro Asn Thr
1               5

<210>  308
<211>  108
<212>  PRT
<213>  Homo sapiens

<400>  308

Glu Ile Val Leu Thr Gln Ser Pro Gly Thr Val Ser Leu Ser Pro Gly
1               5                   10                  15

Glu Arg Ala Thr Leu Ser Cys Arg Ala Ser Gln Ser Val Pro Lys Asn
            20                  25                  30

Tyr Leu Ala Trp Phe Gln Gln Lys Pro Gly Gln Ala Pro Arg Leu Val
        35                  40                  45

Ile His Thr Ala Ser Ser Arg Ala Pro Gly Ile Pro Asp Arg Phe Thr
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Thr Ser Pro
                85                  90                  95

Asn Thr Phe Gly Gln Gly Thr Lys Leu Asp Ile Lys
            100                 105

<210>  309
<211>  366
<212>  DNA
<213>  Homo sapiens

<400>  309
caggtgcagc tggtggagtc tgggggaggc gtggtccagc ctgggaggtc cctgagactc      60

tcctgtgcag cctctggatt caccttcagt agctatgcta tgcactgggt ccgccaggct     120

ccaggcaagg ggctggagtg ggtggcagtt atatcatatg atggaagcaa taaatactac     180

gcagactccg tgaagggccg attcaccatc tccagagaca attccaagaa cacgctgtat     240

ctgcaaatga acagcctgag agctgaggac acggctgtgt attactgtgc gagagtcgta     300

gcagcagctg acctcactcg ctactttgac tactggggcc agggaaccct ggtcaccgtc     360

tcctca                                                                366

<210>  310
<211>  324
<212>  DNA
<213>  Homo sapiens

<400>  310
gaaattgtgt tgacgcagtc tccaggcacc gtgtctttgt ctccagggga aagagccacc      60

ctctcctgca gggccagtca gagtgttcct aagaactact tagcctggtt ccagcagaaa     120

cctggccagg ctcccaggct cgtcatccat actgcatcca gcagggcccc tggcatccca     180

gacaggttca ctggcagcgg gtctgggaca gacttcactc ttaccatcag cagactggag 240

cctgaagatt ttgcagtata ttactgtcag cagtatggca cctcaccaaa cacttttggc 300

caggggacca agctggacat caaa 324

<210> 311
<211> 5
<212> PRT
<213> Homo sapiens

<400> 311

Gly Tyr Tyr Met His
1               5

<210> 312
<211> 17
<212> PRT
<213> Homo sapiens

<400> 312

Trp Met Asn Pro Asn Ser Gly Asn Thr Gly Tyr Ala Gln Lys Phe Gln
1               5                   10                  15

Gly

<210> 313
<211> 11
<212> PRT
<213> Homo sapiens

<400> 313

Gly Gly Gln Gln Gln Leu Val Leu Asp Asp Tyr
1               5                   10

<210> 314
<211> 120
<212> PRT
<213> Homo sapiens

<400> 314

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Met Asn Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Gln Gln Gln Leu Val Leu Asp Asp Tyr Trp Gly Gln
            100                 105                 110

Gly Thr Leu Val Thr Val Ser Ser
        115                 120

<210>  315
<211>  16
<212>  PRT
<213>  Homo sapiens

<400>  315

Arg Ser Ser Gln Ser Leu Leu His Ser Asn Gly Tyr Asn Tyr Leu Asp
1               5                   10                  15

<210>  316
<211>  7
<212>  PRT
<213>  Homo sapiens

<400>  316

Leu Gly Ser Asn Arg Ala Ser
1               5

<210>  317
<211>  9
<212>  PRT
<213>  Homo sapiens

<400>  317

Met Gln Ala Leu Gln Thr Pro Arg Thr
1               5

<210>  318
<211>  112
<212>  PRT
<213>  Homo sapiens

<400>  318

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Leu His Ser
            20                  25                  30

Asn Gly Tyr Asn Tyr Leu Asp Trp Tyr Leu Gln Lys Pro Gly Gln Ser
        35                  40                  45

Pro Gln Leu Leu Ile Tyr Leu Gly Ser Asn Arg Ala Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Met Gln Ala
                85                  90                  95

Leu Gln Thr Pro Arg Thr Phe Gly Pro Gly Thr Lys Val Asp Ile Lys
            100                 105                 110

<210>  319
<211>  360
<212>  DNA
<213>  Homo sapiens

<400>  319
caagtgcagc tggtacagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatggggtgg atgaatccta acagtggcaa cacaggctat     180

gcacagaagt tccagggcag agtcaccatg accaggaaca cctccataag cacagcctac     240

atggagctga gcagcctgag atctgaggac acggccgtgt attactgtgc gagaggcgga     300

cagcagcagc tggtactgga cgactactgg ggccagggaa ccctggtcac cgtctcctca     360

<210>  320
<211>  336
<212>  DNA
<213>  Homo sapiens

<400>  320
gacatcgtga tgacccagtc tccactctcc ctgcccgtca cccctggaga gccggcctcc      60

atctcctgca ggtctagtca gagcctcctg catagtaatg gatacaacta tttggattgg     120

tacctgcaga agccagggca gtctccacag ctcctgatct atttgggttc taatcgggcc     180

tccggggtcc ctgacaggtt cagtggcagt ggatcaggca cagattttac actgaaaatc     240

agcagagtgg aggctgagga tgttggggtt tattactgca tgcaagctct acaaacccct     300

cggactttcg gccctgggac caaagtggat atcaaa                               336

<210>  321
<211>  5
<212>  PRT
<213>  Homo sapiens

<400>  321

Ser Tyr Gly Ile Ser
1               5


<210>  322
<211>  17
<212>  PRT
<213>  Homo sapiens

<400>  322

Trp Ile Ser Ala Tyr Asn Gly Asn Thr Asn Tyr Ala Gln Lys Leu Gln
1               5                   10                  15


Gly


<210>  323
<211>  12
<212>  PRT
<213>  Homo sapiens

<400>  323

Asp His Ser Ile Val Gly Ala Thr Thr Phe Asp Tyr
1               5                   10


<210>  324
<211>  121
<212>  PRT
<213>  Homo sapiens

<400>  324

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Ser Tyr
            20                  25                  30


Gly Ile Ser Trp Val Arg Gln Ala Pro Gly Gln Gly Leu Glu Trp Met
        35                  40                  45


Gly Trp Ile Ser Ala Tyr Asn Gly Asn Thr Asn Tyr Ala Gln Lys Leu
    50                  55                  60


Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Asp His Ser Ile Val Gly Ala Thr Thr Phe Asp Tyr Trp Gly
            100                 105                 110

Gln Gly Thr Leu Val Thr Val Ser Ser
        115                 120

<210>  325
<211>  11
<212>  PRT
<213>  Homo sapiens

<400>  325

Arg Ala Ser Gln Ser Ile Ser Ser Trp Leu Ala
1               5                   10

<210>  326
<211>  7
<212>  PRT
<213>  Homo sapiens

<400>  326

Asp Ala Ser Ser Leu Glu Ser
1               5

<210>  327
<211>  9
<212>  PRT
<213>  Homo sapiens

<400>  327

Gln Gln Tyr Asn Ser Tyr Pro Trp Thr
1               5

<210>  328
<211>  107
<212>  PRT
<213>  Homo sapiens

<400>  328

Asp Ile Val Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile

35                  40                  45

Tyr Asp Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ser Tyr Pro Trp
                85                  90                  95

Thr Phe Gly Gln Gly Thr Lys Val Glu Ile Lys
            100                 105

<210>  329
<211>  363
<212>  DNA
<213>  Homo sapiens

<400>  329
caggtccagc tggtgcagtc tggagctgag gtgaagaagc ctggggcctc agtgaaggtc      60

tcctgcaagg cttctggtta cacctttacc agctacggta tcagctgggt gcgacaggcc     120

cctggacaag ggcttgagtg gatgggatgg atcagcgctt acaatggtaa cacaaactat     180

gcacagaagc tccagggcag agtcaccatg accacagaca catccacgag cacagcctac     240

atggagctga ggagcctgag atctgacgac acggccgtgt attactgtgc gagagatcac     300

agtatagtgg gagctactac gtttgactac tggggccagg gaaccctggt caccgtctcc     360

tca                                                                   363

<210>  330
<211>  321
<212>  DNA
<213>  Homo sapiens

<400>  330
gacatcgtga tgacccagtc tccttccacc ctgtctgcat ctgtaggaga cagagtcacc      60

atcacttgcc gggccagtca gagtattagt agctggttgg cctggtatca gcagaaacca     120

gggaaagccc ctaagctcct gatctatgat gcctccagtt tggaaagtgg ggtcccatca     180

aggttcagcg gcagtggatc tgggacagaa ttcactctca ccatcagcag cctgcagcct     240

gatgattttg caacttatta ctgccaacag tacaatagtt atccgtggac gttcggccaa     300

gggaccaagg tggaaatcaa a                                               321

<210>  331
<211>  5
<212>  PRT
<213>  Homo sapiens

<400>  331

Ser Tyr Thr Met Asn
1               5


<210>  332
<211>  17
<212>  PRT
<213>  Homo sapiens

<400>  332

Ser Ile Ser Ser Ile Gly Thr Tyr Ile Tyr Tyr Ala Asp Ser Val Lys
1               5                   10                  15


Gly


<210>  333
<211>  13
<212>  PRT
<213>  Homo sapiens

<400>  333

Val Leu Leu Ser Gly Ser Tyr Tyr Gly Tyr Phe Asp Ser
1               5                   10


<210>  334
<211>  122
<212>  PRT
<213>  Homo sapiens

<400>  334

Gln Met Gln Leu Val Gln Ser Gly Gly Gly Leu Val Lys Pro Gly Gly
1               5                   10                  15


Ser Leu Arg Leu Ser Cys Ala Ala Ser Gly Phe Thr Phe Ser Ser Tyr
            20                  25                  30


Thr Met Asn Trp Val Arg Gln Ala Pro Gly Lys Gly Leu Glu Trp Val
        35                  40                  45


Ser Ser Ile Ser Ser Ile Gly Thr Tyr Ile Tyr Tyr Ala Asp Ser Val
    50                  55                  60


Lys Gly Arg Phe Thr Ile Ser Arg Asp Asn Ala Lys Asn Ser Leu Tyr
65                  70                  75                  80


Leu Gln Met Asn Ser Leu Arg Ala Glu Asp Thr Ala Val Tyr Tyr Cys
                85                  90                  95

Ala Arg Val Leu Leu Ser Gly Ser Tyr Tyr Gly Tyr Phe Asp Ser Trp
            100                 105                 110

Gly Gln Gly Thr Leu Val Thr Val Ser Ser
        115                 120

<210>  335
<211>  14
<212>  PRT
<213>  Homo sapiens

<400>  335

Thr Gly Ser Ser Ser Asn Ile Gly Ala Gly Tyr Asp Val His
1               5                   10

<210>  336
<211>  7
<212>  PRT
<213>  Homo sapiens

<400>  336

Gly Asn Ser Asn Arg Pro Ser
1               5

<210>  337
<211>  11
<212>  PRT
<213>  Homo sapiens

<400>  337

Gln Ser Tyr Asp Ser Ser Leu Ser Gly Tyr Val
1               5                   10

<210>  338
<211>  111
<212>  PRT
<213>  Homo sapiens

<400>  338

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Asp Val His Trp Tyr Gln Gln Leu Pro Gly Thr Ala Pro Lys Leu
        35                  40                  45

Phe Ile Tyr Gly Asn Ser Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Ser Ser
                85                  90                  95

Leu Ser Gly Tyr Val Phe Gly Thr Gly Thr Lys Val Thr Val Leu
            100                 105                 110

<210>  339
<211>  366
<212>  DNA
<213>  Homo sapiens

<400>  339
cagatgcagc tggtacagtc tgggggaggc ctggtcaagc ctggggggtc cctgagactc      60

tcctgtgcag cctctggatt caccttcagt agctatacca tgaactgggt ccgccaggct     120

ccagggaagg ggctggagtg ggtctcatcc attagtagta ttggtactta catatactac     180

gcagactcag tgaagggccg attcaccatc tccagagaca acgccaagaa ctcactgtat     240

ctgcaaatga acagcctgag agccgaggac acggctgtgt attactgtgc gagagtcctg     300

ttaagtggga gctactacgg ctactttgac tcctggggcc agggaaccct ggtcaccgtc     360

tcctca                                                                366

<210>  340
<211>  333
<212>  DNA
<213>  Homo sapiens

<400>  340
cagtctgtgc tgactcagcc accctcagtg tctggggccc cagggcagag ggtcaccatc      60

tcctgcactg ggagcagctc caacatcggg gcaggttatg atgtgcactg gtaccagcag     120

cttccaggaa cagcccccaa actcttcatc tatggtaaca gcaatcggcc ctcaggggtc     180

cctgaccgat tctctggctc caagtctggc acctcagcct ccctggccat cactgggctc     240

caggctgagg atgaggctga ttattactgc cagtcctatg acagcagcct gagtggttat     300

gtcttcggaa ctgggaccaa ggtcaccgtc cta                                  333

<210>  341
<211>  5
<212>  PRT
<213>  Homo sapiens

<400>  341

Gly Tyr Tyr Met His
1               5

<210>  342
<211>  17
<212>  PRT
<213>  Homo sapiens

<400>  342

Trp Ile Asn Pro Asn Ser Gly Asn Thr Gly Tyr Ala Gln Lys Phe Gln
1               5                   10                  15

Gly

<210>  343
<211>  12
<212>  PRT
<213>  Homo sapiens

<400>  343

Gly Arg Leu Glu Arg Gly Tyr Trp Tyr Phe Asp Leu
1               5                   10

<210>  344
<211>  121
<212>  PRT
<213>  Homo sapiens

<400>  344

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Arg Leu Glu Arg Gly Tyr Trp Tyr Phe Asp Leu Trp Gly
            100                 105                 110

Arg Gly Thr Leu Val Thr Val Ser Ser
        115                 120

<210>  345
<211>  13
<212>  PRT
<213>  Homo sapiens

<400>  345

Thr Arg Ser Ser Gly Ser Ile Thr Ser Asn Tyr Val Gln
1               5                   10

<210>  346
<211>  7
<212>  PRT
<213>  Homo sapiens

<400>  346

Glu Asp Lys Glu Arg Pro Ser
1               5

<210>  347
<211>  11
<212>  PRT
<213>  Homo sapiens

<400>  347

Gln Ser Tyr Gly Gly Thr Ser Gln Gly Val Leu
1               5                   10

<210>  348
<211>  112
<212>  PRT
<213>  Homo sapiens

<400>  348

Asn Phe Met Leu Thr Gln Pro His Ser Val Ser Glu Ser Pro Gly Arg
1               5                   10                  15

Thr Val Thr Ile Ser Cys Thr Arg Ser Ser Gly Ser Ile Thr Ser Asn
            20                  25                  30

Tyr Val Gln Trp Tyr Gln Gln Arg Pro Gly Ser Ala Pro Thr Ile Leu
        35                  40                  45

Ile Tyr Glu Asp Lys Glu Arg Pro Ser Glu Val Pro Asp Arg Phe Ser
    50                  55                  60

Gly Ser Ile Asp Ile Ser Ser Asn Ser Ala Ser Leu Thr Ile Ser Gly
65                  70                  75                  80

Leu Lys Thr Glu Asp Glu Ala Asp Tyr Tyr Cys Gln Ser Tyr Gly Gly
                85                  90                  95

Thr Ser Gln Gly Val Leu Phe Gly Gly Gly Thr Lys Val Thr Val Leu
            100                 105                 110

<210>  349
<211>  363
<212>  DNA
<213>  Homo sapiens

<400>  349
caggtccagc tggtgcagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggatgg atcaacccta acagtggtaa cacaggctat     180

gcacagaagt tccagggcag agtcaccatg accaggaaca cctccataag cacagcctac     240

atggagctga gcagcctgag atctgaggac acggccgtgt attactgtgc gagaggccgt     300

ctggaacgcg gatactggta cttcgatctc tggggccgtg gcaccctggt caccgtctcc     360

tca                                                                   363

<210>  350
<211>  336
<212>  DNA
<213>  Homo sapiens

<400>  350
aattttatgc tgactcagcc ccactctgtg tcggagtctc cggggaggac ggtaaccatc      60

tcctgcaccc gcagcagtgg cagcattacc agcaactatg tccagtggta ccagcagcgc     120

ccgggcagtg cccctaccat cctaatctat gaggataagg aaagaccctc tgaggtccct     180

gatcgcttct ctggctccat cgacatttcc tccaactctg cctccctcac catctctggc     240

ctgaagacgg aggacgaggc tgactactac tgtcagtctt atggtggcac cagtcaaggg     300

gtgttattcg gcggagggac caaggtgacc gtccta                               336

<210>  351
<211>  5
<212>  PRT
<213>  Homo sapiens

<400>  351

Gly Tyr Tyr Met His
1               5

<210>  352
<211>  17
<212>  PRT

<213>  Homo sapiens

<400>  352

Trp Ile Asn Pro Asn Ser Gly Gly Thr Asn Tyr Ala Gln Lys Phe Gln
1               5                   10                  15

Gly

<210>  353
<211>  11
<212>  PRT
<213>  Homo sapiens

<400>  353

Gly Gly Gln Gln Gln Leu Val Leu Asp Asp Tyr
1               5                   10

<210>  354
<211>  120
<212>  PRT
<213>  Homo sapiens

<400>  354

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Gly Thr Asn Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Gln Gln Gln Leu Val Leu Asp Asp Tyr Trp Gly Gln
            100                 105                 110

Gly Thr Leu Val Thr Val Ser Ser
        115                 120

<210>  355

<211>  11
<212>  PRT
<213>  Homo sapiens

<400>  355

Arg Ala Ser Gln Ser Ile Ser Ser Tyr Leu Asn
1               5                   10


<210>  356
<211>  7
<212>  PRT
<213>  Homo sapiens

<400>  356

Ala Ala Ser Ser Leu Gln Ser
1               5


<210>  357
<211>  9
<212>  PRT
<213>  Homo sapiens

<400>  357

Gln Gln Ser Tyr Ser Thr Pro Arg Thr
1               5


<210>  358
<211>  107
<212>  PRT
<213>  Homo sapiens

<400>  358

Asp Ile Val Met Thr Gln Ser Pro Ser Ser Leu Ser Ala Ser Val Gly
1               5                   10                  15


Asp Arg Val Thr Ile Thr Cys Arg Ala Ser Gln Ser Ile Ser Ser Tyr
            20                  25                  30


Leu Asn Trp Tyr Gln Gln Lys Pro Gly Lys Ala Pro Lys Leu Leu Ile
        35                  40                  45


Tyr Ala Ala Ser Ser Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Ser Tyr Ser Thr Pro Arg
                85                  90                  95

Thr Phe Gly Gln Gly Thr Lys Val Glu Ile Lys
            100                 105

<210>  359
<211>  360
<212>  DNA
<213>  Homo sapiens

<400>  359
caggtgcagc tggtgcaatc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggatgg atcaacccta acagtggtgg cacaaactat     180

gcacagaagt tccagggcag agtcaccatg accaggaaca cctccataag cacagcctac     240

atggagctga gcagcctgag atctgaggac acggccgtgt attactgtgc gagaggcgga     300

cagcagcagc tggtactgga cgactactgg ggccagggaa ccctggtcac cgtctcctca     360

<210>  360
<211>  321
<212>  DNA
<213>  Homo sapiens

<400>  360
gacatcgtga tgacccagtc tccatcctcc ctgtctgcat ctgtaggaga cagagtcacc      60

atcacttgcc gggcaagtca gagcattagc agctatttaa attggtatca gcagaaacca     120

gggaaagccc ctaagctcct gatctatgct gcatccagtt tgcaaagtgg ggtcccatca     180

aggttcagtg gcagtggatc tgggacagat ttcactctca ccatcagcag tctgcaacct     240

gaagattttg caacttacta ctgtcaacag agttacagta cccctcgaac gttcggccaa     300

gggaccaagg tggaaatcaa a                                               321

<210>  361
<211>  5
<212>  PRT
<213>  Homo sapiens

<400>  361

Gly Tyr Tyr Met His
1               5

<210>  362
<211>  17
<212>  PRT
<213>  Homo sapiens

<400>  362

Trp Ile Asn Pro Asn Ser Gly Asn Thr Gly Tyr Ala Gln Lys Phe Gln
1               5                   10                  15

Gly

<210>  363
<211>  11
<212>  PRT
<213>  Homo sapiens

<400>  363

Gly Gly Gln Gln Gln Leu Val Leu Asp Asp Tyr
1               5                   10

<210>  364
<211>  120
<212>  PRT
<213>  Homo sapiens

<400>  364

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Tyr Met His Trp Val Arg Gln Ala Pro Gly Gln Gly Leu Glu Trp Met
        35                  40                  45

Gly Trp Ile Asn Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Gly Gly Gln Gln Gln Leu Val Leu Asp Asp Tyr Trp Gly Gln
            100                 105                 110

Gly Thr Leu Val Thr Val Ser Ser
        115                 120

<210>  365
<211>  13
<212>  PRT
<213>  Homo sapiens

<400>  365

Thr Gly Ser Ser Gly Ser Ile Ala Ser Asn Tyr Val Gln

1               5                   10


<210>  366
<211>  7
<212>  PRT
<213>  Homo sapiens

<400>  366

Glu Asp Asn Gln Arg Pro Ser
1               5


<210>  367
<211>  10
<212>  PRT
<213>  Homo sapiens

<400>  367

Gln Ser Tyr Asp Ser Ser Asn Gln Arg Val
1               5                   10


<210>  368
<211>  111
<212>  PRT
<213>  Homo sapiens

<400>  368

Asn Phe Met Leu Thr Gln Pro His Ser Val Ser Glu Ser Pro Gly Lys
1               5                   10                  15


Thr Val Thr Ile Ser Cys Thr Gly Ser Ser Gly Ser Ile Ala Ser Asn
            20                  25                  30


Tyr Val Gln Trp Tyr Gln Gln Arg Pro Gly Ser Ala Pro Thr Thr Val
        35                  40                  45


Ile Tyr Glu Asp Asn Gln Arg Pro Ser Gly Val Pro Asp Arg Phe Ser
    50                  55                  60


Gly Ser Ile Asp Ser Ser Ser Asn Ser Ala Ser Leu Thr Ile Ser Gly
65                  70                  75                  80


Leu Lys Thr Glu Asp Glu Ala Asp Tyr Tyr Cys Gln Ser Tyr Asp Ser
                85                  90                  95


Ser Asn Gln Arg Val Phe Gly Gly Gly Thr Lys Leu Thr Val Leu
            100                 105                 110


<210>  369
<211>  360
<212>  DNA

<213>  Homo sapiens

<400>  369
caggttcagc tggtgcagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggatgg atcaacccta acagtggtaa cacaggctat     180

gcacagaaat tccagggcag agtcaccatg accaggaaca cctccataag cacagcctac     240

atggagctga gcagcctgag atctgaggac acggccgtgt attactgtgc gagaggcgga     300

cagcagcagc tggtactgga cgactactgg ggccagggaa ccctggtcac cgtctcctca     360


<210>  370
<211>  333
<212>  DNA
<213>  Homo sapiens

<400>  370
aattttatgc tgactcagcc ccactctgtg tcggagtctc cggggaagac ggtaaccatc      60

tcctgcaccg gcagcagtgg cagcattgcc agcaactatg tgcagtggta ccagcagcgc     120

ccgggcagtg cccccaccac tgtgatctat gaggataacc aaagaccctc tggggtccct     180

gatcggttct ctggctccat cgacagctcc tccaactctg cctccctcac catctctgga     240

ctgaagactg aggacgaggc tgactactac tgtcagtctt atgatagcag caatcagagg     300

gtgttcggcg gagggaccaa gctgaccgtc cta                                  333


<210>  371
<211>  431
<212>  PRT
<213>  Homo sapiens

<400>  371

Met Thr Ser Gln Arg Ser Pro Leu Ala Pro Leu Leu Leu Leu Ser Leu
1               5                   10                  15


His Gly Val Ala Ala Ser Leu Glu Val Ser Glu Ser Pro Gly Ser Ile
            20                  25                  30


Gln Val Ala Arg Gly Gln Pro Ala Val Leu Pro Cys Thr Phe Thr Thr
        35                  40                  45


Ser Ala Ala Leu Ile Asn Leu Asn Val Ile Trp Met Val Thr Pro Leu
    50                  55                  60


Ser Asn Ala Asn Gln Pro Glu Gln Val Ile Leu Tyr Gln Gly Gly Gln
65                  70                  75                  80


Met Phe Asp Gly Ala Pro Arg Phe His Gly Arg Val Gly Phe Thr Gly

                85                  90                  95

Thr Met Pro Ala Thr Asn Val Ser Ile Phe Ile Asn Asn Thr Gln Leu
            100                 105                 110

Ser Asp Thr Gly Thr Tyr Gln Cys Leu Val Asn Asn Leu Pro Asp Ile
        115                 120                 125

Gly Gly Arg Asn Ile Gly Val Thr Gly Leu Thr Val Leu Val Pro Pro
    130                 135                 140

Ser Ala Pro His Cys Gln Ile Gln Gly Ser Gln Asp Ile Gly Ser Asp
145                 150                 155                 160

Val Ile Leu Leu Cys Ser Ser Glu Glu Gly Ile Pro Arg Pro Thr Tyr
                165                 170                 175

Leu Trp Glu Lys Leu Asp Asn Thr Leu Lys Leu Pro Pro Thr Ala Thr
            180                 185                 190

Gln Asp Gln Val Gln Gly Thr Val Thr Ile Arg Asn Ile Ser Ala Leu
        195                 200                 205

Ser Ser Gly Leu Tyr Gln Cys Val Ala Ser Asn Ala Ile Gly Thr Ser
    210                 215                 220

Thr Cys Leu Leu Asp Leu Gln Val Ile Ser Pro Gln Pro Arg Asn Ile
225                 230                 235                 240

Gly Leu Ile Ala Gly Ala Ile Gly Thr Gly Ala Val Ile Ile Ile Phe
                245                 250                 255

Cys Ile Ala Leu Ile Leu Gly Ala Phe Phe Tyr Trp Arg Ser Lys Asn
            260                 265                 270

Lys Glu Glu Glu Glu Glu Glu Ile Pro Asn Glu Ile Arg Glu Asp Asp
        275                 280                 285

Leu Pro Pro Lys Cys Ser Ser Ala Lys Ala Phe His Thr Glu Ile Ser
    290                 295                 300

Ser Ser Asp Asn Asn Thr Leu Thr Ser Ser Asn Ala Tyr Asn Ser Arg
305                 310                 315                 320

Tyr Trp Ser Asn Asn Pro Lys Val His Arg Asn Thr Glu Ser Val Ser
                325                 330                 335

His Phe Ser Asp Leu Gly Gln Ser Phe Ser Phe His Ser Gly Asn Ala
            340                 345                 350

Asn Ile Pro Ser Ile Tyr Ala Asn Gly Thr His Leu Val Pro Gly Gln
        355                 360                 365

His Lys Thr Leu Val Val Thr Ala Asn Arg Gly Ser Ser Pro Gln Val
    370                 375                 380

Met Ser Arg Ser Asn Gly Ser Val Ser Arg Lys Pro Arg Pro Pro His
385                 390                 395                 400

Thr His Ser Tyr Thr Ile Ser His Ala Thr Leu Glu Arg Ile Gly Ala
                405                 410                 415

Val Pro Val Met Val Pro Ala Gln Ser Arg Ala Gly Ser Leu Val
            420                 425                 430

<210>  372
<211>  430
<212>  PRT
<213>  Homo sapiens

<400>  372

Met Ser Leu Val Glu Leu Leu Leu Trp Trp Asn Cys Phe Ser Arg Thr
1               5                   10                  15

Gly Val Ala Ala Ser Leu Glu Val Ser Glu Ser Pro Gly Ser Ile Gln
            20                  25                  30

Val Ala Arg Gly Gln Pro Ala Val Leu Pro Cys Thr Phe Thr Thr Ser
        35                  40                  45

Ala Ala Leu Ile Asn Leu Asn Val Ile Trp Met Val Thr Pro Leu Ser
    50                  55                  60

Asn Ala Asn Gln Pro Glu Gln Val Ile Leu Tyr Gln Gly Gly Gln Met
65                  70                  75                  80

Phe Asp Gly Ala Pro Arg Phe His Gly Arg Val Gly Phe Thr Gly Thr
                85                  90                  95

Met Pro Ala Thr Asn Val Ser Ile Phe Ile Asn Asn Thr Gln Leu Ser
            100                 105                 110

Asp Thr Gly Thr Tyr Gln Cys Leu Val Asn Asn Leu Pro Asp Ile Gly
        115                 120                 125

Gly Arg Asn Ile Gly Val Thr Gly Leu Thr Val Leu Val Pro Pro Ser
    130                 135                 140

Ala Pro His Cys Gln Ile Gln Gly Ser Gln Asp Ile Gly Ser Asp Val
145                 150                 155                 160

Ile Leu Leu Cys Ser Ser Glu Glu Gly Ile Pro Arg Pro Thr Tyr Leu
                165                 170                 175

Trp Glu Lys Leu Asp Asn Thr Leu Lys Leu Pro Pro Thr Ala Thr Gln
            180                 185                 190

Asp Gln Val Gln Gly Thr Val Thr Ile Arg Asn Ile Ser Ala Leu Ser
        195                 200                 205

Ser Gly Leu Tyr Gln Cys Val Ala Ser Asn Ala Ile Gly Thr Ser Thr
    210                 215                 220

Cys Leu Leu Asp Leu Gln Val Ile Ser Pro Gln Pro Arg Asn Ile Gly
225                 230                 235                 240

Leu Ile Ala Gly Ala Ile Gly Thr Gly Ala Val Ile Ile Ile Phe Cys
                245                 250                 255

Ile Ala Leu Ile Leu Gly Ala Phe Phe Tyr Trp Arg Ser Lys Asn Lys
            260                 265                 270

Glu Glu Glu Glu Glu Glu Ile Pro Asn Glu Ile Arg Glu Asp Asp Leu
        275                 280                 285

Pro Pro Lys Cys Ser Ser Ala Lys Ala Phe His Thr Glu Ile Ser Ser
    290                 295                 300

Ser Asp Asn Asn Thr Leu Thr Ser Ser Asn Ala Tyr Asn Ser Arg Tyr
305                 310                 315                 320

Trp Ser Asn Asn Pro Lys Val His Arg Asn Thr Glu Ser Val Ser His
                325                 330                 335

Phe Ser Asp Leu Gly Gln Ser Phe Ser Phe His Ser Gly Asn Ala Asn
            340                 345                 350

Ile Pro Ser Ile Tyr Ala Asn Gly Thr His Leu Val Pro Gly Gln His
        355                 360                 365

Lys Thr Leu Val Val Thr Ala Asn Arg Gly Ser Ser Pro Gln Val Met
    370                 375                 380

Ser Arg Ser Asn Gly Ser Val Ser Arg Lys Pro Arg Pro Pro His Thr
385                 390                 395                 400

His Ser Tyr Thr Ile Ser His Ala Thr Leu Glu Arg Ile Gly Ala Val
                405                 410                 415

Pro Val Met Val Pro Ala Gln Ser Arg Ala Gly Ser Leu Val
            420                 425                 430

<210>  373
<211>  406
<212>  PRT
<213>  Homo sapiens

<400>  373

Met Ser Leu Val Glu Leu Leu Leu Trp Trp Asn Cys Phe Ser Arg Thr
1               5                   10                  15

Gly Val Ala Ala Ser Leu Glu Val Ser Glu Ser Pro Gly Ser Ile Gln
            20                  25                  30

Val Ala Arg Gly Gln Pro Ala Val Leu Pro Cys Thr Phe Thr Thr Ser
        35                  40                  45

Ala Ala Leu Ile Asn Leu Asn Val Ile Trp Met Val Thr Pro Leu Ser
    50                  55                  60

Asn Ala Asn Gln Pro Glu Gln Val Ile Leu Tyr Gln Gly Gly Gln Met
65                  70                  75                  80

Phe Asp Gly Ala Pro Arg Phe His Gly Arg Val Gly Phe Thr Gly Thr
                85                  90                  95

Met Pro Ala Thr Asn Val Ser Ile Phe Ile Asn Asn Thr Gln Leu Ser
            100                 105                 110

Asp Thr Gly Thr Tyr Gln Cys Leu Val Asn Asn Leu Pro Asp Ile Gly
        115                 120                 125

Gly Arg Asn Ile Gly Val Thr Gly Leu Thr Val Leu Val Pro Pro Ser
    130                 135                 140

Ala Pro His Cys Gln Ile Gln Gly Ser Gln Asp Ile Gly Ser Asp Val
145                 150                 155                 160

Ile Leu Leu Cys Ser Ser Glu Glu Gly Ile Pro Arg Pro Thr Tyr Leu
                165                 170                 175

Trp Glu Lys Leu Asp Asn Thr Leu Lys Leu Pro Pro Thr Ala Thr Gln
        180                 185                 190

Asp Gln Val Gln Gly Thr Val Thr Ile Arg Asn Ile Ser Ala Leu Ser
    195                 200                 205

Ser Ala Gln Pro Arg Asn Ile Gly Leu Ile Ala Gly Ala Ile Gly Thr
    210                 215                 220

Gly Ala Val Ile Ile Ile Phe Cys Ile Ala Leu Ile Leu Gly Ala Phe
225                 230                 235                 240

Phe Tyr Trp Arg Ser Lys Asn Lys Glu Glu Glu Glu Glu Glu Ile Pro
                245                 250                 255

Asn Glu Ile Arg Glu Asp Asp Leu Pro Pro Lys Cys Ser Ser Ala Lys
            260                 265                 270

Ala Phe His Thr Glu Ile Ser Ser Ser Asp Asn Asn Thr Leu Thr Ser
        275                 280                 285

Ser Asn Ala Tyr Asn Ser Arg Tyr Trp Ser Asn Asn Pro Lys Val His
    290                 295                 300

Arg Asn Thr Glu Ser Val Ser His Phe Ser Asp Leu Gly Gln Ser Phe
305                 310                 315                 320

Ser Phe His Ser Gly Asn Ala Asn Ile Pro Ser Ile Tyr Ala Asn Gly
                325                 330                 335

Thr His Leu Val Pro Gly Gln His Lys Thr Leu Val Val Thr Ala Asn
            340                 345                 350

Arg Gly Ser Ser Pro Gln Val Met Ser Arg Ser Asn Gly Ser Val Ser
        355                 360                 365

Arg Lys Pro Arg Pro Pro His Thr His Ser Tyr Thr Ile Ser His Ala
    370                 375                 380

Thr Leu Glu Arg Ile Gly Ala Val Pro Val Met Val Pro Ala Gln Ser
385                 390                 395                 400

Arg Ala Gly Ser Leu Val
                405

<210>  374
<211>  219

<212>  PRT
<213>  Homo sapiens

<400>  374

Leu Glu Val Ser Glu Ser Pro Gly Ser Ile Gln Val Ala Arg Gly Gln
1               5                   10                  15

Pro Ala Val Leu Pro Cys Thr Phe Thr Thr Ser Ala Ala Leu Ile Asn
            20                  25                  30

Leu Asn Val Ile Trp Met Val Thr Pro Leu Ser Asn Ala Asn Gln Pro
        35                  40                  45

Glu Gln Val Ile Leu Tyr Gln Gly Gly Gln Met Phe Asp Gly Ala Pro
    50                  55                  60

Arg Phe His Gly Arg Val Gly Phe Thr Gly Thr Met Pro Ala Thr Asn
65                  70                  75                  80

Val Ser Ile Phe Ile Asn Asn Thr Gln Leu Ser Asp Thr Gly Thr Tyr
                85                  90                  95

Gln Cys Leu Val Asn Asn Leu Pro Asp Ile Gly Gly Arg Asn Ile Gly
            100                 105                 110

Val Thr Gly Leu Thr Val Leu Val Pro Pro Ser Ala Pro His Cys Gln
        115                 120                 125

Ile Gln Gly Ser Gln Asp Ile Gly Ser Asp Val Ile Leu Leu Cys Ser
    130                 135                 140

Ser Glu Glu Gly Ile Pro Arg Pro Thr Tyr Leu Trp Glu Lys Leu Asp
145                 150                 155                 160

Asn Thr Leu Lys Leu Pro Pro Thr Ala Thr Gln Asp Gln Val Gln Gly
                165                 170                 175

Thr Val Thr Ile Arg Asn Ile Ser Ala Leu Ser Ser Gly Leu Tyr Gln
            180                 185                 190

Cys Val Ala Ser Asn Ala Ile Gly Thr Ser Thr Cys Leu Leu Asp Leu
        195                 200                 205

Gln Val Ile Ser Pro Gln Pro Arg Asn Ile Gly
    210                 215

<210>  375
<211>  114

<212>  PRT
<213>  Homo sapiens

<400>  375

Leu Glu Val Ser Glu Ser Pro Gly Ser Ile Gln Val Ala Arg Gly Gln
1               5                   10                  15

Pro Ala Val Leu Pro Cys Thr Phe Thr Thr Ser Ala Ala Leu Ile Asn
            20                  25                  30

Leu Asn Val Ile Trp Met Val Thr Pro Leu Ser Asn Ala Asn Gln Pro
        35                  40                  45

Glu Gln Val Ile Leu Tyr Gln Gly Gly Gln Met Phe Asp Gly Ala Pro
    50                  55                  60

Arg Phe His Gly Arg Val Gly Phe Thr Gly Thr Met Pro Ala Thr Asn
65                  70                  75                  80

Val Ser Ile Phe Ile Asn Asn Thr Gln Leu Ser Asp Thr Gly Thr Tyr
                85                  90                  95

Gln Cys Leu Val Asn Asn Leu Pro Asp Ile Gly Gly Arg Asn Ile Gly
            100                 105                 110

Val Thr



<210>  376
<211>  91
<212>  PRT
<213>  Homo sapiens

<400>  376

Pro Ser Ala Pro His Cys Gln Ile Gln Gly Ser Gln Asp Ile Gly Ser
1               5                   10                  15

Asp Val Ile Leu Leu Cys Ser Ser Glu Glu Gly Ile Pro Arg Pro Thr
            20                  25                  30

Tyr Leu Trp Glu Lys Leu Asp Asn Thr Leu Lys Leu Pro Pro Thr Ala
        35                  40                  45

Thr Gln Asp Gln Val Gln Gly Thr Val Thr Ile Arg Asn Ile Ser Ala
    50                  55                  60

Leu Ser Ser Gly Leu Tyr Gln Cys Val Ala Ser Asn Ala Ile Gly Thr
65                  70                  75                  80

Ser Thr Cys Leu Leu Asp Leu Gln Val Ile Ser
                85                  90

<210>  377
<211>  431
<212>  PRT
<213>  Homo sapiens

<400>  377

Met Thr Ser Arg Arg Ser Pro Leu Ala Pro Leu Leu Leu Leu Ser Leu
1               5                   10                  15

His Gly Val Ala Ala Ser Leu Glu Val Ser Glu Ser Pro Gly Ser Ile
            20                  25                  30

Gln Val Ala Arg Gly Gln Thr Ala Val Leu Pro Cys Thr Phe Thr Thr
        35                  40                  45

Ser Ala Ala Leu Ile Asn Leu Asn Val Ile Trp Met Val Thr Pro Leu
    50                  55                  60

Ser Asn Ala Asn Gln Pro Glu Gln Val Ile Leu Tyr Gln Gly Gly Gln
65                  70                  75                  80

Met Phe Asp Gly Ala Pro Arg Phe His Gly Arg Val Gly Phe Thr Gly
                85                  90                  95

Thr Met Pro Ala Thr Asn Val Ser Val Phe Ile Asn Asn Thr Gln Leu
            100                 105                 110

Ser Asp Thr Gly Thr Tyr Gln Cys Leu Val Asn Asn Leu Pro Asp Ile
        115                 120                 125

Gly Gly Arg Asn Ile Gly Val Thr Gly Leu Thr Val Leu Val Pro Pro
    130                 135                 140

Ser Ala Pro His Cys Gln Ile Gln Gly Ser Gln Asp Ile Gly Ser Asp
145                 150                 155                 160

Val Ile Leu Leu Cys Ser Ser Glu Glu Gly Ile Pro Arg Pro Thr Tyr
                165                 170                 175

Leu Trp Glu Lys Leu Asp Asn Thr Leu Lys Leu Pro Pro Thr Ala Thr
            180                 185                 190

Gln Asp Gln Val Gln Gly Thr Val Thr Ile Arg Asn Ile Ser Thr Leu
        195                 200                 205

Thr Ser Gly Leu Tyr Gln Cys Val Ala Ser Asn Ala Ile Gly Thr Ser
    210                 215                 220

Thr Cys Leu Leu Asp Leu Gln Val Ile Ser Pro Gln Pro Arg Asn Ile
225                 230                 235                 240

Gly Leu Ile Ala Gly Ala Val Gly Thr Gly Ala Val Ile Ile Ile Phe
                245                 250                 255

Cys Ile Ala Leu Ile Leu Gly Ala Phe Phe Tyr Trp Arg Ser Lys Asn
            260                 265                 270

Lys Glu Glu Glu Glu Glu Glu Ile Pro Asn Glu Ile Arg Glu Asp Asp
        275                 280                 285

Leu Pro Pro Lys Cys Ser Ser Ala Lys Ala Phe His Thr Glu Ile Ser
    290                 295                 300

Ser Ser Asp Asn Asn Thr Leu Thr Ser Ser Asn Thr Tyr Asn Ser Arg
305                 310                 315                 320

Tyr Trp Ser Asn Asn Pro Lys Val His Arg Asn Thr Glu Ser Val Asn
                325                 330                 335

His Phe Ser Asp Leu Gly Gln Ser Phe Ser Leu Arg Ser Gly Asn Ala
            340                 345                 350

Ser Ile Pro Ser Ile Tyr Ala Asn Gly Ser His Leu Leu Pro Gly Gln
        355                 360                 365

His Lys Thr Leu Val Val Thr Ala Asn Arg Gly Ser Ser Pro Gln Val
    370                 375                 380

Met Ser Arg Ser Asn Gly Ser Val Ser Arg Lys Pro Arg Pro Pro His
385                 390                 395                 400

Ser His Ser Tyr Thr Ile Ser His Ala Thr Leu Glu Arg Ile Gly Ala
                405                 410                 415

Val Pro Val Met Val Pro Ala Gln Ser Arg Ala Gly Ser Leu Val
            420                 425                 430

<210>  378
<211>  428
<212>  PRT
<213>  Homo sapiens

<400>  378

Met Thr Arg Arg Arg Ser Ala Pro Ala Ser Trp Leu Leu Val Ser Leu
1               5                   10                  15

Leu Gly Val Ala Thr Ser Leu Glu Val Ser Glu Ser Pro Gly Ser Val
            20                  25                  30

Gln Val Ala Arg Gly Gln Thr Ala Val Leu Pro Cys Ala Phe Ser Thr
        35                  40                  45

Ser Ala Ala Leu Leu Asn Leu Asn Val Ile Trp Met Val Ile Pro Leu
    50                  55                  60

Ser Asn Ala Asn Gln Pro Glu Gln Val Ile Leu Tyr Gln Gly Gly Gln
65                  70                  75                  80

Met Phe Asp Gly Ala Leu Arg Phe His Gly Arg Val Gly Phe Thr Gly
                85                  90                  95

Thr Met Pro Ala Thr Asn Val Ser Ile Phe Ile Asn Asn Thr Gln Leu
            100                 105                 110

Ser Asp Thr Gly Thr Tyr Gln Cys Leu Val Asn Asn Leu Pro Asp Arg
        115                 120                 125

Gly Gly Arg Asn Ile Gly Val Thr Gly Leu Thr Val Leu Val Pro Pro
    130                 135                 140

Ser Ala Pro Gln Cys Gln Ile Gln Gly Ser Gln Asp Leu Gly Ser Asp
145                 150                 155                 160

Val Ile Leu Leu Cys Ser Ser Glu Glu Gly Ile Pro Arg Pro Thr Tyr
                165                 170                 175

Leu Trp Glu Lys Leu Asp Asn Thr Leu Lys Leu Pro Pro Thr Ala Thr
            180                 185                 190

Gln Asp Gln Val Gln Gly Thr Val Thr Ile Arg Asn Ile Ser Ala Leu
        195                 200                 205

Ser Ser Gly Leu Tyr Gln Cys Val Ala Ser Asn Ala Ile Gly Thr Ser
    210                 215                 220

Thr Cys Leu Leu Asp Leu Gln Val Ile Ser Pro Gln Pro Arg Ser Val
225                 230                 235                 240

Gly Val Ile Ala Gly Ala Val Gly Thr Gly Ala Val Leu Ile Val Ile
                245                 250                 255

Cys Leu Ala Leu Ile Ser Gly Ala Phe Phe Tyr Trp Arg Ser Lys Asn
        260                 265                 270

Lys Glu Glu Glu Glu Glu Glu Ile Pro Asn Glu Ile Arg Glu Asp Asp
    275                 280                 285

Leu Pro Pro Lys Cys Ser Ser Ala Lys Ala Phe His Thr Glu Ile Ser
    290                 295                 300

Ser Ser Glu Asn Asn Thr Leu Thr Ser Ser Asn Thr Tyr Asn Ser Arg
305                 310                 315                 320

Tyr Trp Asn Asn Asn Pro Lys Pro His Arg Asn Thr Glu Ser Phe Asn
                325                 330                 335

His Phe Ser Asp Leu Arg Gln Ser Phe Ser Gly Asn Ala Val Ile Pro
        340                 345                 350

Ser Ile Tyr Ala Asn Gly Asn His Leu Val Leu Gly Pro His Lys Thr
    355                 360                 365

Leu Val Val Thr Ala Asn Arg Gly Ser Ser Pro Gln Val Leu Pro Arg
    370                 375                 380

Asn Asn Gly Ser Val Ser Arg Lys Pro Trp Pro Gln His Thr His Ser
385                 390                 395                 400

Tyr Thr Val Ser Gln Met Thr Leu Glu Arg Ile Gly Ala Val Pro Val
                405                 410                 415

Met Val Pro Ala Gln Ser Arg Ala Gly Ser Leu Val
        420                 425

<210>  379
<211>  311
<212>  PRT
<213>  Homo sapiens

<400>  379

Met Gly Val Pro Thr Ala Leu Glu Ala Gly Ser Trp Arg Trp Gly Ser
1               5                   10                  15

Leu Leu Phe Ala Leu Phe Leu Ala Ala Ser Leu Gly Pro Val Ala Ala
            20                  25                  30

Phe Lys Val Ala Thr Pro Tyr Ser Leu Tyr Val Cys Pro Glu Gly Gln
        35                  40                  45

Asn Val Thr Leu Thr Cys Arg Leu Leu Gly Pro Val Asp Lys Gly His
    50                  55                  60

Asp Val Thr Phe Tyr Lys Thr Trp Tyr Arg Ser Ser Arg Gly Glu Val
65                  70                  75                  80

Gln Thr Cys Ser Glu Arg Arg Pro Ile Arg Asn Leu Thr Phe Gln Asp
                85                  90                  95

Leu His Leu His His Gly Gly His Gln Ala Ala Asn Thr Ser His Asp
            100                 105                 110

Leu Ala Gln Arg His Gly Leu Glu Ser Ala Ser Asp His His Gly Asn
        115                 120                 125

Phe Ser Ile Thr Met Arg Asn Leu Thr Leu Leu Asp Ser Gly Leu Tyr
    130                 135                 140

Cys Cys Leu Val Val Glu Ile Arg His His His Ser Glu His Arg Val
145                 150                 155                 160

His Gly Ala Met Glu Leu Gln Val Gln Thr Gly Lys Asp Ala Pro Ser
                165                 170                 175

Asn Cys Val Val Tyr Pro Ser Ser Ser Gln Asp Ser Glu Asn Ile Thr
            180                 185                 190

Ala Ala Ala Leu Ala Thr Gly Ala Cys Ile Val Gly Ile Leu Cys Leu
        195                 200                 205

Pro Leu Ile Leu Leu Leu Val Tyr Lys Gln Arg Gln Ala Ala Ser Asn
    210                 215                 220

Arg Arg Ala Gln Glu Leu Val Arg Met Asp Ser Asn Ile Gln Gly Ile
225                 230                 235                 240

Glu Asn Pro Gly Phe Glu Ala Ser Pro Pro Ala Gln Gly Ile Pro Glu
                245                 250                 255

Ala Lys Val Arg His Pro Leu Ser Tyr Val Ala Gln Arg Gln Pro Ser
            260                 265                 270

Glu Ser Gly Arg His Leu Leu Ser Glu Pro Ser Thr Pro Leu Ser Pro
        275                 280                 285

Pro Gly Pro Gly Asp Val Phe Phe Pro Ser Leu Asp Pro Val Pro Asp

290                 295                 300

Ser Pro Asn Phe Glu Val Ile
305                 310

<210>  380
<211>  162
<212>  PRT
<213>  Homo sapiens

<400>  380

Phe Lys Val Ala Thr Pro Tyr Ser Leu Tyr Val Cys Pro Glu Gly Gln
1               5                   10                  15

Asn Val Thr Leu Thr Cys Arg Leu Leu Gly Pro Val Asp Lys Gly His
            20                  25                  30

Asp Val Thr Phe Tyr Lys Thr Trp Tyr Arg Ser Ser Arg Gly Glu Val
        35                  40                  45

Gln Thr Cys Ser Glu Arg Arg Pro Ile Arg Asn Leu Thr Phe Gln Asp
    50                  55                  60

Leu His Leu His His Gly Gly His Gln Ala Ala Asn Thr Ser His Asp
65                  70                  75                  80

Leu Ala Gln Arg His Gly Leu Glu Ser Ala Ser Asp His His Gly Asn
                85                  90                  95

Phe Ser Ile Thr Met Arg Asn Leu Thr Leu Leu Asp Ser Gly Leu Tyr
            100                 105                 110

Cys Cys Leu Val Val Glu Ile Arg His His His Ser Glu His Arg Val
        115                 120                 125

His Gly Ala Met Glu Leu Gln Val Gln Thr Gly Lys Asp Ala Pro Ser
    130                 135                 140

Asn Cys Val Val Tyr Pro Ser Ser Ser Gln Asp Ser Glu Asn Ile Thr
145                 150                 155                 160

Ala Ala

<210>  381
<211>  136
<212>  PRT
<213>  Homo sapiens

<400>  381

Phe Lys Val Ala Thr Pro Tyr Ser Leu Tyr Val Cys Pro Glu Gly Gln
1               5                   10                  15

Asn Val Thr Leu Thr Cys Arg Leu Leu Gly Pro Val Asp Lys Gly His
            20                  25                  30

Asp Val Thr Phe Tyr Lys Thr Trp Tyr Arg Ser Ser Arg Gly Glu Val
        35                  40                  45

Gln Thr Cys Ser Glu Arg Arg Pro Ile Arg Asn Leu Thr Phe Gln Asp
    50                  55                  60

Leu His Leu His His Gly Gly His Gln Ala Ala Asn Thr Ser His Asp
65                  70                  75                  80

Leu Ala Gln Arg His Gly Leu Glu Ser Ala Ser Asp His His Gly Asn
                85                  90                  95

Phe Ser Ile Thr Met Arg Asn Leu Thr Leu Leu Asp Ser Gly Leu Tyr
            100                 105                 110

Cys Cys Leu Val Val Glu Ile Arg His His His Ser Glu His Arg Val
        115                 120                 125

His Gly Ala Met Glu Leu Gln Val
    130                 135


<210>  382
<211>  303
<212>  PRT
<213>  Homo sapiens

<400>  382

Met Gly Val Pro Thr Ala Pro Glu Ala Gly Cys Trp Arg Trp Gly Ser
1               5                   10                  15

Leu Leu Phe Ala Leu Phe Leu Ala Ala Ser Leu Gly Pro Val Ala Ala
            20                  25                  30

Phe Lys Val Ala Thr Leu Tyr Ser Leu Tyr Val Cys Pro Glu Gly Gln
        35                  40                  45

Asn Val Thr Leu Thr Cys Arg Phe Phe Gly Pro Val Asp Lys Gly His
    50                  55                  60

Asp Val Thr Phe Tyr Lys Thr Trp Tyr Arg Ser Ser Arg Gly Glu Val
65                  70                  75                  80

Gln Thr Cys Ser Glu Arg Arg Pro Ile Arg Asn Leu Thr Phe Gln Asp
                85                  90                  95

Leu His Leu His His Gly Gly His Gln Ala Ala Asn Thr Ser His Asp
            100                 105                 110

Leu Ala Gln Arg His Gly Leu Glu Ser Ala Ser Asp His His Gly Asn
        115                 120                 125

Phe Ser Ile Thr Met Arg Asn Leu Thr Leu Leu Asp Ser Gly Leu Tyr
    130                 135                 140

Cys Cys Leu Val Val Glu Ile Arg His His His Ser Glu His Arg Val
145                 150                 155                 160

His Gly Ala Met Glu Leu Gln Val Gln Thr Gly Lys Asp Ala Pro Ser
                165                 170                 175

Ser Cys Val Ala Tyr Pro Ser Ser Ser Gln Glu Ser Glu Asn Ile Thr
            180                 185                 190

Ala Ala Ala Leu Ala Thr Gly Ala Cys Ile Val Gly Ile Leu Cys Leu
        195                 200                 205

Pro Leu Ile Leu Leu Leu Val Tyr Lys Gln Arg Gln Ala Ala Ser Asn
    210                 215                 220

Arg Arg Asp Asn Thr Gln Gly Ile Glu Asn Pro Gly Phe Glu Ala Ser
225                 230                 235                 240

Ser Pro Ala Gln Gly Ile Leu Glu Ala Lys Val Arg His Pro Leu Ser
                245                 250                 255

Tyr Val Ala Gln Arg Gln Pro Ser Glu Ser Gly Arg His Leu Leu Ser
            260                 265                 270

Glu Pro Gly Thr Pro Leu Ser Pro Pro Gly Pro Gly Asp Val Phe Phe
        275                 280                 285

Pro Ser Leu Asp Pro Val Pro Asp Ser Pro Asn Phe Glu Val Ile
    290                 295                 300

<210>  383
<211>  308
<212>  PRT
<213>  Homo sapiens

<400>  383

Met Gly Val Pro Ala Val Pro Glu Ala Ser Ser Pro Arg Trp Gly Thr
1               5                   10                  15

Leu Leu Leu Ala Ile Phe Leu Ala Ala Ser Arg Gly Leu Val Ala Ala
            20                  25                  30

Phe Lys Val Thr Thr Pro Tyr Ser Leu Tyr Val Cys Pro Glu Gly Gln
        35                  40                  45

Asn Ala Thr Leu Thr Cys Arg Ile Leu Gly Pro Val Ser Lys Gly His
    50                  55                  60

Asp Val Thr Ile Tyr Lys Thr Trp Tyr Leu Ser Ser Arg Gly Glu Val
65                  70                  75                  80

Gln Met Cys Lys Glu His Arg Pro Ile Arg Asn Phe Thr Leu Gln His
                85                  90                  95

Leu Gln His His Gly Ser His Leu Lys Ala Asn Ala Ser His Asp Gln
            100                 105                 110

Pro Gln Lys His Gly Leu Glu Leu Ala Ser Asp His His Gly Asn Phe
        115                 120                 125

Ser Ile Thr Leu Arg Asn Val Thr Pro Arg Asp Ser Gly Leu Tyr Cys
    130                 135                 140

Cys Leu Val Ile Glu Leu Lys Asn His His Pro Glu Gln Arg Phe Tyr
145                 150                 155                 160

Gly Ser Met Glu Leu Gln Val Gln Ala Gly Lys Gly Ser Gly Ser Thr
                165                 170                 175

Cys Met Ala Ser Asn Glu Gln Asp Ser Asp Ser Ile Thr Ala Ala Ala
            180                 185                 190

Leu Ala Thr Gly Ala Cys Ile Val Gly Ile Leu Cys Leu Pro Leu Ile
        195                 200                 205

Leu Leu Leu Val Tyr Lys Gln Arg Gln Val Ala Ser His Arg Arg Ala
    210                 215                 220

Gln Glu Leu Val Arg Met Asp Ser Asn Thr Gln Gly Ile Glu Asn Pro
225                 230                 235                 240

Gly Phe Glu Thr Thr Pro Pro Phe Gln Gly Met Pro Glu Ala Lys Thr

245                 250                 255

Arg Pro Pro Leu Ser Tyr Val Ala Gln Arg Gln Pro Ser Glu Ser Gly
            260                 265                 270

Arg Tyr Leu Leu Ser Asp Pro Ser Thr Pro Leu Ser Pro Pro Gly Pro
        275                 280                 285

Gly Asp Val Phe Phe Pro Ser Leu Asp Pro Val Pro Asp Ser Pro Asn
    290                 295                 300

Ser Glu Ala Ile
305

<210>  384
<211>  19
<212>  RNA
<213>  Artificial

<220>
<223>  siRNA

<400>  384
caacauacca uccauuuau                                                    19

<210>  385
<211>  19
<212>  RNA
<213>  Artificial

<220>
<223>  siRNA

<400>  385
ggaacgaauu ggugcagua                                                    19

<210>  386
<211>  19
<212>  RNA
<213>  Artificial

<220>
<223>  siRNA

<400>  386
gaacaucagu gcccugucu                                                    19

<210>  387
<211>  19
<212>  RNA
<213>  Artificial

<220>
<223>  siRNA

<400> 387
caggaacauu ggacuaaua 19

## Description

The invention is based on the surprising finding of antibodies that bind to IGSF11 (VSIG3) can also inhibit the interaction between IGSF11 and IGSF11 receptors such as VSIR (VISTA), the inhibition of such interaction can sensitise tumour cells to anti-tumour immune responses. In particular, the invention provides products, compositions and methods for treating diseases using modulators of IGSF11, especially antigen binding proteins targeting IGSF11, including those being inhibitors of IGSF11-interaction with VSIR. Also provided are methods of sensitising cells involved with a proliferative disorder against the cytotoxic effect of cell-mediated immune responses, and/or to kill such cells and/or methods for treating proliferative diseases, using an IGSF11 inhibitor such as an IGSF11-binding antibody, as well as certain related aspects including detection, diagnostic and screening methods.

In the treatment of cancer there are a number of approaches by which therapies may lead to the elimination of tumour cells, including those that involve or exploit one or more components of the immune system, either directly or indirectly. One of the limitations associated with such therapies is that cancerous cells often exploit immune-checkpoints to evade a patient's immune system, such as by preventing immune-recognition or down-regulating a tumour-specific cytotoxic T cell (CTL) response, thereby generating resistance against an immune response (Rabinovich et al 2007, Annu Rev Immunol 25:267; Zitvogel et al 2006, Nat Rev Immunol 6:715). Under normal conditions, such immune-regulatory checkpoints are crucial for the maintenance of self-tolerance under physiological conditions but there is an increasing recognition of the important role that they can also play in cancer (Hanahan and Weinberg 2011, Cell; 144:646); cancerous cells can take over these mechanisms to evade and suppress the immune system in order to develop into a tumour (Drake et al 2006, Adv Immunol 90:51).

Current state of the art cancer therapies include blockade of those few immune-regulatory checkpoints presently known and for which their mechanism of action is understood. For example, blocking antibodies against surface-expressed immune-regulatory proteins, such as CTLA4 and PD-L1 (Chambers et al 2001, Annu Rev Immunol 19:565; Blank et al 2004, Cancer Res 64:1140), can boost anti-tumour immunity and have shown clinical success against many cancer types (Page et al 2014, Annu Rev Med 65:185). However, a large proportion of cancer patients does not respond to such checkpoint blockage therapy (Bu et al 2016, Trends Mol Med 22:448; Hugo et al 2016, Cell 165:35; Topalian et al 2012, New Engl J Med 366:2443), indicating that other immune-checkpoint pathways may be active. Indeed, synergistic cooperation between several immune-regulatory pathways maintains immune tolerance against tumours, which might explain why blocking only one immune-regulatory checkpoint node can still result in tumour escape (Woo et al 2012, Cancer Res 72:917; Berrien-Elliott et al 2013, Cancer Res 73:605). However, little is known about the molecular factors that are central to the mechanism of action of such immune-regulatory pathways. Indeed, successful cancer immunotherapy requires a systematic delineation of the entire immune-regulatory circuit - the 'immune modulatome' - expressed by tumours. Therefore, today, there is still an unmet need for identifying further molecular targets that may serve as immune-regulatory checkpoints and in particular an unmet need for means and methods to modulate, detect and otherwise utilise such possible checkpoint targets, such as in medicine, diagnosis and research.

V-set immunoregulatory receptor (VSIR), initially described and designated as "V-domain Ig suppressor of T cell activation" (VISTA) by Wang et al (2011; J Exp Med 208:777), is an immunoglobulin (Ig) superfamily ligand that negatively regulates T cell responses. VISTA is primarily expressed on hematopoietic cells, and VISTA expression is highly regulated on myeloid antigen-presenting cells (APCs) and T cells. Wang et al described that a soluble VISTA-Ig fusion protein or VISTA expression on APCs inhibited T cell proliferation and cytokine production in vitro, and that a VISTA-specific monoclonal antibody interfered with VISTA-induced suppression of T cell responses by VISTA-expressing APCs in vitro. These findings showed that VISTA had functional activities that were non-redundant with other Ig superfamily members, and Wang et al postulated further that VISTA might play a role in the development of autoimmunity and immune surveillance in cancer.

VISTA is since known as a broad-spectrum negative checkpoint regulator for cancer immunotherapy (Lines et al, 2014; Cancer Immunol Res 2:510). For example, initial studies described VISTA as a potent negative regulator of T-cell function that is expressed on hematopoietic cells. VISTA levels are heightened within the tumour microenvironment (TME), in which its blockade can enhance anti-tumour immune responses in mice. Results have established VISTA as a negative checkpoint regulator that suppresses T-cell activation, that induces Foxp3 expression, and is highly expressed within the tumour microenvironment, leading to the suggestion that VISTA blockade may offer an immunotherapeutic strategy for human cancer (Lines et al, 2014; Cancer Res 74:1924).

Indeed, VISTA blockade has been shown to impair the suppressive function and reduce the emergence of tumour-specific Foxp3+CD4+ regulatory T cells. Consequently, VISTA mAb administration as a monotherapy significantly suppressed the growth of both transplantable and inducible melanoma. Initial studies exploring a combinatorial regimen using VISTA blockade and a peptide-based cancer vaccine with TLR agonists as adjuvants suggested that VISTA blockade synergised with the vaccine to effectively impair the growth of established tumours. These studies thereby established a foundation for designing VISTA-targeted approaches either as a monotherapy or in combination with additional immune-targeted strategies for cancer immunotherapy (Le Mercier et al, 2014; Cancer Res 74:1933).

Subsequently, VISTA has been associated with acquired resistance to anti-PD-1 therapy in metastatic melanoma patients (Kakavand et al, 2017; Modern Pathol 89, doi:10.1038/modpathol.2017.89; published online 4-Aug-2017), and as a compensatory inhibitory pathway in prostate tumours after ipilimumab therapy (Gao et al, 2017; Nat Med 23:551). Furthermore, the immune-checkpoint protein VISTA has been described to critically regulate the IL-23/IL-17 inflammatory axis (Li et al, 2017; Sci Rep 7:1485).

WO2016/090347 describes V-set and immunoglobulin domain-containing protein 8 (VSIG8) as the receptor for VISTA, as well as the use of VSIG8 in the identification or synthesis of agonist or antagonist compounds, preferably antibodies, polypeptides and fusion proteins which agonise or antagonise the effects of VSIG8 and/or VISTA and/or the VSIG8/VISTA binding interaction. Such VSIG8 antagonists were postulated therein to be used to suppress VISTA's suppressive effects on T cell immunity, and more particularly used in the treatment of cancer, or infectious disease; and such agonist compounds were postulated therein to be used to potentiate or enhance VISTA's suppressive effects on T cell immunity and thereby suppress T cell immunity, such as in the treatment of autoimmunity, allergy or inflammatory conditions. Screening assays for identifying agonists and antagonist of and/or VISTA and/or the VSIG8/VISTA binding interaction compounds were also described in WO2016/090347.

Therefore, there is a need, from one or more of the above perspectives, for novel approaches to render cells involved with certain disorders (such as a tumour) more (or less) susceptible to the immune system, and in particular to circumvent tumour immune escape mechanisms. The present invention seeks to provide, in particular, novel therapeutic approaches and methods involving existing or novel compounds; for example, compounds and ABPs that sensitise such cells towards a cytotoxic response of the immune system or components thereof. Furthermore, the invention seeks to provide novel strategies to diagnose, prognose and/or monitor cell resistance to such an immune response or components, as wells as screening approaches for the identification of compounds that are useful in the treatment of certain disorders. Accordingly, it is an object of the present invention to provide alternative, improved, simpler, cheaper and/or integrated means or methods that address one or more of these or other problems. Such an object underlying the present invention is solved by the subject matter as disclosed or defined anywhere herein, for example by the subject matter of the attached claims.

Generally, and by way of brief description, the main aspects of the present invention can be described as follows:

In **a first aspect,** the invention relates to **an antigen binding protein (ABP)** which specifically binds to IGSF11 (VSIG3) protein (eg to the extra cellular domain (ECD) of IGSF11 protein) and, optionally, wherein the ABP is able to inhibit the binding of VSIR (VISTA) protein or a variant thereof to IGSF11 protein or a variant thereof.

In **a second aspect,** the invention relates to **an ABP which competes with** an ABP of a first aspect for binding to IGSF11 protein (eg to the ECD of IGSF11 protein). In a **related aspect,** the invention relates to **an ABP which binds to the same epitope** as an ABP of a first aspect.

In **another aspect,** the invention relates to **an antigen binding domain (ABD)** of an ABP of the invention.

In **a third aspect,** the invention relates to **a nucleic acid** encoding for an ABP or ABD of the invention or of components thereof, and in **related aspects,** the invention relates to **a nucleic acid construct (NAC)** comprising such a nucleic acid, and relates to **a host cell** comprising a nucleic acid or NAC of the invention.

In **a fourth aspect,** the invention relates to **a pharmaceutical composition** comprising an ABP, ABD, nucleic acid, NAC or host cell of the invention, or comprising a compound that is a modulator of the expression, function, activity and/or stability of immunoglobulin superfamily member 11 (IGSF11, or VSIG3), or of a variant of IGSF11, and a pharmaceutically acceptable carrier, stabiliser and/or excipient.

In **a fifth aspect,** the invention relates to **a method for the treatment** of certain diseases, disorders or conditions in a subject by administering a product to the subject, wherein the product is selected from the list consisting of an ABP, ABD, nucleic acid, NAC and host cell of the invention, or is a compound that is a modulator of the expression, function, activity and/or stability of immunoglobulin superfamily member 11 (IGSF11, or VSIG3), or of a variant of IGSF11. In **related aspects,** the invention relates to **a product for use in medicine,** and relates to the **use of a product for the manufacture of a medicament,** wherein the product is selected from the list consisting of an ABP, ABD, nucleic acid, NAC or host cell of the invention, or is a compound that is modulator of the expression, function, activity and/or stability of immunoglobulin superfamily member 11 (IGSF11, or VSIG3), or of a variant of IGSF11.

The invention also relates to various **methods to produce** a recombinant cell line or ABP of the invention, a hybridoma or host cell capable of producing an ABP of the present invention, as well as relating to various **determination** and/or **diagnostic methods** or **uses,** and to **kits** useful for such determination and/or diagnostic methods, as well as to various **methods for identifying and/or charactering** compounds, such as those suitable for use in medicine.

The figures show:

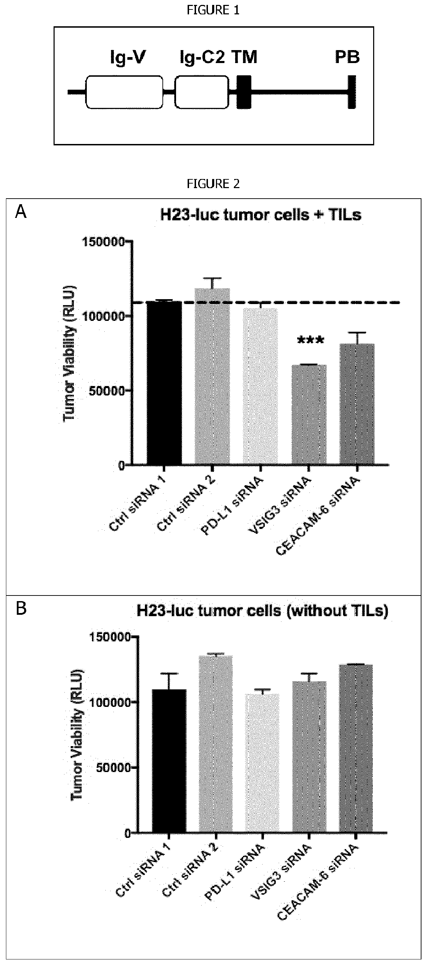

**Figure 1****:** A pictorial representation of the domain structure of IGSF11 (VSIG3). Ig-V/C2 = immunoglobulin V/C2-like; TM = transmembrane; and PB = PDZ-binding. Adapted from Jang et al (2016; Nat Neurosci 19:84).
**Figure 2****:** IGSF11 (VSIG3) knockdown sensitises lung tumour cells towards tumour infiltrating lymphocytes (TIL)-mediated cytotoxicity. H23 NSCLC cell lines stably transfected with a pEGFP-luc reporter plasmid were treated with the noted siRNAs and then co-cultured in the presence **(A)** or absence **(B)** of patient-derived TILs before the viability of tumour cells was measured for remaining luciferase activity.

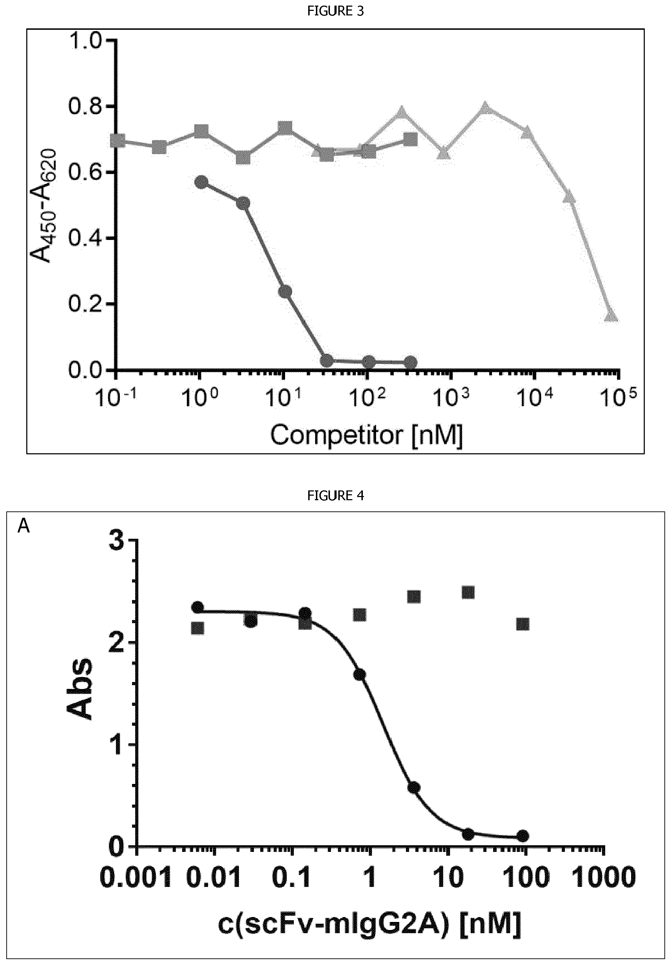

**Figure 3****:** ELISA assay to detect inhibition of binding between IGSF11 (VSIG3) and VSIR (VISTA). Purified and immobilised extracellular domain (ECD) of human IGSF11 (VSIG3) (HIS6-tagged) can interact with VSIR (VISTA), and this interaction can be blocked with a mouse anti-VISTA monoclonal antibody (circles) but not isotype control antibody (squares). Also, the soluble ECD of IGSF11 (triangles) can inhibit the interaction.

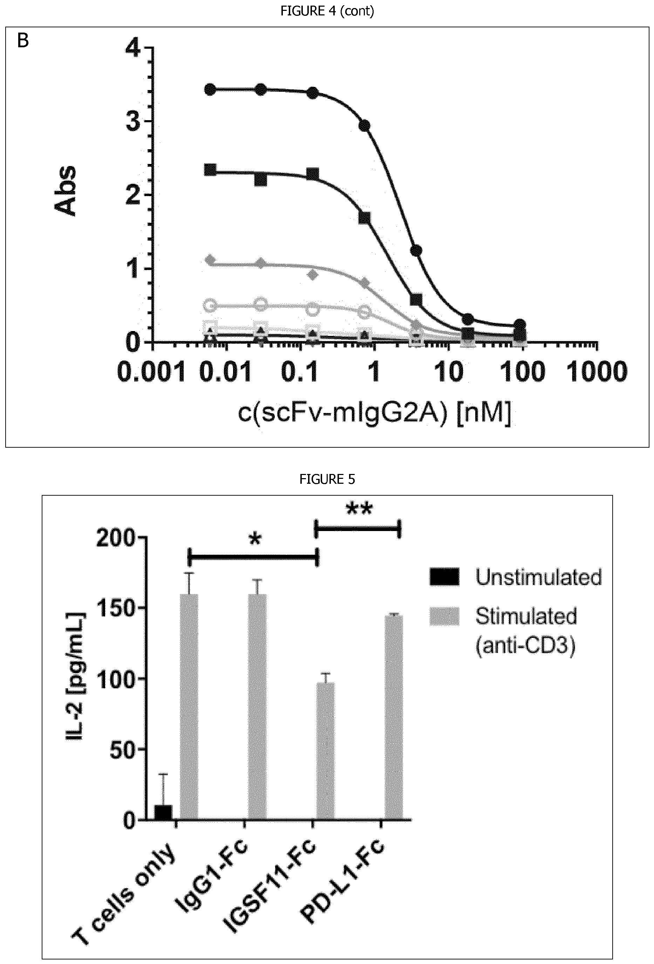

**Figure 4****:** scFv-Fc-format ABPs of the invention can inhibit the interaction between IGSF11 and VSIR (Fc-protein): **(A)** with an IC50 of less than about 1.5nM at a VSIR-Fc concentration of 6.6ug/mL (about 74nM dimer concentration). Circles = scFv-Fc-format of antibody A-015 of the invention, Squares = unrelated antibody isotype control; and **(B)** with IC50s from about 2.2nM to 1.6nM at VSIR-Fc concentrations from about 20ug/mL to 1.6ug/mL (about from 22nM to 8nM dimer concentration), respectively. VSIR-Fc concentrations: solid circles = 20ug/mL, solid squares = 6.66ug/mL, solid diamonds = 2.22ug/mL, open circles = 0.74ug/mL, open squares = 0.062ug/mL and open triangles = 0.027ug/mL, corresponding to VSIR-Fc dimer concentrations or about 222nM, 74nM, 24.7nM, 8.2nM, 2.7nM, 0.9nM and 0.3nM, respectively.
**Figure 5****:** IGSF11 (Fc protein) can inhibit the production of IL-2 by stimulated T cells. "*" = P<0.05, "**" = P<0.01.

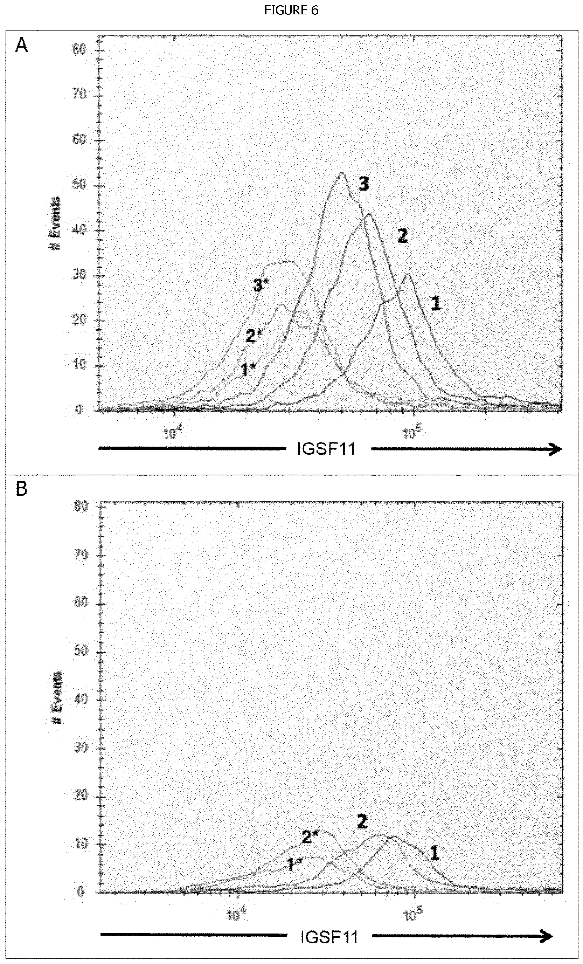

**Figure 6****:** IGSF11 can be detected on the surface of monocytes from PBMC of healthy volunteers. FACS histogram curves show detection of IGSF11 on monocytes of two volunteers (A and B) at the following concentrations of the anti-IGSF11 scFv-Fc format of antibody A-015: 1 = 150ug/mL, 2= 37.5ug/mL, 3= 9.38ug/mL. Those marked by "*" are histogram curves from mouse IgG2a isotype control used at the corresponding concentration.

The present invention, and particular non-limiting aspects and/or embodiments thereof, can be described in more detail as follows:

In **a first aspect,** and as may be further described, defined, claimed or otherwise disclosed herein, the invention relates to **an antigen binding protein (ABP)** which specifically binds to IGSF11 (VSIG3) protein (eg to the extra cellular domain (ECD) of IGSF11 protein) and, optionally, wherein the ABP and is able to inhibit (eg, inhibits) the interaction between VSIR (VISTA) protein or a variant thereof and IGSF11 protein or a variant thereof, for example, the ABP is optionally able to inhibit (eg, inhibits) the binding of IGSF11 protein or a variant thereof to VSIR (VISTA) protein or a variant thereof.

### Antigen binding proteins targeting IGSF11

An "antigen binding protein" ("ABP") as used herein means a protein that specifically binds to a target antigen, such as to one or more epitope(s) displayed by or present on a target antigen. The antigen of the ABPs of the invention is IGSF11 or an orthologue (or paralogue) or other variant thereof; and the ABP can, optionally bind to one or more extracellular domains of said IGSF11 or variant (such as the epitope(s) can be displayed by or present on one or more extracellular domains of said IGSF11 or variant). Typically, an antigen binding protein is an antibody (or a fragment thereof), however other forms of antigen binding protein are also envisioned by the invention. For example, the ABP may be another (non-antibody) receptor protein derived from small and robust non-immunoglobulin "scaffolds", such as those equipped with binding functions for example by using methods of combinatorial protein design (Gebauer & Skerra, 2009; Curr Opin Chem Biol, 13:245). Particular examples of such non-antibody ABPs include: Affibody molecules based on the Z domain of Protein A (Nygren, 2008; FEBS J 275:2668); Affilins based on gamma-B crystalline and/or ubiquitin (Ebersbach et al, 2007; J Mo Biol, 372:172); Affimers based on cystatin (Johnson et al, 2012; Anal Chem 84:6553); Affitins based on Sac7d from Sulfolobus acidcaldarius (Krehenbrink et al, 2008; J Mol Biol 383:1058); Alphabodies based on a triple helix coiled coil (Desmet et al, 2014; Nature Comms 5:5237); Anticalins based on lipocalins (Skerra, 2008; FEBS J 275:2677); Avimers based on A domains of various membrane receptors (Silverman et al, 2005; Nat Biotechnol 23:1556); DARPins based on an ankyrin repeat motif (Strumpp et al, 2008; Drug Discov Today, 13:695); Fynomers based on an SH3 domain of Fyn (Grabulovski et al, 2007; J Biol Chem 282:3196); Kunitz domain peptides based on Kunitz domains of various protease inhibitors (Nixon et al, Curr opin Drug Discov Devel, 9:261) and Centyrins and Monobodies based on a 10th type III domain of fibronectin (Diem et al., 2014; Protein Eng Des Sel 27:419 doi: 10.1093/protein/gzu016; Koide & Koide, 2007; Methods Mol Biol 352:95). In the context of an ABP of the present invention that specifically binds IGSF11, such an ABP is not a protein being V-set immunoregulatory receptor "VSIR" (or VISTA), or a IGSF11-binding fragment or other variant of VSIR (VISTA) (in particular, a variant having more than 70%, 80% or 90% sequence identify to the amino acid sequence of human VSIR (VISTA).

The term "epitope" includes any determinant capable of being bound by an antigen binding protein, such as an antibody. An epitope is a region of an antigen that is bound by an antigen binding protein that targets that antigen, and when the antigen is a protein, includes specific amino acids that bind the antigen binding protein (such as via an antigen binding domain of said protein). Epitope determinants can include chemically active surface groupings of molecules such as amino acids, sugar side chains, phosphoryl or sulfonyl groups, and can have specific three dimensional structural characteristics, and/or specific charge characteristics. Generally, antigen binding proteins specific for a particular target antigen will preferentially recognise an epitope on the target antigen in a complex mixture of proteins and/or macromolecules.

The term "extracellular domain" ("ECD" or "EC" domain) as used herein refers to the region or regions of the protein which are exposed to the extracellular space and which are typically responsible for ligand binding. Immunoglobulin (Ig) superfamily genes typically have an Immunoglobulin-like ECD, such as a Ig-like V-type domain.

An antigen binding protein is "specific" when it binds to one antigen (such as IGSF11; eg human IGSF, orthologues and other variants thereof) more preferentially (eg, more strongly or more extensively) than it binds to a second antigen. The term "specifically binds" (or "binds specifically" and the like) used herein in the context of an ABP means that said ABP will preferentially bind to the desired antigen (eg IGSF11, in particular an ECD of IGSF11) than to bind to other proteins (or other molecules), such as preferentially binding to such IGSF11 compared to one or more of other Immunoglobulin (Ig) superfamily genes. Therefore, preferably, the binding affinity of the ABP to the one antigen (e.g. IGSF11) is at least 2-fold, 5-fold, at least 10-fold, at least 20-fold, at least 50-fold, at least 100-fold, at least 200-fold, at least 500-fold, at least 1000-fold, at least 2000-fold, at least 5000-fold, at least 10000-fold, at least 10⁵-fold or even at least 10⁶-fold, most preferably at least 2-fold, compared to its affinity to the other targets (e.g. unrelated proteins such as mouse or human Fc domain, or streptavidin).

Immunoglobulin superfamily member 11 (IGSF11) - also known as Brain and testis-specific immunoglobulin superfamily protein (Bt-IGSF or BTIGSE), V-set and immunoglobulin domain-containing protein 3 (VSIG3) and coxsackie virus and adenovirus receptor-like 1 (CXADRL1) - was first described by Suzu et al (2002; Biochem Biophys Res Comm 296:1215) as "BT-IgSF", a novel gene from both human and mouse that encoded a new member of the immunoglobulin superfamily that was preferentially expressed in both brain and testis. (hence, BT-IgSF: brain- and testis-specific immunoglobulin superfamily), and that showed significant homology to coxsackie and adenovirus receptor (CAR) and endothelial cell-selective adhesion molecule (ESAM). Human Bt-IgSF protein (431 amino acids) was described to be 88% identical to the mouse protein (428 amino acids). The human gene and protein from such research was the subject of at least EP1321475 (eg SEQ ID NOs 1 and 2 thereof), describing a gene useful for diagnosis and treatment of aplasia of corpus callosum and aspermatogensis and use thereof. However, alternative variants of the same sequence were the subject of earlier patent applications based on the predicted protein sequence from large-scale cDNA-sequencing (eg, 422 amino acids, SEQ ID NO 667 of WO2001/54474; 431 amino acids, SEQ ID NO 22 of WO2003/027228) as well as in later analogous large-scale projects (eg 428 amino acids, SEQ ID NO 4,513 of US 2004/0005560). Katoa & Katoa (2003; Int J Onc 23:525) described two isoforms of the IGSF11 gene (differing in the first 17 amino acids), that the gene encoded adhesion molecules homologous to CXADR, FLJ22415 and ESAM, and was frequently up-regulated in intestinal-type gastric cancer; further suggesting that IGSF11 might be a target for early diagnosis (eg by antibodies) of intestinal-type gastric cancer as well as for drug delivery to cancer cells. Harada et al (2005; J Cell Physiol 204:919) described BT-IgSF as a novel immunoglobulin superfamily protein that mediated homophilic adhesion in a calcium-independent manner.

Suppression of IGSF11 (VSIG3) by siRNA retarded the growth of gastric cancer cells, suggesting that IGSF11 (VSIG3) is a good candidate for cancer immunotherapy using IGSF11 peptides as cancer antigen, in particular for cancers of the stomach, colon and liver (Watanabe et al, 2005; Cancer Sci 96:498; WO2003/104275 and SEQ ID NO 2 thereof, 431 amino acids). WO2004/022594 described the cloning of the human second isoform of IGSF11 (eg, SEQ ID NO 6 thereof, 430 amino acids; and designated such protein therein as "B7-H5" [note, this was a patent nomenclature, and not to be confused with the "B7-H5" used as a synonym for VSIR]) and production of soluble (secreted) forms of human and mouse such "B7-H5". In particular, Example 13 of WO2004/022594 described the stimulation of B cell proliferation but not T cell proliferation by such mouse "B7-H5", Examples 15 and 16 described modulation of B cells in vivo following administration of such murine "B7-H5-Fc" fusion protein, and further prophetic examples of WO2004/022594 postulated other immunologic effects of such "B7-H5" including in therapy.

An extensive functional ELISA binding screening assay revealed that IGSF11 (VSIG3) binds the (eg, IGSF11 interacts with) B7 family member V-set immunoregulatory receptor (VSIR) (which was initially described and designated as "V-domain Ig suppressor of T cell activation" (VISTA)) but did not interact with other known members of the B7 family (Wang et al, 2017; J Immunol 198 [1 Supplement] 154.1, poster published 2016). VSIR (VSIG3) was described therein to inhibit human T cell proliferation in the presence of T cell receptor signalling, and to significantly reduce cytokine and certain chemokine production by human T cells. Furthermore, anti-VISTA neutralisation antibodies attenuated the binding of IGSF11 (VSIG3) to VSIR (VISTA), as well as VSIR-induced T cell inhibition. Thus, Wang et al proposed that they had identified a novel B7 pathway able to inhibit human T cell proliferation and cytokine production, and that this IGSF11/VSIR (VSIG3/VISTA) co-inhibitory pathway may provide new strategies for the treatment of human cancers, autoimmune disorders, infection, and transplant rejection, and may help to design better vaccines.

The interaction between IGSF11 (VSIG3) and VSIR (VISTA) has subsequently been independently described using a high throughput screen for receptor parings (Yang et al, 2017; J Biotech http://dx.doi.org/10.1016/j.jbiotec.2017.08.023). Yang et al speculated that cancer cells utilise IGSF11 to supress the activation of T cells and escape the immune surveillance of immune cells, and further that IGSF11 may be a potential target for cancer immunotherapy due to it being a binding partner for VSIR (VISTA).

"Immunoglobulin superfamily member 11" - or "IGSF11" (or "VSIG3") - as a protein is, in the context of the invention, an immunoglobulin superfamily member and, typically, one that is capable of binding (eg binds) to VSIR (VISTA). Pertinent information on the human IGSF11 gene is found at Entrez Gene ID: 152404; HGNC ID:16669; Genome Coordinates for assembly GRCh38:CM000665.2: Chromosome 3: 118,900,557-119,146,068 reverse strand, and information on human IGSF11 protein is accessible on UniProt: Q5DX21 (eg, Entry version 115 of 25 Oct 2017). An IGSF11 protein in the context of the invention has, typically, the domain structure shown in **Figure 1****,** and preferably (eg as a human IGSF11 protein) comprises an amino acid sequence of one of its isoforms as shown in any of SEQ ID NOs: 371 to 373, more preferably SEQ ID NOs. 371 or 372. With reference to SEQ ID NO. 371, amino acids 1 to 22 represent an N-terminal signal peptide, amino acids 23 to 241 form the extra cellular domain (SEQ ID NO. 374), amino acids 242 to 262 form the helical transmembrane (TM) region and amino acids 263 to 431 form the cytoplasmic domain. As described in more detail elsewhere herein, the extracellular domain (ECD) of (human) IGSF11 forms two (sub)domains, with amino acids 23 to 136 (SEQ ID NO. 375) forming an Ig-like V-type domain, and amino acids 144 to 234 (SEQ ID NO. 376) forming an Ig-like C2-type domain.

"IgV domain" (or "Ig-like V domain") and "IgC domain" (or "Ig-like C domain") as used herein, refer broadly to Ig superfamily member domains. These domains correspond to structural units that have distinct folding patterns called Ig-like folds. Ig-like folds are comprised of a sandwich of two sheets of antiparallel beta strands, with a conserved disulphide bond between the two sheets in most, but not all, domains. IgC domains of Ig, TCR, and MHC molecules share the same types of sequence patterns and are called the C1 set domains within the Ig superfamily. Other IgC domains fall within the IgC2 set domain. IgV domains also share sequence patterns and are called V set domains.

The human IGSF11 gene is located at chromosomal position 3q13.32, and has orthologues (eg, is conserved) in many species such as in chimpanzee and other great apes, Rhesus, Cynomolgus and green monkeys, marmoset, dog, pig, cow, mouse etc. In particular, the amino acid sequence for the IGSF protein in Cynomolgus monkey (UniProt identifier G7NXN0, Entry version 14 of 25 Oct 2017; 97.0% identical to human) is as shown in SEQ ID NO. 377 and in mouse (UniProt identifier P0C673, Entry version 78 of 25 Oct 2017; 88.4% identical to human) is shown in SEQ ID NO. 378. The closest human paralogue to human IGSF11 is coxsackievirus and adenovirus receptor, CXAR (33.3% identity to human IGSF11). The term IGSF11 in some embodiments of the invention may also pertain to variants of the human IGSF11 protein having an amino acid sequence that is substantially identical to, or of at least 70%, 75% or 80%, preferably 85%, more preferably at least 90%, 95%, 96%, 97%, 98%, 99%, or 100% sequence identity (such as at least 90% or 95% sequence identity) to, the amino acid sequence shown in any of SEQ ID NOs. 371 to 373, as determined using, e.g., the "Blast 2 sequences" algorithm described by Tatusova & Madden 1999 (FEMS Microbiol Lett 174: 247-250), and which (preferably) retain biological activity identical or substantially identical to the respective reference IGSF11 (eg to bind to VSIR (VISTA) protein and/or to suppress T cell (or other immune cell) function/activity. Preferred variants of IGSF11 protein comprise sequence variants thereof due to sequence polymorphism between and within populations of the respective species, as well as mutations compared to the wild-type sequence of IGSF11 (eg SEQ ID NO. 371). A preferred variant of IGSF11 protein is an IGSF11 variant (compared to eg SEQ ID NO. 371) selected from the list consisting of: P39T (corresponding to variant dbSNP:rs2903250), E333D (corresponding to variant dbSNP:rs36052974) and S388N (corresponding to variant dbSNP:rs34908332). The term IGSF11 can mean, as applicable to the context (if not more specifically indicated), an IGSF11 protein (such as one described above) or an mRNA molecule encoding such an IGSF11 protein.

In certain of embodiments, an ABP of the invention that binds to the ECD of human IGSF11 protein is cross reactive to the ECD of an orthologous protein, such as cross reactive to the ECD of cynomolgus IGSF11 protein and/or to the ECD of mouse IGSF11 protein and/or to the ECD of rat IGSF11 protein.

The term "orthologue" as used herein means a variant that descends from the same ancestral gene but which is present in another organism due to a speciation event. Orthologues of IGSF11 are typically expected to retain the same function as (or have a similar function to) human IGSF11. Those orthologues of human IGSF11 include those of chimpanzee (431 amino acids; 99.3% identity), cow (437 amino acids; 91.1% identity), mouse (428 amino acids; 88.4% identity) and rat (428 amino acids; 88.9% identity). A particular orthologue of human IGSF11 is that of cynomolgus monkeys or of mouse. An example of a cynomolgus monkey orthologue of human IGSF11 is described above, and of a mouse orthologue of human IGSF11 is described above.

The term "paralogue" as used herein means a variant in the same organism that descends from the same ancestral gene by a duplication event. A paralogue of IGSF11 is typically expected to be an immunoglobulin superfamily protein, in particular one having at least 70%, 80% 85% or 90% sequence identity to the amino acid sequence of the IGSF11 (if any such a paralogue exists in humans).

The term "variant" as used herein in the context of a protein means any natural or non-natural version of such protein which comprises one or more amino acid mutations compared to the reference protein, but which shares significant amino acid sequence identity with the reference protein, e.g. at least 70% or 75% amino acid sequence identity, preferably at least 80% amino acid sequence identity, more preferably at least 90% amino acid sequence identity and most preferably at least 95%, 96%, 97%, 98% or 99% amino acid sequence identity. Preferably, the variant of the protein possesses and/or maintains at least one function/activity that is the same, essentially the same or similar as the reference protein. Variants of IGSF11 may include orthologues to and natural variants of human IGSF11, such as the natural variants P39T, E333D and S388N, and others variants such as Y267H, V374A and K395E. Variants of IGSF11 may also correspond to human IGSF11 with one or more amino acid residues inserted into, or deleted from the amino acid sequence, such as those variants of IGSF11 naturally found within a population or those made by genetic manipulation, such as to specifically engineer amino acid changes into one or more domains (such as extracellular domains) of the variant. Variants of IGSF11 include fusion proteins of IGSF11 (for example, a human IGSF11 fused to a heterologous polypeptide chain, such as Fc immunoglobulin domains or tags), and/or IGSF11 conjugated to another chemical moiety such as an effector group or a labelling group. A variant of IGSF11 can, in certain embodiments, comprise a fragment of IGSF11, for example a polypeptide that consists of one or more EC domains (or regions or (sub)domains thereof) of IGSF11 without one or other (or any other) EC, TM or intracellular domains of IGSF11. Preferred such variants of IGSF11 that are fragments include those that comprise an ECD of IGSF11 without any of the TM or intracellular domains of IGSF11; more preferably those that comprise the Ig-like V-type domain (SEQ ID NO. 375), or the Ig-like C2-type domain (SEQ ID NO. 376,) of IGSF11 without one or more (or all) of the other ECD, TM or intracellular domains of IGSF11. Variants of IGSF11 also include versions of human IGSF11 (or orthologues thereof) that have been modified to display only specific domains (such as extracellular), or not to display one or more other domains, and/or to display certain domains (e.g. ECDs) of human IGSF11 in combination with domains from paralogues and/or orthologues of human IGSF11, or from other immunoglobulin superfamily proteins. Methods describing the engineering of domain or amino acid variants of IGSF11 are known to the person of ordinary skill. In certain embodiments, the variant of IGSF11 is a functional variant thereof. A "functional variant" of IGSF11 (such as a functional fragment of an IGSF11 protein) is a variant of the protein of IGSF11 that provides, possesses and/or maintains one or more of the herein described functions/activities of the non-variant protein of IGSF11. For example, such functional variant may bind VSIR (VISTA) protein and/or to suppress T cell (or other immune cell) function/activity as IGSF11 protein, such as having the same, essentially the same or similar specificity and/or function as a receptor as IGSF11 protein. In other embodiments, such a functional variant may possess other activities than those possessed by the non-variant IGSF11 protein, as long as, preferably, it provides, possesses and/or maintains at least one function/activity that is the same, essentially the same or similar as IGSF11 protein. In more preferred embodiments, a functional variant of IGSF11 may act as an immune checkpoint inhibitor, such as by inhibiting one or more cell-based immune response(s) to a tumour or cancer cell that expresses such functional variant.

The term "identity" refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) are preferably addressed by a particular mathematical model or computer program (i.e., an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al., 1988, SIAM J. Applied Math. 48:1073.

In calculating percent identity, the sequences being compared are typically aligned in a way that gives the largest match between the sequences. One example of a computer program that can be used to determine percent identity is the GCG program package, which includes GAP (Devereux et al., 1984, Nucl. Acid Res. 12:387; Genetics Computer Group, University of Wisconsin, Madison, WI). The computer algorithm GAP is used to align the two polypeptides or polynucleotides for which the percent sequence identity is to be determined. The sequences are aligned for optimal matching of their respective amino acid or nucleotide (the "matched span", as determined by the algorithm). A gap opening penalty (which is calculated as 3x the average diagonal, wherein the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm.

A standard comparison matrix (see, Dayhoff et al., 1978, Atlas of Protein Sequence and Structure 5:345-352 for the PAM 250 comparison matrix; Henikoff et al., 1992, Proc. Natl. Acad. Sci. U.S.A. 89:10915-10919 for the BLOSUM 62 comparison matrix) may also be used by the algorithm.

Examples of parameters that can be employed in determining percent identity for polypeptides or nucleotide sequences using the GAP program are the following: (i) Algorithm: Needleman et al., 1970, J. Mol. Biol. 48:443-453; (ii) Comparison matrix: BLOSUM 62 from Henikoff et al., 1992, *supra*; (iii) Gap Penalty: 12 (but with no penalty for end gaps); (iv) Gap Length Penalty: 4; (v) Threshold of Similarity: 0.

A preferred method of determining similarity between a protein or nucleic acid and (or between) human IGSF11, a paralogue, orthologue or other variant thereof, is that provided by the Blast searches supported at Uniprot *supra* (e.g., http://www.uniprot.org/uniprot/Q5DX21); in particular for amino acid identity, those using the following parameters: Program: blastp; Matrix: blosum62; Threshold: 10; Filtered: false; Gapped: true; Maximum number of hits reported: 250.

Certain alignment schemes for aligning two amino acid sequences may result in matching of only a short region of the two sequences, and this small aligned region may have very high sequence identity even though there is no significant relationship between the two full-length sequences. Accordingly, the selected alignment method (GAP program) can be adjusted if so desired to result in an alignment that spans at least about 10, 15, 20, 25, 30, 35, 40, 45, 50 or other number of contiguous amino acids of the target polypeptide or region thereof.

In particular embodiments of the invention, the IGSF11 is human IGSF11, preferably a protein comprising an amino acid sequence selected from the group consisting of: SEQ ID NO: 371, SEQ ID NO: 342 and SEQ ID NO: 343 (in particular, SEQ ID NO. 371), or a protein having no more than two, four, six, eight, or ten, for example no more than one, two or three, such as no more than one, amino acid substitutions, insertions or deletions compared to these sequences.

In the context of variants of IGSF11, the invention includes those embodiments where a variant of IGSF11 is a protein comprising an amino acid sequence having at least 80%, 85%, 90%, 92% 95% or 97% sequence identity (in particular, at least 92% or 95% sequence identity) to the sequence of SEQ ID NO: 371.

In the context of other variants of IGSF11, the invention also includes those embodiments where a variant of IGSF11 is selected from the group consisting of an ortholog (or paralog) of IGSF11, and a functional fragment of an IGSF11 protein. In certain of such embodiments, such functional fragment of an IGSF11 protein binds to a VSIR (VISTA) protein, such as a human VSIR protein, or a variant of VSIR (such as one described elsewhere herein). In another of such embodiments, such functional fragment of an IGSF11 protein is capable of inhibiting (eg inhibits) a cell-based immune response to a cell, such as a cancer cell, that expresses such functional fragment. In particular of such embodiments, the variant of IGSF11 comprises an extracellular domain (ECD) of an IGSF11 protein, such as an ECD of a human IGSF11 protein and/or where the variant of VSIR protein is a functional fragment of a VSIR protein such as comprising an ECD of VSIR protein.

In particular embodiments of the present invention, an extracellular domain (ECD) of IGSF11 is an ECD of human IGSF11 protein, such as wherein the ECD of a human IGSF11 protein is an amino acid sequence selected from the group consisting of: SEQ ID NO: 374, SEQ ID NO: 375 and SEQ ID NO: 376 (preferably, SEQ ID NO: 375), or an amino acid sequence having at least 80%, 85%, 90%, 92%, 95% or 97% sequence identity (preferably, at least 92% or 95% sequence identity) to these sequences, and/or having no more than two, four, six or eight, for example no more than one, two or three, such as no more than one, amino acid substitutions, insertions or deletions compared to these sequences.

An ABP of the invention may, in particular embodiments, be able to inhibit (eg, inhibits) the interaction between VSIR (VISTA) protein or a variant thereof and IGSF11 protein or a variant thereof. For example, the ABP is optionally able to inhibit (eg, inhibits) the binding of IGSF11 protein or a variant thereof to VSIR (VISTA) protein or a variant thereof. Without being bound by theory, such an ABP of the invention may, by specifically binding to regions of the (eg ECD of) IGSF11 involved in the inter-molecular binding or complex formed between IGSF11 and VSIR, "block" the interaction between IGSF11 and VSIR. Accordingly, such an ABP of the invention can, in some embodiments, be a blocking ABP.

Information on V-set immunoregulatory receptor (VSIR), initially described and designated as "V-domain Ig suppressor of T cell activation" (VISTA) by Wang et al (2011), is described above, and "V-set immunoregulatory receptor" - or "VSIR" (or "VISTA") - as a protein is, in the context of the invention, an immunoglobulin superfamily member and, typically, one that is capable of binding (eg binds) to IGSF11 (VSIG3). Pertinent information on the human VSIR gene is found at Entrez Gene ID: 64115; HGNC ID: 30085; Genome Coordinates for assembly GRCh38:CM000672.2: Chromosome 10: 71,747,559-71,773,498 reverse strand, and information on human VSIR protein is accessible on UniProt: Q9H7M9 (eg, Entry version 129 of 25 Oct 2017) A VSIR protein in the context of the invention, typically, is approximately 50kDa, is a type I transmembrane protein and has one IgV domain. Preferably (eg as a human VSIR protein) comprises an amino acid sequence as shown in SEQ ID NOs: 379. With reference to SEQ ID NO. 379, amino acids 1 to 32 represent an N-terminal signal peptide, amino acids 33 to 194 form the extra cellular domain (SEQ ID NO. 380), amino acids 195 to 215 form the helical transmembrane (TM) region and amino acids 216 to 311 form the cytoplasmic domain. The extracellular domain (ECD) of (human) VSIR forms an Ig-like V-type domain between amino acids 33 to 168 (SEQ ID NO. 381).

The human VSIR gene is located at chromosomal position 10q22.1, and has orthologues (eg, is conserved) in many species such as in chimpanzee and other great apes, Rhesus, Cynomolgus and green monkeys, marmoset, dog, pig, cow, mouse etc. In particular, the amino acid sequence for the VSIR protein in mouse (UniProt identifier Q9D659, Entry version 122 of 20 Dec 2017; 77.2% identical to human) is shown in SEQ ID NO. 383. The closest human paralogue to human VSIR is programmed cell death 1 ligand 1, CD274 or PD-L1 (24.8% identity to human VSIR). The term VSIR in some embodiments of the invention may also pertain to variants of the human VSIR protein having an amino acid sequence that is substantially identical to, or of at least 70%, 75% or 80%, preferably 85%, more preferably at least 90%, 95%, 96%, 97% 98%, 99% or 100% sequence identity (such as at least 90% or 95% sequence identity) to, the amino acid sequence shown in SEQ ID NO. 379, as determined using, e.g., an algorithm described elsewhere herein, and which (preferably) retain biological activity identical or substantially identical to the respective reference VSIR (eg to bind to IGSF11 (VSIG3) protein and/or to suppress T cell (or other immune cel) function/activity). Preferred variants of VSIR protein comprise sequence variants thereof due to sequence polymorphism between and within populations of the respective species, as well as mutations compared to the wild-type sequence of IGSF11 (eg SEQ ID NO. 379). A preferred variant of VSIR protein is an VSIR variant (compared to eg SEQ ID NO. 379) D187E (corresponding to variant dbSNP:rs3747869). The term VSIR can mean, as applicable to the context (if not more specifically indicated), an VSIR protein (such as one described above) or an mRNA molecule encoding such an VSIR protein.

In particular embodiments of the invention, the VSIR is human VSIR, preferably a protein comprising an amino acid sequence of: SEQ ID NO: 379, or a protein having no more than two, four, six or eight, for example no more than one, two or three, such as no more than one, amino acid substitutions, insertions or deletions compared to this sequence

In the context of variants of VSIR, the invention includes those embodiments where a variant of VSIR is a protein comprising an amino acid sequence having at least 80%, 85%, 90%, 92% 95% or 97% sequence identity (in particular, at least 92% or 95% sequence identity) to the sequence of SEQ ID NO: 379.

In the context of other variants of VSIR, the invention also includes those embodiments where a variant of VSIR is selected from the group consisting of an ortholog (or paralog) of VSIR, and a functional fragment of a VSIR protein, preferably where such functional fragment of a VSIR protein binds to IGSF11 (VSIG3), such as a human IGSF11 protein, or a variant of IGSF11, and/or where such functional fragment of a VSIR protein functions as an immune checkpoint.. In particular of such embodiments, the variant of VSIR comprises an extracellular domain (ECD) of an VSIR protein, such as an ECD of a human VSIR protein and/or where the variant of IGSF11 protein is a functional fragment of a IGSF11 protein such as comprising an ECD of IGSF11 protein.

In particular embodiments of the present invention, an extracellular domain (ECD) of VSIR is an ECD of human VSIR protein, such as wherein the ECD of a human VSIR protein is an amino acid sequence selected from the group consisting of: SEQ ID NO: 380 and SEQ ID NO: 381 (preferably, SEQ ID NO: 381), or an amino acid sequence having at least 80%, 85%, 90%, 92%, 95% or 97% sequence identity (preferably, at least 92% or 95% sequence identity) to these sequences, and/or having no more than two, four, six or eight, for example no more than one, two or three, such as no more than one, amino acid substitutions, insertions or deletions compared to these sequences.

### Modulators of IGSF11 expression, function, activity and/or stability

In particular embodiments of such aspect, the ABP is a modulator of the expression, function, activity and/or stability of IGSF11, or the variant of IGSF11, such as wherein the ABP inhibits the expression, function, activity and/or stability of IGSF11, or the variant of IGSF11, or in particular where the ABP is an inhibitor of the function and/or activity of said IGSF11 or the variant of IGSF11. In one of such embodiments, an ABP of the invention is an inhibitor of the interaction between IGSF11, or the variant of IGSF11, to its endogenous receptor or ligand, such as to VSIR, or a variant of VSIR, in particular an ABP of the invention is capable of inhibiting (eg, inhibits or is an inhibitor of) the binding of VSIR (VISTA) protein or a variant thereof to IGSF11 protein or a variant thereof. Accordingly, ABPs of the invention can be "modulators".

The term "modulator" as used herein, refers to a molecule that changes, modifies or alters one or more characteristics, properties and/or abilities of another molecule or, for example, that changes, modifies or alters an immune response ("immunomodulators"), such as a cell-mediated immune response. For example, a modulator (eg, an inhibiting or antagonistic modulator) can impair or interfere with, or cause a decrease in the magnitude of, expression, function, activity and/or stability, such as a certain activity or function, of a molecule compared to the magnitude of such characteristic, property or ability observed in the absence of the modulator. In an alternative example, a modulator (eg, an activating or agonistic modulator) can enhance or promote, or cause an increase in the magnitude of, expression, function, activity and/or stability, such as a certain activity or function, of a molecule compared to the magnitude of such characteristic, property or ability observed in the absence of the modulator. Certain exemplary characteristics, properties or abilities of a molecule include, but are not limited to, expression, function, activity and/or stability, such as binding ability or affinity, enzymatic activity, and signal transduction; for example, any of the functions or activities of IGSF11 described herein.

Modulatory molecules (in particular, modulatory ABPs) can act as "inhibitors" ("antagonists") against a receptor such as IGSF11, such as by impairing (e.g. blocking) ligand engagement to such receptor, eg by inhibiting the interaction between IGSF11 and VSIR. Alternatively, modulatory molecules (in particular, modulatory ABPs) can act as "activators" ("agonists") for a receptor such as IGSF11, such as by enhancing or promoting function and/or activity of such receptor, for example by triggering the receptor's signalling pathway, such as by mimicking the binding of the endogenous ligand for such receptor.

As used herein, the terms "modulator of IGSF11 expression" and the like (such as an "inhibitor [or antagonist] of IGSF11 expression" and the like) shall relate to any molecule (eg any of the herein disclosed ABPs) which has an effect (such as an antagonistic activity) toward the expression of an IGSF11 protein, that is it alters (e.g. impairs, suppresses, reduces and/or lowers) the expression of an IGSF11 protein such as may be determined by measuring an amount (or change in an amount) of IGSF11 protein or IGSF11 mRNA. A modulator that is an activator or agonist will, typically have the corresponding but inverse effect (to that of an inhibitor or antagonist) on IGSF11 expression, eg that such a modulator enhances, promotes, increases and/or raises IGSF11 expression. The term "expression" means in this context the cellular process of transcribing a gene into an mRNA and the following translation of the mRNA into a protein. "Gene expression" therefore may thus refer only to the generation of mRNA, irrespectively from the fate of the so produced mRNA, or alternatively/additionally to the translation of the expressed mRNA into a protein. The term "protein expression" on the other hand may refer to the complete cellular process of synthesis of proteins. The terms "modulator of expression of a [orthologue][paralogue][variant] of IGSF11" and the like, shall have the corresponding meaning with respect to any such variant of IGSF11.

The terms "modulator of IGSF11 stability" and the like (such as an "inhibitor [or antagonist] of IGSF11 stability" and the like) shall refer to any molecule (eg any of the herein disclosed ABPs) which has an effect (such as a negative activity) towards the stability of an IGSF11 protein. The term, in context of the present disclosure, shall be understood in its broadest sense. Such modulators are included by the term, which, for example, interfere with and reduce the IGSF11 protein half-live or interfere with and disturb IGSF11 protein folding or protein presentation on the surface of the cell. In one preferred example, an inhibiting modulator of the invention, such as an ABP, may induce internalisation, and optionally degradation, of IGSF11 protein from the surface of the cell. A modulatory that is an activator or agonist will, typically have the corresponding but inverse effect (to that of an inhibitor or antagonist) on IGSF11 stability, eg that such a modulator enhances, promotes, increases and/or raises IGSF11 stability. The terms "modulator of stability of a [orthologue][paralogue][variant] of IGSF11" and the like, shall have the corresponding meaning with respect to any such variant of IGSF11.

The terms a "modulator of IGSF11 function [or activity]" and the like (such as an "inhibitor [or antagonist] of IGSF11 function [or activity]" and the like) shall refer to any molecule (eg any of the herein disclosed ABPs) that alters, such as impairs (e.g., induces a decrease or reduction in) the efficiency, effectiveness, amount or rate of one or more activities of IGSF11 (for example, by impairing the expression and/or stability of IGSF11 protein), such as one or more of those activities described herein, for example, the activity of IGSF11 as a modulator of T-cell activation and/or viability. In one embodiment, such a modulating ABP may impair binding of one or more of the endogenous binding partners of IGSF11 protein. For example, such a modulator may impair the interaction between IGSF11 protein and VSIR protein (eg, such a modulator may reduce, inhibit or block the binding between IGSF11 protein and VSIR protein. A modulator that is an activator or agonist will, typically have the corresponding but inverse effect (to that of an inhibitor or antagonist) on IGSF11 function and/or activity, eg that such a modulator enhances, promotes, increases and/or raises IGSF11 function and/or activity. For example, such a modulator may promote or increase the function or activity of IGSF11 receptor, for example by triggering the signalling pathway of IGSF11. The terms "modulator of function of a [orthologue][paralogue][variant] of IGSF11" and the like, shall have the corresponding meaning with respect to any such variant of IGSF11.

A particular embodiment of a modulator of IGSF11 is an "inhibitor of IGSF11" (or "IGSF11 inhibitor"), which meaning includes any moiety that inhibits IGSF11, which can mean inhibition of the expression (eg the amount), function, activity and/or stability of IGSF11, especially of mRNA and/or protein of IGSF11. In one particular of such embodiments, an inhibitor of IGSF11 can reduce the function (and/or activity) of IGSF11 protein, and in another of such embodiments, an inhibitor of IGSF11 can reduce the expression of IGSF11 mRNA and/or protein.

Such an IGSF11 inhibiting moiety can act directly, for example, by binding to IGSF11 and decreasing the amount or rate of one or more of the properties of IGSF11 such as its expression, function, activity and/or stability, in particular by inhibiting (eg blocking) its interaction with VSIR and/or to increase the sensitivity of a tumour cell expressing IGSF11 to a cell-mediated immune response. A IGSF11 inhibitor may also decrease the amount or rate of IGSF11 function or activity by impairing its expression or stability, for example, by binding to IGSF11 protein or mRNA and modifying it, such as by removal or addition of a moiety, or altering its three-dimensional conformation; and by binding to IGSF11 protein or mRNA and reducing its stability or conformational integrity. A IGSF11 inhibitor may, alternatively, act indirectly, for example, by binding to a regulatory molecule or gene region to modulate such regulatory protein or gene region function and hence consequentially affect a decrease in the amount or rate of IGSF11 expression (eg amount), function/activity and/or stability, in particular by impairing one or more activity of IGSF11 protein or mRNA (such as by changing the amount or rate of expression and/or stability of IGSF11 protein or mRNA). Thus, an IGSF11 inhibitor can act by any mechanism(s) that impair, such as result in a decrease in, the amount or rate of IGSF11 expression (eg amount), function/activity and/or stability. Non-limiting examples of IGSF11 inhibitors that act directly on IGSF11 include: (i) siRNA or shRNA molecules that bind to and reduce expression of IGSF11 mRNA; and (ii) ABPs that bind to (eg an EC domain) of IGSF11 protein and reduce the ability of IGSF11 protein to interact with (eg bind to) VSIR protein. Non-limiting examples of IGSF11 inhibitors that act indirectly on IGSF11 include siRNA or shRNA molecules that bind to and reduce expression of mRNA or a gene that enhances the expression or activity of IGSF11, consequential reducing the amount (and hence activity) of IGSF11 protein.

General and specific examples of IGSF11 inhibitors (including those that are ABPs of the present invention) are described elsewhere herein, including those as may be characterised by the applicable functional and/or structural features set out herein.

Accordingly, in particular embodiments of the present invention, an ABP of the invention is one that is capable of specifically binding to (eg which specifically binds to) the ECD of IGSF11 (VSIG3), as well as being capable of inhibiting (eg reducing or blocking) the interaction between IGSF11 (VSIG3) protein (or a variant thereof, such as one described above) and VSIR (VISTA) protein (or a variant thereof, such as one described above). In particular, such an ABP is able to inhibit (eg inhibits) the binding of VSIR (VISTA) protein (or a variant thereof, such as one described above) to IGSF11 (VSIG3) protein (or a variant thereof, such as one described above).

Methodologies to determine the interaction (eg binding) between IGSF11 (VSIG3) and VSIR (VISTA) protein (or between variants thereof) are known to the person of ordinary skill, and include ELISA assays (such as described in the examples below), and technologies such as *inter alia*: flow cytometry, surface plasmon resonance, surface acoustic waves and microscale thermophoresis. Such determination methodologies can be used (or adapted) to not only detect the presence of such interaction/binding, but also to measure (eg quantitatively) the degree of binding between the interacting partners IGSF11 and VSIR proteins (or variants thereof). Such (quantitative) measurement of interaction (binding) may be determined or measured in the presence of a competing (eg inhibiting) ABP of the invention, and hence the potential of an ABP of the present invention to inhibit (eg block) such interaction can be measured, and eg reported as an IC50.

Such IC50 values may be determined, such as using ELISA methodology (eg, using an assay correspond to, or substantially as, the ELISA described in **Example 5),** in the presence of a suitable concentration of VSIR protein (or variant thereof) in solution and with surface-bound IGSF11. Suitable concentrations of VSIR protein (or variant thereof) include: about 100pM to about 100uM VSIR protein (or variant thereof), for example about 0.75ug/mL to about 20ug/mL of Fc-VSIR fusion (eg, as described in **Example 5),** which corresponds to about 8.2nM to about 222nM dimer concentration of Fc-VSIR. Preferred suitable concentrations of VSIR protein (or variant thereof) include between about 20nM to about 100nM dimer concentration of Fc-VSIR (eg, as described in **Example 5),** such as about 75nM of such Fc-VSIR.

The IC50 of an (inhibitor/antagonist) modulator (eg, an ABP of the invention) can be determined by examining the effect of increasing concentrations of the inhibitor/antagonist modulator on the function and/or activity being investigated as the biological response (for example, an inhibition of binding of the IGSF (or variant thereof) to the VSIR (or variant thereof), or that results in and/or is measured by enhancement of a cell-mediated immune response and/or an increase in immune cell activity and/or survival, such as may be determined using methodologies correspond to, or substantially as, the those described in **Examples 7 and/or 8),** from a maximum such response. Responses are then normalized to the maximum and plotted against the log concentration of inhibitor/antagonist modulator in order to construct a dose-response curve, from which the concentration can be determined that gives 50% inhibition of the maximum biological response.

In certain of such embodiments of the invention, the ABP of the invention (eg one that binds to [one or more epitope(s) displayed by] an extracellular domain(s) of IGSF11, or a paralogue, orthologue or other variant thereof) is capable of inhibiting (eg will inhibit) the binding of VSIR protein or a variant thereof to IGSF11 protein or a variant thereof with an IC50 of 100nM, 50nM, or preferably 20nM or less, such as 15nM or less, 10nM or less, 5nM or less, 2nM or less, 1nM or less, 500pM or less, 250pM or less, or 100pM or less. In particular of such embodiments, an ABP of the invention is capable of inhibiting (eg will inhibit) the binding of VSIR protein or a variant thereof to IGSF11 protein or a variant thereof with an IC50 of 10nM or less, such as 5nM or less and preferably 2nM or less.

In particular of those embodiments where the ABP of the invention inhibits the interaction (eg the binding) between VSIR and IGSF11 proteins (or variants thereof), the VSIR protein is human VSIR protein and/or the IGSF11 protein is human IGSF11 protein. Preferably (such as in a binding assay to determine the IC50 of such ABP) the VSIR protein is human VSIR protein and the IGSF11 protein is human IGSF11 protein, and in other particular embodiments, the variant of the VSIR protein comprises an ECD of VSIR protein, preferably of a human VSIR protein, and/or the variant of the IGSF11 protein comprises an ECD of IGSF11 protein, preferably of a human IGSF11 protein, such as wherein the variant of the VSIR protein comprises an ECD of human VSIR protein, and the variant of the IGSF11 protein comprises an ECD of human IGSF11 protein. In particular of such embodiments, an ABP of the invention is capable of inhibiting (eg inhibits) the interaction between: (i) an IGSF11 protein variant that is the ECD of human IGSF protein (optionally his tagged for purification), such as described in Example 5; and (ii) a VSIR protein variant that is human VSIR-Fc (human IgG1), such as obtainable from R&D Systems (Cat#7126-B7), in particular where such inhibition of the interaction can be detected in an ELISA assay using such proteins, such as an ELISA assay corresponding to, or substantially as, the ELISA described in **Example 5).**

In other embodiments, a modulator of the invention (eg, an ABP that binds to IGSF11) that is an *inhibitor or antagonist* may instead or also:
- inhibit, impair, reduce or reverse IGSF11-mediated inhibition of a cell-mediated immune response (eg in an in-vitro assay or in a subject, such as one in need thereof); and/or
- inhibit, impair, reduce or reverse IGSF11-mediated inhibition of humoral immunity (eg in an in-vitro assay or in a subject, such as one in need thereof).

The term "cell-mediated immune response", as used herein, may include, but is not limited to, a response in a host organism involving, utilising, and/or promoting any one or combinations of T cell maturation, proliferation, activation, migration, infiltration and/or differentiation, and/or the activation/modulation/migration/infiltration of a macrophage, a natural killer cell, a T lymphocyte (or T cell), a helper T lymphocyte, a memory T lymphocyte, a suppressor T lymphocyte, a regulator T lymphocyte, and/or a cytotoxic T lymphocyte (CTL), and/or the production, release, and/or effect of one or more cell-secretable or cell-secreted factor such as a cytokine or autocoid (in particular a pro-inflammatory cytokine), and/or one or more components of any of such processes (such as a cytokine or autocoid, particular a pro-inflammatory cytokine). The term "cell-mediated immune response," as used herein, may include a cellular response involving a genetically engineered, *in-vitro* cultured, autologous, heterologous, modified, and/or transferred T lymphocyte, or it may include a cell-secretable or cell-secreted factor (such as a cytokine or autocoid, in particular a pro-inflammatory cytokine) produced by genetic engineering. A cell-mediated immune response is preferably not a humoral immune response, such as an immune response involving the release of antibodies. In certain embodiments, in particular when the proliferative disorder is a cancer or tumour, the cell-mediated immune response is an anti-tumour cell-mediated immune response. For example, one that leads to a reduction in tumour (cell) growth, such as a cytotoxic cell-mediated immune response (such as a cytotoxic T cell exposure) that kills cells of the cancer or tumour.

In certain embodiments, the cell mediating the cell-mediated immune response may be mediated by a cell, such as an immune cell, capable of secreting (eg secreting) pro-inflammatory cytokine, such as one selected from the group consisting of: interleukin-1 (IL-1), IL-2, IL-12, IL-17 and IL-18, tumour necrosis factor (TNF) [alpha], interferon gamma (IFN-gamma), and granulocyte-macrophage colony stimulating factor.

In certain embodiments, the cell-mediated immune response can be mediated by a pro-inflammatory cytokine-secreting cell, such as a lymphocyte (eg a T cell), in particular a cytotoxic T lymphocyte (CTL).

In particular embodiments, the cell-mediated immune response may induce killing of cells associated or involved with a disease, disorder or condition, such as a proliferative disorder (eg a cancer).

The term "humoral immunity" (or "humoral immune response") will also be readily understood by the person of ordinary skill, and includes an aspect of an immune response that is mediated by macromolecules found in extracellular fluids such as secreted antibodies, complement proteins, and certain antimicrobial peptides. Humoral immunity is so named because it involves substances found in the humors, or body fluids. Its aspects involving antibodies can be termed antibody-mediated immunity.

As used herein, a "subject" includes all mammals, including without limitation humans, but also non-human primates such as cynomolgus monkeys. It also includes dogs, cats, horses, sheep, goats, cows, rabbits, pigs and rodents (such as mice and rats). It will be appreciated that a particularly preferred subject according to the invention is a human subject, such as a human suffering from (or at risk of suffering from) a disorder, disease or condition, for example a human patient.

In further other embodiments, a modulator of the invention (eg, an ABP that binds to IGSF11) that is an *inhibitor or antagonist* may instead or also:
- increase B cell proliferation or B cell responses including but not limited to antigen-specific antibody responses (eg in an in-vitro assay or in a subject, such as one in need thereof);
- promote humoral immune responses elicited against an antigen or cell or therapeutic antibody (eg in an in-vitro assay or in a subject, such as one in need thereof);
- promote humoral immune responses elicited by a therapeutic or prophylactic vaccine (eg in an in-vitro assay or in a subject, such as one in need thereof);
- mediate any one or combination of at least one of the following effects: (i) increases immune response, (ii) increases T cell activation, (iii) increases cytotoxic T cell activity, (iv) increases NK cell activity, (v) alleviates T-cell suppression, (vi) increases pro-inflammatory cytokine secretion, (vii) increases IL-2 secretion; (viii) increases interferon-gamma production, (ix) increases Th1 response, (x) decreases Th2 response, (xi) decreases or eliminates cell number and/or activity of at least one of regulatory T cells (Tregs), myeloid derived suppressor cells (MDSCs), iMCs, mesenchymal stromal cells, TIE2-expressing monocytes, (xii) reduces regulatory cell activity, and/or the activity of one or more of myeloid derived suppressor cells (MDSCs), iMCs, mesenchymal stromal cells, TIE2-expressing monocytes, (xiii) decreases or eliminates M2 macrophages, (xiv) reduces M2 macrophage pro-tumorigenic activity, (xv) decreases or eliminates N2 neutrophils, (xvi) reduces N2 neutrophils pro-tumorigenic activity, (xvii) reduces inhibition of T cell activation, (xviii) reduces inhibition of CTL activation, (xix) reduces inhibition of NK cell activation, (xx) reverses T cell exhaustion, (xxi) increases T cell response, (xxii) increases activity of cytotoxic cells, (xxiii) stimulates antigen-specific memory responses, (xxiv) elicits apoptosis or lysis of cancer cells, (xxv) stimulates cytotoxic or cytostatic effect on cancer cells, (xxvi) induces direct killing of cancer cells, (xxvii) increases Th17 activity and/or (xxviii) induces complement dependent cytotoxicity and/or antibody dependent cell-mediated cytotoxicity; (eg in an in-vitro assay or in a subject, such as one in need thereof) with the optional proviso that said modulator may elicit an opposite effect to one or more of (i)-(xxviii).

In alterative embodiments, a modulator of the invention (eg, an ABP that binds to IGSF11) that is an *activator or agonist* may instead or also:
- enhance, raise, promote or increase IGSF11-mediated inhibition of a cell-mediated immune response (eg in an in-vitro assay or in a subject, such as one in need thereof); and/or
- enhance, raise, promote or increase IGSF11-mediated inhibition of humoral immunity (eg in an in-vitro assay or in a subject, such as one in need thereof)).

In further alterative embodiments, a modulator of the invention (eg, an ABP that binds to IGSF11) that is an *activator or agonist* may instead or also:
- suppress B cell proliferation or B cell responses including but not limited to antigen-specific antibody responses i (eg in an in-vitro assay or in a subject, such as one in need thereof); and/or
- mediate any one or combination of at least one of the following effects: (i) decreases immune response, (ii) decreases T cell activation, (iii) decreases cytotoxic T ceil activity, (iv) decreases natural killer (NK) cell activity, (v) decreases T-cell activity, (vi) decreases pro-inflammatory cytokine secretion, (vii) decreases IL-2 secretion; (viii) decreases interferon-gamma production, (ix) decreases Th1 response, (x) decreases Th2 response, (xi) increases cell number and/or activity of regulatory T cells, (xii) increases regulatory cell activity and/or one or more of myeloid derived suppressor cells (MDSCs), iMCs, mesenchymal stromal cells, TIE2-expressing monocytes, (xiii) increases regulatory cell activity and/or the activity of one or more of myeloid derived suppressor cells (MDSCs), iMCs, mesenchymal stromal cells, TIE2-expressing monocytes, (xiii) increases M2 macrophages, (xiv) increases M2 macrophage activity, (xv) increases N2 neutrophils, (xvi) increases N2 neutrophils activity, (xvii) increases inhibition of T cell activation, (xviii) increases inhibition of CTL activation, (xix) increases inhibition of NK cell activation, (xx) increases T cell exhaustion, (xxi) decreases T cell response, (xxii) decreases activity of cytotoxic cells, (xxiii) reduces antigen-specific memory responses, (xxiv) inhibits apoptosis or lysis of cells, (xxv) decreases cytotoxic or cytostatic effect on ceils, (xxvi) reduces direct killing of cells, (xxvii) decreases Th17 activity, and/or (xxviii) reduces complement dependent cytotoxicity and/or antibody dependent cell-mediated cytotoxicity; (eg in an in-vitro assay or in a subject, such as one in need thereof) with the optional proviso that said modulator may elicit an opposite effect to one or more of (i)-(xxviii).

### ABPs of the invention comprising one or more complementarity determining regions

In particular embodiments, an ABP of the invention can preferentially comprise at least one complementarity determining region (CDR), such as one from an antibody (in particular from a human antibody), and in particular embodiments the ABP can comprise a CDR having an amino acid sequence with at least 80%, 85%, 90% or 95% sequence identity to (preferably, at least 90% sequence identity to), or having no more than three or two, preferably no more than one amino acid substitution(s), deletion(s) or insertion(s) compared to, a CDR sequence set forth in **Table 1A** herein.

The term "complementarity determining region" (or "CDR" or "hypervariable region"), as used herein, refers broadly to one or more of the hyper-variable or complementarily determining regions (CDRs) found in the variable regions of light or heavy chains of an antibody. See, for example: "IMGT", Lefranc et al, 20003, Dev Comp Immunol 27:55; Honegger & Plückthun, 2001, J Mol Biol 309:657, Abhinandan & Martin, 2008, Mol Immunol 45:3832, Kabat, et al. (1987): Sequences of Proteins of Immunological Interest National Institutes of Health, Bethesda, Md. These expressions include the hypervariable regions as defined by Kabat et al (1983) Sequences of Proteins of Immunological Interest, US Dept of Health and Human Services, or the hypervariable loops in 3-dimensional structures of antibodies (Chothia and Lesk, 1987; J Mol Biol 196:901). The CDRs in each chain are held in close proximity by framework regions and, with the CDRs from the other chain, contribute to the formation of the antigen-binding site. Within the CDRs there are select amino acids that have been described as the selectivity determining regions (SDRs) which represent the critical contact residues used by the CDR in the antibody-antigen interaction. (Kashmiri, 2005; Methods 36:25).*]*

As described above, in particular embodiments of the invention, an ABP can comprise at least one complementarity determining region (CDR). In certain of such embodiments, an ABP of the invention comprises at least one complementarity determining region 3 (CDR3), such as one having an amino acid sequence with at least 80%, 85%, 90% or 95% (preferably at least 90%) sequence identity to, or having no more than three or two, preferably no more than one amino acid substitution(s), deletion(s) or insertion(s) compared to, a sequence selected from those heavy and light chain CDR3 sequences shown in **Table 1A** (eg, a sequence selected from the list consisting of SEQ ID Nos: 3, 7, 13, 17, 23, 27, 33, 37, 43, 47, 53, 57, 63, 67, 73, 77, 83, 87, 93, 97, 103, 107, 113, 117, 123, 127, 133, 137, 143, 147, 153, 157, 163, 167, 173, 177, 183, 187, 193, 197, 203, 207, 213, 217, 223, 227, 233, 237, 243, 247, 253, 257, 263, 267, 273, 277, 283, 287, 293, 297, 303, 307, 313, 317, 323, 327, 333, 337, 343, 347, 353, 357, 363, and 367).

An ABP of the invention may, alternatively or as well as a CDR3 sequence, comprise at least one CDR1, and/or at least one CDR2 (such as one from an antibody, in particular from a human antibody). Preferably, and ABP of the invention comprises at least one such CDR3, as well as at least one such CDR1 and at least one such CDR2, more preferably where each of such CDRs having an amino acid sequence with at least 80%, 85%, 90% or 95% (preferably at least 90%) sequence identity to, or having no more than three or two, preferably no more than one amino acid substitution(s), deletion(s) or insertion(s) compared to, a sequence selected from the corresponding (heavy and light chain) CDR1, CD2 and CD3 sequences shown in **Table 1A.**

In particular embodiments, an ABP of the invention can be an antibody or an antigen binding fragment thereof.

As used herein, the term "antibody" may be understood in the broadest sense as any immunoglobulin (Ig) that enables binding to its epitope. An antibody as such is a species of an ABP. Full length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chain of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to cells or factors, including various cells of the immune system (e.g., effector cells) and the first component (C1q) of the classical complement system. Other forms of antibodies include heavy-chain antibodies, being those which consist only of two heavy chains and lack the two light chains usually found in antibodies. Heavy-chain antibodies include the hcIgG (IgG-like) antibodies of camelids such as dromedaries, camels, llamas and alpacas, and the IgNAR antibodies of cartilaginous fishes (for example sharks). And yet other forms of antibodies include single-domain antibodies (sdAb, called Nanobody by Ablynx, the developer) being an antibody fragment consisting of a single monomeric variable antibody domain. Single-domain antibodies are typically produced from heavy-chain antibodies, but may also be derived from conventional antibodies.

Antibodies (or those from which fragments thereof can be isolated) can include, for instance, chimeric, humanized, (fully) human, or hybrid antibodies with dual or multiple antigen or epitope specificities, antibody fragments and antibody sub-fragments, e.g., Fab, Fab' or F(ab')2 fragments, single chain antibodies (scFv) and the like (described below), including hybrid fragments of any immunoglobulin or any natural, synthetic or genetically engineered protein that acts like an antibody by binding to a specific antigen to form a complex. VSIR is an immunoglobulin-like protein, and as such it is not (nor its variants) considered - for the purposes of the present invention - an antibody that binds to IGSF11.

Accordingly, in certain embodiments an ABP of the invention can comprise an antibody heavy chain, or an antigen binding fragment thereof, and/or an antibody light chain, or an antigen binding fragment thereof.

In further embodiments, an ABP of the invention can comprise an antibody heavy chain variable region, or an antigen binding fragment thereof, and/or an antibody light chain variable region, or an antigen binding fragment thereof, and in yet further embodiments, an ABP of the invention can comprise an antibody heavy chain variable region CDR1, CDR2, and CDR3, and/or an antibody light chain variable region CDR1, CDR2, and CDR3.

In particular embodiments of the invention, when the ABP comprises an antibody heavy chain sequence and/or an antibody light chain sequence, or an antigen binding fragment thereof; the antibody heavy chain sequence, or the fragment thereof, can comprise a CDR3 having at least 80%, 85%, 90%; or 95% (preferably at least 90%) sequence identity to, or having no more than three or two, preferably no more than one amino acid substitution(s), deletion(s) or insertion(s) compared to, a CDR3 sequence selected from those heavy chain CDR3 sequences shown in **Table 1A** (eg, a sequence selected from the list consisting of SEQ ID Nos: 3, 13, 23, 33, 43, 53, 63, 73, 83, 93, 103, 113, 123, 133, 143, 153, 163, 173, 183, 193, 203, 213, 223, 233, 243, 253, 263, 273, 283, 293, 303, 313, 323, 333, 343, 353, and 363), and/or wherein antibody light chain sequence, or the fragment thereof, can comprise a CDR3 having at least 80%, 85%, 90%; or 95% (preferably at least 90%) sequence identity to, or having no more than three or two, preferably no more than one amino acid substitution(s), deletion(s) or insertion(s) compared to, a CDR3 sequence selected from those light chain CDR3 sequences shown in **Table 1A** (eg, a sequence selected from the list consisting of SEQ ID Nos: 7, 17, 27, 37, 47, 57, 67, 77, 87, 97, 107, 117, 127, 137, 147, 157, 167, 177, 187, 197, 207, 217, 227, 237, 247, 257, 267, 277, 287, 297, 307, 317, 327, 337, 347, 357, and 367.

In further embodiments of the invention, when the ABP comprises an antibody heavy chain, or an antigen binding fragment thereof, the antibody heavy chain sequence, or the fragment thereof, can further comprise a CDR1 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 1, 11, 21, 31, 41, 51, 61, 71, 81, 91, 101, 111, 121, 131, 141, 151, 161, 171, 181, 191, 201, 211, 221, 231, 241, 251, 261, 271, 281, 291, 301, 311, 321, 331, 341, 351, and 361 (eg a heavy chain CDR1 sequence disclosed in **Table 1A);** and/or a CDR2 having at 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 2, 12, 22, 32, 42, 52, 62, 72, 82, 92, 102, 112, 122, 132, 142, 152, 162, 172, 182, 192, 202, 212, 222, 232, 242, 252, 262, 272, 282, 292, 302, 312, 322, 332, 342, 352, and 362 (eg a CDR2 sequence disclosed in **Table 1A).**

In yet further embodiments of the present invention, an ABP of the invention comprises an antibody light chain, or an antigen binding fragment thereof, wherein the antibody light chain sequence, or the fragment thereof, further comprises a CDR1 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 5, 15, 25, 35, 45, 55, 65, 75, 85, 95, 105, 115, 125, 135, 145, 155, 165, 175, 185, 195, 205, 215, 225, 235, 245, 255, 265, 275, 285, 295, 305, 315, 325, 335, 345, 355, and 365 361 (eg a light chain CDR1 sequence disclosed in **Table 1A);** and/or a CDR2 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 6, 16, 26, 36, 46, 56, 66, 76, 86, 96, 106, 116, 126, 136, 146, 156, 166, 176, 186, 196, 206, 216, 226, 236, 246, 256, 266, 276, 286, 296, 306, 316, 326, 336, 346, 356, and 366 (eg a light chain CDR2 sequence disclosed in **Table 1A).**

In other embodiments of the present invention, an ABP of the invention can comprise an antibody variable chain sequence having at least 80%, 85%, 90%; or 95% (preferably at least 90%) sequence identity to, or having no more than ten, nine, eight, seven, six, five, four, three, two or one, preferably no more than three, two or one amino acid substitution(s), deletion(s) or insertion(s) compared to, a sequence selected from SEQ ID NOs. 4, 8, 14, 18, 24, 28, 34, 38, 44, 48, 54, 58, 64, 68, 74, 78, 84, 88, 94, 98, 104, 108, 114, 118, 124, 128, 134, 138, 144, 148, 154, 158, 164, 168, 174, 178, 184, 188, 194, 198, 204, 208, 214, 218, 224, 228, 234, 238, 244, 248, 254, 258, 264, 268, 274, 278, 284, 288, 294, 298, 304, 308, 314, 318, 324, 328, 334, 338, 344, 348, 354, 358, 364, and 368 (eg, a VH or VL sequence disclosed in **Table 1A).**

In particular embodiments of the invention, an ABP of the invention comprises an antigen binding fragment of an antibody, wherein the antigen binding fragment comprises CDR1, CDR2 and CDR3. In certain of such embodiments, the CDR1 is selected from those disclosed in **Table 1A,** the CDR2 is selected from those disclosed in **Table 1A** and the CDR3 is selected from those disclosed in **Table 1A** (eg, the CDR1, CDR2 and CDR3 are selected from the CDR1, CDR2 and CDR3 sequences having the respective amino acid sequences of SEQ ID Nos. 1, 2, 3, or 5, 6, 7, or 11, 12, 13, or 15, 16, 17, or 21, 22, 23, or 25, 26, 27, or 31, 32, 33, or 35, 36, 37, or 41, 42, 43, or 45, 46, 47, or 51, 52, 53, or 55, 56, 57, or 61, 62, 63, or 65, 66, 67, or 71, 72, 73, or 75, 76, 77, or 81, 82, 83, or 85, 86, 87, or 91, 92, 93, or 95, 96, 97, or 101, 102, 103, or 105, 106, 107, or 111, 112, 113, or 115, 116, 117, or 121, 122, 123, or 125, 126, 127, or 131, 132, 133, or 135, 136, 137, or 141, 142, 143, or 145, 146, 147, or 151, 152, 153, or 155, 156, 157, or 161, 162, 163, or 165, 166, 167, or 171, 172, 173, or 175, 176, 177, or 181, 182, 183, or 185, 186, 187, or 191, 192, 193, or 195, 196, 197, or 201, 202, 203, or 205, 206, 207, or 211, 212, 213, or 215, 216, 217, or 221, 222, 223, or 225, 226, 227, or 231, 232, 233, or 235, 236, 237, or 241, 242, 243, or 245, 246, 247, or 251, 252, 253, or 255, 256, 257, or 261, 262, 263, or 265, 266, 267, or 271, 272, 273, or 275, 276, 277, or 281, 282, 283, or 285, 286, 287, or 291, 292, 293, or 295, 296, 297, or 301, 302, 303, or 305, 306, 307, or 311, 312, 313, or 315, 316, 317, or 321, 322, 323, or 325, 326, 327, or 331, 332, 333, or 335, 336, 337, or 341, 342, 343, or 345, 346, 347, or 351, 352, 353, or 355, 356, 357, or 361, 362, 363, or 365, 366, 367); in each case independently, optionally with no more than three or two, preferably no more than one, amino acid substitution(s), insertion(s) or deletion(s) compared to these sequences.

In further particular embodiments of the present invention, an ABP of the invention can comprise an antibody heavy chain variable region CDR1, CDR2, and CDR3, and/or an antibody light chain variable region CDR1, CDR2, and CDR3, wherein the CDR1 has an amino acid sequence of a heavy or light chain CDR1 shown in **Table 1A** (eg has an amino acid sequence selected from the list consisting of SEQ ID No 1, 5, 11, 15, 21, 25, 31, 35, 41, 45, 51, 55, 61, 65, 71, 75, 81, 85, 91, 95, 101, 105, 111, 115, 121, 125, 131, 135, 141, 145, 151, 155, 161, 165, 171, 175, 181, 185, 191, 195, 201, 205, 211, 215, 221, 225, 231, 235, 241, 245, 251, 255, 261, 265, 271, 275, 281, 285, 291, 295, 301, 305, 311, 315, 321, 325, 331, 335, 341, 345, 351, 355, 361, and 365), and wherein the CDR2 has an amino acid sequence of a heavy or light chain CDR2 shown in **Table 1A** (eg has an amino acid sequence selected from the list consisting of SEQ ID No 2, 6, 12, 16, 22, 26, 32, 36, 42, 46, 52, 56, 62, 66, 72, 76, 82, 86, 92, 96, 102, 106, 112, 116, 122, 126, 132, 136, 142, 146, 152, 156, 162, 166, 172, 176, 182, 186, 192, 196, 202, 206, 212, 216, 222, 226, 232, 236, 242, 246, 252, 256, 262, 266, 272, 276, 282, 286, 292, 296, 302, 306, 312, 316, 322, 326, 332, 336, 342, 346, 352, 356, 362, and 366), and wherein the CDR3 has an amino acid sequence of a heavy or light chain CDR3 shown in **Table 1A** (eg has an amino acid sequence selected from the list consisting of SEQ ID No 3, 7, 13, 17, 23, 27, 33, 37, 43, 47, 53, 57, 63, 67, 73, 77, 83, 87, 93, 97, 103, 107, 113, 117, 123, 127, 133, 137, 143, 147, 153, 157, 163, 167, 173, 177, 183, 187, 193, 197, 203, 207, 213, 217, 223, 227, 233, 237, 243, 247, 253, 257, 263, 267, 273, 277, 283, 287, 293, 297, 303, 307, 313, 317, 323, 327, 333, 337, 343, 347, 353, 357, 363, and 367); in each case independently, optionally with no more than three or two, preferably no more than one, amino acid substitution(s), insertion(s) or deletion(s) compared to these sequences.

In preferred of such embodiments, the ABP may be an antibody, or an antigen binding fragment thereof, composed of at least one, preferably two, antibody heavy chain sequences, and at least one, preferably two, antibody light chain sequences, wherein at least one, preferably both, of the antibody heavy chain sequences and at least one, preferably both, of the antibody light chain sequences comprise CDR1 to CDR3 sequences in a combination selected from any of the combinations of heavy chain CDRs shown in **Table B** and/or selected from any of the combinations of light chain CDRs shown in **Table B** (in each case, combinations CDRs-A-001 to CDRs-A-037); in each case independently, optionally with no more than three or two, preferably no more than one, amino acid substitution(s), insertion(s) or deletion(s) compared to these sequences. Preferably, the combination of both the heavy chain CDRs and the light chain CDRs is one selected from a row marked by any one of the combinations CDRs-A-001 to CDRs-A-037, in each CDR independently optionally with no more than three or two, preferably no more than one, amino acid substitution(s), insertion(s) or deletion(s) compared to these sequences.

**Table B: preferred combinations of heavy chain CDRs and preferred combinations of light chain CDRs**

| **Combination (ID)** | **Heavy Chain CDR1 to CDR3 (SEQ ID NO)** | | | **Light Chain CDR1 to CDR3 (SEQ ID NO)** | | |
|---|---|---|---|---|---|---|
| CDRs-A-001 | 1 | 2 | 3 | 5 | 6 | 7 |
| CDRs-A-002 | 11 | 12 | 13 | 15 | 16 | 17 |
| CDRs-A-003 | 21 | 22 | 23 | 25 | 26 | 27 |
| CDRs-A-004 | 31 | 32 | 33 | 35 | 36 | 37 |
| CDRs-A-005 | 41 | 42 | 43 | 45 | 46 | 47 |
| CDRs-A-006 | 51 | 52 | 53 | 55 | 56 | 57 |
| CDRs-A-007 | 61 | 62 | 63 | 65 | 66 | 67 |
| CDRs-A-008 | 71 | 72 | 73 | 75 | 76 | 77 |
| CDRs-A-009 | 81 | 82 | 83 | 85 | 86 | 87 |
| CDRs-A-010 | 91 | 92 | 93 | 95 | 96 | 97 |
| CDRs-A-011 | 101 | 102 | 103 | 105 | 106 | 107 |
| CDRs-A-012 | 111 | 112 | 113 | 115 | 116 | 117 |
| CDRs-A-013 | 121 | 122 | 123 | 125 | 126 | 127 |
| CDRs-A-014 | 131 | 132 | 133 | 135 | 136 | 137 |
| CDRs-A-015 | 141 | 142 | 143 | 145 | 146 | 147 |
| CDRs-A-016 | 151 | 152 | 153 | 155 | 156 | 157 |
| CDRs-A-017 | 161 | 162 | 163 | 165 | 166 | 167 |
| CDRs-A-018 | 171 | 172 | 173 | 175 | 176 | 177 |
| CDRs-A-019 | 181 | 182 | 183 | 185 | 186 | 187 |
| CDRs-A-020 | 191 | 192 | 193 | 195 | 196 | 197 |
| CDRs-A-021 | 201 | 202 | 203 | 205 | 206 | 207 |
| CDRs-A-022 | 211 | 212 | 213 | 215 | 216 | 217 |
| CDRs-A-023 | 221 | 222 | 223 | 225 | 226 | 227 |
| CDRs-A-024 | 231 | 232 | 233 | 235 | 236 | 237 |
| CDRs-A-025 | 241 | 242 | 243 | 245 | 246 | 247 |
| CDRs-A-026 | 251 | 252 | 253 | 255 | 256 | 257 |
| CDRs-A-027 | 261 | 262 | 263 | 265 | 266 | 267 |
| CDRs-A-028 | 271 | 272 | 273 | 275 | 276 | 277 |
| CDRs-A-029 | 281 | 282 | 283 | 285 | 286 | 287 |
| CDRs-A-030 | 291 | 292 | 293 | 295 | 296 | 297 |
| CDRs-A-031 | 301 | 302 | 303 | 305 | 306 | 307 |
| CDRs-A-032 | 311 | 312 | 313 | 315 | 316 | 317 |
| CDRs-A-033 | 321 | 322 | 323 | 325 | 326 | 327 |
| CDRs-A-034 | 331 | 332 | 333 | 335 | 336 | 337 |
| CDRs-A-035 | 341 | 342 | 343 | 345 | 346 | 347 |
| CDRs-A-036 | 351 | 352 | 353 | 355 | 356 | 357 |
| CDRs-A-037 | 361 | 362 | 363 | 365 | 366 | 367 |

In other preferred embodiments of the invention, the ABP may be an antibody, or an antigen binding fragment thereof, composed of at least one, preferably two, antibody heavy chain sequence, and at least one, preferably two, antibody light chain sequence, wherein the antibody heavy chain sequence and the antibody light chain sequence each comprises a variable region sequence in a combination of heavy and light chain variable domain shown in **Table C** (eg, selected from any of the variable chain combinations Chains-A-001 to Chains-A-037); in each case independently, optionally with no more than ten, nine, eight, seven, six, five, four, preferably no more than three, two or one, amino acid substitution(s), insertion(s) or deletion(s) compared to these sequences.

**Table C: preferred combinations of heavy and light chain variably domains**

| **Combination (ID)** | **Heavy Chain Variable Domain (SEQ ID NO)** | **Light Chain Variable Domain (SEQ ID NO)** |
|---|---|---|
| Chains-A-001 | 4 | 8 |
| Chains-A-002 | 14 | 18 |
| Chains-A-003 | 24 | 28 |
| Chains-A-004 | 34 | 38 |
| Chains-A-005 | 44 | 48 |
| Chains-A-006 | 54 | 58 |
| Chains-A-007 | 64 | 68 |
| Chains-A-008 | 74 | 78 |
| Chains-A-009 | 84 | 88 |
| Chains-A-010 | 94 | 98 |
| Chains-A-011 | 104 | 108 |
| Chains-A-012 | 114 | 118 |
| Chains-A-013 | 124 | 128 |
| Chains-A-014 | 134 | 138 |
| Chains-A-015 | 144 | 148 |
| Chains-A-016 | 154 | 158 |
| Chains-A-017 | 164 | 168 |
| Chains-A-018 | 174 | 178 |
| Chains-A-019 | 184 | 188 |
| Chains-A-020 | 194 | 198 |
| Chains-A-021 | 204 | 208 |
| Chains-A-022 | 214 | 218 |
| Chains-A-023 | 224 | 228 |
| Chains-A-024 | 234 | 238 |
| Chains-A-025 | 244 | 248 |
| Chains-A-026 | 254 | 258 |
| Chains-A-027 | 264 | 268 |
| Chains-A-028 | 274 | 278 |
| Chains-A-029 | 284 | 288 |
| Chains-A-030 | 294 | 298 |
| Chains-A-031 | 304 | 308 |
| Chains-A-032 | 314 | 318 |
| Chains-A-033 | 324 | 328 |
| Chains-A-034 | 334 | 338 |
| Chains-A-035 | 344 | 348 |
| Chains-A-036 | 354 | 358 |
| Chains-A-037 | 364 | 368 |

In particularly preferred embodiment, an ABP of the invention can comprise a combination of heavy chain CDR1, CDR2 and CDR3 sequences and a combination of light chain CDR1, CDR2 and CDR3 sequences in the combination shown by antibody A-015, such as shown in **Table B** by row CDRs-A-015 (eg, heavy chain CDR1, CDR2 and CDR2 having a sequence shown by SEQ ID Nos, 141, 142 and 143, respectively, and light chain CDR1, CDR2 and CDR2 having a sequence shown by SEQ ID Nos, 145, 146 and 147, respectively), in each CDR independently, optionally with no more than three or two, preferably no more than one, amino acid substitution(s), insertion(s) or deletion(s) compared to these sequences. In another particularly preferred embodiment, an ABP of the invention can be an antibody, or an antigen binding fragment thereof, composed of at least one, preferably two, antibody heavy chain sequences, and at least one, preferably two, antibody light chain sequences, wherein at least one, preferably both, of the antibody heavy chain sequences each comprises heavy chain CDR1 to CDR3 sequences in the combination CDRs-A-015 and at least one, preferably both, of the antibody light chain sequences each comprises light chain CDR1 to CDR3 sequences in the combination shown in the row of **Table B** marked by CDRs-A-015, in each CDR independently, optionally with no more than one amino acid substitution(s), insertion(s) or deletion(s) compared to these sequences. In yet another particularly preferred embodiment, an ABP of the invention can be an antibody, or an antigen binding fragment thereof, composed of at least one, preferably two, antibody heavy chain sequence, and at least one, preferably two, antibody light chain sequence, wherein the antibody heavy chain sequence and the antibody light chain sequence each comprises a variable region sequence in a combination of heavy and light chain variable domain shown the row of **Table B** marked by Chains-A-015. In each of such particularly preferred embodiments of the ABP, optionally, the ABP is able to inhibit the binding of VSIR protein or a variant thereof to IGSF11 protein or a variant thereof with an IC50 of 20nM or less or 10nM or less, such as 5nM or less, or preferably 2nM or less. Such IC50s can be determined using the methods described elsewhere herein.

### Further aspects and embodiments of ABPs of the invention

In **a second aspect,** the invention relates to **an ABP which competes with** an ABP of a first aspect for binding to IGSF11 protein (eg to the ECD of IGSF11 protein) or variant thereof, in particular can relate to an ABP that competes with one of the particularly preferred ABPs described above for binding to the IGSF11 protein or variant.

The term "compete" when used in the context of ABPs (e.g., modulator ABPs) that compete for binding for the same antigen (or epitope displayed by such antigen) means competition between ABPs as may be determined by an assay in which the ABP (e.g., antibody or binding fragment thereof) being tested prevents or inhibits (e.g., reduces) binding of a reference ABP (e.g., a ligand, or a reference antibody) to a common antigen (e.g., IGSF11 or a fragment thereof such as an ECD of IGSF11).

In **a related aspect,** the invention relates to **an ABP which binds to the same epitope** as an ABP of a first aspect.

ABPs of a second aspect of the invention may include one or more features (or specific combinations thereof) of the ABPs described above. In particular, an ABP of a second aspect of the invention may be capable of inhibiting (eg inhibits) the binding of VSIR (VISTA) protein or a variant thereof to IGSF11 (VSIG3) protein or a variant thereof, such as described in more details above, and/or an ABP of a second aspect of the invention may modulate the expression, function, activity and/or stability of IGSF11, or the variant of IGSF11 (such as in anyway described elsewhere herein).

In particular embodiments of the invention, as well as (or instead of) an ABP of the invention's capability to inhibit (eg block) the interaction between VSIR (VISTA) protein or a variant thereof to IGSF11 (VSIG3) protein or a variant thereof, an ABP of the invention (including those of a first or second aspect as above) may display, exhibit or otherwise possess other functional features, in particular those which are associated with their utility in sensitising cells to a cell-mediated immune response.

In certain of such particular embodiments, an ABP of the invention is capable of reducing (eg it reduces) the amount and/or surface concentration of said IGSF11 or the variant of IGSF11 present on the surface of a mammalian cell; preferably by ABP-induced internalisation, and optionally degradation, of said IGSF11 or the variant of IGSF11 present on the surface of the mammalian cell.

In further of such particular embodiments, an ABP of the invention is capable of enhancing (eg it enhances) killing and/or lysis of cells expressing IGSF11, or a variant of IGSF11, by cytotoxic T cells and/or TILs. Such enhancement can be assessed, for example, using a suitable assay such as one described in **Example 7** hereof.

A particular functional characteristic of an ABP of the invention may be that of increasing (eg, an IBP of the invention increases) the activity of immune cells, such as T-cells, including when such T-cells recognise a mammalian cell expressing the IGSF11 or the variant of IGSF11, or are are bound to the surface of a mammalian cell expressing said IGSF11 or the variant of IGSF11. An increase in eg T cells may be an increase in production of a pro-inflammatory cytokine such as IL-2 (such as may be measured as described in **Examples 8 and/or 9).**

The term "immune cell" is art recognised to describe any cell of an organism involved in the immune system of such organism, in particular of a mammal such as a human. Leukocytes (white blood cells) are immune cells that are involved in the innate immune system, and the cells of the adaptive immune system are special types of leukocytes, known as lymphocytes. B cells and T cells are the major types of lymphocytes and are derived from hematopoietic stem cells in the bone marrow. B cells are involved in the humoral immune response, whereas T cells are involved in cell-mediated immune response. In preferred embodiments of the invention, the immune cell can be a myeloid cell eg a T cell, and in particular (such as when an increase in cell-mediated immune response is required, such as to treat a cancer) the T cell can be a cytotoxic T cell (also known as TC, cytotoxic T lymphocyte, CTL, T-killer cell, cytolytic T cell, CD8+ T-cell or killer T cell). A CTL is a T-cell that is involved in the killing of cancer cells, cells that are infected (particularly with viruses), or cells that are damaged in other ways. Other preferred immune cells for such embodiments can include Tumour-Infiltrating Lymphocytes (TILs). TILs are white blood cells that have left the bloodstream and migrated into a tumour. Typically, TILs are a mix of different types of cells (i.e., T cells, B cells, NK cells) in variable proportions, T cells being the most abundant cells. TILs can often be found in the stroma and within the tumour itself, and are implicated in killing tumour cells. The presence of lymphocytes in tumours is often associated with better clinical outcomes.

Other particular functional characteristics of an ABP of the invention may be that of: (i) enhancing a cell-mediated immune response, such as that mediated by an activated cytotoxic T-cell (CTL), to a mammalian cell expressing said IGSF11 or the variant of IGSF11; and/or (ii) increasing immune cell, such as T-cell, activity and/or survival (and/or proliferation) in the presence of a mammalian cell expressing said IGSF11 or the variant of IGSF11. In some embodiments, the mammalian cell expressing the IGSF11 may be a cell associated with a disease, disorder or condition such as a cancer cell being (directly) associated with the cancer. In other the mammalian cell expressing the IGSF11 may be an immune cell, such as a T cell (see below), for example an immune cell that is directly or indirectly associated with the disease, disorder or condition.

Other particular functional characteristics of an ABP of the invention that is an *inhibitor or antagonist* of IGSF11 expression, function, activity and/or stability can be any one, or a combination or at least one, functional characteristic of the inhibiting or antagonistic modulators described herein, in particular in the section above "Modulators of IGSF11 expression, function, activity and/or stability".

Those particular functional characteristics of an ABP of the invention that is an *activator or agonist* of IGSF11 expression, function, activity and/or stability can be any one, or a combination or at least one, functional characteristic of the activating or agonistic modulators described herein, in particular in the section above "Modulators of IGSF11 expression, function, activity and/or stability".

In preferred embodiments of all ABPs of the invention, the ABP is isolated and/or substantially pure.

The term "isolated" as used herein in the context of a protein, such as an ABP (an example of which could be an antibody), refers to a protein that is purified from proteins or polypeptides or other contaminants that would interfere with its therapeutic, diagnostic, prophylactic, research or other use. An isolated ABP according to the invention may be a recombinant, synthetic or modified (non-natural) ABP. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. Preferably an isolated ABP or nucleic acid or cells is/are substantially pure. In this context, a "recombinant" protein or nucleic acid is one made using recombinant techniques. Methods and techniques for the production of recombinant nucleic acids and proteins are well known in the art.

The term "isolated" as used herein in the context of a protein, such as an ABP (an example of which could be an antibody), refers to a protein that is purified from proteins or polypeptides or other contaminants that would interfere with its therapeutic, diagnostic, prophylactic, research or other use. An isolated ABP according to the invention may be a recombinant, synthetic or modified (non-natural) ABP. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. Preferably an isolated ABP or nucleic acid or cells is/are substantially pure. In this context, a "recombinant" protein or nucleic acid is one made using recombinant techniques. Methods and techniques for the production of recombinant nucleic acids and proteins are well known in the art.

In some embodiments, an ABP of the invention may bind to (e.g., via one or more epitope(s) displayed by one or more EC domain(s) of) IGSF11 or a paralogue, orthologue or other variant thereof (such as any IGSF11 or variant described herein) with a KD that is less than 20nM, such as less than about 10nM, 5nM or 2nM (in particular, less than about 1 nM). In a preferred embodiment, the ABP of the invention will bind (e.g. said epitope(s) of) said IGSF11 or variant with a KD that is less than 100 pM. In a more preferred embodiment, the ABP of the invention will bind said IGSF11 or variant with a KD that is less than 10 pM. In a most preferred embodiment, the ABP of the invention will bind said IGSF11 or variant with a KD that is less than 2 pM. Binding of an ABP of the invention, such as an antibody of the invention, to a human cell line expressing said IGSF11 or variant may, in some embodiments, occur at an EC50 of less than about 10µg/mL, 5µg/mL, 2µg/mL, 1µg/mL, 0.5µg/mL or 0.2µg/mL, preferably with an EC50 of less than 2µg/mL. Binding of an ABP of the invention, such as an antibody of the invention, to a Cynomolgus cell line expressing an orthologue of said IGSF11 or variant may, in some embodiments, occur at an EC50 of less than about 10µg/mL, 5µg/mL, 2µg/mL, 1µg/mL, 0.5µg/mL or 0.2µg/mL, preferably with an EC50 of less than 2µg/mL.

In other embodiments, an ABP of the invention may: (i) bind to the IGSF11, or to the variant of IGSF11, with a KD that is less than 20nM, such as less than about 10nM, 5nM or 2nM (in particular, less than about 1 nM), is less than 100 pM, or is less than 10 pM; and/or (ii) binds to a human cell line expressing the IGSF11 or the variant of IGSF11 with an EC50 of less than 2ug/mL.

The term "KD", as used herein, is intended to refer to the dissociation constant, which is obtained from the ratio of Kd to Ka (i. e., Kd/Ka) and is expressed as a molar concentration (M). KD values for antibodies can be determined using methods well established in the art such as plasmon resonance (BIAcore®), ELISA and KINEXA. A preferred method for determining the KD of an antibody is by using surface plasmon resonance, preferably using a biosensor system such as a BIAcore® system or by ELISA. "Ka" (or "K-assoc"), as used herein, refers broadly to the association rate of a particular antibody-antigen interaction, whereas the term "Kd" (or "K-diss"), as used herein, refers to the dissociation rate of a particular antibody-antigen interaction.

In yet other embodiments, an ABP of the invention may compete for binding to IGSF11, or to the variant of IGSF11, with an endogenous IGSF11 ligand or receptor, preferably wherein said endogenous IGSF11 ligand or receptor is VSIR, or a variant of VSIR. For example, in certain of such embodiments, the ABP of the invention (eg one that binds to [one or more epitope(s) displayed by] an extracellular domain(s) of IGSF11, or a paralogue, orthologue or other variant thereof) is capable of inhibiting (eg will inhibit) the binding of VSIR protein or a variant thereof to IGSF11 protein or a variant thereof with an IC50 of 100nM, 50nM, or preferably 20nM or less, such as 15nM or less, 10nM or less, 5nM or less, 2nM or less, 1nM or less, 500pM or less, 250pM or less, or 100pM or less. In particular of such embodiments, an ABP of the invention is capable of inhibiting (eg will inhibit) the binding of VSIR protein or a variant thereof to IGSF11 protein or a variant thereof with an IC50 of 10nM or less, such as 5nM or less and preferably 2nM or less.

In one embodiment, an ABP of the invention is a polyclonal antibody (mixture), or the antigen binding fragment is a fragment of a polyclonal antibody (mixture).

In another embodiment, an ABP of the invention is not a polyclonal antibody, or the antigen binding fragment is not a fragment of a polyclonal antibody. In more specific embodiments, an ABP of the invention is not an anti-IGSF11 polyclonal sheep IgG (or, is not antibody number AF4915 from R&D Systems), and/or is not an anti-IGSF11 polyclonal rabbit IgG (or, is not antibody number orb1928 from biorbyt and/or is not antibody number MBP1-59503 from Novus Biologicals).

In an alternative, and preferred, embodiment of all ABPs of the invention, the ABP is an antibody or an antigen binding fragment thereof, and the antibody is a monoclonal antibody, or wherein the antigen binding fragment is a fragment of a monoclonal antibody.

The term "monoclonal antibody" or "mAb" as used herein refers to an antibody obtained from a population of substantially identical antibodies based on their amino acid sequence. Monoclonal antibodies are typically highly specific. Furthermore, in contrast to conventional (polyclonal) antibody preparations which typically include different antibodies directed against different determinants (e.g. epitopes) of an antigen, each mAb is typically directed against a single determinant on the antigen. In addition to their specificity, mAbs are advantageous in that they can be synthesized by cell culture (hybridomas, recombinant cells or the like) uncontaminated by other immunoglobulins. The mAbs herein include for example chimeric, humanized or human antibodies or antibody fragments.

Monoclonal antibodies in accordance with the present invention may be prepared by methods well known to those skilled in the art. For example, mice, rats or rabbits may be immunized with an antigen of interest together with adjuvant. Splenocytes are harvested as a pool from the animals that are administered several immunisations at certain intervals with test bleeds performed to assess for serum antibody titers. Splenocytes are prepared that are either used immediately in fusion experiments or stored in liquid nitrogen for use in future fusions. Fusion experiments are then performed according to the procedure of Stewart & Fuller, J. Immunol. Methods 1989, 123:45-53. Supernatants from wells with growing hybrids are screened by eg enzyme-linked immunosorbent assay (ELISA) for mAb secretors. ELISA-positive cultures are cloned either by limiting dilutions or fluorescence-activated cell sorting, typically resulting in hybridomas established from single colonies. The ability of an antibody, including an antibody fragment or sub-fragment, to bind to a specific antigen can be determined by binding assays known in the art, for example, using the antigen of interest as the binding partner.

In a further preferred embodiment, an ABP of the invention is an antibody or an antigen binding fragment thereof, wherein the antibody is a human antibody a humanised antibody or a chimeric-human antibody, or wherein the antigen binding fragment is a fragment of a human antibody a humanised antibody or a chimeric-human antibody.

Human antibodies can also be derived by in vitro methods. Suitable examples include but are not limited to phage display (CAT, Morphosys, Dyax, Biosite/Medarex, Xoma, Yumab, Symphogen, Alexion, Affimed) and the like. In phage display, a polynucleotide encoding a single Fab or Fv antibody fragment is expressed on the surface of a phage particle (see e.g., Hoogenboom et al., J. Mol. Biol., 227: 381 (1991); Marks et al., J Mol Biol 222: 581 (1991); U.S. Patent No. 5,885,793). Phage are "screened" to identify those antibody fragments having affinity for target. Thus, certain such processes mimic immune selection through the display of antibody fragment repertoires on the surface of filamentous bacteriophage, and subsequent selection of phage by their binding to target. In certain such procedures, high affinity functional neutralizing antibody fragments are isolated. A complete repertoire of human antibody genes may thus be created by cloning naturally rearranged human V genes from peripheral blood lymphocytes (see, e.g., Mullinax et al., Proc Natl Acad Sci (USA), 87: 8095-8099 (1990)) or by generating fully synthetic or semi-synthetic phage display libraries with human antibody sequences (see Knappik et al 2000; J Mol Biol 296:57; de Kruif et al, 1995; J Mol Biol 248):97).

The antibodies described herein may alternatively be prepared through the utilization of the XenoMouse® technology. Such mice are capable of producing human immunoglobulin molecules and antibodies and are deficient in the production of murine immunoglobulin molecules and antibodies. In particular, a preferred embodiment of transgenic production of mice and antibodies is disclosed in U.S. Patent Application Serial No. 08/759,620, filed December 3, 1996 and International Patent Application Nos. WO 98/24893, published June 11, 1998 and WO 00/76310, published December 21, 2000. See also Mendez et al., Nature Genetics, 15:146-156 (1997). Through the use of such technology, fully human monoclonal antibodies to a variety of antigens have been produced. Essentially, XenoMouse® lines of mice are immunized with an antigen of interest. e.g. IGSF11 (VSIG3), lymphatic cells (such as B-cells) are recovered from the hyper-immunized mice, and the recovered lymphocytes are fused with a myeloid-type cell line to prepare immortal hybridoma cell lines. These hybridoma cell lines are screened and selected to identify hybridoma cell lines that produce antibodies specific to the antigen of interest. Other "humanised" mice are also commercially available: eg, Medarex - HuMab mouse, Kymab - Kymouse, Regeneron - Velocimmune mouse, Kirin - TC mouse, Trianni - Trianni mouse, OmniAb - OmniMouse, Harbour Antibodies - H2L2 mouse, Merus - MeMo mouse. Also are available are "humanised" other species: rats: OmniAb - OmniRat, OMT - UniRat. Chicken: OmniAb - OmniChicken.

The term "humanised antibody" according to the present invention refers to immunoglobulin chains or fragments thereof (such as Fab, Fab', F(ab')2, Fv, or other antigen-binding sub-sequences of antibodies), which contain minimal sequence (but typically, still at least a portion) derived from non-human immunoglobulin. For the most part, humanised antibodies are human immunoglobulins (the recipient antibody) in which CDR residues of the recipient antibody are replaced by CDR residues from a non-human species immunoglobulin (the donor antibody) such as a mouse, rat or rabbit having the desired specificity, affinity and capacity. As such, at least a portion of the framework sequence of said antibody or fragment thereof may be a human consensus framework sequence. In some instances, Fv framework residues of the human immunoglobulin need to be replaced by the corresponding non-human residues to increase specificity or affinity. Furthermore, humanised antibodies can comprise residues which are found neither in the recipient antibody nor in the imported CDR or framework sequences. These modifications are made to further refine and maximise antibody performance. In general, the humanised antibody will comprise substantially all of at least one, and typically at least two, variable domains, in which all or substantially all of the CDR regions correspond to those of a non-human immunoglobulin and all or substantially all of the framework regions are those of a human immunoglobulin consensus sequence. The humanised antibody optimally also will comprise at least a portion of an immunoglobulin constant region, typically that of a human immunoglobulin, which (eg human) immunoglobulin constant region may be modified (eg by mutations or glycoengineering) to optimise one or more properties of such region and/or to improve the function of the (eg therapeutic) antibody, such as to increase or reduce Fc effector functions or to increase serum half-life. Exemplary such Fc modification (for example, Fc engineering or Fc enhancement) are described elsewhere herein.

The term "chimeric antibody" according to the present invention refers to an antibody whose light and/or heavy chain genes have been constructed, typically by genetic engineering, from immunoglobulin variable and constant regions which are identical to, or homologous to, corresponding sequences of different species, such as mouse and human. Alternatively, variable region genes derive from a particular antibody class or subclass while the remainder of the chain derives from another antibody class or subclass of the same or a different species. It covers also fragments of such antibodies. For example, a typical therapeutic chimeric antibody is a hybrid protein composed of the variable or antigen-binding domain from a mouse antibody and the constant or effector domain from a human antibody, although other mammalian species may be used.

In particular of such embodiments, an ABP of the invention comprises an antigen binding domain of an antibody wherein the antigen binding domain is of a human antibody. Preferably, ABP comprises an antigen binding domain of an antibody or an antigen binding fragment thereof, which is a human antigen binding domain; (ii) the antibody is a monoclonal antibody, or wherein the antigen binding fragment is a fragment of a monoclonal antibody; and (iii) the antibody is a human antibody or a humanised antibody, or wherein the antigen binding fragment is a fragment of a human antibody, a humanised antibody or a chimeric-human antibody.

Light chains of human antibodies generally are classified as kappa and lambda light chains, and each of these contains one variable region and one constant domain. Heavy chains are typically classified as mu, delta, gamma, alpha, or epsilon chains, and these define the antibody's isotype as IgM, IgD, IgG, IgA, and IgE, respectively. Human IgG has several subtypes, including, but not limited to, IgG1, IgG2, IgG3, and IgG4. Human IgM subtypes include IgM, and IgM2. Human IgA subtypes include IgA1 and IgA2. In humans, the IgA and IgD isotypes contain four heavy chains and four light chains; the IgG and IgE isotypes contain two heavy chains and two light chains; and the IgM isotype contains ten or twelve heavy chains and ten or twelve light chains. Antibodies according to the invention may be IgG, IgE, IgD, IgA, or IgM immunoglobulins.

In some embodiments, the ABP of the invention is an IgG antibody or fragment thereof. In some embodiments, the ABP of the invention is an IgE antibody or fragment thereof. In some embodiments, the ABP of the invention is an IgD antibody or fragment thereof. In some embodiments, the ABP of the invention is an IgA antibody or fragment thereof. In some embodiments, the ABP of the invention is an IgM antibody or fragment thereof. Preferably the ABP of the invention is, comprises or is derived from an IgG immunoglobulin or fragment thereof; such as a human, human-derived IgG immunoglobulin, or a rabbit- or rat-derived IgG, and/or an IgG2 immunoglobulin, or fragment thereof. When the ABP of the invention is, comprises or is derived from a rat-derived IgG, then preferably, the ABP is, comprises or is derived from, a rat IgG2a or IgG2b immunoglobulin. When the ABP of the invention is, comprises or is derived from a human-derived IgG, then more preferably, the ABP of the invention is, comprises or is derived from a human IgG1, IgG2 or IgG4, most preferably, the ABP of the invention is, comprises or is derived from a human IgG1 or IgG2

Accordingly, in particular embodiments of the invention, an ABP is an antibody wherein the antibody is an IgG, IgE, IgD, IgA, or IgM immunoglobulin; preferably an IgG immunoglobulin.

An ABP of the invention, where comprising at least a portion of an immunoglobulin constant region (typically that of a human immunoglobulin) may have such (eg human) immunoglobulin constant region modified - for example eg by glycoengineering or mutations - to optimise one or more properties of such region and/or to improve the function of the (eg therapeutic) antibody, such as to increase or reduce Fc effector functions or to increase serum half-life.

ABPs of the invention, in particular those useful in the present methods include antibodies that induce antibody-dependent cytotoxicity (ADCC) of IGSF11-expressing cells. The ADCC of an anti-IGSF11 antibody can be improved by using antibodies that have low levels of or lack fucose. Antibodies lacking fucose have been correlated with enhanced ADCC (antibody- dependent cellular cytotoxicity) activity, especially at low doses of antibody (Shields et ah, 2002, J. Biol. Chem. 277:26733-26740; Shinkawa et ah, 2003, J. Biol. Chem. 278:3466).

Methods of preparing fucose-less antibodies or antibodies with reduced fucose levels include growth in rat myeloma YB2/0 cells (ATCC CRL 1662). YB 2/0 cells express low levels of FUT8 mRNA, which encodes an enzyme (.alpha. 1,6- fucosyltransferase) necessary for fucosylation of polypeptides.

Alternatively, during the expression of such antibodies, an inhibitor against an enzyme relating to the modification of a sugar chain may be used, including: tunicamycin which selectively inhibits formation of GlcNAc-P-P-Dol which is the first step of the formation of a core oligosaccharide which is a precursor of an N-glycoside-linked sugar chain, castanospermin and W-methyl-1-deoxynojirimycin which are inhibitors of glycosidase I, kifunensine which is an inhibitor of mannosidase I, bromocondulitol which is an inhibitor of glycosidase II, 1 - deoxynojirimycin and 1 ,4-dioxy-1 ,4-imino-D-mannitol which are inhibitors of mannosidase I, swainsonine which is an inhibitor of mannosidase II and the like. Examples of an inhibitor specific for a glycosyltransferase include deoxy derivatives of substrates against N-acetylglucosamine transferase V (GnTV) and the like. Also, it is known that 1 -deoxynojirimycin inhibits synthesis of a complex type sugar chain and increases the ration of high mannose type and hybrid type sugar chains (Glycobiology series 2 -Destiny of Sugar Chain in Cell, edited by Katsutaka Nagai, Senichiro Hakomori and Akira Kobata, 1993).

Based on these data, several cell lines have been genetically engineered to produce antibodies containing no or low levels of fucose (Mori et al, 2004; Yamane-Ohnuki et al., 2004) to engineer the glycosylation patterns of IgG in order to select therapeutic monoclonal antibodies exhibiting particular profiles of Fc-gamma-R engagement that could be used in various pathologies.

Umana et al. and Davis et al. showed that an IgG1 antibody engineered to contain increasing amounts of bisected complex oligosaccharides (bisecting A/-acetylglucosamine, GlcNAC) allows triggering a strong ADCC as compared to its parental counterpart (Umana et al., 1999; Davies et al., 2001). Second, a lack of fucose on human IgG1 N-linked oligosaccharides has been shown to improve FCGRIII binding and ADCC.

GLYCART BIOTECHNOLOGY AG (Zurich, CH) has expressed N-acetyl-glucosaminyltransferase III (GnTIII) which catalyses the addition of the bisecting GlcNac residue to the N-linked oligosaccharide, in a Chinese hamster ovary (CHO) cell line, and showed a greater ADCC of IgG1 antibody produced (WO 99/54342; WO 03/01 1878; WO 2005/044859).

WO20070166306 is related to the modification of an antibody anti-CD19 containing 60% N-acetylglucosamine bisecting oligosaccharides and 10% non-fucosylated N-acetylglucosamine bisecting oligosaccharides produced in a mammalian human 293T embryonal kidney cells transfected with (i) the cDNA for the anti-CD19 antibody and (ii) the cDNA for the GnTIII enzyme.

Recombinant human IgG1 produced in YB2/0 cells (Shinkawa et al., 2003; Siberil et al., 2006) or in CHO-Lec13 (Shields et al., 2002) which exhibited a low-fucose content or were deficient in fucose as compared to the same IgG1 produced in wild-type CHO cells, showed an enhanced ability to trigger cellular cytotoxicity. By contrast, a correlation between galactose and ADCC was not observed and the content of bisecting GlcNAC only marginally affected ADCC (Shinkawa et al., 2003).

By removing or supplanting fucose from the Fc portion of the antibody, KYOWA HAKKO KOGYO (Tokyo, Japan) has enhanced Fc binding and improved ADCC, and thus the efficacy of the MAb (US 6,946,292). This improved Fc-gamma-RIIIA-dependent effector functions of low-fucosylated IgG has been shown to be independent from Fc-gamma-RIII allelic form (Niwa et al., 2005). Moreover, it has been recently shown that the antigenic density required to induce an efficient ADCC is lower when the IgG has a low content in fucose as compared to a highly fucosylated IgG (Niwa et al., 2005)

The Laboratoire Francais du Fractionnement et des Biotechnologies (LFB) (France) showed that the ratio Fuc/Gal in MAb oligosaccharide should be equal or lower than 0.6 to get antibodies with a high ADCC (FR 2 861 080).

Cardarelli et al., 2019 produce an anti-CD19 antibody in Ms-704PF CHO cells deficient in the FUT8 gene which encodes alphal-1,6-fucosyltransferase. Non-fucosylation of the antibody in this paper requires the engineering of an enzyme-deficient cell line. This paper does not consider amino acid mutations.

Herbst et al. generated a humanized IgG1 MAb MEDI-551 expressed in a fucosyltransferase-deficient producer CHO cell line This paper does not consider amino acid mutations (Herbst et al., 2010). [0049] S. Siberil et al used the rat myeloma YB2/0 cell line to produce a MAb anti RhD with a low fucose content. Whereas the MAb produced in a wild type CHO exhibited a high fucose content (81 %), the same MAb produced in YB2/0 cell exhibiited a lower fucose content (32%). This paper does consider amino acid mutations (Siberil et al., 2006).

Accordingly, an ABP of the invention may be prepared and/or may have one or more of the characteristics of such glycoengineering (eg afucosylated) approaches/antibodies described above.

Alternative methods for increasing ADDC activity for an ABP of the invention include mutations in an Fc portion of such ABP, particularly mutations which increase antibody affinity for an Fc-gamma-R receptor.

Accordingly, any of the ABPs of the invention described above can be produced with different antibody isotypes or mutant isotypes to control the extent of binding to different Fc-gamma receptors. Antibodies lacking an Fc region (e.g., Fab fragments) lack binding to different Fc-gamma receptors. Selection of isotype also affects binding to different Fc-gamma receptors. The respective affinities of various human IgG isotypes for the three different Fc-gamma receptors, Fc-gamma-RI, Fc- gamma-RII, and Fc- gamma-RIII, have been determined. (See Ravetch & Kinet, Annu. Rev. Immunol. 9, 457 (1991)). Fc- gamma-RI is a high affinity receptor that binds to IgGs in monomeric form, and the latter two are low affinity receptors that bind IgGs only in multimeric form. In general, both IgG1 and IgG3 have significant binding activity to all three receptors, IgG4 to Fc-gamma-RI, and IgG2 to only one type of Fc-gamma-RII called IIaLR (see Parren et al., J. Immunol. 148, 695 (1992). Therefore, human isotype IgG1 is usually selected for stronger binding to Fc-gamma receptors, and IgG2 or IgG4 is usually selected for weaker binding.

A correlation between increased Fc-gamma-R binding with mutated Fc has been demonstrated using targeted cytoxicity cell-based assays (Shields et ah, 2001, J. Biol. Chem. 276:6591-6604; Presta et ah, 2002, Biochem Soc. Trans. 30:487-490). Methods for increasing ADCC activity through specific Fc region mutations include the Fc variants comprising at least one amino acid substitution at a position selected from the group consisting of: 234, 235, 239, 240, 241, 243, 244, 245, 247, 262, 263, 264, 265, 266, 267, 269, 296, 297, 298, 299, 313, 325, 327, 328, 329, 330 and 332, wherein the numbering of the residues in the Fc region is that of the EU index as in Kabat (Kabat et ah, Sequences of Proteins of Immunological Interest (National Institute of Health, Bethesda, Md. 1987).

In certain specific embodiments, said Fc variants comprise at least one substitution selected from the group consisting of L234D, L234E, L234N, L234Q, L234T, L234H, L234Y, L234I, L234V, L234F, L235D, L235S, L235N, L235Q, L235T, L235H, L235Y, L235I, L235V, L235F, S239D, S239E, S239N, S239Q, S239F, S239T, S239H, S239Y, V240I, V240A, V240T, V240M, F241W, F241L, F241Y, F241E, F241R, F243W, F243L, F243Y, F243R, F243Q, P244H, P245A, P247V, P247G, V262I, V262A, V262T, V262E, V263I, V263A, V263T, V263M, V264L, V264I, V264W, V264T, V264R, V264F, V264M, V264Y, V264E, D265G, D265N, D265Q, D265Y, D265F, D265V, D265I, D265L, D265H, D265T, V266I, V266A, V266T, V266M, S267Q, S267L, E269H, E269Y, E269F, E269R, Y296E, Y296Q, Y296D, Y296N, Y296S, Y296T, Y296L, Y296I, Y296H, N297S, N297D, N297E, A298H, T299I, T299L, T299A, T299S, T299V, T299H, T299F, T299E, W313F, N325Q, N325L, N325I, N325D, N325E, N325A, N325T, N325V, N325H, A327N, A327L, L328M, L328D, L328E, L328N, L328Q, L328F, L328I, L328V, L328T, L328H, L328A, P329F, A330L, A330Y, A330V, A330I, A330F, A330R, A330H, I332D, I332E, I332N, I332Q, I332T, I332H, I332Y and I332A, wherein the numbering of the residues in the Fc region is that of the EU index as in Kabat.

Fc variants can also be selected from the group consisting of V264L, V264I, F241W, F241L, F243W, F243L, F241L/F243L/V262I/V264I, F241W/F243W, F241W/F243W/V262A/V264A, F241L/V262I, F243L/V264I, F243L/V262I/V264W, F241Y/F243Y/V262T/V264T, F241E/F243R/V262E/V264R, F241E/F243Q/V262T/V264E, F241R/F243Q/V262T/V264R, F241E/F243Y/V262T/V264R, L328M, L328E, L328F, I332E, L3238M/I332E, P244H, P245A, P247V, W313F, P244H/P245A/P247V, P247G, V264I/I332E, F241E/F243R/V262E/V264R/I332E, F241E/F243Q/V262T/264E/I332E, F241R/F243Q/V262T/V264R/I332E, F241E/F243Y/V262T/V264R/I332E, S298A/I332E, S239E/I332E, S239Q/I332E, S239E, D265G, D265N, S239E/D265G, S239E/D265N, S239E/D265Q, Y296E, Y296Q, T299I, A327N, S267Q/A327S, S267L/A327S, A327L, P329F, A330L, A330Y, I332D, N297S, N297D, N297S/I332E, N297D/I332E, N297E/I332E, D265Y/N297D/I332E, D265Y/N297D/T299L/I332E, D265F/N297E/I332E, L328I/I332E, L328Q/I332E, I332N, I332Q, V264T, V264F, V240I, V263I, V266I, T299A, T299S, T299V, N325Q, N325L, N325I, S239D, S239N, S239F, S239D/I332D, S239D/I332E, S239D/I332N, S239D/I332Q, S239E/I332D, S239E/I332N, S239E/I332Q, S239N/I332D, S239N/I332E, S239N/I332N, S239N/I332Q, S239Q/I332D, S239Q/I332N, S239Q/I332Q, Y296D, Y296N, F241Y/F243Y/V262T/V264T/N297D/I332E, A330Y/I332E, V264I/A330Y/I332E, A330L/I332E, V264I/A330L/I332E, L234D, L234E, L234N, L234Q, L234T, L234H, L234Y, L234I, L234V, L234F, L235D, L235S, L235N, L235Q, L235T, L235H, L235Y, L235I, L235V, L235F, S239T, S239H, S239Y, V240A, V240T, V240M, V263A, V263T, V263M, V264M, V264Y, V266A, V266T, V266M, E269H, E269Y, E269F, E269R, Y296S, Y296T, Y296L, Y296I, A298H, T299H, A330V, A330I, A330F, A330R, A330H, N325D, N325E, N325A, N325T, N325V, N325H, L328D/I332E, L328E/I332E, L328N/I332E, L328Q/I332E, L328V/I332E, L328T/I332E, L328H/I332E, L328I/I332E, L328A, I332T, I332H, I332Y, I332A, S239E/V264I/I332E, S239Q/V264I/I332E, S239E/V264I/A330Y/I332E, S239E/V264I/S298A/A330Y/I332E, S239D/N297D/I332E, S239E/N297D/I332E, S239D/D265V/N297D/I332E, S239D/D265I/N297D/I332E, S239D/D265L/N297D/I332E, S239D/D265F/N297D/I332E, S239D/D265Y/N297D/I332E, S239D/D265H/N297D/I332E, S239D/D265T/N297D/I332E, V264E/N297D/I332E, Y296D/N297D/I332E, Y296E/N297D/I332E, Y296N/N297D/I332E, Y296Q/N297D/I332E, Y296H/N297D/I332E, Y296T/N297D/I332E, N297D/T299V/I332E, N297D/T299I/I332E, N297D/T299L/I332E, N297D/T299F/I332E, N297D/T299H/I332E, N297D/T299E/I332E, N297D/A330Y/I332E, N297D/S298A/A330Y/I332E, S239D/A330Y/I332E, S239N/A330Y/I332E, S239D/A330L/I332E, S239N/A330L/I332E, V264I/S298A/I332E, S239D/S298A/I332E, S239N/S298A/I332E, S239D/V264I/I332E, S239D/V264I/S298A/I332E, and S239D/264I/A330L/I332E, wherein the numbering of the residues in the Fc region is that of the EU index as in Kabat. See also WO2004029207, incorporated by reference herein..

In particular embodiments, mutations on, adjacent, or close to sites in the hinge link region (e.g., replacing residues 234, 235, 236 and/or 237 with another residue) can be made, in all of the isotypes, to reduce affinity for Fc-gamma receptors, particularly Fc-gamma-RI receptor (see, eg US6624821). Optionally, positions 234, 236 and/or 237 are substituted with alanine and position 235 with glutamate. (See, eg US5624821.) Position 236 is missing in the human IgG2 isotype. Exemplary segments of amino acids for positions 234, 235 and 237 for human IgG2 are Ala Ala Gly, Val Ala Ala, Ala Ala Ala, Val Glu Ala, and Ala Glu Ala. A preferred combination of mutants is L234A, L235E and G237A, or is L234A, L235A, and G237A for human isotype IgG1. A particular preferred ABP of the invention is an antibody having human isotype IgG1 and one of these three mutations of the Fc region. Other substitutions that decrease binding to Fc-gamma receptors are an E233P mutation (particularly in mouse IgG1) and D265A (particularly in mouse IgG2a). Other examples of mutations and combinations of mutations reducing Fc and/or C1q binding are E318A/K320A/R322A (particularly in mouse IgG1), L235A/E318A/K320A/K322A (particularly in mouse IgG2a). Similarly, residue 241 (Ser) in human IgG4 can be replaced, eg with proline to disrupt Fc binding.

Additional mutations can be made to a constant region to modulate effector activity. For example, mutations can be made to the IgG1 or IgG2 constant region at A330S, P331S, or both. For IgG4, mutations can be made at E233P, F234V and L235A, with G236 deleted, or any combination thereof. IgG4 can also have one or both of the following mutations S228P and L235E. The use of disrupted constant region sequences to modulate effector function is further described, eg in WO2006118,959 and WO2006036291.

Additional mutations can be made to the constant region of human IgG to modulate effector activity (see, e.g., WO200603291). These include the following substitutions: (i) A327G, A330S, P331S; (ii) E233P, L234V, L235A, G236 deleted; (iii) E233P, L234V, L235A; (iv) E233P, L234V, L235A, G236 deleted, A327G, A330S, P331S; and (v) E233P, L234V, L235A, A327G, A330S, P331S to human IgG1; or in particular, (vi) L234A, L235E, G237A, A330S and P331S (eg, to human IgG1), wherein the numbering of the residues in the Fc region is that of the EU index as in Kabat. See also WO2004029207, incorporated by reference herein.

The affinity of an antibody for the Fc-gamma-R can be altered by mutating certain residues of the heavy chain constant region. For example, disruption of the glycosylation site of human IgG1 can reduce Fc-gamma-R binding, and thus effector function, of the antibody (see, eg WO2006036291). The tripeptide sequences NXS and NXT, where X is any amino acid other than proline, are the enzymatic recognition sites for glycosylation of the N residue. Disruption of any of the tripeptide amino acids, particularly in the CH2 region of IgG, will prevent glycosylation at that site. For example, mutation of N297 of human IgG1 prevents glycosylation and reduces Fc-gamma-R binding to the antibody.

Although activation of ADCC and CDC is often desirable for therapeutic antibodies, there are circumstances in which an ABP of the invention unable to activate effector functions is preferential (eg, an ABP of the invention that is an agnostic modulator). For these purposes IgG4 has commonly been used but this has fallen out of favour in recent years due the unique ability of this sub-class to undergo Fab-arm exchange, where heavy chains can be swapped between IgG4 in vivo as well as residual ADCC activity. Accordingly, Fc engineering approaches can also be used to determine the key interaction sites for the Fc domain with Fc-gamma receptors and C1q and then mutate these positions, such as in an Fc of an ABP of the invention, to reduce or abolish binding. Through alanine scanning Duncan and Winter (1998; Nature 332:738) first isolated the binding site of C1q to a region covering the hinge and upper CH2 of the Fc domain. Researchers at Genmab identified mutants K322A, L234A and L235A, which in combination are sufficient to almost completely abolish Fc-gamma-R and C1q binding (Hezareh et al, 2001; J Virol 75:12161). In a similar manner MedImmune later identified a set of three mutations, L234F/L235E/P331S (dubbed TM), which have a very similar effect (Oganesyan et al, 2008; Acta Crystallographica 64:700). An alternative approach is modification of the glycosylation on asparagine 297 of the Fc domain, which is known to be required for optimal FcR interaction. A loss of binding to Fc-gammaRs has been observed in N297 point mutations (Tao et al, 1989; J Immunol 143:2595), enzymatically degylcosylated Fc domains (Mimura et al, 2001; J Biol Chem 276:45539), recombinantly expressed antibodies in the presence of a glycosylation inhibitor (Walker et al, 1989; Biochem J 259:347) and the expression of Fc domains in bacteria (Mazor et al 2007; Nat Biotechnol 25:563). Accordingly, the invention also includes embodiments of the ABPs in which such technologies or mutations have been used to reduce effector functions.

IgG naturally persists for a prolonged period in (eg human) serum due to FcRn-mediated recycling, giving it a typical half-life of approximately 21 days. Despite this there have been a number of efforts to engineer the pH dependant interaction of the Fc domain with FcRn to increase affinity at pH 6.0 while retaining minimal binding at pH 7.4. Researchers at PDL BioPharma identified the mutations T250Q/M428L, which resulted in an approximate 2-fold increase in IgG half-life in rhesus monkeys (Hinto et al, 2004; J Biol Chem 279:6213), and researchers at MedImmune have identified mutations M252Y/S254T/T256E (dubbed YTE), which resulted in an approximate 4-fold increase in IgG half-life in cynomolgus monkeys (Dall'Acqua, et al 2006; J Biol Chem 281:23514). A combination of the M252Y/S254T/T256E mutations with point mutations H433K/N434F lead to similar effects (Vaccaro et al., 2005, Nat Biotechnol. Oct;23(10):1283-8). ABPs of the invention may also be PEGylated. PEGylation, ie chemical coupling with the synthetic polymer poly-ethylene glycol (PEG), has emerged as an accepted technology for the development of biologics that exercise prolonged action, with around 10 clinically approved protein and peptide drugs to date (Jevsevar et al., 2010; Biotechnol J 5:113). ABPs of the invention may also be subjected to PASylation, a biological alternative to PEGylation for extending the plasma half-life of pharmaceutically active proteins (Schlapschy et al, 2013; Protein Eng Des Sel 26:489; XL-protein GmbH, Germany). Similarily, the XTEN half-life extension technology from Amunix provides another biological alternative to PEGylation (Schellenberger, 2009, Nat Biotechnol.;27(12):1186-90. doi: 10.1038/nbt.1588). Accordingly, the invention also includes embodiments of the ABPs in which such technologies or mutations have been used to prolong serum half-life, especially in human serum.

Antibody fragments include "Fab fragments", which are composed of one constant and one variable domain of each of the heavy and the light chains, held together by the adjacent constant region of the light chain and the first constant domain (CH1) of the heavy chain. These may be formed by protease digestion, e.g. with papain, from conventional antibodies, but similar Fab fragments may also be produced by genetic engineering. Fab fragments include Fab; Fab and "Fab-SH" (which are Fab fragments containing at least one free sulfhydryl group).

Fab' fragments differ from Fab fragments in that they contain additional residues at the carboxy terminus of the first constant domain of the heavy chain including one or more cysteines from the antibody hinge region. Fab' fragments include "Fab'-SH" (which are Fab' fragments containing at least one free sulfhydryl group).

Further, antibody fragments include F(ab')2 fragments, which contain two light chains and two heavy chains containing a portion of the constant region between the CH1 and CH2 domains ("hinge region"), such that an interchain disulphide bond is formed between the two heavy chains. A F(ab')2 fragment thus is composed of two Fab' fragments that are held together by a disulphide bond between the two heavy chains. F(ab')2 fragments may be prepared from conventional antibodies by proteolytic cleavage with an enzyme that cleaves below the hinge region, e.g. with pepsin, or by genetic engineering.

An "Fv region" comprises the variable regions from both the heavy and light chains, but lacks the constant regions. "Single-chain antibodies" or "scFv" are Fv molecules in which the heavy and light chain variable regions have been connected by a flexible linker to form a single polypeptide chain, which forms an antigen binding region.

An "Fc region" comprises two heavy chain fragments comprising the CH2 and CH3 domains of an antibody. The two heavy chain fragments are held together by two or more disulphide bonds and by hydrophobic interactions of the CH3 domains.

Accordingly, in some embodiments, the ABP of the invention is an antibody fragment selected from the list consisting of: Fab', Fab, Fab'-SH, Fab-SH, Fv, scFv and F(ab')2.

In those embodiments of ABPs that are fragments of immunoglobulins, such as an antibody fragment, preferred are those fragments capable of binding to (eg an epitope displayed by) the extracellular domain(s) of IGSF11, or a paralogue, orthologue or other variant thereof, such as any epitope or other binding characteristic as described herein: and more preferably said fragment is a modulator (such as an inhibitor or antagonist) of the expression, function, activity and/or stability of IGSF11 or a paralogue, orthologue or other variant of IGSF11.

In a preferred embodiment, an ABP of the invention is an antibody wherein at least a portion of the framework sequence of said antibody or fragment thereof is a human consensus framework sequence, for example, comprises a human germline-encoded framework sequence.

In some embodiments, an ABP of the invention is modified or engineered to increase antibody-dependent cellular cytotoxicity (ADCC). As will now be understood by the person of ordinary skill, such ABPs of the invention will have particular utility in the therapy of diseases or disorders associated with cellular resistance against immune cells like CTLs (such as an IGSF11-positive cancer); as the ADCC mechanism (a cell-mediated immune defence whereby an effector cell of the immune system actively lyses a target cell, whose membrane-surface antigens have been bound by specific antibodies) would be enhanced in respect of the cells having resistance against immune cells like CTLs, hence leading to an increase in attachment by and/or lysis of such cells by effector cells of the immune system.

As used herein, "therapy" is synonymous with treating a disease, disorder or condition, which includes reducing symptoms of the disease, disorder or condition, inhibiting progression of the disease, disorder or condition, causing regression of the disease, disorder or condition and/or curing the disease, disorder or condition.

Various techniques to modify or engineer an ABP of the invention to increase ADCC are known (Satoh et al, 2006; Expert Opin Biol Ther 6:1161; WO2009/135181), and hence such embodiments include those wherein an ABP of the invention may be afucosylated (GlycArt Biotechnology) e.g., in which antibodies are produced in CHO cells in which the endogenous FUT8 gene has been knocked out; or the ABP may be a "Sugar-Engineered Antibody" (Seattle Genetics), e.g. in which fucose analogues are added to antibody-expressing CHO cells, resulting in a significant reduction in fucosylation. Other afucosylation approaches that may be applied to an ABP of the invention are described elsewhere herein.

Other techniques to modify or engineer an ABP of the invention to increase ADCC include mutations in a Fc portion of the ABP, (such as described in more detail elsewhere herein), in particular where one or more of residues 234, 235, 236 and/or 237, and/or residues 330, 331 of human Fc are so mutated; wherein such numbering of the residues in the Fc region is that of the EU index as in Kabat (Kabat et ah, Sequences of Proteins of Immunological Interest (National Institute of Health, Bethesda, Md. 1987).

Accordingly, in certain embodiments, the ABP of the invention is modified or engineered to increase antibody-dependent cell-mediated cytotoxicity (ADCC), preferably wherein said ABP is afucosylated and/or an Fc of said ABP is mutated. In alternative embodiments, the ABP of the invention is modified or engineered to reduce ADCC (eg where an Fc is mutated using one or more of the following residue changes: L234A, L235E, G237A, A330S and/or P331S).

In other certain embodiments, the ABP of the invention is modified to prolong serum half-life, especially in human serum. For example, an ABP of the invention may be PEGylated and/or PASylated, or has an Fc region with a T250Q/M428L, H433K/N434F/Y436 or M252Y/S254T/T256E/H433K/N434F modification.

In certain embodiments, the ABP of the invention binds to (a) one or more epitopes displayed by an extracellular domain of IGSF11, or the variant of IGSF11; or which binds to (b) two or more epitopes displayed by an extracellular domain of IGSF11, or the variant of IGSF11. Preferably, one or more of said epitopes is displayed between amino acid residues 23 and 241 (ECD of human IGSF11 protein) of SEQ ID NO: 371, such as between amino acid residues 23 and 136 (Ig-like V-type domain of human IGSF11 protein) of SEQ ID NO: 371.

An ABP of the present invention may be mono-specific (i.e, it possesses antigen binding domain(s) that bind to only one antigen) or may be multi-specific (i.e, it possesses two or more different antigen binding domain(s) that bind to different antigens). For example, a "bi-specific", "dual-specific" or "bifunctional" ABP or antibody is a hybrid ABP or antibody, respectively, having two different antigen binding sites. Bi-specific antigen binding proteins and antibodies are a species of multi-specific antigen binding protein antibody and can be produced by a variety of methods including, but not limited to, fusion of hybridomas or linking of Fab' fragments (see, e.g., Songsivilai and Lachmann, 1990; Kostelny et al., 1992). The two binding sites of a bi-specific antigen binding protein or antibody will bind to two different epitopes, which can reside on the same or different protein targets.

In certain of such embodiments, the ABP may be a bi-specific, tri-specific, or tetra-specific antibody, in particular a bi-specific antibody is selected from: a bispecific T-cell engager (BiTE) antibody, a dual-affinity retargeting molecule (DART), a CrossMAb antibody, a DutaMab™ antibody, a DuoBody antibody; a Triomab, a TandAb, a bispecific NanoBody, Tandem scFv, a diabody, a single chain diabody, a HSA body, a (scFv)2 HSA Antibody, an scFv-IgG antibody, a Dock and Lock bispecific antibody, a DVD-IgG antibody, a TBTI DVD-IgG, an IgG-fynomer, a Tetravalent bispecific tandem IgG antibody, a dual-targeting domain antibody, a chemically linked bispecific (Fab')2 molecule, a crosslinked mAb, a Dual-action Fab IgG (DAF-IgG), an orthoFab-IgG, a bispecific CovX-Body, a bispecific hexavalent trimerbody, and an ART-Ig.

Accordingly, in certain embodiments, the ABP of the invention is a multi-specific antibody comprising at least two antigen binding domains, wherein each antigen binding domain specifically binds to a different antigen epitope.

In certain of such embodiments of such an ABP, at least two of the different antigen epitopes are epitopes displayed by the ECD of IGSF11 (VSIG3) protein, or wherein at least one of the different antigen epitopes is an epitope displayed by the ECD of IGSF11 (VSIG3) protein, and at least one of the different antigen epitopes is an epitope displayed by a protein other than IGSF11 (VSIG3), and preferably other than an epitope displayed by a protein other than VSIR (VISTA).

Accordingly, in some embodiments, the ABP of the invention binds (e.g. via one or more first antigen binding domain(s)) to an extracellular domain(s) of the IGSF11 (VSIG3), paralogue, orthologue or other variant when expressed on the surface of a mammalian cell, and in addition comprises one or more additional antigen binding domain(s) that bind(s) to antigen(s) other than said IGSF11 (VSIG3) or variant. Such other antigen may, in certain embodiments of the inventive ABP, be another immunoglobulin superfamily gene (preferably not VSIR); and/or such other antigen may be an antigen present on a mammalian T-cell. Antigens present on a mammalian T-cell, that may be bound by such an additional antigen binding domain, include CD3, CD40, OX-40, ICOS and 4-1BB. Such other antigen may, in certain embodiments of the inventive ABP, also be albumin, e.g., human albumin. It may also be another component of blood or blood serum the binding of which by the ABP will confer an extended serum half-life upon the ABP, e.g., a half-life similar to that when bound to albumin.

In other embodiments, an ABP of the invention can comprise two or more antigen binding regions, preferably comprising two, three or four antigen binding regions.

In yet other embodiments, an ABP of the invention can comprise a chimeric antigen receptor (CAR), and preferably comprises an extracellular antigen binding region, a membrane anchor such as a transmembrane domain, and an intracellular region, for example, an intracellular signalling region.

In preferred embodiments, an ABP of the invention can comprise at least one antibody constant domain, in particular wherein at least one antibody constant domain is a CH1, CH2, or CH3 domain, or a combination thereof.

In further of such embodiments, an ABP of the invention having antibody constant domain comprises a mutated Fc region, for example for increasing interaction of the Fc region with a Fc receptor (Fc receptor on an immune effector cell (eg Saxena & Wu, 2016; Front Immunol 7:580). Examples and embodiments thereof are described elsewhere herein.

In other embodiments, an ABP of the invention may comprises an effector group and/or a labelling group.

The term "effector group" means any group, in particular one coupled to another molecule such as an antigen binding protein, that acts as a cytotoxic agent. Examples for suitable effector groups are radioisotopes or radionuclides. Other suitable effector groups include toxins, therapeutic groups, or chemotherapeutic groups. Examples of suitable effector groups include calicheamicins, auristatins, geldanamycins, alpha-amanitine, pyrrolobenzodiazepines and maytansines.

The term "label" or "labelling group" refers to any detectable label. In general, labels fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, β-galactosidase, luciferase, alkaline phosphatase); e) biotinylated groups; and f) predetermined polypeptide epitopes recognized by a secondary reporter (e.g., leucine zipper pair sequences, binding sites for secondary antibodies, metal binding domains, epitope tags, etc.).

In some embodiments, an effector group or a labelling group is coupled to another molecule (such as the ABP) via spacer arms of various lengths to reduce potential steric hindrance.

In **another aspect,** the invention relates to **an antigen binding domain (ABD)** of an ABP of the invention, such as of any ABP as described above or elsewhere herein. In certain embodiments, an ABD of the invention is capable, when comprised in an applicable scaffold, of binding to the ECD of IGSF11 (or variant thereof). An ABD of the invention may, in certain embodiments, be isolated and/or substantial pure.

### Nucleic acids, nucleic acid constructs and (host) cells

In **a third aspect,** the invention relates to **a nucleic acid** encoding for an ABP (or ABD) of the invention (such as one described above) or of components thereof. For example, the component encoded by a nucleic acid of the invention may be all or part of one chain of an antibody of the invention; or the component may be a scFV of said ABP. The component encoded by such a nucleic acid may be all or part of one or other of the chains of an antibody of the invention; for example, the component encoded by such a nucleic acid may be an ABD of the invention. The nucleic acids of the invention may also encode a fragment, derivative, mutant, or variant of an ABP of the invention, and/or represent components that are polynucleotides suitable and/or sufficient for use as hybridisation probes, polymerase chain reaction (PCR) primers or sequencing primers for identifying, analyzing, mutating or amplifying a polynucleotide encoding a polypeptide, anti-sense or inhibitory nucleic acids (such as RNAi/siRNA/shRNA or gRNA molecules) for inhibiting expression of a polynucleotide, and complementary sequences of the foregoing.

In particular embodiments of the invention, a nucleic acid of the invention comprises a nucleic acid having a sequence encoding a heavy or light chain CDR, a combination of heavy and/or light chain CDR1, CDR2 and CDR3 or a heavy or light chain variable domain, in each case as displayed in **Table 1A,** or a functional fragment thereof. In other embodiments, a nucleic acid of the invention comprises a nucleic acid sequence at least 60%, 65%, 70%, 75%, 80%, 85%, 90%; or 95% (preferably at least 75%) sequence identity to (or having no more than fifty, forty, thirty, twenty, fifteen, ten or five, preferably no more than three, two or one, base substitution(s), insertion(s) or deletion(s), preferably at the third base of a codon of) a nucleic acid sequence selected from the list consisting of SEQ IDS Nos. 9, 10, 19, 20, 29, 30, 39, 40, 49, 50, 59, 60, 69, 70, 79, 80, 89, 90, 99, 100, 109, 110, 119, 120, 129, 130, 139, 140, 149, 150, 159 160, 169, 170, 179, 180, 189, 190, 199, 200, 209, 210, 219, 220, 229, 230, 239, 240, 249, 250, 259, 260, 269, 270, 279, 280, 289, 290, 299, 300, 309, 310, 319, 320, 329, 330, 339, 340, 349, 350, 359, 360, 369 and 370; preferably wherein such nucleic acid encodes a heavy or light chain variable domain of an ABP of the invention, such as encodes the corresponding heavy or light chain variable domain having the amino acid sequence set forth in **Table 1A,** and optionally having no more than ten, nine, eight, seven, six, five, four, preferably no more than three, two or one, amino acid substitution(s), insertion(s) or deletion(s) compared to these sequences.

The nucleic acid according to the invention may be a DNA or RNA of genomic, mRNA, cDNA, or synthetic origin or some combination thereof, optionally linked to a polynucleotide to which it is not linked in nature. In some embodiments, such nucleic acid may comprise one or more (such as 2, 3, 4, 5, 6, 7, 8, 9, 10 or more than 20, in particular between 1 and about 5, or preferably all instances of a particular nucleotide in the sequence) unnatural (e.g. synthetic) nucleotides; and/or such nucleic acid may comprise (e.g. is conjugated to) another chemical moiety, such as a labelling group or an effector group; for example, a labelling group or an effector group as described elsewhere herein.

In one embodiment, the nucleic acid of the invention may be isolated or substantially pure. In another embodiment, the nucleic acid of the invention may be recombinant, synthetic and/or modified, or in any other way non-natural. For example, a nucleic acid of the invention may contain at least one nucleic acid substitution (or deletion) modification (such as 2, 3, 4, 5, 6, 7, 8, 9, 10 or more than 10 such modifications, in particular between 1 and about 5 such modifications, preferably 2 or 3 such modifications) relative to a product of nature, such as a human nucleic acid.

The nucleic acids can be any suitable length, such as about 10, 15, 20, 25, 30, 35, 40, 45, 50, 75, 100, 125, 150, 175, 200, 250, 300, 350, 400, 450, 500, 750, 1,000, 1,500, 3,000, 5,000 or more nucleotides in length. For example: siRNA nucleic acids may, preferably, be between about 15 to about 25 base pairs in length (preferably between about 19 and about 21 base pairs in length); shRNA nucleic acids may, preferably, comprise a 20-30 base pair stem, a loop of at least 4 nucleotides, and a dinucleotide overhang at the 3' end; microRNA may, preferably, be about 22 base pairs in length; an mRNA or DNA sequence encoding an ABP or a component thereof (such as a heavy or light chain or an IgG antibody) of the invention may, preferably, be between about 500 and 1,500 nucleotides. More preferably, a nucleic acid encoding a mammalian light chain of an antibody may be between about 630 and about 650 nucleotides, and one encoding a mammalian heavy chain of an antibody may be between about 1,300 and about 1,650 nucleotides. A nucleic acid can comprise one or more additional sequences, for example, regulatory sequences, and/or be part of a larger nucleic acid. The nucleic acids can be single-stranded or double-stranded and can comprise RNA and/or DNA nucleotides, and artificial variants thereof (e.g., peptide nucleic acids).

Nucleic acids encoding antibody polypeptides (e.g., heavy or light chain, variable domain only, or full length) may be isolated from B-cells of mice, rats, llamas, alpacas, chicken or rabbits that have been immunized with an IGSF11 (VSIG3) antigen or fragment thereof, such as one or more EC domains (or a polynucleotide encoding and capable of expressing an IGSF11 antigen or fragment thereof). The nucleic acid may be isolated by conventional procedures such as PCR.

Changes can be introduced by mutation into the sequence of a nucleic acid of the invention. Such changes, depending on their nature and location in a codon, can lead to changes in the amino acid sequence of a polypeptide (e.g., an antigen binding protein) that it encodes. Mutations can be introduced using any technique known in the art.

In one embodiment, one or more particular amino acid residues may be changed using, for example, a site-directed mutagenesis protocol. In another embodiment, one or more randomly selected residues may be changed using, for example, a random mutagenesis protocol. However, it is made, a mutant polypeptide can be expressed and screened for a desired property. Mutations can be introduced into a nucleic acid without significantly altering the biological activity of a polypeptide that it encodes. For example, one can make nucleotide substitutions leading to amino acid substitutions at non-essential amino acid residues.

Other changes that may be made (e.g. by mutation) to the sequence of a nucleic acid of the invention may not alter the amino acid sequence of the encoded polypeptide, but may lead to changes to its stability and/or effectiveness of expression of the encoded polypeptide. For example, by codon optimisation, the expression of a given polypeptide sequence may be improved by utilising the more common codons for a given amino acid that are found for the species in which the nucleotide is to be expressed. Methods of codon optimisation, and alternative methods (such as optimisation of CpG and G/C content), are described in, for example, Hass et al, 1996 (Current Biology 6:315); WO1996/09378; WO2006/015789 and WO 2002/098443).

In one **related aspect,** the invention relates to **a nucleic acid construct (NAC)** comprising at least one nucleic acid of the invention (such as described above). Such an NAC can comprise one or more additional features permitting the expression of the encoded ABP or component of said ABP (eg the ABD) in a cell (such as in a host cell). Examples of NACs of the invention include, but are not limited to, plasmid vectors, viral vectors, mRNA, non-episomal mammalian vectors and expression vectors, for example, recombinant expression vectors. The nucleic acid constructs of the invention can comprise a nucleic acid of the invention in a form suitable for expression of the nucleic acid in a cell, such as a host cell, (see below). The nucleic acid constructs of the invention will be, typically, recombinant nucleic acids, and/or may be isolated and/or substantially pure. Recombinant nucleic acids will, typically, be non-natural; particularly if they comprise portions that are derived from different species and/or synthetic, in-vitro or mutagenic methods.

In some embodiments, an NAC of the invention comprises one or more constructs either of which includes a nucleic acid encoding either a heavy or a light antibody chain. In some embodiments, the NAC of the invention comprises two constructs, one of which includes a nucleic acid encoding the heavy antibody chain, the other of which includes a nucleic acid encoding the light antibody chain, such that expression from both constructs can generate a complete antibody molecule. In some embodiments, the NAC of the invention comprises a construct which includes nucleic acids encoding both heavy and light antibody chains, such that a complete antibody molecule can be expressed from one construct. In other embodiments, an NAC of the invention can comprise a single construct that encodes a single chain which is sufficient to form an ABP of the invention; for example, if the encoded ABP is a scFv or a single-domain antibody (such as a camelid antibody).

In some embodiments, the NAC of the invention includes sequences encoding all or part of a constant region, enabling an entire, or a part of, a heavy and/or light chain to be expressed.

An NAC according to the invention may comprise (or consist of) a mRNA molecule which includes an open reading frame encoding an ABP of the invention, and for example together with upstream and downstream elements (such as 5' and/or 3' UTRs and/or poly-A stretch) that enables expression of the ABP, and preferably enhancing stability of the mRNA and/or expression of the ABP. The use of mRNA as NACs to introduce into and express polynucleotides in cells is described, for example, in Zangi et al in Nat. Biotechnol. vol. 31, 898-907 (2013), Sahin et al (2014) Nature Reviews Drug Discovery 13:759 and by Thess et al in Mol. Ther. vol. 23 no.9, 1456-1464 (2015). Particular UTRs that may be comprised in an mRNA NAC of the invention include: 5'UTR of a TOP gene (WO2013/143699), and/or a histone stem-loop (WO 2013/120629). An mRNA NAC of the invention may further comprise one or more chemical modifications (EP 1 685 844); including a 5'-cap, such as m7G(5')ppp, (5'(A,G(5')ppp(5')A or G(5')ppp(5')G and/or at least one nucleotide that is an analogue of naturally occurring nucleotides, such as phosphorothioates, phosphoroamidates, peptide nucleotides, methylphosphonates, 7-deaza-guanosine, 5-methylcytosine or inosine.

NACs, such as DNA-, retroviral- and mRNA-based NACs of the invention may be used in genetic therapeutic methods in order to treat or prevent diseases of the immune system (see Methods of Treatment below), whereby an NAC that comprises an expressible sequence encoding an ABP of the invention is administered to the cell or organism (e.g. by transfection). In particular, the use of mRNA therapeutics for the expression of antibodies is known from WO2008/083949.

In another **related aspect,** the invention relates to **a cell** (such as a host cell and/or a recombinant host cell) comprising one or more nucleic acid or NAC of the invention. Preferably, such cell is capable of expressing the ABP (or component thereof) encoded by said NAC(s). For example, if an ABP of the invention comprises two separate polypeptide chains (e.g. a heavy and light chain of an IgG), then the cell of the invention may comprise a first NAC that encodes (and can express) the heavy chain of such ABP as well as a second NAC that encodes (and can express) the light chain of such ABP; alternatively, the cell may comprise a single NAC that encodes both chains of such ABP. In these ways, such a cell of the invention would be capable of expressing a functional (e.g. binding and/or inhibitory) ABP of the invention. A (host) cell of invention may be one of the mammalian, prokaryotic or eukaryotic host cells as described elsewhere herein, in particularly where the cell is a Chinese hamster ovary (CHO) cell.

In certain embodiments of such aspect, the (host) cell is a human cell; in particular it may be a human cell that has been sampled from a specific individual (eg an autologous human cell). In such embodiments, such human cell can be propagated and/or manipulated in-vitro so as to introduce a NAC of the present invention. The utility of a manipulated human cell from a specific individual can be to produce an ABP of the invention, including to reintroduce a population of such manipulated human cells into a human subject, such as for use in therapy. In certain of such uses, the manipulated human cell may be introduced into the same human individual from which it was first sampled; for example, as an autologous human cell.

The human cell that is subject to such manipulation can be of any germ cell or somatic cell type in the body. For example, the donor cell can be a germ cell or a somatic cell selected from the group consisting of fibroblasts, B cells, T cells, dendritic cells, keratinocytes, adipose cells, epithelial cells, epidermal cells, chondrocytes, cumulus cells, neural cells, glial cells, astrocytes, cardiac cells, oesophageal cells, muscle cells, melanocytes, hematopoietic cells, macrophages, monocytes, and mononuclear cells. The donor cell can be obtained from any organ or tissue in the body; for example, it can be a cell from an organ selected from the group consisting of liver, stomach, intestines, lung, pancreas, cornea, skin, gallbladder, ovary, testes, kidneys, heart, bladder, and urethra.

### Pharmaceutical compositions

To be used in therapy, the ABPs, nucleic acids or NACs (or the cells, such as host cells) of the invention may be formulated into a pharmaceutical composition appropriate to facilitate administration to animals or humans. The term "pharmaceutical composition" means a mixture of substances including a therapeutically active substance (such as an ABP of the invention) for pharmaceutical use.

Accordingly, in **a fourth aspect,** the invention relates to **a pharmaceutical composition** comprising a compound that is modulator of the expression, function, activity and/or stability of immunoglobulin superfamily member 11 (IGSF11, or VSIG3), or of a variant of IGSF11 and a pharmaceutically acceptable carrier, stabiliser and/or excipient. For example, the IGSF11 modulator is an ABP of the invention, and/or at least one NAC of the invention, and/or a (host) cell of the invention. Accordingly, in **a related aspect,** herein provided is a **pharmaceutical composition** comprising an ABP of the invention, and/or at least one NAC of the invention, and/or a (host) cell of the invention, and a pharmaceutically acceptable excipient or carrier.

In a preferred embodiment, the pharmaceutical composition comprises an ABP of the invention, for example in such embodiment, the IGSF11 modulator is an ABP of the invention (eg an IGSF11-inhibitory ABP of the invention).

By way of example, the pharmaceutical composition of the invention may comprise between 0.1% and 100% (w/w) active ingredient (for example, an IGSF11 modulator), such as about 0.5%, 1%, 2%, 3%, 4%, 5%, 6%, 8% 10%, 15%, 20%, 25%, 30%, 40%, 50%, 60%, 70%, 80%, 90%, 95%, 96%, 97%, 98% or 99%, preferably between about 1% and about 20%, between about 10% and 50% or between about 40% and 90%.

As used herein the language "pharmaceutically acceptable" excipient, stabiliser or carrier is intended to include any and all solvents, solubilisers, fillers, stabilisers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of (or for use with) the invention is, typically, formulated to be compatible with its intended route of administration. Examples of routes of administration include oral, parenteral, e.g., intrathecal, intra-arterial, intravenous, intradermal, subcutaneous, oral, transdermal (topical) and transmucosal administration.

Solutions or suspensions used for parenteral, intradermal, or subcutaneous application, as well as comprising a compound of (or for use with) the invention (eg an IGSF11 modulator), can include the following components: a sterile diluent such as water for injection, saline solution, fixed oils, polyethylene glycols, glycerine; propylene glycol or other synthetic solvents; anti-bacterial agents such as benzyl alcohol or methyl parabens; antioxidants such as ascorbic acid or sodium bisulphate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Kolliphor® EL (formerly Cremophor EL™; BASF, Parsippany, N.J.) or phosphate buffered saline (PBS). In all cases, the injectable composition should, typically, be sterile and be fluid to the extent that easy syringability exists. It should, typically, be stable under the conditions of manufacture and storage and be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminium monostearate and gelatin.

Sterile injectable solutions can be prepared by incorporating the compound of (or for use with) the invention (e.g., an IGSF11 modulator) in the required amount in an appropriate solvent with one or a combination of ingredients described herein, as required, followed by filtered sterilisation. Generally, dispersions are prepared by incorporating the active compound into a sterile vehicle which contains a basic dispersion medium and the required other ingredients from those described herein. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions, as well as comprising a compound of (or for use with) the invention (eg an IGSF11 inhibitor), generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavouring agent such as peppermint, methyl salicylate, or orange flavouring.

Furthermore, the compounds of (or for use with) the invention (eg an IGSF11 modulator) can be administered rectally. A rectal composition can be any rectally acceptable dosage form including, but not limited to, cream, gel, emulsion, enema, suspension, suppository, and tablet. One preferred dosage form is a suppository having a shape and size designed for introduction into the rectal orifice of the human body. A suppository usually softens, melts, or dissolves at body temperature. Suppository excipients include, but are not limited to, theobroma oil (cocoa butter), glycerinated gelatin, hydrogenated vegetable oils, mixtures of polyethylene glycols of various molecular weights, and fatty acid esters of polyethylene glycol.

For administration by inhalation, the compounds of (or for use with) the invention (eg an IGSF11 modulator) are typically delivered in the form of an aerosol spray from pressured container or dispenser which contains a suitable propellant, e.g., a gas such as carbon dioxide, or a nebuliser.

Cells, such as immune cells (eg CAR T cells) for use with the invention can be included in pharmaceutical formulations suitable for administration into the bloodstream or for administration directly into tissues or organs. A suitable format is determined by the skilled person (such as a medical practitioner) for each patient, tissue, and organ, according to standard procedures. Suitable pharmaceutically acceptable carriers and their formulation are known in the art (see, e.g. Remington's Pharmaceutical Sciences 16th edition, Osol, A. Ed., 1980). Such cells, when formed in a pharmaceutical composition, are preferably formulated in solution at a pH from about 6.5 to about 8.5. Excipients to bring the solution to isotonicity can also be added, for example, 4.5% mannitol or 0.9% sodium chloride, pH buffered with art-known buffer solutions, such as sodium phosphate. Other pharmaceutically acceptable agents can also be used to bring the solution to isotonicity, including, but not limited to, dextrose, boric acid, sodium tartrate, propylene glycol, polyols (such as mannitol and sorbitol) or other inorganic or organic solutes. In one embodiment, a media formulation is tailored to preserve the cells while maintaining cell health and identity. For example, a premixture including an aqueous solution of anticoagulant (ACD-A), an equal amount of dextrose (50%), and phosphate buffered saline (PBS), or the like is pre-mixed and aliquoted in a volume to typically match or approximate the cellular matrix or environment from which the cell was extracted from the tissue or organ.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the pharmaceutical compositions can be formulated into ointments, salves, gels, or creams as generally known in the art.

In certain embodiments, the pharmaceutical composition is formulated for sustained or controlled release of a compound of (or for use with) the invention (eg an IGSF11 modulator). Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially (including liposomes targeted to infected cells with monoclonal antibodies to viral antigens) can also be used as pharmaceutically acceptable carriers. These can be prepared according to methods known to those skilled in the art.

It is especially advantageous to formulate oral, rectal or parenteral compositions in dosage unit form for ease of administration and uniformity of dosage. Dosage unit form as used herein includes physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

In some embodiments, the pharmaceutical composition comprising an IGSF11 modulator is in unit dose form of between 10 and 1000mg IGSF11 modulator. In some embodiments, the pharmaceutical composition comprising an IGSF11 modulator is in unit dose form of between 10 and 200mg IGSF11 modulator. In some embodiments, the pharmaceutical composition comprising an ABP is in unit dose form of between 200 and 400mg IGSF11 modulator. In some embodiments, the pharmaceutical composition comprising an IGSF11 modulator is in unit dose form of between 400 and 600mg IGSF11 modulator. In some embodiments, the pharmaceutical composition comprising an IGSF11 modulator is in unit dose form of between 600 and 800mg IGSF11 modulator. In some embodiments, the pharmaceutical composition comprising an IGSF11 modulator is in unit dose form of between 800 and 100 mg IGSF11 modulator.

Exemplary unit dosage forms for pharmaceutical compositions comprising IGSF11 modulators are tablets, capsules (eg as powder, granules, microtablets or micropellets), suspensions or as single-use pre-loaded syringes. In certain embodiments, kits are provided for producing a single-dose administration unit. The kit can contain both a first container having a dried active ingredient and a second container having an aqueous formulation. Alternatively, the kit can contain single and multi-chambered pre-loaded syringes.

Toxicity and therapeutic efficacy (eg effectiveness) of such active ingredients can be determined by standard pharmaceutical procedures in cell cultures or experimental animals, eg, for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Active agents which exhibit large therapeutic indices are preferred. While compounds that exhibit toxic side effects may be used, care should be taken to design a delivery system that targets such compounds to the site of affected tissue in order to minimise potential damage to uninfected cells and, thereby, reduce side effects.

The data obtained from the cell culture assays and animal studies can be used in formulating a range of dosage of the active ingredients (eg an IGSF11 modulator), such as for use in humans. The dosage of such active ingredients lies preferably within a range of circulating concentrations that include the ED50 with little or no toxicity. The dosage may vary within this range depending upon the dosage form employed and the route of administration utilised. For any active ingredients used in the therapeutic approaches of the invention, the (therapeutically) effective dose can be estimated initially from cell culture assays. A dose may be formulated in animal models to achieve a circulating plasma concentration range that includes the IC50 (ie, the concentration of the active ingredients which achieves a half-maximal inhibition of symptoms) as determined in cell culture. Such information can be used to more accurately determine useful (eg effective) amounts or doses, such as for administration to humans. The pharmaceutical compositions can be included in a container, pack, or dispenser together with instructions for administration.

In the context of the invention, an effective amount of the IGSF11 modulator or the pharmaceutical composition can be one that will elicit the biological, physiological, pharmacological, therapeutic or medical response of a cell, tissue, system, body, animal, individual, patient or human that is being sought by the researcher, scientist, pharmacologist, pharmacist, veterinarian, medical doctor, or other clinician, eg, lessening of the effects/symptoms of a disorder, disease or condition, such as a proliferative disorder, for example, a cancer or tumour, or killing or inhibiting growth of a cell involved with a proliferative disorder, such as a tumour cell. The effective amount can be determined by standard procedures, including those described below.

In accordance with all aspects and embodiments of the medical uses and methods of treatment provided herein, the effective amount administered at least once to a subject in need of treatment with a IGSF11 modulator is, typically, between about 0.01mg/kg and about 100mg/kg per administration, such as between about 1mg/kg and about 10mg/kg per administration. In some embodiments, the effective amount administered at least once to said subject of a IGSF11 modulator is between about 0.01mg/kg and about 0.1mg/kg per administration, between about 0.1mg/kg and about 1mg/kg per administration, between about 1mg/kg and about 5mg/kg per administration, between about 5mg/kg and about 10mg/kg per administration, between about 10mg/kg and about 50mg/kg per administration, or between about 50mg/kg and about 100mg/kg per administration.

For the prevention or treatment of disease, the appropriate dosage of a IGSF11 modulator (or a pharmaceutical composition comprised thereof) will depend on the type of disease to be treated, the severity and course of the disease, whether the IGSF11 modulator and/or pharmaceutical composition is administered for preventive or therapeutic purposes, previous therapy, the patient's clinical history, age, size/weight and response to the IGSF11 modulator and/or pharmaceutical composition, and the discretion of the attending physician. The IGSF11 modulator and/or pharmaceutical composition is suitably administered to the patient at one time or over a series of treatments. If such IGSF11 inhibitor and/or pharmaceutical composition is administered over a series of treatments, the total number of administrations for a given course of treatment may consist of a total of about 2, 3, 4, 5, 6, 7, 8, 9, 10 or more than about 10 treatments. For example, a treatment may be given once every day (or 2, 3 or 4 times a day) for a week, a month or even several months. In certain embodiments, the course of treatment may continue indefinitely.

The amount of the IGSF11 modulator and/or pharmaceutical composition administered will depend on variables such as the type and extent of disease or indication to be treated, the overall health, age, size/weight of the patient, the in vivo potency of the IGSF11 modulator and/or pharmaceutical composition, and the route of administration. The initial dosage can be increased beyond the upper level in order to rapidly achieve the desired blood-level or tissue level. Alternatively, the initial dosage can be smaller than the optimum, and the daily dosage may be progressively increased during the course of treatment. Human dosage can be optimised, e.g., in a conventional Phase I dose escalation study designed to run from relatively low initial doses, for example from about 0.01mg/kg to about 20mg/kg of active ingredient. Dosing frequency can vary, depending on factors such as route of administration, dosage amount and the disease being treated. Exemplary dosing frequencies are once per day, once per week and once every two weeks. Formulation of an IGSF11 modulator of (or for use with) the present is within the ordinary skill in the art. In some embodiments of the invention such IGSF11 modulator is lyophilised and reconstituted in buffered saline at the time of administration. The IGSF11 modulator and/or pharmaceutical composition of may further result in a reduced relapsing of the disease to be treated or reduce the incidence of drug resistance or increase the time until drug resistance is developing; and in the case of cancer may result in an increase in the period of progression-free survival and/or overall survival.

### Modulators of the expression, function, activity and/or stability of IGSF11 (VSIG3), including for use in pharmaceutical compositions and therapy

In one embodiment of these aspects, the compound that is a modulator of the expression, function, activity and/or stability of immunoglobulin superfamily member 11 (IGSF11, or VSIG3), or of a variant of IGSF11 (such as described above), is a compound that is an an *inhibitor or antagonist* of expression, function, activity and/or stability of IGSF11, or of the variant of IGSF1, in particular a compound that inhibits the binding of VSIR protein (or a variant thereof) to IGSF11 protein (or a variant thereof), in particular inhibits the binding of human VSIR protein (or a variant thereof) to human IGSF11 protein (or a variant thereof), such as inhibits the binding between the ECDs of such proteins, as for example, as described above.

In an alternative embodiment of these aspects, the compound that is a modulator of the expression, function, activity and/or stability of immunoglobulin superfamily member 11 (IGSF11, or VSIG3), or of a variant of IGSF11 (such as described above), is a compound that is an an *activator or agonist* of expression, function, activity and/or stability of IGSF11, or of the variant of IGSF1, in particular a compound that triggers the receptor signaling pathway of the IGSF11 or variant of.

In either of such embodiments, the compound can be one selected from a polypeptide, peptide, glycoprotein, a peptidomimetic, an antigen binding protein (ABP) (for example, an antibody, antibody-like molecule or other antigen binding derivative, or an or antigen binding fragment thereof), a nucleic acid such as a DNA or RNA, for example an antisense or inhibitory DNA or RNA, a ribozyme, an RNA or DNA aptamer, RNAi, siRNA, shRNA and the like, including variants or derivatives thereof such as a peptide nucleic acid (PNA), a genetic construct for targeted gene editing, such as a CRISPR/Cas9 construct and/or a guide nucleic acid (gRNA or gDNA) and/or tracrRNA and a hetero bi-functional compound such as a PROTAC or HyT molecule.

In a preferred embodiment, the compound is an antigen binding protein (ABP) of the invention, such as one of the first or second aspects.

In particular embodiments, the compound enhances killing and/or lysis of cells expressing IGSF11, or a variant of IGSF11, by cytotoxic T-cells and/or TILs.

In other particular embodiments, a compound modulator of the invention that is an *inhibitor or antagonist* of IGSF11 expression, function, activity and/or stability can mediate any one, or a combination or at least one, functional characteristic of the inhibiting or antagonistic modulators described herein, in particular in the section above "Modulators of IGSF11 expression, function, activity and/or stability".

A compound modulator of the invention that is an *activator or agonist* of IGSF11 expression, function, activity and/or stability can mediate any one, or a combination or at least one, functional characteristic of the activating or agonistic modulators described herein, in particular in the section above "Modulators of IGSF11 expression, function, activity and/or stability".

The compound can, in one embodiment, comprise an ECD of an IGSF11 protein or of a VSIR protein, in particular of a human IGSF11 protein or of a human VSIR protein. Such ECDs are described elsewhere herein.

The compound can, in a preferred embodiment, comprise an ABP (for example, an antibody, antibody-like molecule or other antigen binding derivative, or an antigen binding fragment thereof), that binds said IGSF11, or the variant of IGSF11, in particular an ABP of the invention described elsewhere herein.

Alternatively, in another preferred embodiment, the compound can be a nucleic acid (for example an anti-sense nucleotide molecule such as a siRNA or shRNA molecule) that binds to a nucleic acid that encodes or regulates the expression of a gene that controls the expression, function, activity and/or stability of IGSF11, or of a variant of IGSF11. For example, in particular of such embodiments, the nucleic acid (for example an anti-sense nucleotide molecule such as a siRNA or shRNA molecule) that binds to a nucleic acid that encodes IGSF11, or of a variant of IGSF11, or that binds to a nucleic acid that regulates the expression of encodes IGSF11, or of a variant of IGSF11, or that binds to a nucleic acid that encodes for a gene that regulates the expression of encodes IGSF11, or of a variant of IGSF11.

### Nucleic acid modulating compounds

As described above, in one particular set of embodiments, the compound modulator is a nucleic acid.

The terms "nucleic acid", "polynucleotide" and "oligonucleotide" are used, in this context, interchangeably throughout and include DNA molecules (e.g., cDNA or genomic DNA), RNA molecules (e.g., mRNA), analogues of the DNA or RNA generated using nucleotide analogues (e.g., peptide nucleic acids and non-naturally occurring nucleotide analogues), and hybrids thereof. The nucleic acid molecule can be single-stranded or double-stranded.

In the case of IGSF11 modulator compounds being CRISPR/Cas9 constructs and/or guide RNA/DNAs (gRNA/gDNA) and/or tracrRNAs, the basic rules for the design of CRISPR/Cas9 mediated gene editing approaches are known to the skilled artisan and for example reviewed in Wiles MV et al (Mamm Genome 2015, 26:501) or in Savić N and Schwank G (Transl Res 2016, 168:15). Alternatively, gene-specific guide RNAs (gRNAs) useful to knockout the target gene using CRISPR/Cas9 technology can be designed using the online algorithm developed by the Broad Institute (https://portals.broadinstitute.org/gpp/public/analysis-tools/sgrna-design).

In particular embodiments, the IGSF11 modulator compounds may be an inhibitory nucleic acid molecule, such as antisense nucleotide molecule including a siRNA or shRNA molecule, for example as described in detail herein below.

A modulator (eg an inhibitor) compound of IGSF11 that is a nucleic acid can be, for example, an anti-sense nucleotide molecule, an RNA, DNA or PNA molecule, or an aptamer molecule. An anti-sense nucleotide molecule can, by virtue of it comprising an anti-sense nucleotide sequence, bind to a target nucleic acid molecule (eg based on sequence complementarity) within a cell and modulate the level of expression (transcription and/or translation) of IGSF11, or it may modulate expression of another gene that controls the expression, function and/or stability of IGSF11. Similarly, an RNA molecule, such as a catalytic ribozyme, can bind to and alter the expression of the IGSF11 gene, or it can bind to and alter the expression of other genes that control the expression, function and/or stability of IGSF11, such as a transcription factor for or repressor protein of IGSF11. An aptamer is a nucleic acid molecule that has a sequence that confers it an ability to form a three-dimensional structure capable of binding to a molecular target.

A modulator (eg an inhibitor) modulator compound of IGSF11 that is a nucleic acid can be, for example, can further be a double-stranded RNA molecule for use in RNA interference. RNA interference (RNAi) is a process of sequence-specific gene silencing by post-transcriptional RNA degradation or silencing (prevention of translation). RNAi is initiated by use of double-stranded RNA (dsRNA) that is homologous in sequence to the target gene to be silenced. A suitable double-stranded RNA (dsRNA) for RNAi contains sense and antisense strands of about 21 contiguous nucleotides corresponding to the gene to be targeted that form 19 RNA base pairs, leaving overhangs of two nucleotides at each 3'end (Elbashir et al., Nature 411:494-498 (2001); Bass, Nature 411:428-429 (2001); Zamore, Nat. Struct. Biol. 8:746-750 (2001)). dsRNAs of about 25-30 nucleotides have also been used successfully for RNAi (Karabinos et al., Proc. Natl. Acad. Sci. USA 98:7863-7868 (2001). dsRNA can be synthesised in vitro and introduced into a cell by methods known in the art.

A particularly preferred example of an antisense molecule of the invention is a small interfering RNA (siRNA) or endoribonuclease- prepared siRNA (esiRNA). An esiRNA is a mixture of siRNA oligos resulting from cleavage of a long double-stranded RNA (dsRNA) with an endoribonuclease such as Escherichia coli RNase III or dicer. esiRNAs are an alternative concept to the usage of chemically synthesised siRNA for RNA Interference (RNAi). An esiRNAs is the enzymatic digestion of a long double stranded RNA in vitro.

As described above, a modulator of the invention that is an RNAi molecule (such as an siRNA) may bind to and directly inhibit or antagonise the expression of mRNA of IGSF11. However, a modulator of the invention that is an RNAi molecule (such as an siRNA) may bind to and inhibit or antagonise the expression of mRNA of another gene that itself controls the expression (or function or stability) of IGSF11. Such other genes may include transcription factors or repressor proteins of IGSF11.

The sequence identity of the antisense molecule according to the invention in order to target a IGSF11 mRNA (or to target mRNA of a gene controlling expression, function and/or stability of IGSF11), is with increasing preference at least 75%, at least 80%, at least 85%, at least 90%, at least 95%, at least 98%, at least 99% and 100% identity to a region of a sequence encoding the IGSF11 protein, as disclosed herein (or of such other controlling gene). Preferably, the region of sequence identity between the target gene and the modulating antisense molecule is the region of the target gene corresponding to the location and length of the modulating antisense molecule. For example, such a sequence identity over a region of about 19 to 21bp of length corresponding to the modulating siRNA or shRNA molecule). Means and methods for determining sequence identity are known in the art. Preferably, the BLAST (Basic Local Alignment Search Tool) program is used for determining the sequence identity with regard to one or more IGSF11 RNAs as known in the art. On the other hand, preferred antisense molecules such as siRNAs and shRNAs of the present invention are preferably chemically synthesised using appropriately protected ribonucleoside phosphoramidites and a conventional RNA synthesiser. Suppliers of RNA synthesis reagents include Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK).

The ability of antisense molecules, siRNA, and shRNA to potently, but reversibly, silence genes *in vivo* make these molecules particularly well suited for use in the pharmaceutical composition of the invention which will be also described herein below. Ways of administering siRNA to humans are described in De Fougerolles et al., Current Opinion in Pharmacology, 2008, 8:280-285. Such ways are also suitable for administering other small RNA molecules like shRNA. Accordingly, such pharmaceutical compositions may be administered directly formulated as a saline, via liposome based and polymer-based nanoparticle approaches, as conjugated or complexation pharmaceutical compositions, or via viral delivery systems. Direct administration comprises injection into tissue, intranasal and intratracheal administration. Liposome based and polymer- based nanoparticle approaches comprise the cationic lipid Genzyme Lipid (GL) 67, cationic liposomes, chitosan nanoparticles and cationic cell penetrating peptides (CPPs). Conjugated or complexation pharmaceutical compositions comprise PEI-complexed antisense molecules, siRNA, shRNA or miRNA. Further, viral delivery systems comprise influenza virus envelopes and virosomes.

The antisense molecules, siRNAs, shRNAs may comprise modified nucleotides such as locked nucleic acids (LNAs). The ribose moiety of an LNA nucleotide is modified with an extra bridge connecting the 2' oxygen and 4' carbon. The bridge "locks" the ribose in the 3'-endo (North) conformation, which is often found in the A-form duplexes. LNA nucleotides can be mixed with DNA or RNA residues in the oligonucleotide whenever desired. Such oligomers are synthesised chemically and are commercially available. The locked ribose conformation enhances base stacking and backbone pre-organisation. This significantly increases the hybridisation properties (melting temperature) of oligonucleotides. Particularly preferred example of siRNAs is GapmeR (LNA™ GapmeRs (Exiqon)). GapmeRs are potent antisense oligonucleotides used for highly efficient inhibition of IGSF11 mRNA (or of mRNA of a gene controlling expression, function and/or stability of IGSF11). GapmeRs contain a central stretch of DNA monomers flanked by blocks of LNAs. The GapmeRs are preferably 14-16 nucleotides in length and are optionally fully phosphorothioated. The DNA gap activates the RNAse H-mediated degradation of targeted RNAs and is also suitable to target transcripts directly in the nucleus.

Preferred antisense molecules for targeting IGSF11 are antisense molecules or constructs having a sequence complementary to a region (such as one described above) of a nucleic acid sequence of an IGSF11 mRNA, preferably a sequence complementary to a region of a sequence encoding the amino acid sequence shown in SEQ ID NOs: 371 to 373, more preferably, a sequence complementary to a region of between about 15 to 25 bp (such as between about 19 and 21 bp) of a sequence encoding the amino acid sequence shown in SEQ ID NO: 371 to 373.

In particular embodiments, an antisense molecule for targeting IGSF11 may be an siRNA selected from the IGSF11 siRNA molecules identified as "s1", "s2", "s3, or "s4" herein (eg in **Table A;** SEQ ID NOs: 384, 385, 386 and 387, respectively).

**Table A: exemplary siRNA sequences used**

| Gene | siRNA ID | siRNA sequence | Order number (Dharmacon/GE Lifesciences) | SEQ ID NO. |
|---|---|---|---|---|
| IGSF11 | s1 | CAACAUACCAUCCAUUUAU | D-010688-01 | 384 |
| IGSF11 | s2 | GGAACGAAUUGGUGCAGUA | D-010688-02 | 385 |
| IGSF11 | s3 | GAACAUCAGUGCCCUGUCU | D-010688-03 | 386 |
| IGSF11 | s4 | CAGGAACAUUGGACUAAUA | D-010688-04 | 387 |
| IGSF11 | Pool | As above: s1, s2, s3 and s4 | As above | N/A |
| PD-L1 | Smart Pool | | M-015836-01 | N/A |
| CEACAM6 | Smart Pool | | M-015306-01 | N/A |
| Control | siCtrl/siCtrl1 | | 4390844 (ThermoScientific) | N/A |

In particular embodiments, an antisense molecule for targeting IGSF11 may be an shRNA molecule identified as "shIGSF11" herein (eg as may be purchased from Sigma-Aldrich, eg The RNAi Consortium (TRC) numbers: TRCN0000431895, TRCN0000428521 or TRCN0000425839 for IGSF11 CDS, and SHC002 for control shRNA).

In one embodiment, the antisense molecules of the invention may be isolated. In another embodiment, the antisense molecules of the invention may be recombinant, synthetic and/or modified, or in any other way non-natural or not a product of nature. For example, a nucleic acid of the invention may contain at least one nucleic acid substitution (or deletion) modification such as between 1 and about 5 such modifications, preferably no more than 1, 2 or 3 such modifications) relative to a product of nature, such as a human nucleic acid. As described above, the antisense molecules of the invention may be modified by use of non-natural nucleotides, or may be conjugated to another chemical moiety. For example, such chemical moieties may be a heterologous nucleic acid conferring increased stability or cell/nucleus penetration or targeting, or may be a non-nucleic acid chemical moiety conferring such properties, of may be a label.

Certain preferred embodiments pertain to a genetic construct for gene editing that is used as a modulator (eg an inhibitor) of expression, function and/or stability of IGSF11 in the context of the herein described invention. By using genome editing constructs it is possible to modulate the expression, stability and/or activity of IGSF11. Genome editing approaches are well known in the art and may be easily applied when the respective target genomic sequences are known. Preferably, such approaches may be used in gene therapy using e.g. viral vectors, which specifically target tumour cells in accordance with the above descriptions.

In case of genome editing, DNA is inserted, replaced, or removed, from a genome using artificially engineered nucleases, or so called "molecular scissors". The nucleases create specific double- stranded break (DSBs) at desired locations in the genome, and harness the cell's endogenous mechanisms to repair the induced break by natural processes of homologous re-combination (HR) and non-homologous end-joining (NHEJ). For doing so, engineered nucleases such as zinc finger nucleases (ZFNs), Transcription Activator-Like Effector Nucleases (TALENs), the CRISPR/Cas system, and engineered meganuclease re-engineered homing endonucleases are routinely used for genome editing. According to another preferred embodiment, for genome editing approaches for modulating/inhibiting IGSF11, the rare-cutting endonuclease is Cas9, Cpfl, TALEN, ZFN, or a homing endonuclease may be used. Also, it may be convenient to engineer using DNA-guided Argonaute interference systems (DAIS). Basically, said Argonaute (Ago) protein is heterologously expressed from a polynucleotide introduced into said cell in the presence of at least one exogenous oligonucleotide (DNA guide) providing specificity of cleavage to said Ago protein to a preselected locus. The TALEN and Cas9 systems are respectively described in WO 2013/176915 and WO 2014/191128. The Zinc-finger nucleases (ZFNs) are initially described in Kim, YG; Cha, J.; Chandrasegaran, S. ("Hybrid restriction enzymes: zinc finger fusions to Fok I cleavage domain" (1996). Proc Natl Acad Sci USA 93 (3): 1156-60). Cpfl is class 2 CRISPR Cas System described by Zhang et al. (Cpfl is a single RNA-guided Endonuclease of a Class 2 CRIPR-Cas System (2015) Cell 163:759). The argonaute (AGO) gene family was initially described in Guo S, Kemphues KJ. ("par-1, a gene required for establishing polarity in C. elegans embryos, encodes a putative Ser/Thr kinase that is asymmetrically distributed" (1995) Cell 81:611).

The use of the CRISPR/Cas9, CRISPR/Cpfl or the Argonaute genome-editing systems is particularly adapted to be used in combination with the transfection of guide RNA or guide DNA sequences. In this context the guide-RNAs and a nucleic acid sequence coding for Cas9 nickase (or similar enzymes), is transfected into a target cell (preferably a tumour cell) so that they form a complex able to induce a nick event in double-stranded nucleic acid targets in order to cleave the genetic sequence between said nucleic acid targets.

In certain embodiments, it may be useful to deliver the guide RNA-nanoparticle formulations separately from the Cas9. In such an instance, a dual-delivery system is provided such that the Cas9 may be delivered via a vector and the guide RNA is provided in a nanoparticle formulation, where vectors are considered in the broadest sense simply as any means of delivery, rather than specifically viral vectors. Separate delivery of the guide RNA-nanoparticle formulation and the Cas9 may be sequential, for example, first Cas9 vector is delivered via a vector system followed by delivery of sgRNA-nanoparticle formulation) or the sgRNA-nanoparticle formulation and Cas9 may be delivered substantially contemporaneously (i.e., co-delivery). Sequential delivery may be done at separate points in time, separated by days, weeks or even months. In certain embodiments, multiple guide RNAs formulated in one or more delivery vehicles (e.g., where some guide RNAs are provided in a vector and others are formulated in nanoparticles) may be provided with a Cas9 delivery system. In certain embodiments, the Cas9 is also delivered in a nanoparticle formulation. In such an instance, the guide RNA-nanoparticle formulation and the Cas9 nanoparticle formulation may be delivered separately or may be delivered substantially contemporaneously (i.e., co- delivery). As will now be apparent to the person of ordinary skill, the gene target of such genome-editing approaches may be the gene of IGSF11. Alternatively, the gene target of such editing may be another gene that controls the expression, function and/or stability of IGSF11, for example a transcription factor for or repressor protein of IGSF11.

In preferred embodiments of the invention, the compounds for genome editing approaches according to the invention comprise at least the use of a guide RNA or DNA complementary to a region (such as one described above) of a IGSF11 sequence. In some additional embodiments, the compounds for use in genome editing approaches of the invention may include donor sequences homologous to such a region of IGSF11, as templates for homology directed repair. The donor sequences comprise a mutated sequence of IGSF11 that when used in the CRISPR induced repair mechanism in a target cell, is by homologous recombination inserted/copied into the IGSF11 genomic locus, and therefore yields into a mutated IGSF11 gene which is characterised by a reduced expression, function and/or stability of the expressed IGSF11. CRISPR/Cas9 genome editing in cancer therapy is reviewed for ex-ample in Khan FA et al: "CRISPR/Cas9 therapeutics: a cure for cancer and other genetic diseases." (Oncotarget. 2016 May 26. doi: 10.18632/oncotarget.9646; incorporated by reference in its entirety).

### Hetero-bi-functional modulating compounds

In particular embodiments, modulating (eg inhibiting) compounds of IGSF11 may be a hetero-bi-functional compound that contains two ligands connected by a linker, wherein one ligand binds to the target protein (in this case, IGSF11 or a gene that controls the expression, amount, function, activity and/or stability of IGSF11) and the other ligand binds to and/or recruits a component of the cellular protein degradation machinery such as binding to a ubiquitin ligase protein (eg E3 ubiquitin ligase) or such as recruiting a chaperone protein. Examples of such hetero-bi-functional compounds include PROTACs ("PROteolysis TAgeting Chimera) or HyT ("hydrophobic tagging") molecules, in each case designed to bind to the target protein for the present invention. The general principles of PROTACs and HyT molecules are reviewed in Huang & Dixit 2016 (Cell Research 26:484) and exemplified specifically in, for example, WO 2016/146985A1.

A PROTAC that binds to the target protein (eg IGSF11) with one ligand and with the other ligand to an E3 ubiquitin ligase protein thereby brings the ligase and the target into close proximity. Without being bound by any particular theory it is generally understood that it is this close proximity which in turn triggers the poly-ubiquitination and subsequent proteasome-dependent degradation of the target protein of interest. Supporting evidence for a PROTAC approach on a general level is provided by known proof-of-concept examples where alternative PROTACs have been used to degrade: the Estrogen- receptor (Cyrus et al 2010, Chem Bio Chem 11:1531); the Androgen-receptor (Sakamoto et al 2003, Mol Cell Proteomics 2:1350); methionine aminopeptidease-2 (Sakamoto et al 2001, PNAS 98:8554); as well as the Aryl Hydrocarbon Receptor (Lee et al 2007, Chem Bio Chem 8:2058).

The concept of hydrophobic tagging is similar to that of PROTAC, but instead of using a ligand to recruit a specific E3 ligase, a synthetic hydrophobic group, such as adamantane, linked to a chemical moiety that specifically recognizes the target protein (eg IGSF11), assumes the role of "recruiter" for the degradation machinery. Upon binding to the target protein, the hydrophobic tag mimics or induces a misfolded state. Without being bound by any particular theory it is generally understood that modification of the target protein with a bulky hydrophobic side-group attracts the chaperone machinery, the primary goal of which is to help refold misfolded proteins. Since the covalent modification cannot be easily removed, the target protein remains unfolded and is eventually cleared by ubiquitin-proteasome mediated degradation.

### IGSF11 modulating uses, medical uses and methods of treatment

Modulating compounds of IGSF11 (VSIG3), or of a variant of IGSF11, and/or the ABPs, NAC, (host) cells and the pharmaceutical compositions of the invention can be used in various ways to modulate the expression, function, activity and/or stability of the IGSF11 (or variant thereof), including their use in therapy or for prophylaxis.

Accordingly, **in a further aspect,** herein provided is a **method of modulating** the expression, function, activity and/or stability of of IGSF11 (VSIG3), or of a variant of IGSF11 comprising contacting a cell that expresses said IGSF11 or variant with a modulating compound as described above, in particular an ABP of the invention or an NAC encoding said ABP. When such ABP is a modulator of the expression, function, activity and/or stability of said IGSF11 or variant, thereby the expression, function, activity and/or stability of said IGSF11 or variant is modulated. Such method may be practiced on cells that are present *ex-vivo,* that is where said cells are contained in receptacles or containers, such as those used in research facilities. Accordingly, in such embodiments such method of the invention can be described as an *in-vitro* method of modulating the expression, function, activity and/or stability of IGSF11 (VSIG3), or of a variant of IGSF11. However, in alternative embodiments, the method may be practiced using cells within the body, for example an in-vivo method of modulating the expression, function, activity and/or stability of IGSF11 (VSIG3), or of a variant of IGSF11.

In particular of such embodiments, such an in-vitro (or in-vivo) method comprises the *inhibition* of the function and/or activity of the IGSF11 or variant, when such modulating compound (eg the ABP) is an *inhibitor of and*/*or antagonist of* such function and/or activity. In some embodiments of such method, it further comprises the step of contacting the cell with an immune cell, such as a CTL or TIL. Preferably, the ABP is an antibody, or an antibody fragment, and is an *inhibitor or antagonist of* the function and/or activity of the IGSF11 or variant.

In particular of such embodiments, such an in-vitro (or in-vivo) method comprises the *activation* of the function and/or activity of the IGSF11 or variant, when such modulating compound (eg the ABP) is an *activator of and*/*or agonist* of such function and/or activity. In some embodiments of such method, it further comprises the step of contacting the cell with an immune cell, such as a CTL or TIL. Preferably, the ABP is an antibody, or an antibody fragment, and is an *activator and*/*or agonist* of the function and/or activity of the IGSF11 or variant. In certain embodiments of these aspects, the method of modulating comprises contacting a cell that expresses said IGSF11 or variant with a modulating compound as described above that is an *activator and*/*or agonist* of the function and/or activity of the IGSF11 or variant, and the method mediates any one or combination of at least one of the functional characteristic or effects of the activating or agonistic modulators described herein, in particular as set forth in the section above "Modulators of IGSF11 expression, function, activity and/or stability.

In other certain embodiments of these aspects, the method of modulating comprises contacting a cell that expresses said IGSF11 or variant with a modulating compound as described above that is an *inhibitor and*/*or antagonist* of the function and/or activity of the IGSF11 or variant, and the method mediates any one or combination of at least one of the functional characteristic or effects of the inhibitor or antagonist modulators described herein, in particular as set forth in the section above "Modulators of IGSF11 expression, function, activity and/or stability.

In particular embodiments, the modulating compound (in particular, an ABP) is an *inhibitor and*/*or antagonist* of the function and/or activity of the IGSF11 or variant and inhibits the interaction between of VSIR (VISTA) protein or a variant thereof and IGSF11 protein or a variant thereof; that is, such a compound inhibits the binding function and/or activity of the IGSF11 protein or variant thereof.

In preferred embodiments of the therapeutic aspects, the modulating compound (such as one that is an inhibitor or antagonist of expression, function, activity and/or stability of the IGSF11 or variant) for example an ABP, or an NAC encoding said ABP, is capable of: (i) modulating the expression, function, activity and/or stability of the IGSF11 or variant; and/or (ii) enhancing a cell-mediated immune response to a mammalian cell, decreases or reduces the resistance of cells (such as tumour cells that express the IGSF11 or variant), to an immune response. In other certain preferred embodiments of the invention, the ABP (such as one that is an inhibitor or antagonist of expression, function, activity and/or stability of the IGSF11 or variant, in particular one that inhibits the binding function and/or activity of the IGSF11 protein or variant thereof to VSIR (VISTA) protein or a variant thereof), or an NAC encoding said ABP, enhances or increases the sensitivity of cells (such as tumour cells that express the IGSF11 or variant), to an immune response.

The term "resistance" refers to an acquired or natural resistance of a cell involved with (eg of or affected by) a disease (eg a proliferative disorder), such as tumour or cancer cell, to a patient's own immune response (such as a cell-mediated immune response), or to immune responses aided by immune therapy such as adoptive T-cell transfer or treatment with checkpoint blockers. Therefore, a resistant cell (eg a resistant tumour or cancer cell) is more likely to escape and survive humoural and/or cellular immune defence mechanisms in a subject having the disorder (such as the tumour or cancer). A treatment of a resistant proliferative disease, such as tumour/cancer resistance, in context of the invention shall be effective if, compared to a non-treated control, the cell involved with the proliferative disease (such as a cell of the tumour of cancer) becomes more sensitive or susceptible to an immune response (such as a cell-mediated immune response) - in other words will be more likely to be recognised and/or neutralised (for example by cytotoxic processes such as apoptosis) by the subject's immune response.

Accordingly, in particular embodiments of the invention, cell(s) involved with the disease may be resistant against (to) a cell-mediated immune response; and/or such cell(s) may have or display a resistant phenotype.

In preferred embodiments of the invention, the terms "cellular resistance", "cell resistance" and the like refers to a resistance of the subject cell(s) (such as a tumour or cancer cell) to a cell-mediated immune response, such as a cytotoxic T lymphocyte (CTL) response (eg, the tumour or tumour cell being nonresponsive to, or having reduced or limited response to a CTL targeting a tumour cell). A tumour cell may show a reduced or limited response when contacted with a CTL specific for an antigen expressed on that tumour cell. A reduced or limited response is a reduction to a 90% cytotoxic T cell response, preferably a reduction to 80%, 70%, 60%, 50% or more preferably a reduction to 40%, 30%, 20% or even less. In this case, 100% would denote the state wherein the CTLs can kill all of the subject cells involved with the proliferative disorder in a sample. Whether or not a subject cell (eg a tumour cell) is resistant to a patient's (cell-mediated) immune response may be tested in-vitro by contacting a sample of the subjects such cells (eg autologous tumour cells) with (eg autologous) T-cells and thereafter quantifying the survival/proliferation rate of the (eg) tumour cells. As an alternative, the reduction in (cell-mediated) immune response is determined by comparing cancer samples of the same cancer before and after the resistance is acquired (for example induced by therapy), or by comparing with a cancer sample derived from a different cancer which is known to have no resistance to the CTL. On the other hand, the treatments of the present invention include the sensitisation of cells involved with the proliferative disorder against CTL and therefor to decrease resistance of such cells. A decrease of (eg tumour) cell resistance against CTL is preferably a significant increase of CTL toxicity, preferably a 10% increase, more preferably 20%, 30%, 40%, 50%, 60%, 70%, 80% or more, even more preferably 2 fold increase, 3 fold, 4 fold, 5 fold or more.

In particular embodiments, a resistant phenotype of the cells involved with the proliferative disorder is displayed by such cells when a subject suffering from the proliferative disorder (eg a cancer or tumour) has been previously treated with an (immune)therapy and, for example, such proliferative disorders has progressed despite such prior (immune)therapy. For example, a class of subject suitable for the various therapeutic methods of the invention can be those whose tumour (or cancer) has progressed (such as has relapsed or recurred, or has not responded to) after prior treatment with a cancer immunotherapy. In certain embodiments, such prior treatment may be any immunotherapy as described elsewhere herein, including adoptive immune cell transfer (eg TCR or CAR T cell therapy), an anti-tumour vaccine, an antibody binding to an immune checkpoint molecule (such as CTLA-4, PD-1 or PD-L1). In other embodiments, the subject may suffer from a tumour or cancer, and such cancer may have progressed (such as has relapsed or recurred, or has not responded to) after prior radiotherapy.

The immune response, is, in particular of such embodiments, a cell-mediated immune response such as one mediated by T-cells including cytotoxic T-cells and/or TILs; and/or the immune response is the lysis and/or killing of the cells, in particular those that express IGSF11 or a variant thereof) that is mediated by cytotoxic T-cells and/or TILs. In other particular of such embodiments, the immune response is a cytotoxic immune response against cells (such as tumour cells and/or cells the IGSF11 or variant), in particular a cell-mediated cytotoxic immune response such as one mediated by T-cells including cytotoxic T-cells and/or TILs.

Specifically, in certain preferred embodiments of such therapeutic aspects the modulating compound as disclosed herein, in particular the ABP (such as one that is an inhibitor or antagonist of expression, function, activity and/or stability of the IGSF11 or variant thereof), or an NAC encoding said ABP, enhances or increases killing and/or lysis of cells expressing IGSF11 or variant thereof, (such as tumour cells); preferably killing and/or lysis being mediated by cytotoxic T-cells and/or TILs, and/or mediated by an enhancement of or increase in the sensitivity of the cells expressing the IGSF11 or variant thereof to a (cytotoxic) immune response, such an immune response described above, and/or mediated by a decrease in or reduction of the resistance of the cells expressing the IGSF11 or variant thereof to a (cytotoxic) immune response, such an immune response described above

The cells that express IGSF11 or variant thereof are, in certain of such preferred embodiments, cancer cells or are cells that originated from a tumor cell. Exemplary cancer or tumor cells can be those as described or exemplified elsewhere herein.

In other certain preferred embodiments of such therapeutic aspects, the modulating compounds, in particular the ABP (such as one that is an inhibitor or antagonist of expression, function, activity and/or stability of IGSF11 or variant thereof, in particular that is an inhibitor of the VSIR-binding function of the IGSF11 or variant), or an NAC encoding said ABP, increases T-cell activity and/or survival (and/or increases T-cell proliferation), which in certain embodiments, may lead to an enhancement of a (cytotoxic) immune response mediated by such T-cells.

Lines et al (2014) described that T cells both express and *respond* to VSIR (VISTA), indicating that T cells and/or other components of the immune system, in certain circumstances, may express IGSF11 (eg, acting as a receptor for VSIR). Without being bound to theory, Figure 1 of Lines et al 2014, indicates that in the tumour microenvironment (TME) the VSIR receptor ("VISAT-R", eg, now to include IGSF11) may also be expressed on CTLs. The present inventors hypothesise that (analogous to VSIR expression being found on various types of immune cells, as described above) IGSF11 may be expressed on other cells involved in the regulation of the immune response (eg, IGSF11 expression by monocytes and/or Tregs), and the immune regulatory effects mediated by the IGSF11-VSIR axis between immune cells may also lead to a reduction in the (eg anti-tumour) immune response (eg a cell-mediated immune response), manifesting itself in a "resistance" of cells involved in a disease to a cell-mediated immune response. Such an inter-immune cell IGSF11-VSIR axis may play a role at the site of the tumour, for example in the TME (eg tumour bed) between eg VSIR-expressing T cells and IGSF11-expressing monocytes or Tregs that are present at or associated with the site of the tumour, or may play a role outside of the TME, such as in one or more component of the peripheral immune system, in particular by the expression of IGSF11 on monocytes driving T cell suppression in lymph nodes. In particular, the presence of tumour associated macrophages (TAMs) in cancers is associated with poor prognosis, as they are believed to facilitate tumour invasions, angiogenesis and metastasis, and "subvert" the adaptive immune response potentially via their T cell recruitment/activation ability. (Wlliams et al, 2016; NPJ Breast Cancer, 2:15025; Nielsen & Schmid, 2017; Mediators of Inflammation Article ID 9624760). Accordingly, inhibition of the IGSF11-VSIR interaction (eg, by compounds or ABPs of the present invention) in particular where both IGSF11 and VSIR are being expressed by different components (eg different cell types) of a cellular immune response - can also lead to attenuation of the inhibition of the immune response mediated by such an IGSF11-VSIR interaction. Therefore, it is also envisioned by the present invention, those embodiments where IGSF11 is expressed by cells other than eg cancer cells. In particular by immune cells such as monocytes (eg, see **Example 6**) or Tregs. For example, the cells described herein as being "associated with" the disease, disorder or condition, includes not only eg cancer cells (being directly involved in a proliferative disorder), but may also include non-cancer cells, eg regulatory immune cells which may be involved in the (over) inhibition of eg T cell activation, such as monocytes and/or Tregs (either inside, or outside of, the TME) but hence such regulatory immune cells are therefore indirectly associated with the development or (or lack of response of) a proliferative disorder to a cellular immune response. The present inventors also hypothesise that given the potential for a role of the IGSF11-VSIR axis in regulation of the immune system, and in particular by expression of IGSF11 (VSIG3) on immune cells (such as T cells) or monocytes, that IGSF11 (VSIG3) expression on T cells or monocytes can be immunosuppressive by interacting with VSIR (VISTA) present on other immune cells. Accordingly, that an activator or agonist of IGSF11 (VSIG3) will have utility as an immune suppression agent, and hence suitable for the treatment of diseases, disorders or conditions associated with an over-active immune system or an immune system displaying undesired activity, such as autoimmunity, allergy or inflammatory conditions, in particular for the treatment or prevention of allergy, autoimmunity, transplant rejection, inflammation, graft vs host disease or sepsis (or a condition associated with such diseases, disorders or conditions).

Accordingly, in **a fifth aspect,** the invention relates to **a method for the treatment** of a disease, disorder or condition in a mammalian subject by administering a product to the subject wherein the product is a modulator of the expression, function, activity and/or stability of immunoglobulin superfamily member 11 (IGSF11, or VSIG3), or of a variant of IGSF11. In **a related aspect,** the invention relates to **a product for use in medicine,** wherein the product is a compound that is modulator of the expression, function, activity and/or stability of immunoglobulin superfamily member 11 (IGSF11, or VSIG3), or of a variant of IGSF11. In particular embodiments, of these medical/treatment claims, the modulating compound (such as an ABP) is an inhibitor of the binding VSIR-binding function of IGSF11 or variant thereof; and/or wherein the product is selected from the list consisting of an ABP, ABD, nucleic acid, NAC or recombinant host cell of the invention, in particular an ABP of the invention.

In a **related aspect,** the invention also relates to **method of treating or preventing** a disease, disorder or condition in a mammalian subject in need thereof, comprising administering to said subject at least once an effective amount of modulating compound as desired above, or, and in particular administering to said subject at least once an effective amount of the ABP, the NAC, the (host) cells, or the pharmaceutical composition as described above.

In another **related aspect,** the invention also relates to the **use of a product** of the invention as describe above, or a modulating compound as described above (in particular an ABP of the invention) **for the manufacture of a medicament,** in particular for the treatment of a disease, disorder or condition in a mammalian subject, in particular where the disease, disorder or condition is one as set out herein.

The term "treatment" in the present invention is meant to include therapy, e.g. therapeutic treatment, as well as prophylactic or suppressive measures for a disease (or disorder or condition). Thus, for example, successful administration of a IGSF11 inhibitor prior to onset of the disease results in treatment of the disease. "Treatment" also encompasses administration of a IGSF11 inhibitor after the appearance of the disease in order to ameliorate or eradicate the disease (or symptoms thereof). Administration of a IGSF11 inhibitor after onset and after clinical symptoms, with possible abatement of clinical symptoms and perhaps amelioration of the disease, also comprises treatment of the disease. Those "in need of treatment" include subjects (such as a human subject) already having the disease, disorder or condition, as well as those prone to or suspected of having the disease, disorder or condition, including those in which the disease, disorder or condition is to be prevented.

In particular embodiments of these aspects, the modulating compound is one described above, and/or is an ABP, NAC, a (host) cell, or a pharmaceutical composition of the present invention; in particular is an ABP of the invention, and/or is an inhibitory nucleic acid of the invention.

Such a compound can for example in preferred embodiments, be an inhibitor or antagonist of expression, function, activity and/or stability of IGSF11, or of the variant of IGSF11. In particular, the compound inhibits the binding of VSIR protein (or a variant thereof) to IGSF11 protein (or a variant thereof), in particular inhibits the binding of human VSIR protein (or a variant thereof) to human IGSF11 protein (or a variant thereof), such as inhibits the binding between the ECDs of such proteins; preferably wherein such proteins (or variants) and the inhibitions is described as above.

Such a compound can, for example, be a compound (such as an ABP or inhibitors nucleic acid) that enhances killing and/or lysis of cells expressing IGSF11, or a variant of IGSF11, by cytotoxic T-cells and/or TILs.

In other aspects described elsewhere herein, are provided methods to detect and/or diagnose a disease, disorder or condition in a mammalian subject.

In one particular embodiment, the disease, disorder or condition that is characterised by a pathological immune response.

In a further particular embodiment, the disease, disorder or condition is characterised by expression of IGSF11, or a variant of IGSF11, in particular by expression of IGSF11, or a variant of IGSF11 by cells associated with the disease, disorder or condition, such as cancer cells. For example, the disease, disorder or condition can be associated with the undesired presence of IGSF11-positive cells or cells positive for a variant of IGSF11 and or VSIR positive immune cells, in particular VSIR positive monocytes and/or macrophages (in particular, TAMs).

In a yet further particular embodiment, a subject suffering from, or suspected of suffering from, a disease, disorder or condition is characterised as: (i) having an IGSF11 positive cancer or a cancer positive for a variant of IGSF11, and/or (ii) having VSIR positive immune cells, in particular VSIR positive monocytes and/or macrophages; and/or (iii) having IGSF11 positive immune cells, in particular IGSF11 positive monocytes (or Tregs); preferably wherein such IGSF11 positive immune cells are present at or associated with the site of a cancer or tumour (such as being present in the tumour bed or tumour micro environment (TME) of such cancer or tumour, in particular with the presence of TAMs and/or MDSCs).

A disorder, disorder or condition treatable by the subject matter of the invention is, in certain alterative embodiments, one characterised by expression of IGSF11; in particular, one characterised by such expression that is aberrant, for example over- (or under-) expression or representation or activity of IGSF11 (in particular of phosphorylated IGSF11) in a given cell or tissue (such as those cells or tissues involved with the proliferative disease of the subject) compared to that in a healthy subject or a normal cell.

In yet a further particular embodiment, the disease, disorder or condition is characterised by expression and/or activity of IGSF11, in particular such cells express mRNA and/or protein of IGSF11, and/or are positive for such IGSF11 expression and/or activity.

In another particular embodiment, the disease, disorder or condition is a proliferative disorder (or a condition associated with such disorder or disease), in particular when the product or modulating compound (such as a ABP, ABD, nucleic acid, NAC or recombinant host cell of the invention, in particular an ABP of the invention) is an *inhibitor and*/*or antagonist* of the expression, function, activity and/or stability of IGSF11 (VSIG3) or a variant of IGSF11.

A "proliferative disorder" refers to a disorder characterised by abnormal proliferation of cells. A proliferative disorder does not imply any limitation with respect to the rate of cell growth, but merely indicates loss of normal controls that affect growth and cell division. Thus, in some embodiments, cells of a proliferative disorder can have the same cell division rates as normal cells but do not respond to signals that limit such growth. Within the ambit of "proliferative disorder" is neoplasm or tumour, which is an abnormal growth of tissue or cells. Cancer is art understood, and includes any of various malignant neoplasms characterised by the proliferation of cells that have the capability to invade surrounding tissue and/or metastasise to new colonisation sites. Proliferative disorders include cancer, atherosclerosis, rheumatoid arthritis, idiopathic pulmonary fibrosis and cirrhosis of the liver. Non-cancerous proliferative disorders also include hyperproliferation of cells in the skin such as psoriasis and its varied clinical forms, Reiter's syndrome, pityriasis rubra pilaris, and hyperproliferative variants of disorders of keratinization (e.g., actinic keratosis, senile keratosis), scleroderma, and the like.

In more particular embodiments, the proliferative disorder is a cancer or tumour, in particular a solid tumour (or a condition associated with such cancer or tumour). Such proliferative disorders include, but are not limited to, head and neck cancer, squamous cell carcinoma, multiple myeloma, solitary plasmacytoma, renal cell cancer, retinoblastoma, germ cell tumors, hepatoblastoma, hepatocellular carcinoma, melanoma, rhabdoid tumor of the kidney, Ewing Sarcoma, chondrosarcoma, any haemotological malignancy (e.g., chronic lymphoblastic leukemia, chronic myelomonocytic leukemia, acute lymphoblastic leukemia, acute lymphocytic leukemia, acute myelogenous leukemia, acute myeloblasts leukemia, chronic myeloblastic leukemia, Hodgkin's disease, non- Hodgkin's lymphoma, chronic lymphocytic leukemia, chronic myelogenous leukemia, myelodysplastic syndrome, hairy cell leukemia, mast cell leukemia, mast cell neoplasm, follicular lymphoma, diffuse large cell lymphoma, mantle cell lymphoma, marginal zone lymphoma, Burkitt Lymphoma, mycosis fungoides, seary syndrome, cutaneous T-cell lymphoma, peripheral T cell lymphoma, chronic myeloproliferative disorders, myelofibrosis, myeloid metaplasia, systemic mastocytosis), and cental nervous system tumors (e.g., brain cancer, glioblastoma, non- glioblastoma brain cancer, meningioma, pituitary adenoma, vestibular schwannoma, a primitive neuroectodermal tumor, medulloblastoma, astrocytoma, anaplastic astrocytoma, oligodendroglioma, ependymoma and choroid plexus papilloma), myeloproliferative disorders (e.g., polycythemia vera, thrombocythemia, idiopathic myelofibrosis), soft tissue sarcoma, thyroid cancer, endometrial cancer, carcinoid cancer, or liver cancer.

In one preferred embodiment, the various aspects of the invention relate to, for example the ABPs of the invention used to detect/diagnose, prevent and/or treat, such proliferative disorders that include but are not limited to carcinoma (including breast cancer, prostate cancer, gastric cancer, lung cancer, colorectal and/ or colon cancer, hepatocellular carcinoma, melanoma), lymphoma (including non-Hodgkin's lymphoma and mycosis fungoides), leukemia, sarcoma, mesothelioma, brain cancer (including glioma), germinoma (including testicular cancer and ovarian cancer), choriocarcinoma, renal cancer, pancreatic cancer, thyroid cancer, head and neck cancer, endometrial cancer, cervical cancer, bladder cancer, or stomach cancer.

Accordingly, in a preferred embodiment, the proliferative disease is a cancer, for example lung cancer, breast cancer, colorectal cancer, gastric cancer, hepatocellular carcinoma, pancreatic cancer, ovarian cancer, melanoma, myeloma, kidney cancer, head and neck cancer, Hodgkin lymphoma, bladder cancer or prostate cancer, in particular one selected from the list consisting of: melanoma, lung cancer (such as non-small cell lung cancer), bladder cancer (such as urothelial carcinoma), kidney cancer (such as renal cell carcinoma), head and neck cancer (such as squamous cell cancer of the head and neck) and Hodgkin lymphoma. Preferably, the proliferative disease is melanoma, or lung cancer (such as non-small cell lung cancer).

In a particularly preferred embodiment, the disease, disorder or condition is a IGSF11-positive cancer or a cancer positive for the variant of IGSF11 and/or is a cancer characterised by the presence of VSIR positive immune cells, in particular VSIR positive monocytes and/or macrophages (in particular, TAMs) and/or is a cancer (or other proliferative disorder) characterised by being resistant and/or refractory to blockade of an immune checkpoint molecule (eg resistant and/or refractory to therapy for blockade of an immune checkpoint molecule), such as blockade using a ligand to an immune checkpoint molecule (as further described below, such as blockade of PD1/CTLA4; analogous to Gao et al, 2017). For example, in one such embodiment, the disease, disorder or condition can be a proliferative disorder (such as cancer) resistant and/or refractory to PD1/CTLA4 blockade therapy.

In a further particular embodiment, the disease, disorder or condition is an infectious disease (or a condition associated with such disorder or disease), in particular when the product or modulating compound (such as a ABP, ABD, nucleic acid, NAC or recombinant host cell of the invention, in particular an ABP of the invention) is an *inhibitor and*/*or antagonist* of the expression, function, activity and/or stability of IGSF11 (VSIG3) or a variant of IGSF11.

The term "infectious disease" is art recognised, and as used herein includes those diseases, disorders or conditions associated with (eg resulting from or caused by) by any pathogen or agent that infects mammalian cells, preferable human cells. Examples of such pathogens include bacteria, yeast, fungi, protozoans, mycoplasma, viruses, prions, and parasites. Examples of infectious disease include(a) viral diseases such as, for example, diseases resulting from infection by an adenovirus, a herpesvirus (e.g., HSV-I, HSV-II, CMV, or VZV), a poxvirus (e∼g-, an orthopoxvirus such as variola or vaccinia, or molluscum contagiosum), a picornavirus (e.g., rhinovirus or enterovirus), an orthomyxovirus (e.g., influenza virus), a paramyxovirus (e.g., parainfluenza virus, mumps virus, measles virus, and respiratory syncytial virus (RSV)), a cononavirus (e.g., SARS), a papovavirus (e.g., papillomaviruses, such as those that cause genital warts, common warts, or plantar warts), a hepadnavirus (e.g., hepatitis B virus), a flavi virus (e.g., hepatitis C virus or Dengue virus), or a retrovirus (e.g., a lentivirus such as HIV); (b) bacterial diseases such as, for example, diseases resulting from infection by bacteria of, for example, the genus Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Pneumococcus, Neisseria, Clostridium, Bacillus, Corynebacterium, Mycobacterium, Campylobacter, Vibrio, Serratia, Providencia, Chromobacterium, Brucella, Yersinia, Haemophilus, or Bordetella; (c) other infectious diseases, such chlamydia, fungal diseases including but not limited to candidiasis, aspergillosis, histoplasmosis, cryptococcal meningitis, parasitic diseases including but not limited to malaria, Pneumocystis camii pneumonia, leishmaniasis, cryptosporidiosis, toxoplasmosis, and trypanosome infection and prions that cause human disease such as Creutzfeldt-Jakob Disease (CJD), variant Creutzfeldt-Jakob Disease (vCJD), Gerstmann-Straeussler-Scheinker syndrome, Fatal Familial Insomnia and kuru.

In yet another particular embodiment, the disease, disorder or condition is one associated with an over-active or immune system or an immune system displaying undesired activity, such as autoimmunity, allergy or inflammatory conditions, in particular for allergy, autoimmunity, transplant rejection, inflammation, graft vs host disease or sepsis (or a condition associated with such diseases, disorders or conditions), in particular when the product or modulating compound (such as a ABP, ABD, nucleic acid, NAC or recombinant host cell of the invention, in particular an ABP of the invention) is an *activator and*/*or agonist* of the expression, function, activity and/or stability of IGSF11 (VSIG3) or a variant of IGSF11.

According to the medical uses and methods of treatment disclosed herein, the subject is a mammal, and may include mice, rats, rabbits, monkeys and humans. In a preferred embodiment, the mammalian subject is a human patient.

In one embodiment, cells involved in the proliferative disorder are resistant to a cell-mediated immune response. For example, cells involved in the proliferative disorder (eg cells of a cancer or tumour) are resistant and/or refractory to blockade of an immune checkpoint molecule such as blockade using a ligand to an immune checkpoint molecule, in exemplary instances blockade of PD1/CTLA4 (analogous to Gao et al, 2017).

In particular, the treatment methods may be applied to a proliferative disorder that has been subjected to prior immunotherapy (such as therapy for blockade of an immune checkpoint molecule, eg blockade of PD1/CTLA4), in particular prior immunotherapy with a ligand to an immune checkpoint molecule. For example, in certain embodiments the IGSF11 (eg antagonist) modulator, such as an ABP of the present invention, can be for use in the treatment of a proliferative disorder in a subject in need thereof, and the subject has been subjected to to prior immunotherapy, in particular prior administration of a ligand to an immune checkpoint molecule.

In other methods, the modulating (eg inhibiting) compound (eg an ABP, such as one of the present invention) may be used is in combination with a different anti-proliferative therapy, in particular a different anticancer therapy, in particular where the different anti-proliferative therapy is immunotherapy, in particular immunotherapy with a ligand to an immune checkpoint molecule. Accordingly, the composition can be for use in the treatment of a proliferative disorder in a subject in need thereof, where the subject is subjected to to co-treatment by immunotherapy, in particular co-therapy (eg combination treatment) with a ligand to an immune checkpoint molecule.

In such embodiments, the ligand is one that binds to an immune (inhibitory) checkpoint molecule. For example, such checkpoint molecule may be one selected from the group consisting of: A2AR, B7-H3, B7-H4, CTLA-4, IDO, KIR, LAG3, PD-1 (or one of its ligands PD-L1 and PD-L2), TIM-3 (or its ligand galectin-9), TIGIT and VISTA. In particular of such embodiments, the ligand binds to a checkpoint molecule selected from: CTLA-4, PD-1 and PD-L1. In other more particular embodiments, the ligand is an antibody selected from the group consisting of: ipilimumab, nivolumab, pembrolizumab, BGB-A317, atezolizumab, avelumab and durvaluma; in particular an antibody selected from the group consisting of: ipilimumab (YERVOY), nivolumab (OPDIVO), pembrolizumab (KEYTRUDA) and atezolizumab (TECENTRIQ).

When a method or use in therapy of the present invention (eg, one involving an ABP of the invention) is used in combination treatments together with any of such other procedures (eg, another agent or a cancer immunotherapy, such as a ligand that binds to an immune (inhibitory) checkpoint molecule), then such method or use being a combination treatment regimen may comprise embodiments where such exposures/administrations are concomitant. In alternative embodiments, such administrations may be sequential; in particular those embodiments where the IGSF11 modulator (eg an ABP of the invention) is administered before such other procedure. For example, such IGSF11 modulator may be sequentially administered within about 14 days of (eg before) the other procedure, such as within about 10 days, 7 days, 5 days, 2 days or 1 day of (eg before) the other procedure; and further including where the IGSF11 modulator may be sequentially administered within about 48 hours, 24 hours, 12 hours, 8 hours, 6 hours, 4 hours, 2 hours, 1 hours, 30 mins, 15 mins or 5 mins of (eg before) the other procedure.

In certain embodiments, the medical uses or compositions are for use in enhancing an immune response in the subject, preferably for use in aiding a cell-mediated immune response in the subject such as the subject's T cell mediated immune response, for example for treating a proliferative disease such as a cancer disease.

In particular embodiments, the treatment can comprise a transfer of cells to the subject, preferably a transfer of immune cells to the subject, more preferably an adoptive T-cell transfer. For example, such cells can be autologous cells of the subject, for example autologous immune cells, such as T-cells, dendritic cells or Natural Killer (NK)-cells, of the subject.

In a preferred embodiment of the medical uses or compositions, the modulating compound (eg ABP of the invention) is an inhibitor or antagonist of expression, function, activity and/or stability of said IGSF11, or the variant of IGSF11, and wherein the inhibition of the expression, function, activity and/or stability of said IGSF11, or the variant of IGSF11, enhances an immune response, preferably enhances a cell-mediated immune response in the subject such as a T-cell mediated immune response in the subject, for example for treating an infectious disease or a proliferative disease such as a cancer disease, in particular where the composition is an ABP of the invention.

In such embodiments, the immune response can be enhanced by an increase in T cell activity, proliferation and/or survival, in particular wherein the increase in T cell activity comprises an increase in production of one or more pro-inflammatory cytokines by such T cells (such as TILs). Preferably in such embodiments, the cytokine is one selected from the group consisting of: interleukin-1 (IL-1), IL-2, IL-12, IL-17, and IL-18, tumour necrosis factor (TNF) [alpha], interferon gamma (IFN-gamma), and granulocyte-macrophage colony stimulating factor, such as IL-2 (and/or IL-17 or IFN-gamma).

Such an increase in T cell activity, proliferation and/or survival, can be associated with the inhibition of the interaction between IGSF11 and VSIR, in particular mediated by IGSF11-mediated VISTA signaling, or IGSF11-variant-mediated VISTA signaling.

Administration of the modulating (eg inhibiting) compound (eg an ABP of the invention) is, in certain embodiments, associated with the inhibition of the interaction between IGSF11 and VSIR, in particular mediated by IGSF11-mediated VISTA signaling, or IGSF11-variant-mediated VISTA signaling.

In other certain embodiments, administration of the modulating compound (eg an ABP of the invention), decreases or reduces the resistance of cells (such as tumour cells and/or cells that express IGSF11, or the variant of IGSF11), to an immune response, preferably wherein the compound enhances or increases the sensitivity of cells (such as tumour cells and/or cells that express IGSF11, or the variant of IGSF11), to an immune response.

In preferred embodiments, the medical uses are for the treatment of a proliferative disorder (such as a cancer described herein) in a mammalian subject in need thereof. In certain of such embodiments, the subject is a mouse, rat, guinea pig, rabbit, cat, dog, monkey, or preferably a human, for example a human patient.

### Cells and methods of producing the ABPs/NACs of the invention

As described above, in one aspect, herein provided is a cell, such as (recombinant) host cell or a hybridoma capable of expressing an ABP as described above. In an alternative aspect, herein provided is a cell, which comprises at least one NAC encoding an ABP or a component of an ABP as described above. Cells of the invention can be used in methods provided herein to produce the ABPs and/or NACs of the invention.

In certain embodiments, the cell is isolated or substantially pure, and/or is a recombinant cell and/or is a non-natural cell (i.e., it is not found in, or is a product of, nature), such as a hybridoma.

Accordingly, in **another aspect** the invention relates to **a method of producing a recombinant cell line** capable of expressing an ABP specific for IGSF11, or for a IGSF11 variant, the method comprising the steps of:
- providing a suitable host cell;
- providing at least one genetic construct comprising coding sequence(s) encoding the ABP of the present invention;
- introducing into said suitable host cell said genetic construct(s); and
- optionally, expressing said genetic construct(s) by said suitable host cell under conditions that allow for the expression of the ABP.

In **yet another aspect,** herein provided is a **method of producing an ABP** as described above, for example comprising culturing one or more cells of the invention under conditions allowing the expression of said ABP.

Accordingly, in **another aspect,** the invention relates to **a method of producing an ABP** specific for IGSF11, or for a IGSF11 variant, the method comprising the steps of:
- providing a hybridoma or (host) cell capable of expressing an ABP according to the invention, for example a recombinant cell line comprising at least one genetic construct comprising coding sequence(s) encoding said compound or ABP; and
- culturing said hybridoma or host cell under conditions that allow for the expression of the ABP.

For producing the recombinant ABPs of the invention, the DNA molecules encoding the proteins (e.g. for antibodies, light and/or heavy chains or fragments thereof) are inserted into an expression vector (or NAC) such that the sequences are operatively linked to transcriptional and translational control sequences. Alternatively, DNA molecules encoding the ABP can be chemically synthesized. Synthetic DNA molecules can be ligated to other appropriate nucleotide sequences, including, e.g., constant region coding sequences, and expression control sequences, to produce conventional gene expression constructs encoding the desired ABP. For manufacturing the ABPs of the invention, the skilled artisan may choose from a great variety of expression systems well known in the art, e.g. those reviewed by Kipriyanow and Le Gall, 2004. Expression vectors include, but are not limited to, plasmids, retroviruses, cosmids, EBV-derived episomes, and the like. The term "expression vector" or "NAC" comprises any vector suitable for the expression of a foreign DNA. Examples of such expression vectors are viral vectors, such as adenovirus, vaccinia virus, baculovirus and adeno-associated virus vectors. In this connection, the expression "virus vector" is understood to mean both a DNA and a viral particle. Examples of phage or cosmid vectors include pWE15, M13, λEMBL3, λEMBL4, λFIXII, λDASHII, λZAPII, λgT10, λgt11, Charon4A and Charon21A. Examples of plasmid vectors include pBR, pUC, pBluescriptII, pGEM, pTZ and pET groups. Various shuttle vectors may be used, e.g., vectors which may autonomously replicate in a plurality of host microorganisms such as E. coli and Pseudomonas sp. In addition, artificial chromosome vectors are considered as expression vectors. The expression vector and expression control sequences are selected to be compatible with the cell, such as a host cell. Examples of mammalian expression vectors include, but are not limited to, pcDNA3, pcDNA3.1(+/-), pGL3, pZeoSV2(+/-), pSecTag2, pDisplay, pEF/myc/cyto, pCMV/myc/cyto, pCR3.1, pSinRepS, D H26S, D HBB, pNMT1, pNMT41, pNMT81, which are available from InvitrogenTM, pCI which is available from Promega, pMbac, pPbac, pBK-RSV and pBK-CMV which are available from Agilent Technologies, pTRES which is available from Clontech, and their derivatives.

For manufacturing antibodies, the antibody light chain gene and the antibody heavy chain gene can be inserted into separate vectors. In certain embodiments, both DNA sequences are inserted into the same expression vector. Convenient vectors are those that encode a functionally complete human CH or CL immunoglobulin sequence, with appropriate restriction sites engineered so that any VH or VL sequence can be easily inserted and expressed, as described above, wherein the CH1 and/or upper hinge region comprises at least one amino acid modification of the invention. The constant chain is usually kappa or lambda for the antibody light chain. The recombinant expression vector may also encode a signal peptide that facilitates secretion of the antibody chain from a (host) cell. The DNA encoding the antibody chain may be cloned into the vector such that the signal peptide is linked in-frame to the amino terminus of the mature antibody chain DNA. The signal peptide may be an immunoglobulin signal peptide or a heterologous peptide from a non-immunoglobulin protein. Alternatively, the DNA sequence encoding the antibody chain may already contain a signal peptide sequence.

In addition to the DNA sequences encoding the ABP (antibody) chains, the recombinant expression vectors carry regulatory sequences including promoters, enhancers, termination and polyadenylation signals and other expression control elements that control the expression of the antibody chains in a (host) cell. Examples for promoter sequences (exemplified for expression in mammalian cells) are promoters and/or enhancers derived from CMV (such as the CMV Simian Virus 40 (SV40) promoter/enhancer), adenovirus, (e.g., the adenovirus major late promoter (AdMLP)), polyoma and strong mammalian promoters such as native immunoglobulin and actin promoters. Examples for polyadenylation signals are BGH polyA, SV40 late or early polyA; alternatively, 3'UTRs of immunoglobulin genes etc. can be used.

The recombinant expression vectors may also carry sequences that regulate replication of the vector in (host) cells (e.g. origins of replication) and selectable marker genes. Nucleic acid molecules encoding the heavy chain or an antigen-binding portion thereof and/or the light chain or an antigen-binding portion thereof of an antibody of the present invention, and vectors comprising these DNA molecules can be introduced into (host) cells, e.g. bacterial cells or higher eukaryotic cells, e.g. mammalian cells, according to transfection methods well known in the art, including liposome-mediated transfection, polycation-mediated transfection, protoplast fusion, microinjections, calcium phosphate precipitation, electroporation or transfer by viral vectors.

For antibodies or fragments thereof, it is within ordinary skill in the art to express the heavy chain and the light chain from a single expression vector or from two separate expression vectors. Preferably, the DNA molecules encoding the heavy chain and the light chain are present on two vectors which are co-transfected into the (host) cell, preferably a mammalian cell

Mammalian cell lines available as hosts for expression are well known in the art and include, inter alia, Chinese hamster ovary (CHO, CHO-DG44, BI-HEX-CHO) cells, NSO, SP2/0 cells, HeLa cells, HEK293 cells, baby hamster kidney (BHK) cells, monkey kidney cells (COS), human carcinoma cells (e.g., Hep G2), A549 cells, 3T3 cells or the derivatives/progenies of any such cell line. Other mammalian cells, including but not limited to human, mice, rat, monkey and rodent cells lines, or other eukaryotic cells, including but not limited to yeast, insect and plant cells, or prokaryotic cells such as bacteria may be used. The antibody molecules of the invention are produced by culturing the host cells for a period of time sufficient to allow for expression of the antibody molecule in the host cells.

According to some embodiments of the method of producing an ABP, following expression, the intact antibody (or the antigen-binding fragment of the antibody) can be harvested and isolated using purification techniques well known in the art, e.g., Protein A, Protein G, affinity tags such as glutathione-S-transferase (GST) and histidine tags.

ABPs are preferably recovered from the culture medium as a secreted polypeptide or can be recovered from host cell lysates if for example expressed without a secretory signal. It is necessary to purify the ABP molecules using standard protein purification methods used for recombinant proteins and host cell proteins in a way that substantially homogenous preparations of the ABP are obtained. By way of example, state-of-the art purification methods useful for obtaining the ABP molecule of the invention include, as a first step, removal of cells and/or particulate cell debris from the culture medium or lysate. The ABP is then purified from contaminant soluble proteins, polypeptides and nucleic acids, for example, by fractionation on immunoaffinity or ion-exchange columns, ethanol precipitation, reverse phase HPLC, Sephadex chromatography, chromatography on silica or on a cation exchange resin. Preferably, ABPs are purified by standard protein A chromatography, e.g., using protein A spin columns (GE Healthcare). Protein purity may be verified by reducing SDS PAGE. ABP concentrations may be determined by measuring absorbance at 280nm and utilizing the protein specific extinction coefficient. As a final step in the process for obtaining an ABP molecule preparation, the purified ABP molecule may be dried, e.g. lyophilized, for therapeutic applications.

Accordingly, certain embodiments of such aspects, the method comprises a further step of isolation and/or purification of the ABP.

In another aspect, herein provided is a method of manufacturing a pharmaceutical composition comprising an ABP as described above, comprising formulating the ABP isolated by the methods described above into a pharmaceutically acceptable form.

In an alternative aspect, herein provided is a method of manufacturing a pharmaceutical composition comprising an NAC as described above, comprising formulating the NAC prepared by the methods described above into a pharmaceutically acceptable form.

According to some embodiments, the methods of manufacturing a pharmaceutical composition comprise a further step of combining said ABP and/or NAC with a pharmaceutically acceptable excipient or carrier.

In some embodiments of the method of manufacturing a pharmaceutical composition comprising an ABP, the ABP typically will be labelled with a detectable labelling group before being formulated into a pharmaceutically acceptable form. Various methods for labelling proteins are known in the art and may be used. Suitable labelling groups include, but are not limited to, the following: radioisotopes or radionuclides (e.g., 3H, 14C, 15N, 35S, 90Y, 99Tc, 111In, 1251, 1311), fluorescent groups (e.g., FITC, rhodamine, lanthanide phosphors), enzymatic groups (e.g., horseradish peroxidase, β- galactosidase, luciferase, alkaline phosphatase), chemiluminescent groups, biotinyl groups, or predetermined polypeptide epitopes recognized by a secondary reporter (e.g., leucine zipper pair sequences, binding sites for secondary antibodies, metal binding domains, epitope tags). In some embodiments, the labelling group is coupled to the ABP via spacer arms of various lengths to reduce potential steric hindrance.

Accordingly, in certain embodiments of such aspects, the ABP is a modified antibody and the method comprises a further step of addition of a functional moiety selected from a detectable labelling group or a cytotoxic moiety.

### Detection/diagnostic/monitoring methods

IGSF11 can be used for diagnostic purposes to detect, diagnose, or monitor diseases, disorders and/or conditions associated with the undesired presence of IGSF11-positive cells or cells positive for a variant of IGSF11 and/or associated with cellular resistance against a cell-mediated immune response; and in particular aberrant and/or localised expression/activity of IGSF11 (in particular phosphorylated IGSF11) can be so used. The disease, disorder and/or conditions so detected, diagnosed, or monitored, can be one of those described elsewhere herein. In preferred embodiments of the detection and diagnosis methods of the invention, the diseases, disorders or conditions is a proliferative disorder, such as cancer or tumour (eg a solid tumour), including one or more of those described elsewhere herein; more preferably one or more of lung cancer, breast cancer, colorectal cancer, pancreatic cancer, gastric cancer, hepatocellular carcinoma, ovarian cancer, melanoma, myeloma, kidney cancer, head and neck cancer, Hodgkin lymphoma, bladder cancer or prostate cancer, in particular one selected from the list consisting of: melanoma, lung cancer (such as non-small cell lung cancer), bladder cancer (such as urothelial carcinoma), kidney cancer (such as renal cell carcinoma), head and neck cancer (such as squamous cell cancer of the head and neck) and Hodgkin lymphoma. Preferably, the proliferative disease is melanoma, or lung cancer (such as non-small cell lung cancer).

Accordingly, in **a sixth aspect,** the invention related to a **method for determining** (eg an in vitro method) whether a subject has, or is at risk of, developing a phenotype (eg a disease, disorder or condition) that is associated with the undesired presence of IGSF11-positive cells or cells positive for a variant of IGSF11 and/or that is associated with cellular resistance against a cell-mediated immune response and/or that is associated with (eg aberrant) expression or activity of IGSF11 (or a variant thereof), the method comprising the step of:
- detecting (for example, detecting in vitro), eg protein and/or mRNA of IGSF11 (or a variant thereof), in particular the presence (or an amount) of or expression and/or activity of IGSF11 (or the variant), in a biological sample from said subject,
wherein the detection of IGSF11 (or the variant thereof) in the sample indicates such phenotype (eg such disease, disorder or condition), or a risk of developing such phenotype (eg such disease, disorder or condition), in the subject.

In certain embodiments of such aspect, the detection of the IGSF11 (or the variant) may comprise determining the presence or an amount of the IGSF11 (or the variant), or activity thereof, in the sample, in particular the IGSF11 (or the variant) associated with or of tumour cells (or immune cells present at the site of the tumour) of the subject. In other (alternative or further) embodiments, the detection may comprise determining the presence or an amount of one or more of other applicable biomarkers such as VSIR protein and/or mRNA.

In a preferred embodiment, protein IGSF11 (or a variant thereof) is detected with a ABP of the invention, and in an alternative embodiment mRNA IGSF11 (or a variant thereof) is detected.

In **a related aspect,** the invention relates to **a method for determining** the presence or an amount of IGSF11 (or a variant thereof) in a biological sample from a subject, the method comprising the steps of:
- contacting said sample with an ABP capable of binding to IGSF11 (or the variant); and
- detecting binding between the IGSF11 (or the variant) in the biological sample and the ABP.

In a preferred embodiment, protein IGSF11 (or a variant thereof) is detected with a ABP of the invention.

In certain embodiments, a biological sample will (preferably) comprise cells or tissue of the subject, or an extract of such cells or tissue, in particular where such cells are those involved with the proliferative disorder (eg tumour cells such as cells of a solid tumour, or immune cells present at the site of the tumour). The tumour or cell thereof, may be one or, or derived from, one of the tumours described elsewhere herein.

In particular embodiments of such aspect, the method will also comprise a step of:
- providing (such as by obtaining) the biological sample from the subject, in particular where such step is conducted prior to the detection step.

In particular embodiments, such detection and/or determination methods can be practiced as a method of diagnosis, such as a method of diagnosis whether a mammalian subject (such as a human subject or patient) has a disease, disorder or condition (such as one described above), in particular a proliferative disorder such as a cancer or tumour (or has a risk of developing such a disease, disorder or condition) that is associated with the undesired presence of IGSF11-positive cells or cells positive for a variant of IGSF11 and/or that is associated with cellular resistance against a cell-mediated immune response and/or that is associated with (eg aberrant) expression or activity of IGSF11 (or a variant thereof); in particular a (solid) tumour, such as one having cellular resistance against a cell-mediated immune response.

In certain embodiments of these detection, determination and/or diagnostic methods, the cellular resistance against a cell-mediated immune response is cellular resistance against a T cell-mediated immune response.

In certain embodiments, the biological sample is one obtained from a mammalian subject like a human patient. The term "biological sample" is used in its broadest sense and can refer to a bodily sample obtained from the subject (eg, a human patient). For example, the biological sample can include a clinical sample, i.e., a sample derived from a subject. Such samples can include, but are not limited to: peripheral bodily fluids, which may or may not contain cells, e.g., blood, urine, plasma, mucous, bile pancreatic juice, supernatant fluid, and serum; tissue or fine needle biopsy samples; tumour biopsy samples or sections (or cells thereof), and archival samples with known diagnosis, treatment and/or outcome history. Biological samples may also include sections of tissues, such as frozen sections taken for histological purposes. The term "biological sample" can also encompass any material derived by processing the sample. Derived materials can include, but are not limited to, cells (or their progeny) isolated from the biological sample, nucleic acids and/or proteins extracted from the sample. Processing of the biological sample may involve one or more of, filtration, distillation, extraction, amplification, concentration, fixation, inactivation of interfering components, addition of reagents, and the like.

In some embodiments, these detection, determination and/or diagnostic methods may be a computer-implemented method, or one that is assisted or supported by a computer. In some embodiments, information reflecting the presence or an amount of the IGSF11 (or variant thereof) to be determined (or activity thereof) in a sample is obtained by at least one processor, and/or information reflecting the presence or an amount of the IGSF11 or variant (or activity thereof) in a sample is provided in user readable format by another processor. The one or more processors may be coupled to random access memory operating under control of or in conjunction with a computer operating system. The processors may be included in one or more servers, clusters, or other computers or hardware resources, or may be implemented using cloud-based resources. The operating system may be, for example, a distribution of the Linux™ operating system, the Unix™ operating system, or other open-source or proprietary operating system or platform. Processors may communicate with data storage devices, such as a database stored on a hard drive or drive array, to access or store program instructions other data. Processors may further communicate via a network interface, which in turn may communicate via the one or more networks, such as the Internet or other public or private networks, such that a query or other request may be received from a client, or other device or service. In some embodiments, the computer-implemented method of detecting the presence or an amount of the IGSF11 or variant (or activity thereof) in a sample is provided as a kit.

Such detection, determination and/or diagnosis methods can be conducted as an in-vitro method, and can be, for example, practiced using the kit of the present invention (or components thereof).

In some embodiments of these detection, determination and/or diagnosis methods, the biological sample is a tissue sample from the subject, such as a sample of a tumour or a cancer from the subject. Such a sample may contain tumour cells and/or blood cells (eg monocytes and T cells). As described above, such tissue sample may be a biopsy sample of the tumour or a cancer such as a needle biopsy samples, or a tumour biopsy sections or an archival sample thereof. Such a tissue sample may comprise living, dead or fixed cells, such as from the tumour or a cancer, and such cells may be suspected of expressing (e.g. aberrantly or localised) the applicable biomarker to be determined.

In other embodiments of these detection, determination and/or diagnosis methods, the biological sample is a blood sample from the subject, such as a sample of immune cells present in blood (eg monocytes and T cells).

In some embodiments, determination and/or diagnosis method of the invention can comprise, such as in a further step, comparing the detected amount (or activity of) of (eg protein or mRNA of) the applicable biomarker (ie IGSF11 or a variant thereof) with a standard or cut-off value; wherein a detected amount greater than the standard or cut-off value indicates a phenotype (or a risk of developing a phenotype) that is associated with the undesired presence of IGSF11-positive cells (or cells positive for a variant of IGSF11) and/or that itassociated with cellular resistance against the cell-mediated immune response in the subject and/or is associated with (eg aberrant) expression or activity of IGSF11 (or the variant) in the subject. Such a standard or cut-off value may be determined from the use of a control assay, or may be pre-determined from one or more values obtained from a study or a plurality of samples having known phenotypes. For example, a cut-off value for a diagnostic test may be determined by the analysis of samples taken from patients in the context of a controlled clinical study, and determination of a cut-off depending on the desired (or obtained) sensitivity and/or specificity of the test.

Examples of methods useful in the detection of (such as the presence or absence of, or an amount of) the applicable biomarker (ie the IGSF11 or variant thereof) include immunoassays, such as the enzyme linked immunosorbent assay (ELISA) and the radioimmunoassay (RIA), which employ ABP (eg of the present invention) such as an antibody or an antigen-binding fragment thereof, that specifically binds to such applicable biomarker.

For such methods, a monoclonal antibody or a polyclonal antibody may be employed. Examples of monoclonal antibodies are described elsewhere herein. The term "polyclonal antibody" as used herein refers to a mixture of antibodies which are genetically different since produced by plasma cells derived from multiple somatic recombination and clonal selection events and which, typically, recognise a different epitope of the same antigen.

Alternatively, the presence of the applicable biomarker (ie IGSF11 or variant thereof) may be detected by detection of the presence of mRNA that encodes such applicable biomarker, or fragments of such mRNA. Methods to detect the presence of such mRNA (or fragments) can include, PCR (such as quantitative RT-PCR), hybridisation (such as to Illumina chips), nucleic-acid sequencing etc. Such methods may involve or comprise steps using one or more nucleic acids as described herein, such as PCR primers or PCR probes, or hybridisation probes, that bind (eg specifically) to such mRNA.

For such detection, determination or diagnostic applications, the ABP or nucleic acid, typically, will be labelled with a detectable labelling group. In general, labelling groups fall into a variety of classes, depending on the assay in which they are to be detected: a) isotopic labels, which may be radioactive or heavy isotopes; b) magnetic labels (e.g., magnetic particles); c) redox active moieties; d) optical dyes; enzymatic groups (e.g. horseradish peroxidase, beta-galactosidase, luciferase, alkaline phosphatase); e) biotinylated groups; and f) predetermined polypeptide epitopes recognised by a secondary reporter (e.g., leucine zipper pair sequences, binding sites for secondary antibodies, metal binding domains, epitope tags, etc.).Suitable labelling groups include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent groups (e.g., FITC, rhodamine, lanthanide phosphors), enzymatic groups (e.g., horseradish peroxidase, beta-galactosidase, luciferase, alkaline phosphatase), chemiluminescent groups, biotinyl groups, or predetermined polypeptide epitopes recognised by a secondary reporter (eg, leucine zipper pair sequences, binding sites for secondary antibodies, metal binding domains, epitope tags). In some embodiments, the labelling group is coupled to the ABP or nucleic acid via spacer arms of various lengths to reduce potential steric hindrance. Various methods for labelling proteins are known in the art and may be used. For example, the ABP or nucleic acid may be labelled with a secondary reporter (e.g., leucine zipper pair sequences, binding sites for secondary antibodies, metal binding domains, epitope tags, etc.).

Accordingly, in particular embodiments of the detection/diagnostic methods (or the kits therefor), the means (eg ABP or nucleic acid) for the detection (eg detector) of protein or mRNA of the applicable biomarker (eg IGSF11), is labelled, for example is coupled to a detectable label. The term "label" or "labelling group" refers to any detectable label, including those described herein.

In certain embodiments, the detection/diagnostic methods of the invention involve an immunohistochemistry (IHC) assay or an immunocytochemistry (IC) assay. The terms "IHC" and "ICC" are art recognised, and include the meanings of techniques employed to localise antigen expression that are dependent on specific epitope-antibody interactions. IHC typically refers to the use of tissue sections, whereas ICC typically describes the use of cultured cells or cell suspensions. In both methods, positive staining is typically visualised using a molecular label (eg, one which may be fluorescent or chromogenic). Briefly, samples are typically fixed to preserve cellular integrity, and then subjected to incubation with blocking reagents to prevent non-specific binding of the antibodies. Samples are subsequently typically incubated with primary (and sometimes secondary) antibodies, and the signal is visualised for microscopic analysis.

Accordingly, such embodiments of the detection/diagnostic methods of the invention may include a step of preparing a subject IHC or ICC preparation tissue or cells (such as those present in the biological samples obtained from a subject); and preferably wherein the detection of binding of an ABP to the applicable biomarker (ie IGSF11 or a variant thereof) expressed by the tissues of cells said IHC or ICC preparation indicates: (i) a phenotype such as a disease, disorder or condition (or a risk of developing such a phenotype) that is associated with the undesired presence of IGSF11-positive cells (or cells positive for a variant of IGSF11) and/or associated with cellular resistance against the cell-mediated immune response in the subject; and/or (ii) said subject has or has a risk of developing disease, condition or disorder that is associated with (eg aberrant) expression or activity of the IGSF11 or variant.

In such IHC/ICC methods is used an ABP that binds to (preferably specifically to) the applicable biomarker (ie IGSF11 or a variant thereof) and that does not bind (eg does not detectably bind) to a validation IHC or ICC preparation of mammalian tissues or cells other than to (detectably) bind to the applicable biomarker (ie IGSF11 or a variant thereof) that is expressed by the tissue cells or of said validation IHC or ICC preparation.

In certain of such embodiment, said validation and/or subject IHC or ICC preparation is one selected from the list consisting of: a frozen section, a paraffin section, and a resin section, in each case of the tissues and/or cells; and/or wherein the tissues and/or cell comprised in either (or both) said IHC or ICC preparations are fixed. The tissues and/or cells or such IHC or ICC preparation(s) may be fixed by an alcohol, an aldehyde, a mercurial agent, an oxidising agent or a picrate.

In one preferred of such embodiments, said validation and/or subject IHC or ICC preparation is a formalin-fixed paraffin embedded (FFPE) section of said tissues and/or cells; and/or wherein said validation and/or subject IHC or ICC preparation is subjected to antigen retrieval (AR). Such AR may comprise protease-induced epitope retrieval (PIER) or heat-induced epitope retrieval (HIER).

The ABP used in such methods is, preferably, validated. For example, the ABP is validated to (detectably) bind to the applicable biomarker (ie IGSF11 or a variant thereof) expressed by the cells and/or tissues of said validation IHC or ICC preparation, but does not (detectably) bind to a control IHC or ICC preparation of control cells and/or tissues that do not express such applicable biomarker. Preferably, said control cells are gene knock-down or gene knock-out cells and/or tissues for the applicable biomarker (ie IGSF11 or a variant thereof); more preferably, wherein said gene knock-down or gene knock-out cells and/or tissues are siRNA or shRNA gene knock-down or gene knock-out for such applicable biomarker. Such control cells may comprise control cells and/or tissues that do not express such applicable biomarker (ie IGSF11 or a variant thereof) comprise cells of said cell line that have been transfected with a IGSF11 siRNA selected from those of **Table A** (or transfected with a shRNA as described above); and/or said validation IHC or ICC preparation comprises cells transduced with shIGSF11 lentiviral vectors. Alternatively, the selectivity of such ABP can be determined by binding to recombinant, cell surface expressed IGSF11 or a variant thereof versus no binding to the same cell line expressing an irrelevant recombinant antigen.

In such IHC/ICC methods, the ABP is used with said validation and/or subject IHC or ICC preparation at a working concentration of less than about 50ug/mL, 25ug/mL, 20ug/mL, 15ug/mL, 10ug/mL, 7.5ug/mL, 5ug/mL, 2.5ug/mL, 1ug/mL, 0.5ug/mL, 0.2ug/ml or 0.1ug/ml, in particular less than about 5ug/mL, and more particularly at less than 2.5ug/mL; preferably, at a concentration that is about 2-fold, 5-fold, 10-fold, 20-fold or 50-fold higher than said working concentration, said ABP does not (detectable) bind to said validation immunohistochemistry (IHC) preparation of mammalian cells or tissues other than to (detectably) bind to the applicable biomarker (ie IGSF11 or a variant thereof) expressed by the mammalian cells or tissue of said IHC preparation, in particular at a concentration that is about 2-fold higher than said working concentration, and more particularly at a concentration that is about 5-fold higher than said working concentration

In the detection/diagnostic methods of the invention, the ABP used may be a polyclonal antibody; and preferably may be a rabbit antibody.

In **another aspect,** the invention relates to a **method for determining** whether a subject has, or has a risk of developing, a disease, disorder or condition that is associated with the undesired presence of IGSF11-positive cells or cells positive for a variant of IGSF11 and/or that is associated with cellular resistance against a cell-mediated immune response and/or that is associated with (eg aberrant) expression or activity of IGSF11 (or a variant thereof), the method comprising the steps of:
- contacting cells of the subject involved with the disease, disorder or condition with an (eg IGSF11 inhibitory) ABP of the invention, or a (eg IGSF11 inhibitory) product or another (eg IGSF11 inhibitory) modulating compound of the invention, in the presence of a cell-mediated immune response, preferably wherein the cell-mediated immune response comprises immune cells selected from the group consisting of: lymphocytes, T-cells, CTLs and TILs; and
- determining the cell-mediated immune response against such cells of the subject,
wherein an *enhancement* of the cell-mediated immune response against such cells of the subject indicates that the subject has or has a risk of developing such disease, disorder or condition, such as a proliferative disorder or infectious disease (preferably, a proliferative disorder such as a cancer).

In an **alternative aspect,** the invention relates to a **method for determining** whether a subject has, or has a risk of developing, a disease, disorder or condition that is associated with the undesired presence of IGSF11-positive cells or cells positive for a variant of IGSF11 and/or that is associated with (eg aberrant) expression or activity of IGSF11 (or a variant thereof), the method comprising the steps of:
- contacting cells of the subject involved with the disease, disorder or condition with an (eg IGSF11 activating) ABP of the invention, or a (eg IGSF11 activating) product or another (eg IGSF11 activating) modulating compound of the invention, in the presence of a cell-mediated immune response, preferably wherein the cell-mediated immune response comprises immune cells selected from the group consisting of: lymphocytes, T-cells, CTLs and TILs; and
- determining the cell-mediated immune response against such cells of the subject,
wherein a *reduction* of the cell-mediated immune response against such cells of the subject indicates that the subject has or has a risk of developing such disease, disorder or condition, such as autoimmunity, allergy or inflammatory conditions.

In **a related aspect,** the invention relates to **a method for determining** the resistance of a cell involved with a proliferative disease (eg a cancer or tumour) to a cell-mediated immune response, the method comprising the steps or:
- contacting such cells with an (eg IGSF11 inhibitory) ABP of the invention, or a (eg IGSF11 inhibitory) product or another (eg IGSF11 inhibitory) modulating compound of the invention, in the presence of a cell-mediated immune response, such as immune cells selected from the group consisting of: lymphocytes, T-cells, CTLs and TILs; and
- determining the cell-mediated immune response against such cells;
wherein an enhancement of the cell-mediated immune response against such cells (in the presence of the ABP of the invention or other product or modulating compound of the invention) indicates that such cells have a resistance to a cell-mediated immune response.

In certain embodiments, the cells involved with a proliferative disorder are provided as a biological sample obtained from a subject (such as a human subject or patient) that has (or has a risk of developing) a disease, disorder or condition that is associated with the undesired presence of IGSF11-positive cells (or cells positive for a variant of IGSF11) and/or associated with cellular resistance against a cell-mediated immune response and/or that is associated with (eg aberrant) expression or activity of IGSF11 or a variant thereof.

In certain embodiments, the cells of the subject contacted with the cell-mediated immune response are provided (such as by obtaining) a biological sample from the subject, wherein the sample comprises cells of the subject (such as cells of a tumour or cancer of the subject). Particular embodiments of such method also comprise a step of providing (such as by obtaining) a biological sample from the subject, in particular where such step is conducted prior to the contacting step.

In **a related aspect,** the detection, a determination and/or diagnostic method may be used as **a method for monitoring** (or prognosing) the success (or likelihood of success or risk or remission) of treatment of a subject being treated, or intended to be treated, with a treatment method of the invention. For example: (1) if the sample from the subject is determined to contain the presence of (or an indicative amount of) IGSF11 (or a variant thereof), then this indicates that (a future) treatment with a method of the invention (eg administration of an ABP of the invention) may be successful, or more likely to be successful, for such subject; and/or (2) if, during the course of such treatment (eg administration of an ABP of the invention), a reduction in (such as less than an indicative amount of), the absence of, or the functional inhibition of (eg, by monitoring the phosphorylation status), IGSF11 (or a variant thereof), or expression (or activity) thereof, is determined in the sample from the subject, then this indicates that such treatment with a method of the invention (eg administration of an ABP of the invention) is or was successful, or is more likely to be successful if continued, for such subject.

The person of ordinary skill will now readily recognise how the detection, determination and/or diagnostic methods of the present invention (and any embodiments thereof) may be practiced or modified so as to use them as part of the monitoring or prognostic methods of the invention.

In **another aspect,** the invention relates to **a method of diagnosing and treating** a disease, disorder or condition characterised by the undesired presence of IGSF11-positive cells (or cells positive for a variant of IGSF11) and/or by cellular resistance against a cell-mediated immune response and/or by (eg aberrant) expression or activity of IGSF11 (or a variant thereof), (such as a proliferative disorder, eg a tumour or cancer) in a subject, such as a human patient, comprising:
- conducting a detection, determination and/or diagnostic method of the invention (such as one described above), thereby diagnosing if the subject is suffering from such a disease, disorder or condition; and
- administering an effective amount of an ABP of the invention (or another modulating compound of the invention), and/or a pharmaceutical composition of the invention, to the so diagnosed subject, in particular practicing a treatment method of the invention on the subject.

In **yet another aspect,** the invention relates to **an ABP** binding to (preferably specifically to) protein of the applicable biomarker (ie IGSF11 or a variant thereof), or **a nucleic acid** that can bind to (such as specifically to) mRNA of such applicable biomarker, for use in diagnosis, such as in the detection of (or determination of the risk of developing) a disease, disorder or condition in a mammalian subject, such as a human patient, in particular of a disease, disorder or condition that is associated with the undesired presence of IGSF11-positive cells (or cells positive for a variant of IGSF11) and/or that is associated with cellular resistance against a cell-mediated immune response (such as a proliferative disorder, eg a tumour or cancer), and/or that is associated with (eg aberrant) expression or activity of IGSF11 or a variant thereof.

Accordingly, one embodiment of such aspect provides **a use of an ABP** that is capable of binding to or binds to (eg specifically to) IGSF11 or a variant thereof (in particular, an ABP of the invention) for/in (eg, in-vitro) diagnosis. In particular is provided an ABP (such as a monoclonal antibody) that binds to (eg specifically to) IGSF11 or a variant thereof for use in the diagnosis of a disease, disorder or condition that is associated with the undesired presence of IGSF11-positive cells (or cells positive for a variant of IGSF11) and/or that is associated with cellular resistance against a cell-mediated immune response (such as a cancer), and/or that is associated with (eg aberrant) expression or activity of IGSF11 or variant thereof.

The ABP or nucleic acid for use for such detection may be any as described elsewhere herein.

### Detection/diagnostic/monitoring kits:

In **a seventh aspect,** herein provided is **a kit,** such as one for performing the diagnostic methods or the determination methods or the detection methods (or the monitoring or prognostic methods) of the invention, eg, for determining the presence, absence, amount, function, activity and/or expression of the applicable biomarker (ie IGSF11 or a variant thereof) in a sample (eg a biological sample), such as on cells in a sample. The kit comprises an ABP and/or a nucleic acid as described above and, optionally one or more additional components.

In certain embodiments of the kit, an additional component may comprise instructions describing how to use the ABP or a nucleic acid or kit, for detecting the presence of the applicable biomarker in the sample, such as by detecting binding between the ABP and protein such applicable biomarker, and/or detecting binding between the nucleic acid and mRNA of such applicable biomarker. Such instructions may consist of a printed manual or computer readable memory comprising such instructions, or may comprise instructions as to identify, obtain and/or use one or more other components to be used together with the kit.

In other certain embodiments of the kit, the additional component may comprise one or more other claim, component, reagent or other means useful for the use of the kit or practice of a detection method of the invention, including any such claim, component, reagent or means disclosed herein useful for such practice. For example, the kit may further comprise reaction and/or binding buffers, labels, enzymatic substrates, secondary antibodies and control samples, materials or moieties etc.

In a particular such embodiment, the additional component may comprise means of detecting the presence of protein of the applicable biomarker (ie IGSF11 or a variant thereof), such as detecting binding between the ABP and such protein.

Various means for indicating (eg indictors) the binding of an ABP can be used. For example, fluorophores, other molecular probes, or enzymes can be linked to the ABP and the presence of the ABP can be observed in a variety of ways. A method for screening for diseases, disorders or conditions can involve the use of the kit, or simply the use of one of the disclosed ABPs and the determination of the extent to which ABP binds to the protein of the applicable biomarker (ie IGSF11 or a variant thereof), in a sample. As will be appreciated by one of skill in the art, high or elevated levels of protein of such applicable biomarker, will result in larger amounts of the ABP binding thereto in the sample. Thus, degree of ABP binding can be used to determine how much of such applicable biomarker is in a sample. Subjects or samples with an amount of such applicable biomarker that is greater than a predetermined amount (eg, an amount or range that a person without a disorder related to the applicable biomarker (ie IGSF11 or a variant thereof) can be characterised as having a disease, disorder or condition mediated by IGSF11 or a variant thereof (such as one mediated by the (eg aberrant) expression, function, activity and/or stability of IGSF11 or the variant), in particular of IGSF11 or the variant in tumour cells.

In some embodiments, the kit further comprises one or more of the following: standards of protein or mRNA of the applicable biomarker (ie IGSF11 or a variant thereof), positive and/or negative controls for ABP or nucleic acid binding, a vessel for collecting a sample, materials for detecting binding of the ABP or nucleic acid to protein or mRNA (as applicable) of such applicable biomarker in said sample, and reagent(s) for performing said detection.

In **another aspect** herein provided is **the use of a kit as described above** for performing the (eg in vitro) diagnostic or detection methods of the invention; and, **in a related other aspect** the invention related to a **kit as described above for use in** a (eg in vitro) determination/diagnostic method of the present invention.

As described above, kits of the invention may be accompanied by instructions, including those to use them for determining the amount, activity and/or expression of the applicable biomarker (ie IGSF11 or a variant thereof), such as in tumour cells in a sample.

### Screening methods of the invention:

In **an eighth aspect** of the invention is provided, **a method for identifying (and/or characterising)** a compound, such as a compound suitable for use in medicine (such as for the treatment of a disease, disorder or condition, eg a proliferative disorder) that is associated with the undesired presence of IGSF11-positive cells or cells positive for a variant of IGSF11 and/or that is characterised by cellular resistance against a cell-mediated immune response and/or one that is characterised by (aberrant) expression or activity of IGSF11 or a variant thereof, the method comprising the steps of:
- bringing into contact a first cell and the candidate compound, wherein the first cell expresses IGSF11 or a variant thereof (eg, a protein or mRNA of the IGSF11 or variant); and
- determining (i) the expression, activity (eg kinase activity), function and/or stability of the (eg protein or mRNA of) the IGSF11 or variant, in the first cell; and/or (ii) the cell-mediated immune response (eg the cytotoxicity or cytokine production) against the first cell,
wherein: (i) a *reduced* expression, activity function and/or stability of the IGSF11 (or variant), in said first cell contacted with the candidate compound compared to said first cell not contacted with said candidate compound; and/or (ii) an *enhancement* of the cell-mediated immune response against the first cell contacted with the candidate compound compared to the cell-mediated immune response against the first cell not contacted with the candidate compound; indicates that the candidate compound is a compound suitable for the treatment of a disease, disorder or condition such as a proliferative disorder or an infectious disease (preferably, a proliferative disorder such as a cancer); or
wherein: (i) an *enhanced* expression, activity function and/or stability of the IGSF11 (or variant), in said first cell contacted with the candidate compound compared to said first cell not contacted with said candidate compound; and/or (ii) an *reduction* of the cell-mediated immune response against the first cell contacted with the candidate compound compared to the cell-mediated immune response against the first cell not contacted with the candidate compound; indicates that the candidate compound is a compound suitable for the treatment of a disease, disorder or condition such as autoimmunity, allergy or inflammatory conditions.

In certain embodiments of such aspects, the methods also include the step of providing (such as by obtaining) the first cell and/or the candidate compound and/or (components of) the cell-mediated immune response (preferably wherein the cell-mediated immune response comprises immune cells selected from the group consisting of: lymphocytes, T-cells, CTLs and TILs), in particular where each of such steps is conducted prior to the contacting step.

The reduction (or enhancement) of expression, activity function and/or stability or IGSF11 (or variant thereof), or the enhancement (or reduction) of the cell-mediated immune response is, preferably, identified by reference to a control method. In one example, the control method may be one practiced in the absence of any candidate compound, or with compound having a known effect on such expression, function, activity and/or stability (such as a positive or negative control), and/or one practiced in the absence of (one or more components of) a cell-mediated immune response.

In particular of such embodiments, the compound having a known effect on such expression, function, activity and/or stability on the IGSF11 or the variant is an ABP of the invention (or a product or another modulating compound of the invention).

In certain embodiments of the screening method, the (components of) cell-mediated immune response is a second cell which is a cytotoxic immune cell, for example a cytotoxic T-lymphocyte (CTL), capable of immunologically recognising the first cell. Accordingly, the containing step of such embodiment comprises bringing into contact a first cell and the candidate compound and a second cell, wherein the first cell expresses IGSF11 or a variant thereof (eg, a protein or mRNA of the IGSF11 or variant) and the second cell is a cytotoxic immune cell, for example a cytotoxic T-lymphocyte (CTL), capable of immunologically recognising the first cell.

In related but alternative embodiments of the screening method, the (components of) cell-mediated immune response is a cell-free medium that has previously contained immunologically stimulated immune cells, for example cytotoxic T-lymphocytes (CTLs). Such immune cells may be stimulated by samples of the first cell and/or by polyclonal stimulants such as CD3-CD28 bead stimulation. Accordingly, the contacting step of such embodiment comprises bringing into contact a first cell and the candidate compound and a cell-free medium, wherein the first cell expresses IGSF11 (eg, a protein or mRNA of IGSF11) and the cell-free medium had previously contained immunologically stimulated immune cells, for example a cytotoxic T-lymphocyte (CTL), such as those capable of immunologically recognising the first cell.

The first cell is preferably a cell involved with a proliferative disorder (such as a tumour), eg a cell derived from a tumour. The tumour or cell thereof, may be one or, or derived from, one of the tumours described elsewhere herein.

The candidate compound used in the screening methods may be one selected from a polypeptide, peptide, glycoprotein, a peptidomimetic, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a hetero bi-functional compound such as a PROTAC or HyT molecule; a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, or small cell-permeable molecules.

In particular embodiments, the candidate compound is an ABP, such as one described elsewhere herein.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: IGSF11 (VSIG3) knockdown sensitises tumour cells towards TIL-mediated cytotoxicity.

The inventors demonstrate that knockdown of IGFS11 (VSIG3) expression and hence function/activity by inhibitory nucleic acids surprisingly causes a sensitisation of tumour cells to a cell-mediated immune response, even in a lung cancer cell that remains insensitive to tumour infiltrating lymphocytes (TIL)-mediated cytotoxicity despite knockdown of PD-L1. In the presence of TILs, the viability of lung cancer cells is significantly (P=0.0008) reduced for those cells treated with IGSF11 (VSIG3) siRNA (siGENOME, SMARTpool siRNA, Dharmacon, GE Healthcare) compared to those treated with negative control (Ctrl) siRNAs; and even treatment with PD-L1 siRNA shows no decrease in cell viability in the presence of TILs (**Figure 2A****;** CEACAM-6 siRNA as positive control), even though these cells express PD-L1 (data not shown). This observed reduction in viability was not observed in comparable experiments without TILs (**Figure 2B**), and hence sensitivity of the lung cancer cells to the cytotoxic effects of TILs is therefore significantly increased and/or enhanced by the knockdown of IGSF11 (VSIG3).

Cells from the H23 non-small cell lung cancer (NSCLC) cell line (acquired from DSMZ, Germany) were stably transfected with pEGFP-luc plasmid as described in Khandelwal et al, 2015 (EMBO Mol Med 7:450). Approximately 2,000 tumor cells per well were reverse transfected with the described siRNA type (25nM) using RNAiMAX transfection reagent (Thermo Fisher Scientific) as described before by Khandelwal et al (2015). 72 hours after siRNA transfection, the cells were co-cultured with medium alone or approximately 10,000 TILs (derived from a lung adenocarcinoma patient) for an additional 18 hours. The remaining luciferase activity associated with live tumor cells was then read out using a Tecan Spark 20M luminescence reader.

IGSF11 (VSIG3) is confirmed to be expressed in the H23 cells at the mRNA level (eg, by qPCR); at least two non-overlapping IGSF11-specific siRNAs (or the siRNA pool) are tested to confirm they induced an efficient IGSF11 (VSIG3) knockdown at mRNA level as compared to scrambled control siRNA (with data normalised to eg GAPDH). Western blot and/or flow cytometry (FC) analysis can further confirm knockdown or IGSF11 (VSIG3) at the protein level by the same IGSF11-specific individual and pool siRNAs, using anti-IGSF11 antibody (eg, as described in the further examples; or anti-IGSF11 sheep polyclonal Ab cat#: AF4915 R&D Systems) and a labelled secondary antibody (eg, respectively, APC-labelled anti-human IgG, or APC-labelled anti-sheep IgG cat#: F0127 R&D Systems). The expression level of PD-L1 and CEACAM-6 in H23 cells can analogously be confirmed at the mRNA and/or protein level after treatment with gene-specific siRNA or control siRNA. For example, for western blot analysis CEACAM6 and PD-L1 can be detected with the following antibodies: anti-CEACAM6 (Abcam, Cat No: ab98109) with anti-rabbit IgG-HRP as secondary antibody (Abcam, ab97051); anti-PD-L1 (R&D system; Cat. N. 130021), with secondary antibody: antimouse IgG-HRP (Abcam, ab6789). siRNAs which can be used to knock down IGSF11 (VSIG3), PD-L1 and/or CEACAM-6 in H23 cells, and the control siRNA are shown in **Table A.**

The sensitisation of H23 cell lines to TIL-mediated cytotoxicity upon inhibition of IGSF11 (VSIG3) expression and/or function/activity (eg by treatment with IGSF11-specific siRNAs) can be confirmed using other assays. For example, such data are generated from: (i) classical chromium release assays conducted to directly measure specific lysis of H23 cells after co-culture with (eg, patient derived) TILs, using an assay as described by Khandelwal et al (2015); or (ii) real-time live-cell microscopy (Incucyte Zoom - Essen Bioscience) for the evaluation of tumour cell death using YOYO-1 dye, and measuring (eg, after 72h of culture) the area of YOYO-1+ cells/well as a measure of cell death. In particular, the chromium release assay can be used to investigate IGSF11-mediated sensitivity to cell-based immune responses over a range of effector (E) cell to tumour (T) cell ratios, which can confirm that eg infiltrating lymphocytes show weak cytotoxic activity against tumour cells, even at high E:T ratios, when co-cultured in the absence of IGSF11 (VSIG3) knockdown. However, IGSF11 (VSIG3) down-regulation (eg, inhibition of expression and/or activity/function) can dramatically increase TIL-mediated killing of tumour cells. Such data can confirm that the increase in T cell-mediated cytotoxicity, which is dependent on on-target gene silencing of IGSF11 (VSIG3), is observed across a wide range of E:T ratios.

Alternatively, IGSF11 (VSIG3) can be knocked-down using CRISPR/CAS9 technology, briefly as follows: IGSF11-specific guide RNAs (gRNAs) are designed using the online algorithm developed by the Broad Institute (https://portals.broadinstitute.org/gpp/public/analysis-tools/sgrna-design). Approximately 50,000 tumour cells (M579-luc, A549-luc, MCF7-luc,H23-luc etc, as applicable) are reverse transfected with the 10nM ribonucleoprotein (RNP) mix, containing purified gRNA complexed with Cas9 protein (GeneArt Platinum, Invitrogen, Thermo), using the Lipofectamine RNAiMax transfection reagent (Thermo Fisher) in a 96-well plate. Non-targeting gRNAs and luc-specific gRNAs are used as negative and positive controls respectively. Cells are incubated for 2 days at 37oC followed by co-incubation with specific T cells (at 10:1 or 5:1 E:T ratio) for an additional 18 hours. Knockdown of IGSF11 expression can be confirmed at the mRNA or protein level as described above and tumour lysis induced by T cells can be measured using one or more of the assays described above, including the Luc-CTL assay as described earlier (Khandelwal et al., 2015 EMBO Mol Med).

### Example 2 (prophetic): IGSF11 (VSIG3) inhibition sensitises tumour cells of various cancer types to the anti-tumour effects of a cell-mediated immune response.

The inventors are able to demonstrate that IGSF11 (VSIG3) plays a role in the mediation of resistance to an immune response, surprisingly, in other tumour types, and not just the lung cell line used in **Example 1.** Inhibition of IGSF11 (VSIG3) eg by siRNA-based knockdown of IGSF11 (VSIG3) expression and/or function/activity in one or more of the following tumour cell lines: breast (MCF-7, MDA-MB-231, BT-474), colorectal (SW480, HTC-116), pancreatic (PANC-1), ovarian (OVCAR-3), melanoma (M579-A2), lung (A549) and myeloma (KMM1), and the subsequent challenge with survivin-specific T cells, influenza peptide-specific-T cells or (eg HLA-matched) TILs, results in significantly increased tumour cell death compared to co-culture in the absence of the T cells. Such effects can be confirmed using one or more of the assay read-out approaches described in **Example 1** (eg, luc-based, chromium-release or real-time live-cell microscopy), or other suitable assays.

The expression level of IGSF11 (VSIG3) found in the various tumour cell lines tested can be determined at the mRNA or protein level (eg by qPCR or western blot as described in **Example 1**), and IGSF11-expression correlated to the sensitivity of each tumour cell line to TIL-mediated cytotoxicity upon treatment with IGSF11-specific siRNA. It can be shown that cell lines that do not express IGSF11 (VSIG3) do not show a decrease in viability (eg, an increase in cytotoxicity/lysis) when co-cultured with TILs after treatment with IGSF11-specific siRNA. In contrast, it can be shown that cells that do express IGSF11 (VSIG3) are - typically - more sensitive to TIL-mediated cytotoxicity after treatment with IGSF11-specific siRNA.

### Example 3: Generation of antibodies that bind to human IGSF11 (VSIG3).

The inventors identified human scFv antibodies that bind human IGSF11 (VSIG3). Two universal human scFv antibody-phage libraries (Yumab GmbH, Braunschweig, Germany), consisting either solely of human kappa or lambda antibodies and comprising a diversity of more than 1x10e10 different antibody sequences, were screened for binding to the extracellular domain (ECD) of human IGSF11 (VSIG3). Conventional phage-display and panning protocols were used by Yumab in different selection strategies, each strategy comprising three rounds of selection using different variants of the biotinylated ECDs of human or murine IGSF11 protein and/or transfected HEK cells expressing human IGSF11 protein. Panning-derived hits were further selected for preferential binding to the (streptavidin-captured) positive antigens human and mouse IGSF11 compared to a negative antigen of streptavidin and/or murine-Fc domain. Binding of certain of such hits to cynomolgus monkey IGSF11 can also be tested.

scFv antibodies of the invention that selectively bind the ECD of human and murine IGSF11 protein over streptavidin and murine-Fc domain are identified and described in **Tables 1,** showing for each such antibody the heavy chain and light chain CDR sequences and variable region sequences comprised in each such antibody as well as nucleic acid sequences encoding for such variable regions (**Table 1A**)**,** and the identification of the human germline genes for the variable regions (**Table 1B**). The degree of binding of each such antibody to human and murine IGSF11 protein (and to irrelevant antigen), as determined by ELISA, and to human IGSF11 protein expressed by cells, as determined by flow cytometry (FC), is shown in **Table 2.**

**Table 1A: Amino acid sequences of CDR and variable regions of ABPs of the invention, as well as nucleic acid sequences encoding variable regions of ABPs of the invention.**

| **Antibody** | **Region** | **Sequence** | **SEQ ID NO.** |
|---|---|---|---|
| A-001 | H-CDR1 | SYSMN | 1 |
| | H-CDR2 | SISSSSSYIYYADSVKG | 2 |
| | H-CDR3 | SIIVQALGITSVFDI | 3 |
| | VH (aa) | EVQLVESGGGLVKPGGSLRLSCAASGFTFSSYSMNWVRQAPGKGLEWVSSISSSSSY IYYADSVKGRFTISRDNAKNSLYLQMNSLRAEDTAVYYCVRSIIVQALGITSVFDIWG QGTMVTVSS | 4 |
| | L-CDR1 | RASQIISNHLN | 5 |
| | L-CDR2 | AASRLQT | 6 |
| | L-CDR3 | QQSYSNPRT | 7 |
| | VL (aa) | AIRLTQSPSSLSASVGDRVTISCRASQIISNHLNWYQQKPGKAPKLLIYAASRLQTGV PSRFSGSGSETDYTLTISSLQPEDFATYYCQQSYSNPRTFGHGTKVEIK | 8 |
| | VH (DNA) | GAGGTGCAGCTGGTGGAGTCTGGGGGAGGCCTGGTCAAGCCTGGGGGGTCCCTG AGACTCTCCTGTGCAGCCTCTGGATTCACCTTCAGTAGCTATAGCATGAACTGGGT CCGCCAGGCTCCAGGGAAGGGGCTGGAGTGGGTCTCATCCATTAGTAGTAGTAGT AGTTACATATACTACGCAGACTCAGTGAAGGGCCGATTCACCATCTCCAGAGACAA CGCCAAGAACTCACTGTATCTGCAAATGAACAGCCTGAGAGCCGAGGACACGGCT GTATATTACTGTGTCAGGTCTATTATAGTTCAAGCACTTGGCATAACGTCCGTTTT TGATATCTGGGGCCAAGGGACAATGGTCACCGTCTCTTCA | 9 |
| | VL (DNA) | GCCATCCGGTTGACCCAGTCTCCATCCTCCCTGTCTGCATCTGTAGGAGACAGAGT CACCATCTCTTGCCGGGCAAGTCAGATCATTAGCAATCATTTAAATTGGTATCAGC AGAAACCAGGAAAAGCCCCTAAGCTCCTGATCTATGCTGCATCCAGATTGCAAACT GGGGTCCCATCAAGGTTCAGTGGCAGTGGCTCTGAGACAGACTACACTCTCACCA TCAGCAGTCTGCAACCTGAAGATTTTGCAACTTACTACTGTCAACAGAGTTACAGT AACCCCCGGACGTTCGGCCACGGGACCAAGGTGGAAATCAAA | 10 |
| A-002 | H-CDR1 | SNYMS | 11 |
| | H-CDR2 | VIYSGGSTYYADSVKG | 12 |
| | H-CDR3 | GNPYYYGDLQVNFDY | 13 |
| | VH (aa) | EVQLVESGGGLIQPGGSLRLSCAASGFTVSSNYMSWVRQAPGKGLEWVSVIYSGGS TYYADSVKGRFTISRDNSKNTLYLQMNSLRAEDTAVYYCARGNPYYYGDLQVNFDY WGQGTLVTVSS | 14 |
| | L-CDR1 | GSSTGAVTSGNLPN | 15 |
| | L-CDR2 | STSNKHS | 16 |
| | L-CDR3 | LLYYGGAWV | 17 |
| | VL (aa) | QAVVTQEPSLTVSPGGTVTPTCGSSTGAVTSGNLPNWFQQKPGQAPRALIYSTSNK HSWTPARISGSLLGGKAALTLSGVQPEDEAEYYCLLYYGGAWVFGGGTKLTVL | 18 |
| | VH (DNA) | GAAGTGCAGCTGGTGGAGTCTGGAGGAGGCTTGATCCAGCCTGGGGGGTCCCTG AGACTCTCCTGTGCAGCCTCTGGGTTCACCGTCAGTAGCAACTACATGAGCTGGG TCCGCCAGGCTCCAGGGAAGGGGCTGGAGTGGGTCTCAGTTATTTATAGCGGTG GTAGCACATACTACGCAGACTCCGTGAAGGGCCGATTCACCATCTCCAGAGACAAT TCCAAGAACACGCTGTATCTTCAAATGAACAGCCTGAGAGCCGAGGACACGGCCG TGTATTACTGTGCGAGAGGTAACCCATATTACTACGGTGACCTCCAGGTGAACTTT GACTACTGGGGCCAGGGAACCCTGGTCACCGTCTCCTCA | 19 |
| | VL (DNA) | CAGGCTGTGGTGACTCAGGAGCCCTCACTGACTGTGTCCCCAGGAGGGACAGTCA CTCCCACCTGTGGTTCCAGCACTGGAGCAGTCACCAGTGGTAACCTTCCAAACTG GTTCCAGCAGAAACCTGGACAAGCACCCAGGGCACTGATTTATAGTACAAGCAACA AACACTCCTGGACCCCTGCCCGGATCTCAGGCTCCCTCCTTGGGGGCAAAGCTGC CCTGACACTGTCAGGTGTGCAGCCTGAGGACGAGGCTGAATATTACTGTCTACTC TATTATGGTGGTGCTTGGGTGTTCGGCGGAGGGACCAAGCTGACCGTCCTA | 20 |
| A-003 | H-CDR1 | SYAIS | 21 |
| | H-CDR2 | GIIPIFGTANYAQKFQG | 22 |
| | H-CDR3 | PRIQLWAAGGFDY | 23 |
| | VH (aa) | VQLVQSGAEVKKPGSSVKVSCKASGGTFSSYAISWVRQAPGQGLEWMGGIIPIFGTA NYAQKFQGRVTITADESTSTAYMELSSLRSEDTAVYYCASPRIQLWAAGGFDYWGQ GTLVTVSS | 24 |
| | L-CDR1 | TGTSSDVGGYNLVS | 25 |
| | L-CDR2 | DVSNRPS | 26 |
| | L-CDR3 | SSFTTSTTLV | 27 |
| | VL (aa) | QSALTQPASVSGSPGQSITISCTGTSSDVGGYNLVSWYQQHPGKAPKLMIYDVSNRP SGVSNRFSGSKSGNTASLTISGLQAEDEADYYCSSFTTSTTLVFGGGTKLTVL | 28 |
| | VH (DNA) | GTGCAGCTGGTGCAGTCTGGGGCTGAGGTGAAGAAGCCTGGGTCCTCGGTGAAG GTCTCCTGCAAGGCTTCTGGAGGCACCTTCAGCAGCTATGCTATCAGCTGGGTGC GACAGGCCCCTGGACAAGGGCTTGAGTGGATGGGAGGGATCATCCCTATCTTTGG TACAGCAAACTACGCACAGAAGTTCCAGGGCAGAGTCACGATTACCGCGGACGAA TCCACGAGCACAGCCTACATGGAGCTGAGCAGCCTGAGATCTGAGGACACGGCCG TGTATTACTGTGCGAGCCCCAGGATACAGCTATGGGCCGCCGGGGGCTTTGACTA CTGGGGCCAGGGAACCCTGGTCACTGTCTCCTCA | 29 |
| | VL (DNA) | CAGTCTGCCCTGACTCAGCCTGCCTCCGTGTCTGGGTCTCCTGGACAGTCGATCA CCATCTCCTGCACTGGAACCAGCAGTGACGTTGGTGGTTATAACCTTGTCTCCTG GTACCAACAGCACCCAGGCAAAGCCCCCAAACTCATGATTTATGATGTCAGTAATC GGCCCTCAGGGGTTTCTAATCGCTTCTCTGGCTCCAAGTCTGGCAACACGGCCTC CCTGACCATCTCTGGGCTCCAGGCTGAGGACGAGGCTGATTATTATTGCAGCTCA TTTACAACTAGCACCACTCTAGTATTCGGCGGAGGGACCAAACTGACCGTCCTA | 30 |
| A-004 | H-CDR1 | SYSMN | 31 |
| | H-CDR2 | SISSSSSYIYYADSVKG | 32 |
| | H-CDR3 | GQLLWFGESALIDAFDI | 33 |
| | VH (aa) | EVQLVESGGGLVKPGGSLRLSCAASGFTFSSYSMNWVRQAPGKGLEWVSSISSSSSY IYYADSVKGRFTISRDNAKNSLYLQMNSLRAEDTAVYYCARGQLLWFGESALIDAFDI WGQGTMVTVSS | 34 |
| | L-CDR1 | RASQSISSYLN | 35 |
| | L-CDR2 | AASILQS | 36 |
| | L-CDR3 | QQSYSTPRT | 37 |
| | VL (aa) | EIVLTQSPSSLSASVGDRVTITCRASQSISSYLNWYQLKPGKAPKLLIYAASILQSGVP SRFSGSGSGTDFTLTISSLQPEDFAAYYCQQSYSTPRTFGQGTKLEIK | 38 |
| | VH (DNA) | GAAGTGCAGCTGGTGGAGTCTGGGGGAGGCCTGGTCAAGCCTGGGGGGTCCCTG AGACTCTCCTGTGCAGCCTCTGGATTCACCTTCAGTAGCTATAGCATGAACTGGGT CCGCCAGGCTCCAGGGAAGGGGCTGGAGTGGGTCTCATCCATTAGTAGTAGTAGT AGTTACATATACTACGCAGACTCAGTGAAGGGCCGATTCACCATCTCCAGAGACAA CGCCAAGAACTCACTGTATCTGCAAATGAACAGCCTGAGAGCCGAGGACACGGCT GTGTATTACTGTGCGAGAGGTCAATTACTATGGTTCGGGGAGTCAGCACTGATTG ATGCTTTTGATATCTGGGGCCAAGGGACAATGGTCACCGTCTCTTCA | 39 |
| | VL (DNA) | GAAATTGTGTTGACGCAGTCTCCATCCTCCCTGTCTGCATCTGTAGGAGACAGAG TCACCATCACTTGCCGGGCAAGTCAGAGCATTAGCAGCTATTTAAATTGGTATCAG CTGAAACCAGGGAAAGCCCCTAAACTCCTGATCTATGCTGCATCCATTCTGCAAAG TGGGGTCCCATCAAGGTTCAGTGGCAGTGGATCTGGGACAGATTTCACTCTCACC ATCAGCAGTCTGCAACCTGAAGATTTTGCAGCTTACTACTGTCAACAGAGTTACAG TACCCCTCGGACTTTTGGCCAGGGGACCAAGCTGGAGATCAAA | 40 |
| A-005 | H-CDR1 | SNYMS | 41 |
| | H-CDR2 | VIYSGGSTYYADSVKG | 42 |
| | H-CDR3 | GNPYYYGDLQVNFDY | 43 |
| | VH (aa) | EVQLVESGGGLIQPGGSLRLSCAASGFTVSSNYMSWVRQAPGKGLEWVSVIYSGGS TYYADSVKGRFTISRDNSKNTLYLQMNSLRAEDTAVYYCARGNPYYYGDLQVNFDY WGQGTLVTVSS | 44 |
| | L-CDR1 | ASSTGAVTSGYYPN | 45 |
| | L-CDR2 | STSNKDS | 46 |
| | L-CDR3 | LLYYGGAWV | 47 |
| | VL (aa) | QAVVTQEPSLTVSPGETVTLTCASSTGAVTSGYYPNWFQQRPGQAPRALIYSTSNKD SWTPARFSGSLLGGKAALTLSGVQPEDEAEYYCLLYYGGAWVFGGGTKLTVL | 48 |
| | VH (DNA) | GAGGTGCAGCTGGTGGAGTCTGGAGGAGGCTTGATCCAGCCTGGGGGGTCCCTG AGACTCTCCTGTGCAGCCTCTGGGTTCACCGTCAGTAGCAACTACATGAGCTGGG TCCGCCAGGCTCCAGGGAAGGGGCTGGAGTGGGTCTCAGTTATTTATAGCGGTG GTAGCACATACTACGCAGACTCCGTGAAGGGCCGATTCACCATCTCCAGAGACAAT TCCAAGAACACGCTGTATCTTCAAATGAACAGCCTGAGAGCCGAGGACACGGCCG TGTATTACTGTGCGAGAGGTAACCCATATTACTACGGTGACCTCCAGGTGAACTTT GACTACTGGGGCCAGGGAACCCTGGTCACCGTCTCCTCA | 49 |
| | VL (DNA) | CAGGCTGTGGTGACTCAGGAGCCCTCACTGACTGTGTCCCCAGGAGAGACGGTCA CTCTCACCTGTGCTTCCAGCACTGGAGCAGTCACCAGTGGTTACTATCCAAACTGG TTCCAGCAGAGACCTGGACAAGCACCCAGGGCACTGATTTATAGTACAAGCAACAA AGACTCCTGGACCCCTGCCCGGTTCTCAGGCTCCCTCCTTGGGGGCAAAGCTGCC CTGACACTGTCAGGTGTGCAGCCTGAGGACGAGGCTGAGTATTACTGCCTGCTCT ACTATGGTGGTGCTTGGGTGTTCGGCGGAGGGACCAAGCTGACCGTCCTA | 50 |
| A-006 | H-CDR1 | SNYMS | 51 |
| | H-CDR2 | VIYSGGSTYYADSVKG | 52 |
| | H-CDR3 | GNPYYYGDLQVNFDY | 53 |
| | VH (aa) | EVQLVESGGGLIQPGGSLRLSCAASGFTVSSNYMSWVRQAPGKGLEWVSVIYSGGS TYYADSVKGRFTISRDNSKNTLYLQMNSLRAEDTAVYYCARGNPYYYGDLQVNFDY WGQGTLVTVSS | 54 |
| | L-CDR1 | ASSTGAVTSGYYPN | 55 |
| | L-CDR2 | STSNKHS | 56 |
| | L-CDR3 | LLYYGGDWV | 57 |
| | VL (aa) | QAVVTQEPSLTVSPGGTVTLTCASSTGAVTSGYYPNWFQQKPGQAPRPLIYSTSNKH SWTPARFSGSLLGGKAALTLSGVQPDDEAEYYCLLYYGGDWVFGGGTKLTVL | 58 |
| | VH (DNA) | GAGGTGCAGCTGGTGGAGTCTGGAGGAGGCTTGATCCAGCCTGGGGGGTCCCTG AGACTCTCCTGTGCAGCCTCTGGGTTCACCGTCAGTAGCAACTACATGAGCTGGG TCCGCCAGGCTCCAGGGAAGGGGCTGGAGTGGGTCTCAGTTATTTATAGCGGTG GTAGCACATACTACGCAGACTCCGTGAAGGGCCGATTCACCATCTCCAGAGACAAT TCCAAGAACACGCTGTATCTTCAAATGAACAGCCTGAGAGCCGAGGACACGGCCG TGTATTACTGTGCGAGAGGTAACCCATATTACTACGGTGACCTCCAGGTGAACTTT GACTACTGGGGCCAGGGAACCCTGGTCACCGTCTCCTCA | 59 |
| | VL (DNA) | CAGGCTGTGGTGACTCAGGAGCCCTCACTGACTGTGTCCCCAGGAGGGACAGTCA CTCTCACCTGTGCTTCCAGCACTGGAGCAGTCACCAGTGGTTACTATCCAAACTGG TTCCAGCAGAAACCTGGACAAGCACCCAGGCCACTGATTTATAGTACAAGCAACAA ACACTCCTGGACCCCTGCCCGGTTCTCAGGCTCCCTCCTTGGGGGCAAAGCTGCC CTGACACTGTCAGGTGTGCAGCCTGACGACGAGGCTGAATATTACTGCCTGCTCT ACTATGGTGGTGACTGGGTGTTCGGCGGAGGGACCAAGCTGACCGTCCTA | 60 |
| A-007 | H-CDR1 | GYYMH | 61 |
| | H-CDR2 | WINPNSGGTNYAQKFQG | 62 |
| | H-CDR3 | GSNFDY | 63 |
| | VH (aa) | QVQLVQSGAEVKKPGASVKVSCKASGYTFTGYYMHWVRQAPGQGLEWMGWINPN SGGTNYAQKFQGRVTMTRDTSISTAYMELSRLRSDDTAVYYCAAGSNFDYWGQGTL VTVSS | 64 |
| | L-CDR1 | TGSSSDIGSFSYVS | 65 |
| | L-CDR2 | GVNNRPL | 66 |
| | L-CDR3 | SSYTRRSTVI | 67 |
| | VL (aa) | QSALTQPASVSGSPGQSITIACTGSSSDIGSFSYVSWYQQRPGKAPTLIIYGVNNRPL GVSRRFSGSKSGNTASLSISGLQAEDEADYYCSSYTRRSTVIFGGGTKLTVL | 68 |
| | VH (DNA) | CAGGTTCAGCTGGTGCAGTCTGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGATGGATCAACCCTAACAGT GGTGGCACAAACTATGCACAGAAGTTTCAGGGCAGGGTCACCATGACCAGGGACA CGTCCATCAGCACAGCCTACATGGAGCTGAGCAGGCTGAGATCTGACGACACGGC CGTGTATTACTGTGCTGCGGGGTCTAACTTTGACTACTGGGGCCAGGGAACCCTG GTCACCGTCTCCTCA | 69 |
| | VL (DNA) | CAGTCTGCCCTGACTCAGCCTGCCTCCGTGTCTGGGTCTCCGGACAATCGATCA CCATCGCCTGCACTGGAAGCAGTAGTGACATTGGTAGTTTTTCTTATGTCTCCTGG TACCAACAGCGCCCCGGCAAAGCCCCCACACTCATCATTTATGGGGTCAATAATCG ACCCTTAGGGGTGTCTCGGCGCTTCTCTGGCTCCAAGTCTGGCAACACGGCCTCC CTAAGCATCTCTGGGCTCCAGGCTGAGGACGAGGCTGATTATTACTGCAGTTCCT ATACACGCAGAAGCACTGTGATCTTCGGCGGCGGGACCAAGTTGACCGTCCTA | 70 |
| A-008 | H-CDR1 | SSGYYWG | 71 |
| | H-CDR2 | SIYYSGSTYYNPSLKS | 72 |
| | H-CDR3 | HRVRFGEFDAFDI | 73 |
| | VH (aa) | QLQLQESGPGLVKPSETLSLTCTVSGGSISSSGYYWGWIRQPPGKGLEWIGSIYYSG STYYNPSLKSRVTISVDTSKNQFSLKLSSVTAADTAVYYCARHRVRFGEFDAFDIWG QGTMVTVSS | 74 |
| | L-CDR1 | TGTSSNVGADFDVH | 75 |
| | L-CDR2 | GSDNRPS | 76 |
| | L-CDR3 | QAYDVRLSGWV | 77 |
| | VL (aa) | QAVLTQPPSVSGAPGERVTLSCTGTSSNVGADFDVHWYQQFPGTAPRLLIFGSDNRP SGVPDRFSGSKSGTSASLAITGLQVEDEADYYCQAYDVRLSGWVFGGGTKLTVL | 78 |
| | VH (DNA) | CAGCTGCAGCTGCAGGAGTCGGGCCCAGGACTGGTGAAGCCTTCGGAGACCCTGT CCCTCACCTGCACCGTCTCTGGTGGCTCCATCAGCAGTAGTGGTTACTACTGGGG CTGGATCCGCCAGCCCCCAGGGAAGGGGCTGGAGTGGATTGGGAGTATCTATTAT AGTGGGAGCACCTACTACAACCCGTCCCTCAAGAGTCGAGTCACCATATCCGTAGA CACGTCTAAGAACCAGTTCTCCCTGAAGCTGAGCTCTGTGACCGCCGCAGACACG GCTGTGTATTACTGTGCGAGACATAGGGTACGGTTCGGGGAGTTCGATGCTTTTG ATATCTGGGGCCAAGGGACAATGGTCACCGTCTCTTCA | 79 |
| | VL (DNA) | CAGGCTGTGCTGACTCAGCCACCCTCAGTGTCTGGGGCCCCGGAGAGAGGGTCA CCCTCTCTTGTACAGGGACCAGCTCGAACGTCGGGGCAGATTTTGATGTACATTG GTACCAGCAGTTTCCAGGAACAGCCCCCAGACTCCTCATCTTTGGTTCCGACAATC GGCCCTCAGGAGTCCCTGACCGATTCTCTGGCTCCAAGTCTGGCACCTCAGCCTC CCTGGCCATCACTGGGCTCCAGGTTGAGGATGAGGCTGATTATTATTGCCAGGCT TATGACGTCAGGCTGAGTGGCTGGGTGTTCGGCGGGGGGACCAAGCTGACCGTC CTA | 80 |
| A-009 | H-CDR1 | SYAIS | 81 |
| | H-CDR2 | GIIPIFGTANYAQKFQG | 82 |
| | H-CDR3 | GRGFGELYFDY | 83 |
| | VH (aa) | QVQLVQSGAEVKKPGSSVKVSCKASGGTFSSYAISWVRQAPGQGLEWMGGIIPIFGT ANYAQKFQGRVTITADESTSTAYMELSSLRSEDTAVYYCARGRGFGELYFDYWGQG TLVTVSS | 84 |
| | L-CDR1 | RASQGVRSNIA | 85 |
| | L-CDR2 | DSSTRAT | 86 |
| | L-CDR3 | QQYKNWPRT | 87 |
| | VL (aa) | ETTLTQSPATLSVSPGERATLSCRASQGVRSNIAWYQQKPGQAPRLLIYDSSTRATGI PARFSGSGSGTEFTLTISSLQSEDFAVYSCQQYKNWPRTFGQGTKVEIK | 88 |
| | VH (DNA) | CAGGTTCAGCTGGTGCAGTCTGGGGCTGAGGTGAAGAAGCCTGGGTCCTCGGTG AAGGTCTCCTGCAAGGCTTCTGGAGGCACCTTCAGCAGCTATGCTATCAGCTGGG TGCGACAGGCCCCTGGACAAGGGCTTGAGTGGATGGGAGGGATCATCCCTATCTT TGGTACAGCAAACTACGCACAGAAGTTCCAGGGCAGAGTCACGATTACCGCGGAC GAATCCACGAGCACAGCCTACATGGAGCTGAGCAGCCTGAGATCTGAGGACACGG CCGTGTATTACTGTGCGAGAGGGCGAGGGTTCGGGGAGTTATACTTTGACTACTG GGGCCAGGGAACCCTGGTCACCGTCTCCTCA | 89 |
| | VL (DNA) | GAAACGACACTCACGCAGTCTCCAGCCACCCTGTCTGTGTCTCCAGGGGAAAGAG CCACCCTCTCCTGCAGGGCCAGTCAGGGTGTTAGAAGCAATATAGCCTGGTACCA GCAGAAACCTGGCCAGGCTCCCAGGCTCCTCATCTATGATTCATCCACCAGGGCCA CTGGTATCCCAGCCAGGTTCAGTGGCAGTGGGTCTGGGACAGAGTTCACTCTCAC CATCAGCAGCCTGCAGTCTGAAGATTTTGCAGTTTATTCCTGTCAGCAGTATAAGA ACTGGCCTCGGACGTTCGGCCAAGGGACCAAGGTGGAAATCAAA | 90 |
| A-010 | H-CDR1 | SYGIS | 91 |
| | H-CDR2 | WISAYNGNTNYAQKLQG | 92 |
| | H-CDR3 | VPAWSGQFDY | 93 |
| | VH (aa) | QVQLVQSGAEVKKPGASVKVSCKASGYTFTSYGISWVRQAPGQGLEWMGWISAYN GNTNYAQKLQGRVTMTTDTSTSTAYMELRSLRSDDTAVYYCARVPAWSGQFDYWG QGTLVTVSS | 94 |
| | L-CDR1 | TGSSSDVGGYNYVS | 95 |
| | L-CDR2 | DVRSRPS | 96 |
| | L-CDR3 | TSYTSSNTLVI | 97 |
| | VL (aa) | QSALTQPASVSGSPGQSITISCTGSSSDVGGYNYVSWYQQYPGKAPKLMIYDVRSRP SGVSNRFSGSKSGNTASLTISGLQAEDEADYYCTSYTSSNTLVIFGGGTKVTVL | 98 |
| | VH (DNA) | CAGGTTCAGCTGGTGCAGTCTGGAGCTGAGGTGAAGAAGCCTGGGGCCTCAGTG AAGGTCTCCTGCAAGGCTTCTGGTTACACCTTTACCAGCTATGGTATCAGCTGGGT GCGACAGGCCCCTGGACAAGGGCTTGAGTGGATGGGATGGATCAGCGCCTACAAT GGTAACACAAACTATGCACAGAAGCTCCAGGGCAGAGTCACCATGACCACAGACA CATCCACGAGCACAGCCTACATGGAGCTGAGGAGCCTGAGATCTGACGACACGGC CGTGTATTACTGTGCGAGAGTCCCTGCGTGGAGTGGTCAATTTGACTACTGGGGC CAGGGAACCCTGGTCACCGTCTCCTCA | 99 |
| | VL (DNA) | CAGTCTGCCCTGACTCAGCCTGCCTCCGTGTCTGGGTCTCCTGGACAGTCGATCA CCATCTCCTGCACTGGATCCAGCAGTGACGTTGGTGGTTATAACTATGTCTCCTGG TACCAGCAGTACCCAGGCAAAGCCCCCAAACTCATGATTTATGATGTCAGAAGTCG GCCCTCAGGGGTTTCTAATCGCTTCTCTGGCTCCAAGTCTGGCAACACGGCCTCCC TGACCATCTCTGGACTCCAGGCTGAGGACGAGGCTGATTATTACTGCACCTCATAT ACAAGCAGCAACACTCTTGTGATATTCGGCGGAGGGACCAAGGTGACCGTCCTA | 100 |
| A-011 | H-CDR1 | SYAMH | 101 |
| | H-CDR2 | VISYDGSNKYYADSVKG | 102 |
| | H-CDR3 | GGALNYYGMDV | 103 |
| | VH (aa) | QVQLVESGGGVVQPGRSLRLSCAASGFTFSSYAMHWVRQAPGKGLEWVAVISYDGS NKYYADSVKGRFTISRDNSKNTLYLQMNSLRAEDTAVYYCAEGGALNYYGMDVWG QGTTVTVSS | 104 |
| | L-CDR1 | QGDSLRHFYAT | 105 |
| | L-CDR2 | GKNNRPS | 106 |
| | L-CDR3 | QSRDPRNNHLI | 107 |
| | VL (aa) | SSELTQDPGVSVALGQTVRITCQGDSLRHFYATWYQQKPGQAPILVIYGKNNRPSGI PDRFSGSSSEDTASLTITGAQAEDEADYYCQSRDPRNNHLIFGGGTKLIVL | 108 |
| | VH (DNA) | CAGGTGCAGCTGGTGGAGTCTGGGGGAGGCGTGGTCCAGCCTGGGAGGTCCCTG AGACTCTCCTGTGCAGCCTCTGGATTCACCTTCAGTAGCTATGCTATGCACTGGGT CCGCCAGGCTCCAGGCAAGGGGCTGGAGTGGGTGGCAGTTATATCATATGATGGA AGCAATAAATACTACGCAGACTCCGTGAAGGGCCGATTCACCATCTCCAGAGACAA TTCCAAGAACACGCTGTATCTGCAAATGAACAGCCTGAGAGCTGAGGACACGGCT GTGTATTACTGTGCGGAGGGCGGAGCGTTGAACTACTACGGTATGGACGTCTGGG GCCAAGGGACCACGGTCACCGTCTCCTCA | 109 |
| | VL (DNA) | TCTTCTGAGCTGACTCAGGACCCTGGTGTGTCTGTGGCCTTGGGACAGACAGTCA GGATCACATGCCAGGGAGACAGCCTCAGACACTTTTATGCAACCTGGTACCAGCA GAAGCCAGGACAGGCCCCTATACTTGTCATCTATGGTAAAAACAACCGGCCCTCAG GGATCCCAGACCGATTCTCTGGCTCCAGTTCTGAAGACACAGCCTCCTTGACCATC ACTGGGGCTCAGGCGGAGGATGAGGCTGACTACTACTGTCAATCCCGGGACCCCA GGAATAACCATTTGATTTTCGGCGGCGGGACCAAACTGATCGTCCTC | 110 |
| A-012 | H-CDR1 | SNYMS | 111 |
| | H-CDR2 | VIYSGGSTYYADSVKG | 112 |
| | H-CDR3 | GNPYYYGDLQVNFDY | 113 |
| | VH (aa) | EVQLLESGGGLIQPGGSLRLSCAVSGFTVSSNYMSWVRQAPGKGLEWVSVIYSGGST YYADSVKGRFTISRDNSKNTLYLQMNSLRAEDTAVYYCARGNPYYYGDLQVNFDYW GQGTLVTVSS | 114 |
| | L-CDR1 | ASSTGAVTSGYYPN | 115 |
| | L-CDR2 | STSNKHS | 116 |
| | L-CDR3 | LLYYGGAWV | 117 |
| | VL (aa) | QAVVTQEPSLTVSPGGTVTLTCASSTGAVTSGYYPNWFQQKPGQAPRALIYSTSNKH SWTPARFSGSLLGGKAALTLSGVQPEDEAEYYCLLYYGGAWVFGGGTKLTVL | 118 |
| | VH (DNA) | GAGGTGCAGCTGTTGGAGTCTGGAGGAGGCTTGATCCAGCCTGGGGGGTCCCTG AGACTCTCCTGTGCAGTCTCTGGGTTCACCGTCAGTAGCAACTACATGAGCTGGG TCCGCCAGGCTCCAGGGAAGGGGCTGGAGTGGGTCTCAGTTATTTATAGCGGTG GTAGCACATACTACGCAGACTCCGTGAAGGGCCGATTCACCATCTCCAGAGACAAT TCCAAGAACACGCTGTATCTTCAAATGAACAGCCTGAGAGCCGAGGACACGGCCG TGTATTACTGTGCGAGAGGTAACCCATATTACTACGGTGACCTCCAGGTGAACTTT GACTACTGGGGCCAGGGAACCCTGGTCACCGTCTCCTCA | 119 |
| | VL (DNA) | CAGGCTGTGGTGACTCAGGAGCCCTCACTGACTGTGTCCCCAGGAGGGACAGTCA CTCTCACCTGTGCTTCCAGCACTGGAGCAGTCACCAGTGGTTACTATCCAAACTGG TTCCAGCAGAAACCTGGACAAGCACCCAGGGCACTGATTTATAGTACAAGCAACAA ACACTCCTGGACCCCTGCCCGGTTCTCAGGCTCCCTCCTTGGGGGCAAAGCTGCC CTGACACTGTCAGGTGTGCAGCCTGAGGACGAGGCTGAGTATTACTGCCTGCTCT ACTATGGTGGTGCTTGGGTGTTCGGCGGAGGGACCAAGCTGACCGTCCTA | 120 |
| A-013 | H-CDR1 | SNYMS | 121 |
| | H-CDR2 | VIYSGGSTYYADSVKG | 122 |
| | H-CDR3 | GNPYYYGDLQVNFDY | 123 |
| | VH (aa) | EVQLVESGGGLIQPGGSLRLSCAASGFTVSSNYMSWVRQAPGKGLEWVSVIYSGGS TYYADSVKGRFTISRDNSKNTLYLQMNSLRAEDTAVYYCARGNPYYYGDLQVNFDY WGQGTLVTVSS | 124 |
| | L-CDR1 | ASSTGAVTSAYYPN | 125 |
| | L-CDR2 | STSNKHS | 126 |
| | L-CDR3 | LLYYGGAWV | 127 |
| | VL (aa) | QAVVTQEPSLTVSPGGTVTLTCASSTGAVTSAYYPNWFQQKPGQAPRALIYSTSNKH SWTPARFSGSLLGGKAALTLSGVQPEDEADYYCLLYYGGAWVFGGGTKLTVL | 128 |
| | VH (DNA) | GAAGTGCAGCTGGTGGAGTCTGGAGGAGGCTTGATCCAGCCTGGGGGGTCCCTG AGACTCTCCTGTGCAGCCTCTGGGTTCACCGTCAGTAGCAACTACATGAGCTGGG TCCGCCAGGCTCCAGGGAAGGGGCTGGAGTGGGTCTCAGTTATTTATAGCGGTG GTAGCACATACTACGCAGACTCCGTGAAGGGCCGATTCACCATCTCCAGAGACAAT TCCAAGAACACGCTGTATCTTCAAATGAACAGCCTGAGAGCCGAGGACACGGCCG TGTATTACTGTGCGAGAGGTAACCCATATTACTACGGTGACCTCCAGGTGAACTTT GACTACTGGGGCCAGGGAACCCTGGTCACCGTCTCCTCA | 129 |
| | VL (DNA) | CAGGCTGTGGTGACTCAGGAGCCCTCACTGACTGTGTCCCCAGGAGGGACAGTCA CTCTCACCTGTGCTTCCAGCACTGGAGCAGTCACCAGTGCTTACTATCCAAACTGG TTCCAGCAGAAACCTGGACAAGCACCCAGGGCACTGATTTATAGTACAAGCAACAA ACACTCCTGGACCCCTGCCCGGTTCTCAGGCTCCCTCCTTGGGGGCAAAGCTGCC CTGACACTGTCAGGTGTGCAGCCTGAGGACGAGGCTGACTATTACTGCCTGCTCT ACTATGGTGGTGCTTGGGTGTTCGGCGGAGGGACCAAGCTGACCGTCCTA | 130 |
| A-014 | H-CDR1 | DYYIH | 131 |
| | H-CDR2 | WFNPSTGGANYAQKFQG | 132 |
| | H-CDR3 | GNSPDLDY | 133 |
| | VH (aa) | QVQLVQSGAEVKKPGASVKVSCKASGYTFNDYYIHWVRQAPGQGLEWMGWFNPST GGANYAQKFQGRVTMTRDTSISTAYLEVSSLRSDDTAVYYCARGNSPDLDYWGQGT LVTVSS | 134 |
| | L-CDR1 | KSSQSLLHSSNNKNYLA | 135 |
| | L-CDR2 | WASTRQS | 136 |
| | L-CDR3 | QQYYTTTPNT | 137 |
| | VL (aa) | DIQMTQSPDSLTVSLGERATINCKSSQSLLHSSNNKNYLAWYQQRPGQPPKLLIYWA STRQSGVPDRFSGSGSGTDFTLTINSLQADDMAVYYCQQYYTTTPNTFGQGTKLEIK | 138 |
| | VH (DNA) | CAAGTCCAGCTGGTGCAGTCTGGGGCTGAGGTGAAGAAGCCTGGGGCCTCAGTG AAGGTCTCCTGCAAGGCTTCTGGATACACCTTCAACGACTACTATATACACTGGGT GCGGCAGGCCCCTGGACAAGGGCTTGAGTGGATGGGGTGGTTCAACCCTAGCACT GGTGGCGCAAATTATGCACAGAAGTTTCAGGGCAGGGTCACCATGACCAGGGACA CGTCCATCAGCACAGCCTACTTGGAAGTGAGCAGCCTGAGATCTGACGACACGGC CGTGTATTACTGTGCGAGAGGTAATAGCCCGGACCTTGACTACTGGGGCCAGGGA ACCCTGGTCACCGTCTCCTCA | 139 |
| | VL (DNA) | GACATCCAGATGACCCAGTCTCCAGACTCCCTGACTGTGTCTCTGGGCGAGAGGG CCACCATCAACTGCAAGTCGAGCCAGAGTCTTTTACACAGCTCCAACAATAAGAAT TACTTGGCTTGGTACCAGCAGAGACCAGGACAGCCTCCTAAACTGCTCATTTACTG GGCATCCACCCGGCAATCCGGGGTCCCGGACCGCTTCAGTGGCAGCGGGTCTGG GACAGATTTCACTCTCACCATCAACAGCCTGCAGGCTGACGACATGGCAGTTTATT ACTGCCAGCAGTATTATACTACTACTCCGAACACTTTTGGCCAGGGGACCAAGCTG GAGATCAAA | 140 |
| A-015 | H-CDR1 | GYYMH | 141 |
| | H-CDR2 | WINPNSGGTNYAQKFQG | 142 |
| | H-CDR3 | DPDGSGGSSRWFDP | 143 |
| | VH (aa) | QVQLVQSGAEVKKPGASVKVSCKASGYTFTGYYMHWVRQAPGQGLEWMGWINPN SGGTNYAQKFQGRVTMTRDTSISTAYMELSRLRSDDTAVYYCARDPDGSGGSSRWF DPWGQGTLVTVSS | 144 |
| | L-CDR1 | TLSSGHTNYAIA | 145 |
| | L-CDR2 | LNSDGSHTRGG | 146 |
| | L-CDR3 | MIWHNNAWV | 147 |
| | VL (aa) | QPVLTQSPSASASLGASVKLTCTLSSGHTNYAIAWRQQQPGKAPRYLMLLNSDGSHT RGGGIPDRFSGSSSGAERYLTISSLQSEDEADYYCMIWHNNAWVFGGGTKLAVL | 148 |
| | VH (DNA) | CAAGTGCAGCTGGTGCAGTCTGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGATGGATCAACCCTAACAGT GGTGGCACAAACTATGCACAGAAGTTTCAGGGCAGGGTCACCATGACCAGGGACA CGTCCATCAGCACAGCCTACATGGAGCTGAGCAGGCTGAGATCTGACGACACGGC CGTGTATTACTGTGCGAGAGATCCTGATGGGAGTGGTGGTAGTTCCCGGTGGTTC GACCCCTGGGGCCAGGGAACCCTGGTCACCGTCTCCTCA | 149 |
| | VL (DNA) | CAGCCTGTGCTGACTCAATCGCCCTCTGCCTCTGCCTCCCTGGGAGCCTCGGTCA AGCTCACCTGCACTCTGAGCAGTGGGCACACCAACTACGCCATCGCTTGGCGTCA GCAACAGCCTGGGAAGGCCCCTCGATATTTGATGCTGCTTAACAGTGATGGCAGC CACACGAGGGGGGGCGGATCCCTGATCGCTTCTCAGGCTCCAGTTCTGGGGCTG AGCGCTACCTCACCATCTCCAGCCTCCAGTCTGAGGATGAGGCTGACTATTACTGT ATGATTTGGCACAACAACGCTTGGGTGTTCGGCGGAGGGACCAAGCTGGCCGTCC TT | 150 |
| A-016 | H-CDR1 | GYYMH | 151 |
| | H-CDR2 | GIIPIFGTANYAQKFQG | 152 |
| | H-CDR3 | AGMELTRSGAYYYYGMDV | 153 |
| | VH (aa) | QVQLQESGAEVKKPGASVKVSCKASGYTFTGYYMHWVRQAPGQGLEWMGGIIPIFG TANYAQKFQGRVTITADESTSTAYMELSSLRSEDTAVYYCAGAGMELTRSGAYYYYG MDVWGQGTTVTVSS | 154 |
| | L-CDR1 | TGTSSDIGGYTFVS | 155 |
| | L-CDR2 | DVNNRPS | 156 |
| | L-CDR3 | SSVTSTNTYV | 157 |
| | VL (aa) | QSALTQPASVSGSPGQSITISCTGTSSDIGGYTFVSWYQQHPGKAPKVMIHDVNNRP SGISNRFSGSKSGNTASLTISGLQAEDEADYYCSSVTSTNTYVFGTGTKVTVL | 158 |
| | VH (DNA) | CAGGTGCAGCTGCAGGAGTCGGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGAGGGATCATCCCTATCTTT GGTACAGCAAACTACGCACAGAAGTTCCAGGGCAGAGTCACGATTACCGCGGACG AATCCACGAGCACAGCCTACATGGAGCTGAGCAGCCTGAGATCTGAGGACACGGC CGTGTATTACTGTGCCGGGGCGGGGATGGAGCTTACTCGCTCGGGTGCTTACTAC TACTACGGTATGGACGTCTGGGGCCAAGGGACCACGGTCACCGTCTCCTCA | 159 |
| | VL (DNA) | CAGTCTGCCCTGACTCAGCCTGCCTCCGTGTCTGGGTCTCCTGGACAGTCGATCA CCATCTCCTGCACTGGAACCAGCAGTGACATTGGTGGTTATACCTTTGTCTCCTGG TACCAACAACACCCAGGCAAAGCCCCCAAAGTCATGATTCATGATGTCAATAATCG GCCCTCAGGGATTTCTAATCGCTTCTCTGGCTCCAAGTCTGGCAACACGGCCTCCC TGACCATCTCTGGGCTCCAGGCTGAGGACGAGGCTGATTATTACTGCAGCTCAGT TACAAGCACCAACACTTATGTCTTCGGAACTGGGACAAAGGTCACCGTCCTA | 160 |
| A-017 | H-CDR1 | GYYMH | 161 |
| | H-CDR2 | WINPNSGNTGYAQKFQG | 162 |
| | H-CDR3 | GGQQQLVLDDY | 163 |
| | VH (aa) | QVQLVQSGAEVKKPGASVKVSCKASGYTFTGYYMHWVRQAPGQGLEWMGWINPN SGNTGYAQKFQGRVTMTRNTSISTAYMELSSLRSEDTAVYYCARGGQQQLVLDDY WGQGTLVTVSS | 164 |
| | L-CDR1 | QASQDIRHHLN | 165 |
| | L-CDR2 | DSSNLET | 166 |
| | L-CDR3 | QQYDSLPRT | 167 |
| | VL (aa) | DIVLTQSPASLSASVGDRITITCQASQDIRHHLNWFQHKPGKAPKLLISDSSNLETGV PSRFSGSGSGTDFSFTISRLQPEDIATYYCQQYDSLPRTFGQGTKLEIK | 168 |
| | VH (DNA) | CAGGTGCAGCTGGTGCAATCTGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGATGGATCAACCCTAACAGT GGTAACACAGGCTATGCACAGAAGTTCCAGGGCAGAGTCACCATGACCAGGAACA CCTCCATAAGCACAGCCTACATGGAGCTGAGCAGCCTGAGATCTGAGGACACGGC CGTGTATTACTGTGCGAGAGGCGGACAGCAGCAGCTGGTACTGGACGACTACTGG GGCCAGGGAACCCTGGTCACCGTCTCCTCA | 169 |
| | VL (DNA) | GATATTGTGCTGACACAGTCTCCAGCCTCCCTGTCTGCATCTGTCGGCGACAGGA TCACCATCACCTGCCAGGCGAGTCAGGACATTAGGCATCATTTAAATTGGTTTCAG CACAAACCAGGGAAAGCCCCCAAGCTCCTGATCTCCGATTCATCCAACCTGGAAAC AGGAGTCCCGTCAAGGTTCAGTGGAAGTGGGTCTGGGACAGATTTTTCTTTCACC ATCAGCCGCCTGCAGCCTGAAGATATTGCGACTTATTACTGTCAACAATATGATAG TCTGCCTCGAACCTTTGGCCAGGGGACCAAACTGGAGATCAAA | 170 |
| A-018 | H-CDR1 | SYAMH | 171 |
| | H-CDR2 | VISYDGSNKYYADSVKG | 172 |
| | H-CDR3 | ASPSQWLVLGHY | 173 |
| | VH (aa) | QVQLVESGGGVVQPGRSLRLSCAASGFTFSSYAMHWVRQAPGKGLEWVAVISYDGS NKYYADSVKGRFTISRDNSKNTLYLQMNSLRAEDTAVYYCARASPSQWLVLGHYWG QGTLVTVSS | 174 |
| | L-CDR1 | RASQSVSSSYLA | 175 |
| | L-CDR2 | GASSRAT | 176 |
| | L-CDR3 | QQYGSSPWT | 177 |
| | VL (aa) | ETTLTQSPGTLSLSPGERATLSCRASQSVSSSYLAWYQQKPGQAPRLLIYGASSRATG IPDRFSGSGSGTDFTLTISRLEPEDFAVYYCQQYGSSPWTFGQGTKVEIK | 178 |
| | VH (DNA) | CAGGTGCAGCTGGTGGAGTCTGGGGGAGGCGTGGTCCAGCCTGGGAGGTCCCTG AGACTCTCCTGTGCAGCCTCTGGATTCACCTTCAGTAGCTATGCTATGCACTGGGT CCGCCAGGCTCCAGGCAAGGGGCTGGAGTGGGTGGCAGTTATATCATATGATGGA AGCAATAAATACTACGCAGACTCCGTGAAGGGCCGATTCACCATCTCCAGAGACAA TTCCAAGAACACGCTGTATCTGCAAATGAACAGCCTGAGAGCTGAGGACACGGCT GTGTATTACTGTGCGAGAGCCTCTCCATCACAGTGGCTGGTACTCGGGCACTACT GGGGCCAGGGAACCCTGGTCACCGTCTCCTCA | 179 |
| | VL (DNA) | GAAACGACACTCACGCAGTCTCCAGGCACCCTGTCTTTGTCTCCAGGGGAAAGAG CCACCCTCTCCTGCAGGGCCAGTCAGAGTGTTAGCAGCAGCTACTTAGCCTGGTA CCAGCAGAAACCTGGCCAGGCTCCCAGGCTCCTCATCTATGGTGCATCCAGCAGG GCCACTGGCATCCCAGACAGGTTCAGTGGCAGTGGGTCTGGGACAGACTTCACTC TCACCATCAGCAGACTGGAGCCTGAAGATTTTGCAGTGTATTACTGTCAGCAGTAT GGTAGCTCACCGTGGACGTTCGGCCAAGGGACCAAGGTGGAAATCAAA | 180 |
| A-019 | H-CDR1 | GYYMH | 181 |
| | H-CDR2 | WINPNSGNTGYAQKFQG | 182 |
| | H-CDR3 | GGQQQLVLDDY | 183 |
| | VH (aa) | QVQLVQSGAEVKKPGASVKVSCKASGYTFTGYYMHWVRQAPGQGLEWMGWINPN SGNTGYAQKFQGRVTMTRNTSISTAYMELSSLRSEDTAVYYCARGGQQQLVLDDY WGQGTLVTVSS | 184 |
| | L-CDR1 | RASQSVSSNLA | 185 |
| | L-CDR2 | GASTRAT | 186 |
| | L-CDR3 | QQYNNWPWT | 187 |
| | VL (aa) | ETTLTQSPATLSVSPGERATLSCRASQSVSSNLAWYQQKPGQAPRLLIYGASTRATGI PARFSGSGSGTEFTLTISSLQSEDFAVYYCQQYNNWPWTFGQGTKVETK | 188 |
| | VH (DNA) | CAGGTGCAGCTGGTGCAGTCTGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGATGGATCAACCCTAACAGT GGTAACACAGGCTATGCACAGAAGTTCCAGGGCAGAGTCACCATGACCAGGAACA CCTCCATAAGCACAGCCTACATGGAGCTGAGCAGCCTGAGATCTGAGGACACGGC CGTGTATTACTGTGCGAGAGGCGGACAGCAGCAGCTGGTACTGGACGACTACTGG GGCCAGGGAACCCTGGTCACCGTCTCCTCA | 189 |
| | VL (DNA) | GAAACGACACTCACGCAGTCTCCAGCCACCCTGTCTGTGTCTCCAGGGGAAAGAG CCACCCTCTCCTGCAGGGCCAGTCAGAGTGTTAGCAGCAACTTAGCCTGGTACCA GCAGAAACCTGGCCAGGCTCCCAGGCTCCTCATCTATGGTGCATCCACCAGGGCC ACTGGTATCCCAGCCAGGTTCAGTGGCAGTGGGTCTGGGACAGAGTTCACTCTCA CCATCAGCAGCCTGCAGTCTGAAGATTTTGCAGTTTATTACTGTCAGCAGTATAAT AACTGGCCTTGGACGTTCGGCCAAGGGACCAAGGTGGAAACCAAA | 190 |
| A-020 | H-CDR1 | SGGYYWS | 191 |
| | H-CDR2 | YIYYSGSTYYNPSLKS | 192 |
| | H-CDR3 | GGISPSGSSIYYYYGMDV | 193 |
| | VH (aa) | QLQLQESGPGLVKPSQTLSLTCTVSGGSISSGGYYWSWIRQHPGKGLEWIGYIYYSG STYYNPSLKSRVTISVDTSKNQFSLKLSSVTAADTAVYYCARGGISPSGSSIYYYYGM DVWGQGTTVTVSS | 194 |
| | L-CDR1 | TGTSNDVGGYNYVS | 195 |
| | L-CDR2 | EVNKRPS | 196 |
| | L-CDR3 | SSYAGTKEV | 197 |
| | VL (aa) | QPVLTQPPSASGSPGQSVTISCTGTSNDVGGYNYVSWYQQHPGKAPKLIIYEVNKRP SGVPDRFSGSKSGNTASLTVSGLQAVDESDYYCSSYAGTKEVFGGGTKLTVL | 198 |
| | VH (DNA) | CAGCTGCAGCTGCAGGAGTCCGGCCCAGGACTGGTGAAGCCTTCACAGACCCTGT CCCTCACCTGCACTGTCTCTGGTGGCTCCATCAGCAGTGGTGGTTACTACTGGAG CTGGATCCGCCAGCACCCAGGGAAGGGCCTGGAGTGGATTGGGTACATCTATTAC AGTGGGAGCACCTACTACAACCCGTCCCTCAAGAGTCGAGTTACCATATCAGTAGA CACGTCTAAGAACCAGTTCTCCCTGAAGCTGAGCTCTGTGACTGCCGCGGACACG GCCGTGTATTACTGTGCGAGAGGTGGGATATCACCCTCCGGGTCCTCGATCTACT ACTACTACGGTATGGACGTCTGGGGCCAAGGGACCACGGTCACCGTCTCCTCA | 199 |
| | VL (DNA) | CAGCCTGTGCTGACTCAGCCACCCTCCGCGTCCGGGTCTCCTGGACAGTCAGTCA CCATCTCCTGCACTGGAACCAGCAATGACGTTGGTGGTTATAACTATGTCTCCTGG TACCAACAACACCCAGGCAAAGCCCCCAAACTCATAATTTATGAGGTCAATAAGCG GCCCTCAGGGGTCCCTGATCGCTTCTCTGGCTCCAAGTCTGGCAACACGGCCTCC CTGACCGTCTCTGGGCTCCAGGCTGTGGATGAGTCTGATTATTACTGCAGCTCAT ATGCAGGCACCAAGGAGGTCTTCGGCGGAGGGACCAAGCTGACCGTCCTA | 200 |
| A-021 | H-CDR1 | SYGIS | 201 |
| | H-CDR2 | WISAYNGNTNYAQKLQG | 202 |
| | H-CDR3 | VPAWSGQFDY | 203 |
| | VH (aa) | QVQLVQSGAEVKKPGASVKVSCKASGYTFTSYGISWVRQAPGQGLEWMGWISAYN GNTNYAQKLQGRVTMTTDTSTSTAYMELRSLRSDDTAVYYCARVPAWSGQFDYWG QGTLVTVSS | 204 |
| | L-CDR1 | TGTSSDVGGYNYVS | 205 |
| | L-CDR2 | EVTNRPS | 206 |
| | L-CDR3 | NSYTSGPTYVL | 207 |
| | VL (aa) | QSALTQPASVSGSPGQSITISCTGTSSDVGGYNYVSWYQQHPGKAPKLMIYEVTNRP SGVSDRFSGSKSANTASLTISELQAEDEAVYYCN SYTSGPTYVLFGGGTQLTVL | 208 |
| | VH (DNA) | CAGGTGCAGCTGGTGCAGTCTGGAGCTGAGGTGAAGAAGCCTGGGGCCTCAGTG AAGGTCTCCTGCAAGGCTTCTGGTTACACCTTTACCAGCTATGGTATCAGCTGGGT GCGACAGGCCCCTGGACAAGGGCTTGAGTGGATGGGATGGATCAGCGCTTACAAT GGTAACACAAACTATGCACAGAAGCTCCAGGGCAGAGTCACCATGACCACAGACA CATCCACGAGCACAGCCTACATGGAGCTGAGGAGCCTGAGATCTGACGACACGGC CGTGTATTACTGTGCGAGAGTCCCTGCGTGGAGTGGTCAATTTGACTACTGGGGC CAGGGAACCCTGGTCACCGTCTCCTCA | 209 |
| | VL (DNA) | CAGTCTGCCCTGACTCAGCCTGCCTCCGTGTCTGGGTCTCCTGGACAGTCGATCA CCATCTCCTGCACTGGAACCAGCAGTGACGTTGGTGGTTATAACTATGTCTCCTG GTACCAACAGCACCCAGGCAAAGCCCCCAAACTCATGATTTATGAGGTCACTAATC GGCCCTCAGGGGTTTCCGATCGCTTCTCTGGCTCCAAGTCTGCCAACACGGCCTC CCTGACCATCTCTGAGCTCCAGGCTGAAGACGAGGCTGTTTATTACTGCAACTCAT ACACAAGCGGCCCCACTTATGTGCTGTTCGGCGGAGGGACCCAGCTGACCGTCCT A | 210 |
| A-022 | H-CDR1 | SGGYYWS | 211 |
| | H-CDR2 | YIYYSGSTYYNPSLKS | 212 |
| | H-CDR3 | ASRSTDYYFDY | 213 |
| | VH (aa) | EVQLLESGPGLVKPSQTLSLTCTVSGGSISSGGYYWSWIRQHPGKGLEWIGYIYYSG STYYNPSLKSRVTISVDTSKNQFSLKLSSVTAADTAVYYCARASRSTDYYFDYWGQG TLVTVSS | 214 |
| | L-CDR1 | TGNRNNIGDQGAA | 215 |
| | L-CDR2 | RNNNGPS | 216 |
| | L-CDR3 | SAWDSSLRAWV | 217 |
| | VL (aa) | QPGLTQPPSMSYGLGQTATLTCTGNRNNIGDQGAAWLQQHQGHPPKLLSYRNNNG PSGISERLSASRSGNTASLTISGLQPEDEADYYCSAWDSSLRAWVFGGGTKLTVL | 218 |
| | VH (DNA) | GAAGTGCAGCTGTTGGAGTCTGGCCCAGGACTGGTGAAGCCTTCACAGACCCTGT CCCTCACCTGCACTGTCTCTGGTGGCTCCATCAGCAGTGGTGGTTACTACTGGAG CTGGATCCGCCAGCACCCAGGGAAGGGCCTGGAGTGGATTGGGTACATCTATTAC AGTGGGAGCACCTACTACAACCCGTCCCTCAAGAGTCGAGTTACCATATCAGTAGA CACGTCTAAGAACCAGTTCTCCCTGAAGCTGAGCTCTGTGACTGCCGCGGACACG GCCGTGTATTACTGTGCGAGGGCTTCACGATCGACCGATTACTACTTTGACTACTG GGGCCAGGGAACCCTGGTCACCGTCTCCTCA | 219 |
| | VL (DNA) | CAGCCAGGGCTGACTCAGCCACCCTCGATGTCCTACGGCTTGGGACAGACCGCCA CACTCACCTGCACTGGGAACAGAAACAATATTGGCGACCAAGGAGCAGCTTGGCT GCAGCAGCACCAGGGCCACCCTCCCAAACTCCTATCCTACAGGAATAACAACGGGC CCTCAGGGATCTCAGAGAGATTATCTGCATCCAGGTCAGGAAACACAGCCTCCCT GACCATTAGTGGACTCCAGCCTGAGGACGAGGCTGACTATTACTGCTCAGCATGG GACAGCAGCCTCAGGGCTTGGGTGTTCGGCGGAGGGACCAAGCTGACCGTCCTC | 220 |
| A-023 | H-CDR1 | SYAIS | 221 |
| | H-CDR2 | GIIPIFGTANYAQKFQG | 222 |
| | H-CDR3 | PKYSSGWFYYYGMDV | 223 |
| | VH (aa) | QVQLVQSGAEVKKPGSSVKVSCKASGGTFSSYAISWVRQAPGQGLEWMGGIIPIFGT ANYAQKFQGRVTITADESTSTAYMELSSLRSEDTAVYYCARPKYSSGWFYYYGMDV WGQGTTVTVSS | 224 |
| | L-CDR1 | SGSSSNIGNNYVS | 225 |
| | L-CDR2 | DNNKRPS | 226 |
| | L-CDR3 | GTWDSSLSW | 227 |
| | VL (aa) | QSVLTQPPSVSAAPGQKVTISCSGSSSNIGNNYVSWYQQLPGTAPKLLIYDNNKRPS GIPDRFSGSKSGTSATLGITGLQTGDEADYYCGTWDSSLSVVFGGGTKLTVL | 228 |
| | VH (DNA) | CAGGTCCAGCTGGTGCAGTCTGGGGCTGAGGTGAAGAAGCCTGGGTCCTCGGTG AAGGTCTCCTGCAAGGCTTCTGGAGGCACCTTCAGCAGCTATGCTATCAGCTGGG TGCGACAGGCCCCTGGACAAGGGCTTGAGTGGATGGGAGGGATCATCCCTATCTT TGGTACAGCAAACTACGCACAGAAGTTCCAGGGCAGAGTCACGATTACCGCGGAC GAATCCACGAGCACAGCCTACATGGAGCTGAGCAGCCTGAGATCTGAGGACACGG CCGTGTATTACTGTGCGAGACCAAAGTATAGCAGTGGCTGGTTCTACTACTACGG TATGGACGTCTGGGGCCAAGGGACCACGGTCACCGTCTCCTCA | 229 |
| | VL (DNA) | CAGTCTGTGCTGACTCAGCCACCCTCAGTGTCTGCGGCCCCAGGACAGAAGGTCA CCATCTCCTGCTCTGGAAGCAGCTCCAACATTGGGAATAATTATGTATCCTGGTAC CAGCAGCTCCCAGGAACAGCCCCCAAACTCCTCATTTATGACAATAATAAGCGACC CTCAGGGATTCCTGACCGATTCTCTGGCTCCAAGTCTGGCACGTCAGCCACCCTG GGCATCACCGGACTCCAGACTGGGGACGAGGCCGATTATTACTGCGGAACATGGG ATAGCAGCCTGAGTGTGGTATTCGGCGGAGGGACCAAGCTGACCGTCCTA | 230 |
| A-024 | H-CDR1 | SYAIS | 231 |
| | H-CDR2 | WISAYNGNTNYAQKLQG | 232 |
| | H-CDR3 | LGSYGYTGAFDI | 233 |
| | VH (aa) | QMQLVQSGAEVKKPGSSVKVSCKASGGTFSSYAISWVRQAPGQGLEWMGWISAYN GNTNYAQKLQGRVTMTTDTSTSTAYMELRSLRSDDTAVYYCASLGSYGYTGAFDIW GQGTMATVSS | 234 |
| | L-CDR1 | TGTSSDVGGYNYVS | 235 |
| | L-CDR2 | EVSNRPS | 236 |
| | L-CDR3 | SSYTSSSTLW | 237 |
| | VL (aa) | QSALTQPASVSGSPGQSITISCTGTSSDVGGYNYVSWYQQHPGKAPKLMIYEVSNRP SGVSNRFSGSKSGNTASLTISGLQAEDEADYYCSSYTSSSTLVVFGGGTKLTVL | 238 |
| | VH (DNA) | CAAATGCAGCTGGTACAATCTGGGGCTGAGGTGAAGAAGCCTGGGTCCTCGGTGA AGGTCTCCTGCAAGGCTTCTGGAGGCACCTTCAGCAGCTATGCTATCAGCTGGGT GCGACAGGCCCCTGGACAAGGGCTTGAGTGGATGGGATGGATCAGCGCTTACAAT GGTAACACAAACTATGCACAGAAGCTCCAGGGCAGAGTCACCATGACCACAGACA CATCCACGAGCACAGCCTACATGGAGCTGAGGAGCCTGAGATCTGACGACACGGC CGTGTATTACTGTGCGAGCCTGGGGAGCTATGGTTATACAGGGGCTTTTGATATC TGGGGCCAAGGGACAATGGCCACCGTCTCTTCA | 239 |
| | VL (DNA) | CAGTCTGCCCTGACTCAGCCTGCCTCCGTGTCTGGGTCTCCTGGACAGTCGATCA CCATCTCCTGCACTGGAACCAGCAGTGACGTTGGTGGTTATAACTATGTCTCCTG GTACCAACAGCACCCAGGCAAAGCCCCCAAACTCATGATTTATGAGGTCAGTAATC GGCCCTCAGGGGTTTCTAATCGCTTCTCTGGCTCCAAGTCTGGCAACACGGCCTC CCTGACCATCTCTGGGCTCCAGGCTGAGGACGAGGCTGATTATTACTGCAGCTCA TATACAAGCAGCAGCACTCTCGTGGTATTCGGCGGAGGGACCAAGCTGACCGTCC TA | 240 |
| A-025 | H-CDR1 | SYAIS | 241 |
| | H-CDR2 | GIIPIFGTANYAQKFQG | 242 |
| | H-CDR3 | GGWLRQNWFDP | 243 |
| | VH (aa) | QVQLVQSGAEVKKPGSSVKVSCKASGGTFSSYAISWVRQAPGQGLEWMGGIIPIFGT ANYAQKFQGRVTITADESTSTAYMELTSLRSEDTAVYYCARGGWLRQNWFDPWGQ GTLVTVSS | 244 |
| | L-CDR1 | QGDSLRSYYAS | 245 |
| | L-CDR2 | GKNNRPS | 246 |
| | L-CDR3 | NSRDSSGNHPRV | 247 |
| | VL (aa) | SSELTQDPAVSVALGQTVRITCQGDSLRSYYASWYQQKPGQAPVLVIYGKNNRPSGI PDRFSGSSSGNTASLTITGAQAEDEADYYCNSRDSSGNHPRVFGGGTKLTVL | 248 |
| | VH (DNA) | CAGGTGCAGCTGGTGCAGTCTGGGGCTGAGGTGAAGAAGCCTGGGTCCTCGGTG AAGGTCTCCTGCAAGGCTTCTGGAGGCACCTTCAGCAGCTATGCTATCAGCTGGG TGCGACAGGCCCCTGGACAAGGGCTTGAGTGGATGGGAGGGATCATCCCTATCTT TGGTACAGCAAACTACGCACAGAAGTTCCAGGGCAGAGTCACGATAACCGCGGAC GAATCCACGAGCACAGCCTACATGGAGCTGACCAGCCTGAGATCTGAGGACACGG CCGTGTATTACTGTGCGAGAGGGGGGTGGCTACGACAGAACTGGTTCGACCCCTG GGGCCAGGGAACCCTGGTCACCGTCTCCTCA | 249 |
| | VL (DNA) | TCTTCTGAGCTGACTCAGGACCCTGCTGTGTCTGTGGCCTTGGGACAAACAGTCA GGATCACATGCCAAGGAGACAGCCTCAGAAGCTATTATGCAAGCTGGTACCAGCA GAAGCCAGGACAGGCCCCTGTACTTGTCATCTATGGTAAAAACAACCGGCCCTCA GGGATCCCAGACCGATTCTCTGGCTCCAGCTCAGGAAACACAGCTTCCTTGACCAT CACTGGGGCTCAGGCGGAAGATGAGGCTGACTATTACTGTAACTCCCGGGACAGC AGTGGTAACCATCCAAGGGTATTCGGCGGAGGGACCAAGCTGACCGTCCTA | 250 |
| A-026 | H-CDR1 | SGGYYWS | 251 |
| | H-CDR2 | YIYYSGSTYYNPSLKS | 252 |
| | H-CDR3 | WSLGTSNHGWFDP | 253 |
| | VH (aa) | QVQLQESGPGLVKPSQTLSLTCTVSGGSISSGGYYWSWIRQHPGKGLEWIGYIYYSG STYYNPSLKSRVTISVDTSKNQFSLKLSSVTAADTAVYYCASWSLGTSNHGWFDPW GQGTLVTVSS | 254 |
| | L-CDR1 | SGSSSNIGSNTVN | 255 |
| | L-CDR2 | DNNNRPS | 256 |
| | L-CDR3 | QSYDSNLSGWV | 257 |
| | VL (aa) | QSVLTQPPSASGTPGQRVTISCSGSSSNIGSNTVNWYQQLPGTAPKLLISDNNNRPS GVPDRFSGSKSGTSASLAITGLQAEDEADYYCQSYDSNLSGWVFGGGTKLTVL | 258 |
| | VH (DNA) | CAGGTGCAACTGCAGGAGTCGGGCCCAGGACTGGTGAAGCCTTCACAGACCCTGT CCCTCACCTGCACTGTCTCTGGTGGCTCCATCAGCAGTGGTGGTTACTACTGGAG CTGGATCCGCCAGCACCCAGGGAAGGGCCTGGAGTGGATTGGGTACATCTATTAC AGTGGGAGCACCTACTACAACCCGTCCCTCAAGAGTCGAGTTACCATATCAGTAGA CACGTCTAAGAACCAGTTCTCCCTGAAGCTGAGCTCTGTGACCGCCGCAGACACG GCTGTGTATTACTGTGCGAGCTGGTCCCTAGGTACCAGCAACCATGGTTGGTTCG ACCCCTGGGGCCAGGGAACCCTGGTCACCGTCTCCTCA | 259 |
| | VL (DNA) | CAGTCTGTGCTGACTCAGCCACCCTCAGCGTCTGGGACCCCCGGGCAGAGGGTCA CCATCTCTTGTTCTGGAAGCAGCTCCAACATCGGAAGTAATACTGTAAACTGGTAC CAGCAGCTTCCAGGAACAGCCCCCAAACTCCTCATCTCTGATAATAACAATCGGCC CTCAGGGGTCCCTGACCGATTCTCTGGCTCCAAGTCTGGCACCTCAGCCTCCCTG GCCATCACTGGGCTCCAGGCTGAGGATGAGGCTGATTATTACTGCCAGTCCTATG ACAGTAACCTGAGTGGTTGGGTGTTCGGCGGAGGGACCAAGCTGACCGTCCTA | 260 |
| A-027 | H-CDR1 | SYAIS | 261 |
| | H-CDR2 | GIIPIFGTANYAQKFQG | 262 |
| | H-CDR3 | ARGSTWGYFDY | 263 |
| | VH (aa) | QVQLVQSGAEVKKPGSSVKVSCKASGGTFSSYAISWVRQAPGQGLEWMGGIIPIFGT ANYAQKFQGRVTITADKSTSTAYMELSSLRSEDTAVYYCARARGSTWGYFDYWGQG TLVTVSS | 264 |
| | L-CDR1 | RASQSVSNYLA | 265 |
| | L-CDR2 | DASNRAT | 266 |
| | L-CDR3 | QQRDNWPLT | 267 |
| | VL (aa) | EIVLTQSPASLSLSPGERVTLSCRASQSVSNYLAWYQQKPGQAPRLLIYDASNRATGI PARFSGSGSGTDFTLTISSLEPEDFAVYYCQQRDNWPLTFGGGTKVEIK | 268 |
| | VH (DNA) | CAGGTGCAGCTGGTACAATCTGGGGCTGAGGTGAAGAAGCCTGGGTCCTCGGTG AAGGTCTCCTGCAAGGCTTCTGGAGGCACCTTCAGCAGCTATGCTATCAGCTGGG TGCGACAGGCCCCTGGACAAGGGCTTGAGTGGATGGGAGGGATCATCCCTATCTT TGGTACAGCAAACTACGCACAGAAGTTCCAGGGCAGAGTCACGATTACCGCGGAC AAATCCACGAGCACAGCCTACATGGAGCTGAGCAGCCTGAGATCTGAGGACACGG CCGTGTATTACTGTGCGAGAGCGAGGGGCAGCACCTGGGGCTACTTTGACTACTG GGGCCAGGGAACCCTGGTCACCGTCTCCTCA | 269 |
| | VL (DNA) | GAAATAGTGTTGACGCAGTCTCCAGCCTCCCTGTCTTTGTCTCCAGGGGAAAGAG TCACCCTCTCCTGCAGGGCCAGTCAGAGTGTTAGCAATTACTTAGCCTGGTATCAA CAGAAACCTGGCCAGGCTCCCAGGCTCCTCATCTATGATGCATCCAACAGGGCCAC TGGCATCCCAGCCAGGTTCAGTGGCAGTGGGTCTGGGACAGACTTCACTCTCACC ATCAGCAGCCTAGAGCCCGAAGATTTTGCAGTTTATTACTGTCAGCAGCGTGACAA CTGGCCCCTCACTTTCGGCGGAGGGACCAAGGTGGAGATCAAA | 270 |
| A-028 | H-CDR1 | SYAIS | 271 |
| | H-CDR2 | GIIPIFGTANYAQKFQG | 272 |
| | H-CDR3 | VGVEYQLLWYFDY | 273 |
| | VH (aa) | QVQLVQSGAEVKKPGSSVKVSCKASGGTFSSYAISWVRQAPGQGLEWMGGIIPIFGT ANYAQKFQGRVTITADESTSTAYMELSSLRSEDTAVYYCARVGVEYQLLWYFDYWG QGTLVTVSS | 274 |
| | L-CDR1 | RTSQTISNYLN | 275 |
| | L-CDR2 | AASNLQS | 276 |
| | L-CDR3 | QQSYNAS | 277 |
| | VL (aa) | DIQMTQSPSSLSASVGDRVTLTCRTSQTISNYLNWYQQKPGKAPKLLVYAASNLQS WVPSRFSGSGSGTDFTLTISSLQPEDFATYYCQQSYNASFGGGTKVEFK | 278 |
| | VH (DNA) | CAAGTGCAGCTGGTACAGTCTGGGGCTGAGGTGAAGAAGCCTGGGTCCTCGGTG AAGGTCTCCTGCAAGGCTTCTGGAGGCACCTTCAGCAGCTATGCAATCAGCTGGG TGCGACAGGCCCCTGGACAAGGGCTTGAGTGGATGGGAGGGATCATCCCTATCTT TGGTACAGCAAACTACGCACAGAAGTTCCAGGGCAGAGTCACGATTACCGCGGAC GAATCCACGAGCACAGCCTACATGGAGCTGAGCAGCCTGAGATCTGAGGACACGG CCGTGTATTACTGTGCGAGAGTAGGGGTTGAGTACCAGCTGCTATGGTACTTTGA CTACTGGGGCCAGGGAACCCTAGTCACCGTCTCCTCA | 279 |
| | VL (DNA) | GACATCCAGATGACCCAGTCTCCATCCTCCCTGTCTGCATCTGTAGGAGACAGAGT CACCCTCACTTGCCGGACAAGTCAGACCATTAGCAACTATTTAAATTGGTATCAGC AGAAACCAGGGAAAGCCCCTAAGCTCCTGGTCTATGCTGCATCCAATTTGCAAAGT TGGGTCCCATCAAGGTTCAGTGGCAGTGGGTCTGGGACAGATTTCACTCTCACCA TCAGCAGTCTGCAGCCTGAAGATTTTGCAACTTACTACTGTCAACAGAGTTACAAT GCCTCTTTCGGCGGAGGGACCAAGGTGGAGTTCAAA | 280 |
| A-029 | H-CDR1 | SYAIS | 281 |
| | H-CDR2 | GIIPIFGTANYAQKFQG | 282 |
| | H-CDR3 | SYYYYYGMDV | 283 |
| | VH (aa) | QVQLVQSGAEVKKPGSSVKVSCKASGGTFSSYAISWVRQAPGQGLEWMGGIIPIFGT ANYAQKFQGRVTITADESTGTAYMELSSLRSEDTAVYYCARSYYYYYGMDVWGQGT TVTVSS | 284 |
| | L-CDR1 | RSSQSLLHSNGYNYLD | 285 |
| | L-CDR2 | LGSNRAS | 286 |
| | L-CDR3 | MQAVDTPRT | 287 |
| | VL (aa) | DIQMTQSPLSLPVTPGEPASISCRSSQSLLHSNGYNYLDWYLQKPGQSPQLLIHLGSN RASGVPDRFSGSGSGTDFTLKISRVEAEDVGVYYCMQAVDTPRTFGQGTKVDIK | 288 |
| | VH (DNA) | CAGGTCCAGCTGGTGCAGTCTGGGGCTGAGGTGAAGAAGCCTGGGTCCTCGGTG AAGGTCTCCTGCAAGGCTTCTGGAGGCACCTTCAGCAGCTATGCTATCAGCTGGG TGCGACAGGCCCCTGGACAAGGGCTTGAGTGGATGGGAGGGATCATCCCTATCTT TGGTACAGCAAACTACGCACAGAAGTTCCAGGGCAGAGTCACGATTACCGCGGAC GAATCCACGGGCACAGCCTACATGGAGCTGAGCAGCCTGAGATCTGAGGACACGG CCGTGTATTACTGTGCGAGAAGTTACTACTACTACTACGGTATGGACGTCTGGGG CCAAGGGACCACGGTCACCGTCTCCTCA | 289 |
| | VL (DNA) | GACATCCAGATGACCCAGTCTCCACTCTCCCTGCCCGTCACCCCTGGAGAGCCGGC CTCCATCTCCTGCAGGTCTAGTCAGAGCCTCCTGCATAGTAATGGATACAACTATT TGGATTGGTACCTGCAGAAGCCAGGGCAGTCACCACAGCTCCTGATCCATTTGGG TTCTAATCGGGCCTCCGGGGTCCCTGACAGGTTCAGTGGCAGTGGATCAGGCACA GATTTTACACTGAAAATTAGCAGAGTGGAGGCTGAGGATGTTGGGGTTTATTACT GCATGCAAGCTGTAGATACTCCTCGGACGTTCGGCCAAGGGACCAAGGTGGACAT CAAA | 290 |
| A-030 | H-CDR1 | GYYMH | 291 |
| | H-CDR2 | WINPNSGNTGYAQKFQG | 292 |
| | H-CDR3 | GGQQQLVLDDY | 293 |
| | VH (aa) | QVQLVQSGAEVKKPGASVKVSCKASGYTFTGYYMHWVRQAPGQGLEWMGWINPN SGNTGYAQKFQGRVTMTRNTSISTAYMELSSLRSEDTAVYYCARGGQQQLVLDDY WGQGTLVTVSS | 294 |
| | L-CDR1 | RASQSIGGWLA | 295 |
| | L-CDR2 | AASSLQS | 296 |
| | L-CDR3 | RQSYSTPPT | 297 |
| | VL (aa) | DIQLTQSPSTLSASVGDRVTITCRASQSIGGWLAWYQHKPGKAPKLLIYAASSLQSG VPSRFSGSGSGTDFTLTISSLQPEDFATYYCRQSYSTPPTFGQGTKVEIK | 298 |
| | VH (DNA) | CAGGTGCAGCTGGTGCAGTCTGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGATGGATCAACCCTAACAGT GGTAACACAGGCTATGCACAGAAGTTCCAGGGCAGAGTCACCATGACCAGGAACA CCTCCATAAGCACAGCCTACATGGAGCTGAGCAGCCTGAGATCTGAGGACACGGC CGTGTATTACTGTGCGAGAGGCGGACAGCAGCAGCTGGTACTGGACGACTACTGG GGCCAGGGAACCCTGGTCACCGTCTCCTCA | 299 |
| | VL (DNA) | GACATCCAGTTGACCCAGTCTCCTTCCACCCTGTCAGCATCTGTAGGCGACAGAGT CACCATCACTTGCCGGGCCAGTCAGTCTATTGGTGGTTGGTTGGCCTGGTATCAG CACAAACCAGGGAAAGCCCCCAAGCTCCTGATCTATGCTGCATCCAGTTTGCAAAG TGGGGTCCCATCAAGGTTCAGTGGCAGTGGATCTGGGACAGATTTCACTCTCACC ATCAGCAGTCTGCAACCTGAAGATTTTGCAACTTACTACTGTCGACAGAGTTACAG TACCCCTCCGACGTTCGGCCAAGGGACCAAGGTGGAAATCAAA | 300 |
| A-031 | H-CDR1 | SYAMH | 301 |
| | H-CDR2 | VISYDGSNKYYADSVKG | 302 |
| | H-CDR3 | WAAADLTRYFDY | 303 |
| | VH (aa) | QVQLVESGGGVVQPGRSLRLSCAASGFTFSSYAMHWVRQAPGKGLEWVAVISYDGS NKYYADSVKGRFTISRDNSKNTLYLQMNSLRAEDTAVYYCARVVAAADLTRYFDYW GQGTLVTVSS | 304 |
| | L-CDR1 | RASQSVPKNYLA | 305 |
| | L-CDR2 | TASSRAP | 306 |
| | L-CDR3 | QQYGTSPNT | 307 |
| | VL (aa) | EIVLTQSPGTVSLSPGERATLSCRASQSVPKNYLAWFQQKPGQAPRLVIHTASSRAPG IPDRFTGSGSGTDFTLTISRLEPEDFAVYYCQQYGTSPNTFGQGTKLDIK | 308 |
| | VH (DNA) | CAGGTGCAGCTGGTGGAGTCTGGGGGAGGCGTGGTCCAGCCTGGGAGGTCCCTG AGACTCTCCTGTGCAGCCTCTGGATTCACCTTCAGTAGCTATGCTATGCACTGGGT CCGCCAGGCTCCAGGCAAGGGGCTGGAGTGGGTGGCAGTTATATCATATGATGGA AGCAATAAATACTACGCAGACTCCGTGAAGGGCCGATTCACCATCTCCAGAGACAA TTCCAAGAACACGCTGTATCTGCAAATGAACAGCCTGAGAGCTGAGGACACGGCT GTGTATTACTGTGCGAGAGTCGTAGCAGCAGCTGACCTCACTCGCTACTTTGACT ACTGGGGCCAGGGAACCCTGGTCACCGTCTCCTCA | 309 |
| | VL (DNA) | GAAATTGTGTTGACGCAGTCTCCAGGCACCGTGTCTTTGTCTCCAGGGGAAAGAG CCACCCTCTCCTGCAGGGCCAGTCAGAGTGTTCCTAAGAACTACTTAGCCTGGTTC CAGCAGAAACCTGGCCAGGCTCCCAGGCTCGTCATCCATACTGCATCCAGCAGGG CCCCTGGCATCCCAGACAGGTTCACTGGCAGCGGGTCTGGGACAGACTTCACTCT TACCATCAGCAGACTGGAGCCTGAAGATTTTGCAGTATATTACTGTCAGCAGTATG GCACCTCACCAAACACTTTTGGCCAGGGGACCAAGCTGGACATCAAA | 310 |
| A-032 | H-CDR1 | GYYMH | 311 |
| | H-CDR2 | WMNPNSGNTGYAQKFQG | 312 |
| | H-CDR3 | GGQQQLVLDDY | 313 |
| | VH (aa) | QVQLVQSGAEVKKPGASVKVSCKASGYTFTGYYMHWVRQAPGQGLEWMGWMNPN SGNTGYAQKFQGRVTMTRNTSISTAYMELSSLRSEDTAVYYCARGGQQQLVLDDY WGQGTLVTVSS | 314 |
| | L-CDR1 | RSSQSLLHSNGYNYLD | 315 |
| | L-CDR2 | LGSNRAS | 316 |
| | L-CDR3 | MQALQTPRT | 317 |
| | VL (aa) | DIVMTQSPLSLPVTPGEPASISCRSSQSLLHSNGYNYLDWYLQKPGQSPQLLIYLGSN RASGVPDRFSGSGSGTDFTLKISRVEAEDVGVYYCMQALQTPRTFGPGTKVDIK | 318 |
| | VH (DNA) | CAAGTGCAGCTGGTACAGTCTGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGGTGGATGAATCCTAACAGT GGCAACACAGGCTATGCACAGAAGTTCCAGGGCAGAGTCACCATGACCAGGAACA CCTCCATAAGCACAGCCTACATGGAGCTGAGCAGCCTGAGATCTGAGGACACGGC CGTGTATTACTGTGCGAGAGGCGGACAGCAGCAGCTGGTACTGGACGACTACTGG GGCCAGGGAACCCTGGTCACCGTCTCCTCA | 319 |
| | VL (DNA) | GACATCGTGATGACCCAGTCTCCACTCTCCCTGCCCGTCACCCCTGGAGAGCCGG CCTCCATCTCCTGCAGGTCTAGTCAGAGCCTCCTGCATAGTAATGGATACAACTAT TTGGATTGGTACCTGCAGAAGCCAGGGCAGTCTCCACAGCTCCTGATCTATTTGG GTTCTAATCGGGCCTCCGGGGTCCCTGACAGGTTCAGTGGCAGTGGATCAGGCAC AGATTTTACACTGAAAATCAGCAGAGTGGAGGCTGAGGATGTTGGGGTTTATTAC TGCATGCAAGCTCTACAAACCCCTCGGACTTTCGGCCCTGGGACCAAAGTGGATA TCAAA | 320 |
| A-033 | H-CDR1 | SYGIS | 321 |
| | H-CDR2 | WISAYNGNTNYAQKLQG | 322 |
| | H-CDR3 | DHSIVGATTFDY | 323 |
| | VH (aa) | QVQLVQSGAEVKKPGASVKVSCKASGYTFTSYGISWVRQAPGQGLEWMGWISAYN GNTNYAQKLQGRVTMTTDTSTSTAYMELRSLRSDDTAVYYCARDHSIVGATTFDYW GQGTLVTVSS | 324 |
| | L-CDR1 | RASQSISSWLA | 325 |
| | L-CDR2 | DASSLES | 326 |
| | L-CDR3 | QQYNSYPWT | 327 |
| | VL (aa) | DIVMTQSPSTLSASVGDRVTITCRASQSISSWLAWYQQKPGKAPKLLIYDASSLESGV PSRFSGSGSGTEFTLTISSLQPDDFATYYCQQYNSYPWTFGQGTKVEIK | 328 |
| | VH (DNA) | CAGGTCCAGCTGGTGCAGTCTGGAGCTGAGGTGAAGAAGCCTGGGGCCTCAGTG AAGGTCTCCTGCAAGGCTTCTGGTTACACCTTTACCAGCTACGGTATCAGCTGGG TGCGACAGGCCCCTGGACAAGGGCTTGAGTGGATGGGATGGATCAGCGCTTACAA TGGTAACACAAACTATGCACAGAAGCTCCAGGGCAGAGTCACCATGACCACAGAC ACATCCACGAGCACAGCCTACATGGAGCTGAGGAGCCTGAGATCTGACGACACGG CCGTGTATTACTGTGCGAGAGATCACAGTATAGTGGGAGCTACTACGTTTGACTA CTGGGGCCAGGGAACCCTGGTCACCGTCTCCTCA | 329 |
| | VL (DNA) | GACATCGTGATGACCCAGTCTCCTTCCACCCTGTCTGCATCTGTAGGAGACAGAGT CACCATCACTTGCCGGGCCAGTCAGAGTATTAGTAGCTGGTTGGCCTGGTATCAG CAGAAACCAGGGAAAGCCCCTAAGCTCCTGATCTATGATGCCTCCAGTTTGGAAA GTGGGGTCCCATCAAGGTTCAGCGGCAGTGGATCTGGGACAGAATTCACTCTCAC CATCAGCAGCCTGCAGCCTGATGATTTTGCAACTTATTACTGCCAACAGTACAATA GTTATCCGTGGACGTTCGGCCAAGGGACCAAGGTGGAAATCAAA | 330 |
| A-034 | H-CDR1 | SYTMN | 331 |
| | H-CDR2 | SISSIGTYIYYADSVKG | 332 |
| | H-CDR3 | VLLSGSYYGYFDS | 333 |
| | VH (aa) | QMQLVQSGGGLVKPGGSLRLSCAASGFTFSSYTMNWVRQAPGKGLEWVSSISSIGT YIYYADSVKGRFTISRDNAKNSLYLQMNSLRAEDTAVYYCARVLLSGSYYGYFDSWG QGTLVTVSS | 334 |
| | L-CDR1 | TGSSSNIGAGYDVH | 335 |
| | L-CDR2 | GNSNRPS | 336 |
| | L-CDR3 | QSYDSSLSGYV | 337 |
| | VL (aa) | QSVLTQPPSVSGAPGQRVTISCTGSSSNIGAGYDVHWYQQLPGTAPKLFIYGNSNRP SGVPDRFSGSKSGTSASLAITGLQAEDEADYYCQSYDSSLSGYVFGTGTKVTVL | 338 |
| | VH (DNA) | CAGATGCAGCTGGTACAGTCTGGGGGAGGCCTGGTCAAGCCTGGGGGGTCCCTG AGACTCTCCTGTGCAGCCTCTGGATTCACCTTCAGTAGCTATACCATGAACTGGGT CCGCCAGGCTCCAGGGAAGGGGCTGGAGTGGGTCTCATCCATTAGTAGTATTGGT ACTTACATATACTACGCAGACTCAGTGAAGGGCCGATTCACCATCTCCAGAGACAA CGCCAAGAACTCACTGTATCTGCAAATGAACAGCCTGAGAGCCGAGGACACGGCT GTGTATTACTGTGCGAGAGTCCTGTTAAGTGGGAGCTACTACGGCTACTTTGACT CCTGGGGCCAGGGAACCCTGGTCACCGTCTCCTCA | 339 |
| | VL (DNA) | CAGTCTGTGCTGACTCAGCCACCCTCAGTGTCTGGGGCCCCAGGGCAGAGGGTCA CCATCTCCTGCACTGGGAGCAGCTCCAACATCGGGGCAGGTTATGATGTGCACTG GTACCAGCAGCTTCCAGGAACAGCCCCCAAACTCTTCATCTATGGTAACAGCAATC GGCCCTCAGGGGTCCCTGACCGATTCTCTGGCTCCAAGTCTGGCACCTCAGCCTC CCTGGCCATCACTGGGCTCCAGGCTGAGGATGAGGCTGATTATTACTGCCAGTCC TATGACAGCAGCCTGAGTGGTTATGTCTTCGGAACTGGGACCAAGGTCACCGTCC TA | 340 |
| A-035 | H-CDR1 | GYYMH | 341 |
| | H-CDR2 | WINPNSGNTGYAQKFQG | 342 |
| | H-CDR3 | GRLERGYWYFDL | 343 |
| | VH (aa) | QVQLVQSGAEVKKPGASVKVSCKASGYTFTGYYMHWVRQAPGQGLEWMGWINPN SGNTGYAQKFQGRVTMTRNTSISTAYMELSSLRSEDTAVYYCARGRLERGYWYFDL WGRGTLVTVSS | 344 |
| | L-CDR1 | TRSSGSITSNYVQ | 345 |
| | L-CDR2 | EDKERPS | 346 |
| | L-CDR3 | QSYGGTSQGVL | 347 |
| | VL (aa) | NFMLTQPHSVSESPGRTVTISCTRSSGSITSNYVQWYQQRPGSAPTILIYEDKERPSE VPDRFSGSIDISSNSASLTISGLKTEDEADYYCQSYGGTSQGVLFGGGTKVTVL | 348 |
| | VH (DNA) | CAGGTCCAGCTGGTGCAGTCTGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGATGGATCAACCCTAACAGT GGTAACACAGGCTATGCACAGAAGTTCCAGGGCAGAGTCACCATGACCAGGAACA CCTCCATAAGCACAGCCTACATGGAGCTGAGCAGCCTGAGATCTGAGGACACGGC CGTGTATTACTGTGCGAGAGGCCGTCTGGAACGCGGATACTGGTACTTCGATCTC TGGGGCCGTGGCACCCTGGTCACCGTCTCCTCA | 349 |
| | VL (DNA) | AATTTTATGCTGACTCAGCCCCACTCTGTGTCGGAGTCTCCGGGGAGGACGGTAA CCATCTCCTGCACCCGCAGCAGTGGCAGCATTACCAGCAACTATGTCCAGTGGTAC CAGCAGCGCCCGGGCAGTGCCCCTACCATCCTAATCTATGAGGATAAGGAAAGAC CCTCTGAGGTCCCTGATCGCTTCTCTGGCTCCATCGACATTTCCTCCAACTCTGCC TCCCTCACCATCTCTGGCCTGAAGACGGAGGACGAGGCTGACTACTACTGTCAGT CTTATGGTGGCACCAGTCAAGGGGTGTTATTCGGCGGAGGGACCAAGGTGACCGT CCTA | 350 |
| A-036 | H-CDR1 | GYYMH | 351 |
| | H-CDR2 | WINPNSGGTNYAQKFQG | 352 |
| | H-CDR3 | GGQQQLVLDDY | 353 |
| | VH (aa) | QVQLVQSGAEVKKPGASVKVSCKASGYTFTGYYMHWVRQAPGQGLEWMGWINPN SGGTNYAQKFQGRVTMTRNTSISTAYMELSSLRSEDTAVYYCARGGQQQLVLDDY WGQGTLVTVSS | 354 |
| | L-CDR1 | RASQSISSYLN | 355 |
| | L-CDR2 | AASSLQS | 356 |
| | L-CDR3 | QQSYSTPRT | 357 |
| | VL (aa) | DIVMTQSPSSLSASVGDRVTITCRASQSISSYLNWYQQKPGKAPKLLIYAASSLQSGV PSRFSGSGSGTDFTLTISSLQPEDFATYYCQQSYSTPRTFGQGTKVEIK | 358 |
| | VH (DNA) | CAGGTGCAGCTGGTGCAATCTGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGATGGATCAACCCTAACAGT GGTGGCACAAACTATGCACAGAAGTTCCAGGGCAGAGTCACCATGACCAGGAACA CCTCCATAAGCACAGCCTACATGGAGCTGAGCAGCCTGAGATCTGAGGACACGGC CGTGTATTACTGTGCGAGAGGCGGACAGCAGCAGCTGGTACTGGACGACTACTGG GGCCAGGGAACCCTGGTCACCGTCTCCTCA | 359 |
| | VL (DNA) | GACATCGTGATGACCCAGTCTCCATCCTCCCTGTCTGCATCTGTAGGAGACAGAGT CACCATCACTTGCCGGGCAAGTCAGAGCATTAGCAGCTATTTAAATTGGTATCAGC AGAAACCAGGGAAAGCCCCTAAGCTCCTGATCTATGCTGCATCCAGTTTGCAAAGT GGGGTCCCATCAAGGTTCAGTGGCAGTGGATCTGGGACAGATTTCACTCTCACCA TCAGCAGTCTGCAACCTGAAGATTTTGCAACTTACTACTGTCAACAGAGTTACAGT ACCCCTCGAACGTTCGGCCAAGGGACCAAGGTGGAAATCAAA | 360 |
| A-037 | H-CDR1 | GYYMH | 361 |
| | H-CDR2 | WINPNSGNTGYAQKFQG | 362 |
| | H-CDR3 | GGQQQLVLDDY | 363 |
| | VH (aa) | QVQLVQSGAEVKKPGASVKVSCKASGYTFTGYYMHWVRQAPGQGLEWMGWINPN SGNTGYAQKFQGRVTMTRNTSISTAYMELSSLRSEDTAVYYCARGGQQQLVLDDY WGQGTLVTVSS | 364 |
| | L-CDR1 | TGSSGSIASNYVQ | 365 |
| | L-CDR2 | EDNQRPS | 366 |
| | L-CDR3 | QSYDSSNQRV | 367 |
| | VL (aa) | NFMLTQPHSVSESPGKTVTISCTGSSGSIASNYVQWYQQRPGSAPTTVIYEDNQRPS GVPDRFSGSIDSSSNSASLTISGLKTEDEADYYCQSYDSSNQRVFGGGTKLTVL | 368 |
| | VH (DNA) | CAGGTTCAGCTGGTGCAGTCTGGGGCTGAGGTGAAGAAGCCTGGGGCCTCAGTG AAGGTCTCCTGCAAGGCTTCTGGATACACCTTCACCGGCTACTATATGCACTGGGT GCGACAGGCCCCTGGACAAGGGCTTGAGTGGATGGGATGGATCAACCCTAACAGT GGTAACACAGGCTATGCACAGAAATTCCAGGGCAGAGTCACCATGACCAGGAACA CCTCCATAAGCACAGCCTACATGGAGCTGAGCAGCCTGAGATCTGAGGACACGGC CGTGTATTACTGTGCGAGAGGCGGACAGCAGCAGCTGGTACTGGACGACTACTGG GGCCAGGGAACCCTGGTCACCGTCTCCTCA | 369 |
| | VL (DNA) | AATTTTATGCTGACTCAGCCCCACTCTGTGTCGGAGTCTCCGGGGAAGACGGTAA CCATCTCCTGCACCGGCAGCAGTGGCAGCATTGCCAGCAACTATGTGCAGTGGTA CCAGCAGCGCCCGGGCAGTGCCCCCACCACTGTGATCTATGAGGATAACCAAAGA CCCTCTGGGGTCCCTGATCGGTTCTCTGGCTCCATCGACAGCTCCTCCAACTCTGC CTCCCTCACCATCTCTGGACTGAAGACTGAGGACGAGGCTGACTACTACTGTCAGT CTTATGATAGCAGCAATCAGAGGGTGTTCGGCGGAGGGACCAAGCTGACCGTCCT A | 370 |

**Table 1B: Germline of VH and VL for ABPs of the invention.**

| Antibody | Germline of VH | Germline of VL |
|---|---|---|
| A-001 | IGHV3-21*03 | IGKV1-39*01 |
| A-002 | IGHV3-53*01 | IGLV7-43*01 |
| A-003 | IGHV1-69D*01 | IGLV2-14*01 |
| A-004 | IGHV3-21*03 | IGKV1-39*01 |
| A-005 | IGHV3-53*01 | IGLV7-43*01 |
| A-006 | IGHV3-53*01 | IGLV7-43*01 |
| A-007 | IGHV1-2*02 | IGLV2-14*01 |
| A-008 | IGHV4-39*07 | IGLV1-40*01 |
| A-009 | IGHV1-69D*01 | IGKV3D-15*01 |
| A-010 | IGHV1-18*04 | IGLV2-14*01 |
| A-011 | IGHV3-30*11 | IGLV3-19*01 |
| A-012 | IGHV3-53*01 | IGLV7-43*01 |
| A-013 | IGHV3-53*01 | IGLV7-43*01 |
| A-014 | IGHV1-2*02 | IGKV4-1*01 |
| A-015 | IGHV1-2*02 | IGLV4-2*01 |
| A-016 | IGHV1-69D*01 | IGLV2-14*01 |
| A-017 | IGHV1-2*02 | IGKV1D-33*01 |
| A-018 | IGHV3-30*11 | IGKV3-20*01 |
| A-019 | IGHV1-2*02 | IGKV3D-15*01 |
| A-020 | IGHV4-31*02 | IGLV2-8*01 |
| A-021 | IGHV1-18*04 | IGLV2-14*01 |
| A-022 | IGHV4-31*02 | IGLV10-54*01 |
| A-023 | IGHV1-69D*01 | IGLV1-51*01 |
| A-024 | IGHV1-18*04 | IGLV2-14*01 |
| A-025 | IGHV1-69D*01 | IGLV3-19*01 |
| A-026 | IGHV4-31*02 | IGLV1-44*01 |
| A-027 | IGHV1-69*14 | IGKV3-11*01 |
| A-028 | IGHV1-69D*01 | IGKV1-39*01 |
| A-029 | IGHV1-69D*01 | IGKV2-28*01 |
| A-030 | IGHV1-2*02 | IGKV1-39*01 |
| A-031 | IGHV3-30*11 | IGKV3-20*01 |
| A-032 | IGHV1-8*01 | IGKV2-28*01 |
| A-033 | IGHV1-18*04 | IGKV1-5*01 |
| A-034 | IGHV3-21*03 | IGLV1-40*01 |
| A-035 | IGHV1-2*02 | IGLV6-57*02 |
| A-036 | IGHV1-2*02 | IGKV1-39*01 |
| A-037 | IGHV1-2*02 | IGLV6-57*02 |

**Table 2: Binding of ABPs of the invention to various antigens.**

| Antibody | Human IGSF11 | Streptavidin | Mouse IGSF11 | Mouse Fc | Cell binding | MFI |
|---|---|---|---|---|---|---|
| A-001 | *** | - | *** | - | +++ | ND |
| A-002 | ** | - | * | - | + | ND |
| A-003 | * | - | * | - | + | ND |
| A-004 | ** | - | *** | - | +++ | ND |
| A-005 | ** | - | * | - | ++ | ND |
| A-006 | ** | - | ** | - | ++ | ND |
| A-007 | ** | - | ** | - | + | ND |
| A-008 | *** | - | *** | - | ++ | ND |
| A-009 | ** | - | * | - | ++ | ND |
| A-010 | ** | - | ** | - | ++ | ND |
| A-011 | * | - | *** | - | ++ | ND |
| A-012 | ** | - | ** | - | + | ND |
| A-013 | ** | | ** | - | + | ND |
| A-014 | ** | - | * | - | ++ | ND |
| A-015 | ** | - | ** | - | +++ | ND |
| A-016 | *** | - | ** | - | +++ | ND |
| A-017 | ** | - | * | - | ++ | ND |
| A-018 | ** | - | ** | - | ++ | ND |
| A-019 | *** | - | ** | - | ++ | ND |
| A-020 | *** | - | ** | - | +++ | ### |
| A-021 | ** | - | * | - | +++ | ### |
| A-022 | ** | - | *** | - | ++ | ## |
| A-023 | ** | - | * | - | +++ | ### |
| A-024 | ** | - | * | - | +++ | ### |
| A-025 | * | - | * | - | +++ | ## |
| A-026 | *** | - | *** | - | ++ | ## |
| A-027 | * | - | * | - | ++ | ## |
| A-028 | * | - | * | - | +++ | ### |
| A-029 | * | - | * | - | ++ | ## |
| A-030 | * | - | * | - | ++ | ## |
| A-031 | * | - | * | - | ++ | ## |
| A-032 | * | - | * | - | ++ | # |
| A-033 | * | - | * | - | ++ | # |
| A-034 | *** | - | ** | - | ++ | # |
| A-035 | ** | - | *** | - | + | # |
| A-036 | * | - | * | - | + | # |
| A-037 | * | - | * | - | ++ | ## |

| | | | | | | |
|---|---|---|---|---|---|---|
| Binding (relative fluorescent units, RFU) indicated by: "***" = > 1.0; "**" = approx 0.5 to 1.0; "*" = approx 0.1 to 0.5; "-" = < 0.025; "-" = < 0.05; Cell binding (% positive c (ells in FACS) indicated by: "+++" = > 50%; "++" = approx 35% to 50%; "+" = approx < 25%; mean fluorescent intensity (MFI) (RFU by FACS) indicated by: "###" = > 150; "##" = approx 100 to 150; "#" = approx < 100. | | | | | | |

### Example 4: Functional characterisation of ABPs of the invention by inhibition of the interaction between human IGSF11 (VSIG3) and VSIR /VISTA).

Surprisingly, antibodies of the invention that bind to IGSF11 (VSIG3) are also found to function as inhibitors of the interaction between IGSF11 (VSIG3) and VSIR (VISTA), ie the binding of (eg a function and/or activity of) IGSF11 (VSIG3) to VSIR (VISTA).

An ELISA assay was established to measure the inhibition of the binding of IGSF11 (VSIG3) to VSIR (VISTA) by antibodies (eg of the invention) that bind to IGSF11 (VSIG3). **Figure 3** demonstrates that this assay can detect the inhibition of binding of VSIR (VISTA) by competition for binding to IGSF11 (VSIG3). Purified and immobilised ECD of human IGSF11 (VSIG3) (HIS6-tagged) can interact with VSIR (VISTA), and this interaction is detected, briefly as follows: recombinant, purified and biotinylated IGSF11 (VSIG3) is immobilised on a streptavidin-coated plate at (5 µg/mL in PBS); purified Fc-tagged VSIR (VISTA) (R&D Systems, Cat# 7126-B7) is added for binding (eg at 1.8ug/mL; approx. 20nM bivalent VSIR-FC), and after incubation for 1 hour at room temperature, unbound VSIR is removed by washing 3 times with PBS/Tween 0.05%; remaining VSIR bound to IGSF11 is detected using an appropriately labelled antibody against the Fc-tag (eg horseradish peroxidase-conjugated goat anti-human IgG, Jackson ImmunoResearch, Cat# 115-036-098). This interaction can be blocked with soluble ECD of IGSF11, and as the interaction appears relatively weak (KD estimated by Yang et al (2017) to be at the level of 10e-5 M which belongs to low affinity protein - protein interactions between cell surface receptors) it can also easily be blocked by a commercial anti-VSIR (anti-VISTA) antibody (eg, R&D Systems, Cat#: MAB71261, monoclonal mouse IgG2b, or AF7126, polyclonal sheep)..

IGSF11-binding antibodies of the invention from those shown in **Tables 1** were tested in scFv-format for their ability to inhibit the interaction between IGSF11 (VSIG3) and with VSIR (VISTA) in this ELISA assay, and the degree of such inhibition is shown in **Table 3.** Briefly, E.coli-culture supernatant of scFv-producing phage-infected bacteria is added to surface-immobilised ECD of IGFS11 (VSIG3), and the unbound scFv washed away. The binding of VSIR (VISTA) to the scFv-treated IGSF11 is then assessed as described above

**Table 3: Inhibition of the interaction between IGSF11 (VSIG3) and with VSIR (VISTA) by IGSF11-binding antibodies of the invention (ScFv format).**

| Antibody | Binding inhibition |
|---|---|
| A-001 | - |
| A-002 | - |
| A-003 | * |
| A-004 | * |
| A-005 | - |
| A-006 | ** |
| A-007 | *** |
| A-008 | * |
| A-009 | * |
| A-010 | *** |
| A-011 | ** |
| A-012 | ** |
| A-013 | *** |
| A-014 | *** |
| A-015 | *** |
| A-016 | * |
| A-017 | - |
| A-018 | * |
| A-019 | * |

| | |
|---|---|
| Inhibition of binding indicated by % or remaining bound VSIR as follows: "***" = approx <55%; "**" = approx 55% to 75%; "*" = approx 75% to 95%; "-" = > 95%. | |

### Example 5: Functional characterisation of scFv-Fc-format ABPs of the invention.

Antibodies of the invention in scFv-format are re-cloned, genetically fused to mouse Fc domain for mouse IgG2A and expressed in a HEK293 based expression system. The ability of such scFv-Fc-format ABPs of the invention to inhibit the interaction between IGSF11 (VSIG3) and VSIR (VISTA) can be tested in an ELISA-format assay as follows: (1) Recombinant purified human IGSF11 (VSIG3) ECD (HIS-tagged for purification) was immobilised on an ELISA plate (Nunc MaxiSorp) at 5 µg/mL (in PBS), and the plates were then washed and blocked with 2% BSA in PBS/Tween (0.05%); (2) A dilution series (10ug/mL starting concentration, with a set of seven 5-fold dilutions) of anti-IGSF11 scFv-Fc (mouse IgG2A), or control scFv-Fc (mouse IgG2A) antibody of irrelevant specificity, was added for binding, and after plates were then washed of unbound antibody; (3) A dilution series (cross-dilution, 20ug/mL starting concentration, with a set of seven 3-fold dilutions) of human VSIR-Fc (human IgG1) was added (R&D Systems, Cat# 7126-B7), and after binding, unbound VSIR-Fc was removed by washing; (4) VSIR-Fc bound to immobilised IGSF11 was detected with a horseradish peroxidase-conjugated goat anti-human IgG (Fc specific, minimal species cross-reactive to mouse IgG2A of the IGSF11-Fc) (Jackson ImmunoResearch, Cat# 115-036-098), and after washing the ELISA signal was developed with 3,3;5,5'-Tetramethylbenzidine (TMB) substrate. All binding steps were for 1 hour at room temperature, and all washing steps were three times washing with PBS/Tween (0.05%).

Indeed, at least one ABP of the invention is shown to inhibit the IGSF11-VSIR interaction with an IC50 of less than 1.5nM in such an assay, where Fc-VSIR was added at a concentration of approximately 6.6ug/mL (approximately 74nM divalent Fc-VSIR concentration) **(****Figure 4A****).** Indeed, the IC50 of this scFv-Fc-format ABP of the invention estimated in this assay, ranged from about 2.2mM to 1.6mM when Fc-VSIR was added at a concentration ranging from about 20ug/mL to 0.75ug/mL (about 222nM to 8.2nM dimer concentration), respectively (**Figure 4B**)**.**

Analogously, antibodies of the invention in scFv-format are re-cloned and expressed in a human IgG1-format. scFv-Fc-format and/or IgG1-format antibodies re-cloned from those in **Table 3** are found to also inhibit the interaction between IGSF11 (VSIG3) and VSIR (VISTA) in the ELISA assay described in **Example 4.** Alternatively, the IgG1-format antibodies could be tested in an alternative ELISA set-up, where VSIR (VISTA) is immobilised, the antibody/ies for testing are bound to IGSF11 (VSIG3) in solution, the resulting complex is added to the immobilized VSIR (VISTA) and any residual binding of IGSF11 (VSIG3) (eg, His-tagged IGSF11 or IGSF11-Fc fusion protein) to VSIR (VISTA) is detected after washing to detect VSIR-bound IGSF11 using an appropriate anti-IGSF11 primary antibody (eg, anti-IGSF11 sheep polyclonal Ab cat#: AF4915 R&D Systems, or anti-His tag antibody) and a labelled secondary antibody suitable for the primary antibody. As in the set-up described above, reduced ELISA signals indicate antibodies of the invention which inhibit the interaction between IGSF11 and VSIR (eg, a function and/or activity of IGSF11).

### Example 6: Detection of IGSF11 (VSIG3) using an antibody of the present invention.

The inventors are able to demonstrate that not only can antibodies of the invention can detect IGSF11 expressed on the surface of cell, but surprisingly find that IGSF11 is indeed expressed by immune cells (eg monocytes from the PBMC of healthy donors). Monocytes from the PBMC of healthy donors (A and B) were stained with the described concentration of the anti-IGSF11 scFv format of antibody A-015 (see Example 5) or mouse IgG2a isotype control antibody for 30 min at 4oC, and then detected by FACS using a fluorescently-labeled secondary antibody (gated on CD14 monocytic marker). Analogous methodology can be used to investigate (eg to detect) IGSF11 expression on macrophages (especially TAMs, but also MDSCs, immature DCs etc) present in samples (eg tumor samples) from cancer patients.

Antibodies of the invention in IgG1- or scFv-FC-format (eg, those from **Table 3** recloned as described in **Example 5**) can also be used to specifically detect IGSF11 (VSIG3) expressed on the surface of tumour cells. FACS detection of lung H23 cells transiently transfected with negative control siRNA or IGSF11 knock-down siRNA is conducted, using a APC-labelled anti-human IgG as a secondary antibody. Cells knocked-down for IGSF11 (VSIG3) show reduced fluorescence compared to wild-type (ie, IGSF11-positive) cells. Alternatively, HEK-Freestyle or Expi293 cells (Invitrogen) are transfected with empty plasmid construct or plasmid constructs expressing the cDNA of IGSF11. Antibodies specific for IGSF11 show positive surface staining of the IGSF11-overexpressing HEK cells, compared to the control cells mock-transfected with empty plasmid.

Such antibodies of the invention can be used to investigate the protein expression of IGSF11 (VSIG3) on the surface of one or more of the other tumour cell lines tested in **Example 2** (eg, by FC/FACS or immunohistochemistry), and the amount of IGSF11 (VSIG3) detected by such antibodies can be associated with the degree of resistance each cell lines shows to cell-mediated immune response.

Such results demonstrate the ability of antibodies of the present invention to detected the expression of IGSF11 (VSIG3) protein and their utility to determine increased resistance of a cell against an immune response, such as of a cancer cell.

### Example 7 (prophetic): Immuno-modulatory function of antibodies that bind to human IGSF11 (VSIG3).

Antibodies of the invention in IgG1-format (eg, those from **Example 5**) are found to sensitise human tumour cells towards TIL-mediated cytotoxicity. Analogously to the methodology described in **Example 1** for siRNA knockdown, human tumour cells from one or more of the the following cell lines: MCF7, SW480, M579-A2, KMM1, PANC-1, CaCo2, MDA-MB-231, HCT-116, H23, A54 (each, containing a luc reporter) are treated with 0.1, 1, 5, 10, 30 or 60 ug of IgG1- or scFv-FC-format antibodies (eg from those described in **Example 5**) and 72h thereafter are co-cultured with cytotoxic T cells (eg TILs). Compared to samples exposed to non-specific control IgG antibodies, tumour cell lines that are exposed to the IgG1-format antibodies of the invention display increased cytotoxicity (eg, decreased viability).

Such results further demonstrate the ability of antibodies of the present invention to sensitise a cell to an immune response, such as to sensitise a cancer cell to a cell-mediated immune response.

### Example 8 (prophetic): Antibodies that bind to human IGSF11 (VSIG3) attenuate the inhibition of cytokine and chemokine production by human PBMCs.

The inventors demonstrate that IGSF11 (Fc protein) can inhibit the production of IL-2 by stimulated T cells. 96-well plate culture plates were coated with anti-CD3 antibody (clone OKT3; 2.5ug/mL; eBioscience, Cat# 16-0037-81) along with 10ug/mL of the respective recombinant Fc-proteins: either IGSF11-Fc (R&D systems; #9229-VS-050) or PD-L1-Fc (R&D systems; #156-B7) or IgG1-Fc control (R&D systems, #110-HG-100). Approximately 50,000 T cells, purified from PBMC of a healthy donor, were added to each well to test the inhibitory or stimulatory effect of Fc proteins on T cell activation. After 2 days of incubation at 37oC, plates were centrifuged and the supernatant was collected to measure IL production (eg, human IL-2 Quantikine ELISA Kit, R&D Systems, Cat#: D2050). Compared to unstimulated cells (no addition of anti-CD3 antibody or Fc proteins) showed basal level of IL-2 production, which was markedly increased in cells that were stimulated with anti-CD3 antibody alone. In comparison, addition of IGSF11-Fc protein significantly (P<0.05) decreased the IL-2 production from activated T cells, even more than the decrease in IL-2 production upon addition of PD-L1-Fc (P<0.01) (**Figure 5**).

Wang et al (2017) described the inhibition of cytokine and chemokine production by PBMCs, in particular of CCL5/RANTES, MIP-1 alpha/beta, IL-17A and CXCL11/I-TAC. Therefore, using such an assay, it can be further shown that pre-treatment of recombinant human soluble IGSF11 (VSIG3) (ECD-IgG1-Fc fusion) with IgG1-format antibodies of the invention attenuates the inhibition of cytokine and chemokine production by anti-CD3 activated human PBMCs when exposed to such pre-treated IGSF11 (VSIG3); in particular, such pre-treatment increases the relative production of CCL5/RANTES, MIP-1alpha/beta, IL-17A and/or CXCL11/I-TAC of human PBMCs, as measured using the Proteome ProfilerTM Human XL Cytokine Array Kit (R&D Systems, Catalog # ARY022B) and/or Quantikine® ELISA Kits (R&D Systems, Catalog # D1700, DRN00B DCX110, and DMA00).

Such results further demonstrate the ability of antibodies of the present invention to sensitise a cell to an immune response, such as to sensitise a cancer cell to a cell-mediated immune response.

### Example 9 (prophetic): Antibodies that bind to human IGSF11 (VSIG3) attenuate the inhibition of human T cell activation by IGSF11 (VSIG3).

Wang et al (2017) described that IGSF11 (VSIG3) inhibited anti-CD3 induced IL-2, IFN-g, and IL-17 production by human CD3+ T cells in a dose-dependent manner. Analogous to the experiment described by Wang et al, by pre-treatment of the IGSF11 (VSIG3) with IgG1-format antibodies of the invention it can be shown that such antibodies attenuate the inhibition (eg, activate) the production of one or more cytokines by human CD+ T cells, for example pro-inflammatory cytokines such as IL-2, IFN-gamma, and IL-17, as well as attenuate the inhibition of (eg, activate) human CD3+ T cell proliferation.

Such results further demonstrate the ability of antibodies of the present invention to sensitise a cell to an immune response, such as to sensitise a cancer cell to a cell-mediated immune response.

### Example 10 (prophetic): Tumour resistance to adoptive T cell transfer can be overcome by IGSF11 (VSIG3) inhibition in vivo.

The inventors demonstrate that inhibition of IGSF11 (VSIG3) can be used to overcome resistance of tumour cells to anti-tumour immune response(s) in an in vivo model. IGSF11 (VSIG3) is stably knocked down in a primary cancer cell line (eg, H23, A549 or M579-A2) using IGSF11-specific shRNA (shIGSF11) or the control non-targeting shRNA sequence (shCtrl). The transduced cell lines are produced briefly as follows: lentiviral transduction particles expressing an shRNA targeting IGSF11 mRNA or control shRNA (Sigma-Aldrich, eg The RNAi Consortium (TRC) numbers: TRCN0000431895, TRCN0000428521 or TRCN0000425839 for IGSF11 CDS, and SHC002 for control) are used for transduction. 5x10e4 eg H23-Luc cells are seeded in a 6 well plate in DMEM 10% FCS 1% P/S. After 24h, lentiviral particles are added using multiplicity of infection (MOI) = 2. 48h from transduction, cells put under positive selection using 0.4 µg/ml puromycin. First, the shIGSF11 or shCtrl transduced tumour cells are co-cultured with HLA-matched TILs, and the extent of T cell-mediated killing monitored using in vitro real-time live-cell microscopy. TILs that show increased killing efficacy towards shIGSF11 depleted tutor cells compared to shCtrl are identified. Second, the shIGSF11 and shCtrl transduced tumour cells are subcutaneously injected into the left and the right flank, respectively, of NSG immune deficient mice, and adoptive cell transfer of the identified TIL or PBS injection is applied i.v. once per week. TIL-treatment causes retardation of tumour growth in IGSF11-impaired tumour cells compared to shCtrl-transduced cells. Consistent with the in vitro data, no difference in the tumour growth kinetic between shCtrl and shIGSF11 is observed in PBS-treated mice.

### Example 11 (prophetic): Tumour resistance to adoptive T cell transfer can be overcome, in vivo, by treatment with an antibody of the invention.

Analogous to **Example 10,** H23 cells are subcutaneously injected into a flank of NSG immune deficient mice, and adoptive cell transfer of the identified TIL or PBS injection is applied i.v. once per week. Mice then received 50ug/dose or 200ug/dose of an antibody of the invention, or vehicle control, twice per week for 4 weeks. Antibody treatment causes retardation of tumour growth in this adoptive T cell transfer model compared to administration of vehicle alone.

Such results further demonstrate the ability of antibodies of the present invention to sensitise a cell to an immune response, such as to sensitise a cancer cell to a cell-mediated immune response.

The sequences show:
**SEQ ID NOs. 1 to 370** (Amino acid sequences of CDR and variable regions of ABPs of the invention, as well as nucleic acid sequences encoding variable regions of ABPs of the invention): See **Table 1A.**
**SEQ ID NO. 371** (Human IGSF11 protein isoform 1; UniProt identifier Q5DX21-1): 
10 20 30 40 50
MTSQRSPLAP LLLLSLHGVA ASLEVSESPG SIQVARGQPA VLPCTFTTSA
60 70 80 90 100
ALINLNVIWM VTPLSNANQP EQVILYQGGQ MFDGAPRFHG RVGFTGTMPA
110 120 130 140 150
TNVSIFINNT QLSDTGTYQC LVNNLPDIGG RNIGVTGLTV LVPPSAPHCQ
160 170 180 190 200
 
IQGSQDIGSD VILLCSSEEG IPRPTYLWEK LDNTLKLPPT ATQDQVQGTV
210 220 230 240 250
TIRNISALSS GLYQCVASNA IGTSTCLLDL QVISPQPRNI GLIAGAIGTG
260 270 280 290 300
AVIIIFCIAL ILGAFFYWRS KNKEEEEEEI PNEIREDDLP PKCSSAKAFH
310 320 330 340 350
TEISSSDNNT LTSSNAYNSR YWSNNPKVHR NTESVSHFSD LGQSFSFHSG
360 370 380 390 400
NANIPSIYAN GTHLVPGQHK TLVVTANRGS SPQVMSRSNG SVSRKPRPPH
410 420 430
THSYTISHAT LERIGAVPVM VPAQSRAGSL V
**SEQ ID NO. 372** (Human IGSF11 protein isoform 2; UniProt identifier Q5DX21-2): 
10 20 30 40 50
MSLVELLLWW NCFSRTGVAA SLEVSESPGS IQVARGQPAV LPCTFTTSAA
60 70 80 90 100
LINLNVIWMV TPLSNANQPE QVILYQGGQM FDGAPRFHGR VGFTGTMPAT
110 120 130 140 150
NVSIFINNTQ LSDTGTYQCL VNNLPDIGGR NIGVTGLTVL VPPSAPHCQI
160 170 180 190 200
QGSQDIGSDV ILLCSSEEGI PRPTYLWEKL DNTLKLPPTA TQDQVQGTVT
210 220 230 240 250
IRNISALSSG LYQCVASNAI GTSTCLLDLQ VISPQPRNIG LIAGAIGTGA
260 270 280 290 300
VIIIFCIALI LGAFFYWRSK NKEEEEEEIP NEIREDDLPP KCSSAKAFHT
310 320 330 340 350
EISSSDNNTL TSSNAYNSRY WSNNPKVHRN TESVSHFSDL GQSFSFHSGN
360 370 380 390 400
ANIPSIYANG THLVPGQHKT LVVTANRGSS PQVMSRSNGS VSRKPRPPHT
410 420 430
HSYTISHATL ERIGAVPVMV PAQSRAGSLV
**SEQ ID NO. 373** (Human IGSF11 protein isoform 3; UniProt identifier Q5DX21-3): 
10 20 30 40 50
MSLVELLLWW NCFSRTGVAA SLEVSESPGS IQVARGQPAV LPCTFTTSAA
60 70 80 90 100
LINLNVIWMV TPLSNANQPE QVILYQGGQM FDGAPRFHGR VGFTGTMPAT
110 120 130 140 150
NVSIFINNTQ LSDTGTYQCL VNNLPDIGGR NIGVTGLTVL VPPSAPHCQI
160 170 180 190 200
QGSQDIGSDV ILLCSSEEGI PRPTYLWEKL DNTLKLPPTA TQDQVQGTVT
210 220 230 240 250
 
IRNISALSSA QPRNIGLIAG AIGTGAVIII FCIALILGAF FYWRSKNKEE
260 270 280 290 300
EEEEIPNEIR EDDLPPKCSS AKAFHTEISS SDNNTLTSSN AYNSRYWSNN
310 320 330 340 350
PKVHRNTESV SHFSDLGQSF SFHSGNANIP SIYANGTHLV PGQHKTLVVT
360 370 380 390 400
ANRGSSPQVM SRSNGSVSRK PRPPHTHSYT ISHATLERIG AVPVMVPAQS
RAGSLV
**SEQ ID NO. 374** (ECD of human IGSF11 protein; UniProt identifier Q5DX21): 
10 20 30 40 50
LEVSESPGSI QVARGQPAVL PCTFTTSAAL INLNVIWMVT PLSNANQPEQ
60 70 80 90 100
VILYQGGQMF DGAPRFHGRV GFTGTMPATN VSIFINNTQL SDTGTYQCLV
110 120 130 140 150
NNLPDIGGRN IGVTGLTVLV PPSAPHCQIQ GSQDIGSDVI LLCSSEEGIP
160 170 180 190 200
RPTYLWEKLD NTLKLPPTAT QDQVQGTVTI RNISALSSGL YQCVASNAIG
210
TSTCLLDLQV ISPQPRNIG
**SEQ ID NO. 375** (Ig-like V-type domain of human IGSF11 protein; UniProt identifier Q5DX21): 
10 20 30 40 50
LEVSESPGSI QVARGQPAVL PCTFTTSAAL INLNVIWMVT PLSNANQPEQ
60 70 80 90 100
VILYQGGQMF DGAPRFHGRV GFTGTMPATN VSIFINNTQL SDTGTYQCLV
110
NNLPDIGGRN IGVT
**SEQ ID NO. 376** (Ig-like C2-type domain of human IGSF11 protein; UniProt identifier Q5DX21): 
10 20 30 40 50
PSAPHCQIQG SQDIGSDVIL LCSSEEGIPR PTYLWEKLDN TLKLPPTATQ
60 70 80 90
DQVQGTVTIR NISALSSGLY QCVASNAIGT STCLLDLQVI S
**SEQ ID NO. 377** (Cynomolgus monkey IGSF11 protein; UniProt identifier G7NXN0): 
10 20 30 40 50
MTSRRSPLAP LLLLSLHGVA ASLEVSESPG SIQVARGQTA VLPCTFTTSA
60 70 80 90 100
ALINLNVIWM VTPLSNANQP EQVILYQGGQ MFDGAPRFHG RVGFTGTMPA
110 120 130 140 150
 
TNVSVFINNT QLSDTGTYQC LVNNLPDIGG RNIGVTGLTV LVPPSAPHCQ
160 170 180 190 200
IQGSQDIGSD VILLCSSEEG IPRPTYLWEK LDNTLKLPPT ATQDQVQGTV
210 220 230 240 250
TIRNISTLTS GLYQCVASNA IGTSTCLLDL QVISPQPRNI GLIAGAVGTG
260 270 280 290 300
AVIIIFCIAL ILGAFFYWRS KNKEEEEEEI PNEIREDDLP PKCSSAKAFH
310 320 330 340 350
TEISSSDNNT LTSSNTYNSR YWSNNPKVHR NTESVNHFSD LGQSFSLRSG
360 370 380 390 400
NASIPSIYAN GSHLLPGQHK TLVVTANRGS SPQVMSRSNG SVSRKPRPPH
410 420 430
SHSYTISHAT LERIGAVPVM VPAQSRAGSL V
**SEQ ID NO. 378** (Murine IGSF11 protein; UniProt identifier P0C673): 
10 20 30 40 50
MTRRRSAPAS WLLVSLLGVA TSLEVSESPG SVQVARGQTA VLPCAFSTSA
60 70 80 90 100
ALLNLNVIWM VIPLSNANQP EQVILYQGGQ MFDGALRFHG RVGFTGTMPA
110 120 130 140 150
TNVSIFINNT QLSDTGTYQC LVNNLPDRGG RNIGVTGLTV LVPPSAPQCQ
160 170 180 190 200
IQGSQDLGSD VILLCSSEEG IPRPTYLWEK LDNTLKLPPT ATQDQVQGTV
210 220 230 240 250
TIRNISALSS GLYQCVASNA IGTSTCLLDL QVISPQPRSV GVIAGAVGTG
260 270 280 290 300
AVLIVICLAL ISGAFFYWRS KNKEEEEEEI PNEIREDDLP PKCSSAKAFH
310 320 330 340 350
TEISSSENNT LTSSNTYNSR YWNNNPKPHR NTESFNHFSD LRQSFSGNAV
360 370 380 390 400
IPSIYANGNH LVLGPHKTLV VTANRGSSPQ VLPRNNGSVS RKPWPQHTHS
410 420
YTVSQMTLER IGAVPVMVPA QSRAGSLV
**SEQ ID NO. 379** (Human VSIR protein; UniProt identifier Q9H7M9): 
10 20 30 40 50
MGVPTALEAG SWRWGSLLFA LFLAASLGPV AAFKVATPYS LYVCPEGQNV
60 70 80 90 100
TLTCRLLGPV DKGHDVTFYK TWYRSSRGEV QTCSERRPIR NLTFQDLHLH
110 120 130 140 150
HGGHQAANTS HDLAQRHGLE SASDHHGNFS ITMRNLTLLD SGLYCCLVVE
160 170 180 190 200
 
IRHHHSEHRV HGAMELQVQT GKDAPSNCVV YPSSSQDSEN ITAAALATGA
210 220 230 240 250
CIVGILCLPL ILLLVYKQRQ AASNRRAQEL VRMDSNIQGI ENPGFEASPP
260 270 280 290 300
AQGIPEAKVR HPLSYVAQRQ PSESGRHLLS EPSTPLSPPG PGDVFFPSLD
310
PVPDSPNFEV I
**SEQ ID NO. 380** (ECD of human VSIR protein; UniProt identifier Q9H7M9): 
10 20 30 40 50
FKVATPYSLY VCPEGQNVTL TCRLLGPVDK GHDVTFYKTW YRSSRGEVQT
60 70 80 90 100
CSERRPIRNL TFQDLHLHHG GHQAANTSHD LAQRHGLESA SDHHGNFSIT
110 120 130 140 150
MRNLTLLDSG LYCCLVVEIR HHHSEHRVHG AMELQVQTGK DAPSNCVVYP
160
SSSQDSENIT AA
**SEQ ID NO. 381** (Ig-like V-type domain of human VSIR protein; UniProt identifier Q9H7M9): 
10 20 30 40 50
FKVATPYSLY VCPEGQNVTL TCRLLGPVDK GHDVTFYKTW YRSSRGEVQT
60 70 80 90 100
CSERRPIRNL TFQDLHLHHG GHQAANTSHD LAQRHGLESA SDHHGNFSIT
110 120 130
MRNLTLLDSG LYCCLVVEIR HHHSEHRVHG AMELQV
**SEQ ID NO. 382** (Rhesus monkey VSIR protein; UniProt identifier F7GVN3): 
10 20 30 40 50
MGVPTAPEAG CWRWGSLLFA LFLAASLGPV AAFKVATLYS LYVCPEGQNV
60 70 80 90 100
TLTCRFFGPV DKGHDVTFYK TWYRSSRGEV QTCSERRPIR NLTFQDLHLH
110 120 130 140 150
HGGHQAANTS HDLAQRHGLE SASDHHGNFS ITMRNLTLLD SGLYCCLVVE
160 170 180 190 200
IRHHHSEHRV HGAMELQVQT GKDAPSSCVA YPSSSQESEN ITAAALATGA
210 220 230 240 250
CIVGILCLPL ILLLVYKQRQ AASNRRDNTQ GIENPGFEAS SPAQGILEAK
260 270 280 290 300
VRHPLSYVAQ RQPSESGRHL LSEPGTPLSP PGPGDVFFPS LDPVPDSPNF

EVI
**SEQ ID NO. 383** (Murine VSIR protein; UniProt identifier Q9D659): 
10 20 30 40 50
MGVPAVPEAS SPRWGTLLLA IFLAASRGLV AAFKVTTPYS LYVCPEGQNA
60 70 80 90 100
TLTCRILGPV SKGHDVTIYK TWYLSSRGEV QMCKEHRPIR NFTLQHLQHH
110 120 130 140 150
GSHLKANASH DQPQKHGLEL ASDHHGNFSI TLRNVTPRDS GLYCCLVIEL
160 170 180 190 200
KNHHPEQRFY GSMELQVQAG KGSGSTCMAS NEQDSDSITA AALATGACIV
210 220 230 240 250
GILCLPLILL LVYKQRQVAS HRRAQELVRM DSNTQGIENP GFETTPPFQG
260 270 280 290 300
MPEAKTRPPL SYVAQRQPSE SGRYLLSDPS TPLSPPGPGD VFFPSLDPVP

DSPNSEAI
**SEQ ID NO. 384** (siRNA sequence targeting human IGSF11): 
10
CAACAUACCA UCCAUUUAU
**SEQ ID NO. 385** (siRNA sequence targeting human IGSF11): 
10
GGAACGAAUU GGUGCAGUA
**SEQ ID NO. 386** (siRNA sequence targeting human IGSF11): 
10
GAACAUCAGU GCCCUGUCU
**SEQ ID NO. 387** (siRNA sequence targeting human IGSF11):

10
CAGGAACAUU GGACUAAUA

## Claims

1. **An isolated antigen binding protein (ABP)** which specifically binds to the extra cellular domain (ECD) of IGSF11 (VSIG3) protein and wherein the isolated ABP comprises at least one complementarity determining region (CDR) and is able to inhibit the binding of VSIR (VISTA) protein or a variant thereof to IGSF11 protein or a variant thereof.

2. The isolated ABP of claim 1, comprising at least one complementarity determining region 3 (CDR3) having an amino acid sequence with at least 90% sequence identity to, or having no more than three or two, preferably no more than one amino acid substitution(s), deletion(s) or insertion(s) compared to, a sequence selected from SEQ ID Nos. 3, 7, 13, 17, 23, 27, 33, 37, 43, 47, 53, 57, 63, 67, 73, 77, 83, 87, 93, 97, 103, 107, 113, 117, 123, 127, 133, 137, 143, 147, 153, 157, 163, 167, 173, 177, 183, 187, 193, 197, 203, 207, 213, 217, 223, 227, 233, 237, 243, 247, 253, 257, 263, 267, 273, 277, 283, 287, 293, 297, 303, 307, 313, 317, 323, 327, 333, 337, 343, 347, 353, 357, 363, and 367.

3. The isolated ABP of claim 1 or 2, wherein the ABP is an antibody, or an antigen binding fragment thereof, composed of at least one, preferably two, antibody heavy chain sequences, and at least one, preferably two, antibody light chain sequences, wherein at least one, preferably both, of the antibody heavy chain sequences and at least one, preferably both, of the antibody light chain sequences comprise CDR1 to CDR3 sequences in a combination selected from any of the following combinations of heavy and/or light chain CDRs, CDRs-A-001 to CDRs-A-037:
| **Combination (ID)** | **Heavy Chain CDR1 to CDR3 (SEQ ID NO)** | | | **Light Chain CDR1 to CDR3 (SEQ ID NO)** | | |
|---|---|---|---|---|---|---|
| CDRs-A-001 | 1 | 2 | 3 | 5 | 6 | 7 |
| CDRs-A-002 | 11 | 12 | 13 | 15 | 16 | 17 |
| CDRs-A-003 | 21 | 22 | 23 | 25 | 26 | 27 |
| CDRs-A-004 | 31 | 32 | 33 | 35 | 36 | 37 |
| CDRs-A-005 | 41 | 42 | 43 | 45 | 46 | 47 |
| CDRs-A-006 | 51 | 52 | 53 | 55 | 56 | 57 |
| CDRs-A-007 | 61 | 62 | 63 | 65 | 66 | 67 |
| CDRs-A-008 | 71 | 72 | 73 | 75 | 76 | 77 |
| CDRs-A-009 | 81 | 82 | 83 | 85 | 86 | 87 |
| CDRs-A-010 | 91 | 92 | 93 | 95 | 96 | 97 |
| CDRs-A-011 | 101 | 102 | 103 | 105 | 106 | 107 |
| CDRs-A-012 | 111 | 112 | 113 | 115 | 116 | 117 |
| CDRs-A-013 | 121 | 122 | 123 | 125 | 126 | 127 |
| CDRs-A-014 | 131 | 132 | 133 | 135 | 136 | 137 |
| CDRs-A-015 | 141 | 142 | 143 | 145 | 146 | 147 |
| CDRs-A-016 | 151 | 152 | 153 | 155 | 156 | 157 |
| CDRs-A-017 | 161 | 162 | 163 | 165 | 166 | 167 |
| CDRs-A-018 | 171 | 172 | 173 | 175 | 176 | 177 |
| CDRs-A-019 | 181 | 182 | 183 | 185 | 186 | 187 |
| CDRs-A-020 | 191 | 192 | 193 | 195 | 196 | 197 |
| CDRs-A-021 | 201 | 202 | 203 | 205 | 206 | 207 |
| CDRs-A-022 | 211 | 212 | 213 | 215 | 216 | 217 |
| CDRs-A-023 | 221 | 222 | 223 | 225 | 226 | 227 |
| CDRs-A-024 | 231 | 232 | 233 | 235 | 236 | 237 |
| CDRs-A-025 | 241 | 242 | 243 | 245 | 246 | 247 |
| CDRs-A-026 | 251 | 252 | 253 | 255 | 256 | 257 |
| CDRs-A-027 | 261 | 262 | 263 | 265 | 266 | 267 |
| CDRs-A-028 | 271 | 272 | 273 | 275 | 276 | 277 |
| CDRs-A-029 | 281 | 282 | 283 | 285 | 286 | 287 |
| CDRs-A-030 | 291 | 292 | 293 | 295 | 296 | 297 |
| CDRs-A-031 | 301 | 302 | 303 | 305 | 306 | 307 |
| CDRs-A-032 | 311 | 312 | 313 | 315 | 316 | 317 |
| CDRs-A-033 | 321 | 322 | 323 | 325 | 326 | 327 |
| CDRs-A-034 | 331 | 332 | 333 | 335 | 336 | 337 |
| CDRs-A-035 | 341 | 342 | 343 | 345 | 346 | 347 |
| CDRs-A-036 | 351 | 352 | 353 | 355 | 356 | 357 |
| CDRs-A-037 | 361 | 362 | 363 | 365 | 366 | 367 |
in each case independently, optionally with no more than three or two, preferably no more than one, amino acid substitution(s), insertion(s) or deletion(s) compared to these sequences.

4. The isolated ABP of any one of claims 1 to 3, wherein the ABP is an antibody, or an antigen binding fragment thereof, composed of at least one, preferably two, antibody heavy chain sequences, and at least one, preferably two, antibody light chain sequences, wherein at least one, preferably both, of the antibody heavy chain sequences each comprises heavy chain CDR1 to CDR3 sequences in the combination CDRs-A-015 and at least one, preferably both, of the antibody light chain sequences each comprises light chain CDR1 to CDR3 sequences in the combination CDRs-A-015, in each case independently, optionally with no more than one amino acid substitution(s), insertion(s) or deletion(s) compared to these sequences, and preferably wherein the ABP is able to inhibit the binding of VSIR protein or a variant thereof to IGSF11 protein or a variant thereof with an IC50 of 10nM or less.

5. **An isolated ABP** which competes with an ABP as recited in any one of claims 1 to 4 for binding to the ECD of the IGSF11 protein and is able to inhibit the binding of the VSIR protein or the variant thereof to the IGSF11 protein or the variant thereof.

6. The isolated ABP of any one of claims 1 to 5 that enhances killing and/or lysis of cells expressing IGSF11, or a variant of IGSF11, by cytotoxic T cells and/or TILs.

7. The isolated ABP of any one of claims 1 to 6 that (i) enhances a cell-mediated immune response, such as that mediated by an activated cytotoxic T-cell (CTL), to a mammalian cell expressing said IGSF11 or the variant of IGSF11; and/or (ii) increases immune cell, such as T-cell, activity and/or survival in the presence of a mammalian cell expressing said IGSF11 or the variant of IGSF11.

8. The isolated ABP of any one of claims 1 to 7 that is an antibody or an antigen binding fragment thereof, wherein the antibody is a monoclonal antibody, or wherein the antigen binding fragment is a fragment of a monoclonal antibody.

9. The isolated ABP of any one of claims 1 to 8 that is an antibody or an antigen binding fragment thereof, wherein the antibody is a human antibody a humanised antibody or a chimeric-human antibody, or wherein the antigen binding fragment is a fragment of a human antibody a humanised antibody or a chimeric-human antibody.

10. **An isolated nucleic acid** encoding for an ABP, or for an antigen binding fragment or a monomer of an ABP, wherein the ABP is one recited in any one of claims 1 to 9.

11. **A recombinant host cell** comprising a nucleic acid recited in claim 10.

12. **A pharmaceutical composition** comprising: (i) an ABP recited in any one of claims 1 to 9, or (ii) a nucleic acid recited in claim 10, or (iii) a compound that is an inhibitor of the expression, function, activity and/or stability of immunoglobulin superfamily member 11 (IGSF11, or VSIG3), or of a variant of IGSF11, and a pharmaceutically acceptable carrier, stabiliser and/or excipient.

13. **A product for use in medicine,** wherein the product is selected from the list consisting of: (i) an isolated ABP recited in any one of claims 1 to 9, and (ii) an isolated nucleic acid recited in claim 10, and (iii) a compound that is an inhibitor of the expression, function, activity and/or stability of immunoglobulin superfamily member 11 (IGSF11, or VSIG3), or of a variant of IGSF11.

14. The product for use in medicine of claim 13 wherein the product is for use in the treatment of a proliferative disorder that is associated with the undesired presence of IGSF11-positive cells or cells positive for a variant of IGSF11 and/or that is associated with cellular resistance against a cell-mediated immune response and/or that is associated with expression or activity of IGSF11 or a variant thereof of IGSF11.

15. The product for use in medicine of claim 14, wherein cells involved in the proliferative disorder are resistant to a cell-mediated immune response.

16. The product for use in medicine of any one of claims 13 to 15, wherein the product is for use in enhancing an immune response in a mammalian subject, preferably for use in aiding a cell-mediated immune response in the subject such as the subject's T cell mediated immune response, for example for treating a proliferative disease, such as a cancer disease, of for treating an infectious disease.

17. The product for use in medicine of any one of claims 13 to 16, wherein the product is for use in the treatment of a proliferative disorder resistant and/or refractory to PD1/CTLA4 blockade therapy.

18. **An in-vitro method for determining** whether a subject has, or is at risk of, developing a disease, disorder or condition that is associated with the undesired presence of IGSF11-positive cells (or cells positive for a variant of IGSF11) and/or that is associated with cellular resistance against a cell-mediated immune response and/or that is associated with expression or activity of IGSF11 (or a variant thereof), the method comprising the step of:
• detecting IGSF11 (or a variant thereof), in particular the presence (or an amount) of or expression and/or activity of IGSF11 (or a variant thereof), in a biological sample from said subject,
wherein the detection of IGSF11 (or the variant thereof) in the sample indicates such disease, disorder or condition, or a risk of developing such disease, disorder or condition, in the subject; and
optionally, wherein the IGSF11 (or variant thereof) is detected with a ABP of any one of claims 1 to 9.

19. **An in-vitro method for determining** whether a subject has, or has a risk of developing, a disease, disorder or condition that is associated with the undesired presence of IGSF11-positive cells (or cells positive for a variant of IGSF11) and/or that is associated with cellular resistance against a cell-mediated immune response and/or that is associated with expression or activity of IGSF11 (or a variant thereof), the method comprising the steps of:
• contacting cells of the subject involved with the disease, disorder or condition with an ABP of any one of claims 1 to 9, and/or with a product recited in any one of claims 13 to 17, in the presence of a cell-mediated immune response, preferably wherein the cell-mediated immune response comprises immune cells selected from the group consisting of: lymphocytes, T-cells, CTLs and TILs; and
• determining the cell-mediated immune response against such cells of the subject,
wherein an enhancement of the cell-mediated immune response against such cells of the subject indicates that the subject has or has a risk of developing a disease, disorder or condition that is selected from a proliferative disorder or an infectious disease.

20. **An in-vitro method for identifying and/or characterising** a compound suitable for the treatment of a disease, disorder or condition that is associated with the undesired presence of IGSF11-positive cells (or cells positive for a variant of IGSF11) and/or that is **characterised by** cellular resistance against a cell-mediated immune response and/or one that is **characterised by** expression or activity of IGSF11 (or a variant thereof), the method comprising the steps of:
• (a) bringing into contact a first cell expressing IGSF11 (or a variant thereof) and (x) the candidate compound, or (y) the candidate compound and a cell-mediated immune response, preferably wherein the cell-mediated immune response comprises immune cells selected from the group consisting of: lymphocytes, T-cells, CTLs and TILs; and
• (b) determining (i) the expression, activity, function and/or stability of the (eg protein or mRNA of) the IGSF11 (or variant), in the first cell; and/or (ii) the cell-mediated immune response against the first cell,
wherein: (i) a reduced expression, activity function and/or stability of the IGSF11 (or variant), in said first cell contacted with the candidate compound compared to said first cell not contacted with said candidate compound; and/or (ii) an enhancement of the cell-mediated immune response against the first cell contacted with the candidate compound compared to the cell-mediated immune response against the first cell not contacted with the candidate compound; indicates that the candidate compound is a compound suitable for the treatment of a disease, disorder or condition that is selected from a proliferative disorder or an infectious disease; and
optionally, wherein the reduction of expression, activity function and/or stability or IGSF11 and/or the enhancement of the cell-mediated immune response is identified by reference to a control method practised with a compound having a known effect on such expression, function, activity and/or stability, in particular a positive or negative control; and wherein the compound having a known effect on such expression, function, activity and/or stability is an ABP of any one of claims 1 to 9 and/or is a product recited in any one of claims 13 to 17.
